(19) Europäisches Patentamt
European Patent Office
Office européen des brevets

(11) EP 2 899 192 A1

(12) **EUROPEAN PATENT APPLICATION**

(43) Date of publication:
**29.07.2015 Bulletin 2015/31**

(21) Application number: **14152567.5**

(22) Date of filing: **24.01.2014**

(51) Int Cl.:
***C07D 471/08*** *(2006.01)* ***A61K 31/551*** *(2006.01)*
***A61P 3/00*** *(2006.01)* ***A61P 25/00*** *(2006.01)*
***A61P 27/00*** *(2006.01)* ***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventor: **The designation of the inventor has not yet been filed**

(74) Representative: **Arth, Hans-Lothar**
**ABK Patent Attorneys**
**Jasminweg 9**
**14052 Berlin (DE)**

(54) **Diazabicyclo[4.3.1]decane derivatives for treatment of psychiatric disorders**

(57) The present invention relates to diazabicyclo[4.3.1]decane derivatives of formula (I), pharmaceutically acceptable salts of these compounds and pharmaceutical compositions containing at least one of these compounds together with pharmaceutically acceptable carrier, excipient and/or diluents. Said diazabicyclo[4.3.1]decane derivatives are inhibitors of the FK506 binding protein (FKBP's) and can be used for prophylaxis and/or treatment of psychiatric disorders and neurodegenerative diseases, disorders and conditions.

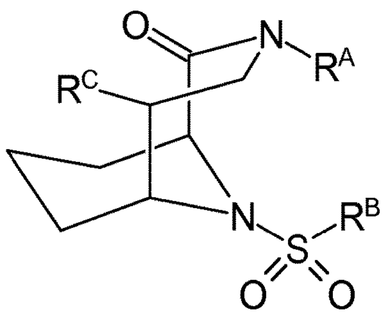

(I)

EP 2 899 192 A1

## Description

## Specification

The present invention relates to diazabicyclo[4.3.1]decane derivatives and stereoisomeric forms, solvates, hydrates and/or pharmaceutically acceptable salts of these compounds as well as pharmaceutical compositions containing at least one of these bicyclic aza-amides derivatives together with pharmaceutically acceptable carrier, excipient and/or diluents. Said diazabicyclo[4.3.1]decane derivatives have been identified as specific inhibitors of the FK506 binding proteins (FKBP's), especially FKBP51 and FKBP52, and are useful for the treatment of psychiatric disorders and neurodegenerative diseases, disorders and conditions, for treating vision disorders and/or improving vision; for treating memory impairment and/or enhancing memory performance, for treating alopecia and promoting hair growth, for treating metabolic disorders and obesity, or for treating prostate cancer or malignant melanoma.

## Background of the invention

The FK506-binding protein (FKBP) family of immunophilins consists of proteins with a variety of protein-protein interaction domains and versatile cellular functions. This highly conserved protein family binds with immunosuppressive drugs, such as FK506 and rapamycin. This protein family displays peptidyl propyl isomerase (PPlase) activity as seen with cyclophilins and parvulins. FKBP12, a 12 kD protein is the most widely studied member of this family.

The immunosuppressant drugs FK506, rapamycin, and cyclosporin are well known as potent T-cell specific immunosuppressants, and are effective against autoimmunity, transplant or graft rejection, inflammation, allergic responses, other autoimmune or immune-mediated diseases, and infectious diseases.

FK506 and rapamycin apart from binding to FKBP12 also interact and inhibit calcineurin (CaN) and mTOR respectively thereby mediating their immunosuppressive action.

The high molecular weight multidomain homologs of FKBP12, FKBP51 and FKBP 52, act as cochaperones for the heat shock protein 90 (Hsp90) and modulate the signal transduction of the glucocorticoid receptor by participating in the Heat shock protein 90 (Hsp90) - steroid receptor complex.

In this complex, FKBP51 and FKBP52 modulate the binding competence and signalling of steroid hormone receptors and thereby regulate the cellular responsiveness to circulating hormone levels. This is supported by a natural animal model (squirrel monkey) and by knockout mice, where the crucial role of FKPB51 and FKBP52 on the Glucocorticoid Receptor (GR) Progesterone Receptor (PR) or Androgen Receptor (AR) activity have been clearly demonstrated. Moreover, polymorphisms in the FKBP51-encoding gene of psychiatric patients have been associated with numerous stress-related psychiatric disorders (Schmidt et al., ChemMedChem 2012, 7, 1351-1359).

The immunosuppressive compounds disclosed in the prior art suppress the immune system, by definition, and also exhibit other toxic side effects. Accordingly, there is a need for non-immunosuppressant, small molecule compounds, and compositions and methods for use of such compounds, that are useful in treating psychiatric disorders and neurodegenerative diseases, disorders and conditions.

Further studies led to $\alpha$-ketoamide analogs of FK506 devoid of immunosuppressive activity. So far there has been only few investigations on the activity of monocyclic, pipecolate or proline-based compounds concerning the larger FKBP's (FKBP51 and 52).

Also, the main physiological role of FKBP51 is believed to be the inhibition of glucocorticoid receptor signaling, especially in stressful situations. However, the FKBP51-GR interplay (glucocorticoid receptor interplay) has been difficult to assess pharmacologically, largely due to lack of appropriate chemical probes.

Therefore, it is the object of the present invention to provide compounds and/or pharmaceutically acceptable salts thereof which inhibit FKBP 51 and/or FKBP 52 more effectively based on significantly increased affinity to FKBP 51 and/or FKBP 52.

Another aspect of the invention is to provide compounds and/or pharmaceutically acceptable salts thereof which can be used as pharmaceutically active agents, especially for the treatment of psychiatric disorders and neurodegenerative diseases, disorders and conditions, for treating vision disorders and/or improving vision; for treating memory impairment and/or enhancing memory performance and for treating alopecia, as well as compositions comprising at least one of those compounds and/or pharmaceutically acceptable salts thereof as pharmaceutically active ingredients.

A further aspect of the invention is to provide methods for preparing said compounds.

The object of the present invention is solved by the teaching of the independent claims. Further advantageous features, aspects and details of the invention are evident from the dependent claims, the description, and the examples of the present application.

## Description of the invention

Thus the present invention relates to the compound of the general formula (I):

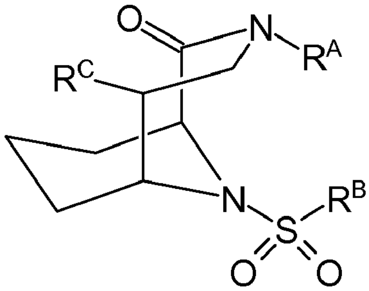

**(I)**

wherein

**$R^A$** represents $-H$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH_2OH$, $-C_2H_4OH$, $-C_3H_6OH$, $-C_4H_8OH$, $-CH(CH_3)-C_2H_4OH$, $-C_5H_{10}OH$, $-CH_2OCH_3$, $-C_2H_4OCH_3$, $-C_3H_6OCH_3$, $-C_4H_8OCH_3$, $-CH(CH_3)-C_2H_4OCH_3$, $-C_5H_{10}OCH_3$, $-CH_2NH_2$, $-C_2H_4NH_2$, $-C_3H_6NH_2$, $-C_4H_8NH_2$, $-CH(CH_3)-C_2H_4NH_2$, $-C_5H_{10}NH_2$, $-C_2H_4NHCH_3$, $-C_2H_4N(CH_3)_2$, $-C_2H_4N(CH_3)_2$, $-C_2H_4NHCH(CH_3)_2$, $-C_2H_4NH(CH_2CH_3)$, $-C_2H_4N(CH_2CH_3)_2$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$,- $CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C\equiv CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C=C-CH(CH_3)-C_2H_5$, $-C=C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$,- $C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-CH_2-Ph$,

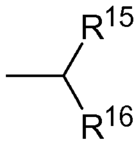,
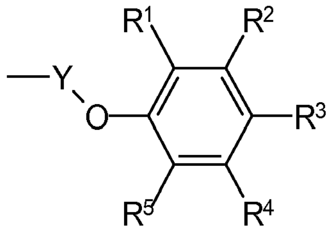,
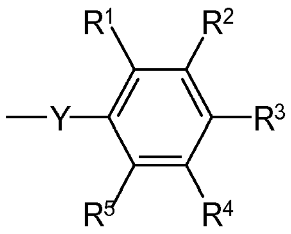
or
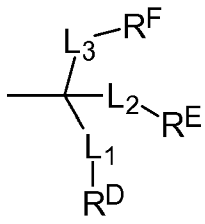;

**$R^D$** represents: **$R^{22}$** or

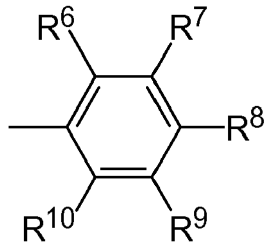,
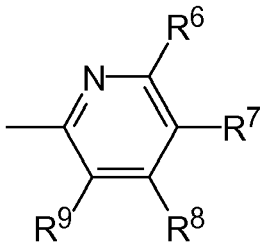,
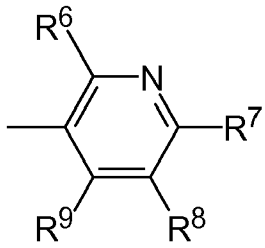,
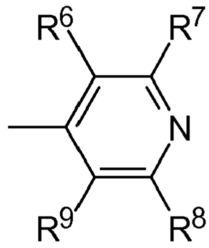,

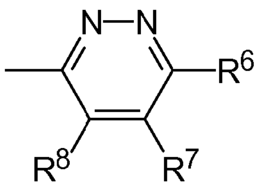,
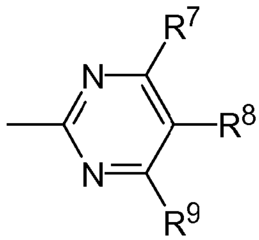,
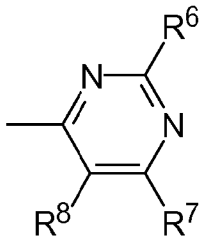,
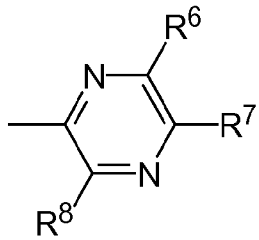,

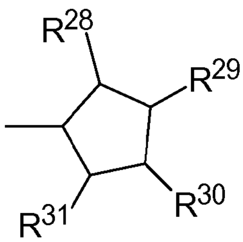,
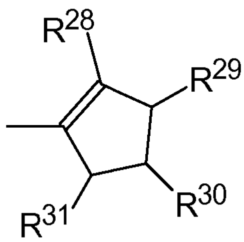,
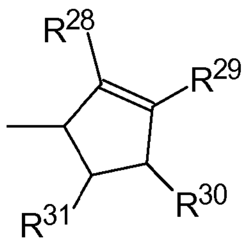,
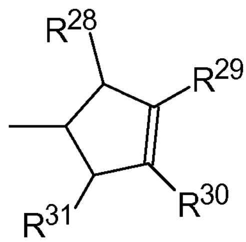,

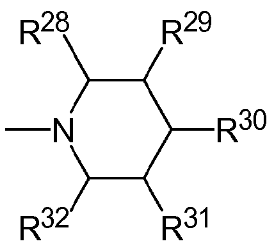,
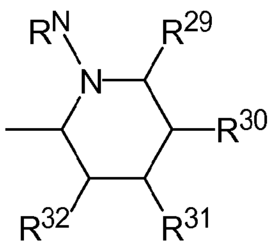,
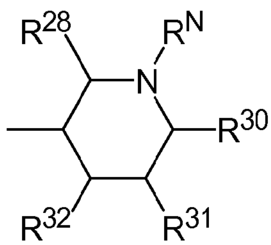,
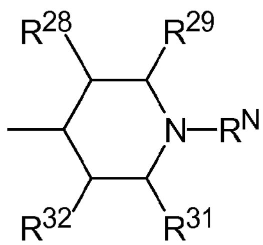,

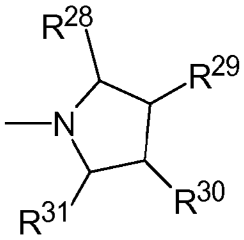 ,
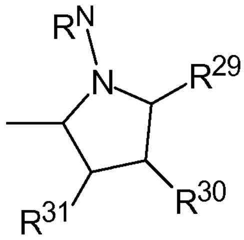 ,
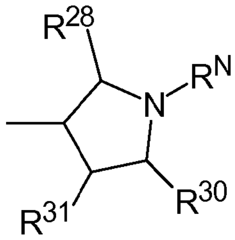 ,
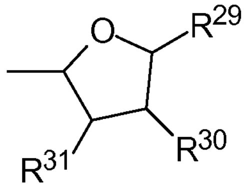 ,

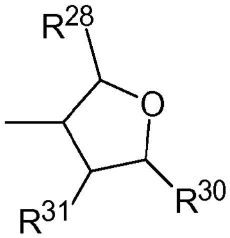 ,
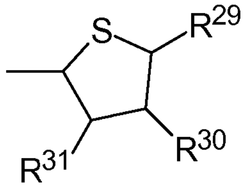 ,
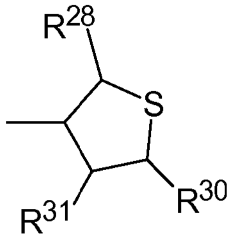 ,
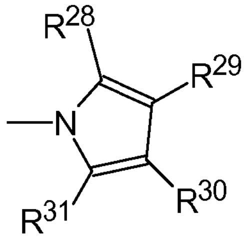 ,

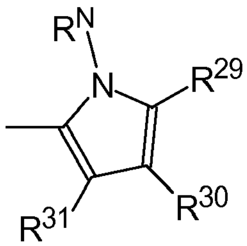 ,
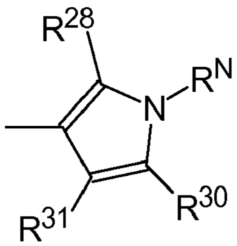 ,
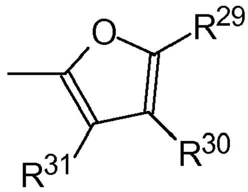 ,
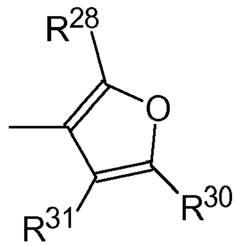 ,

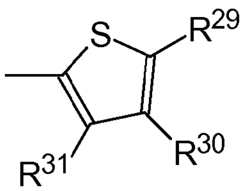 ,
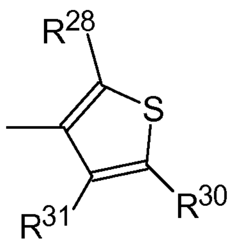 ,
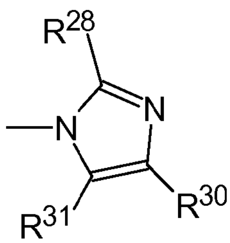 ,
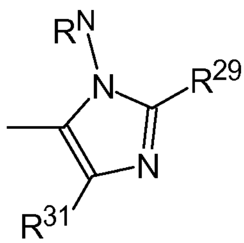 ,

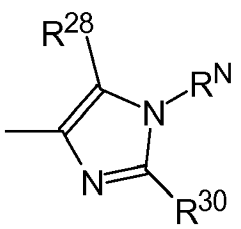 ,
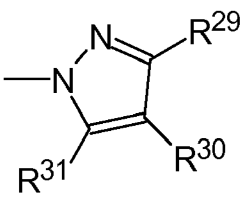 ,
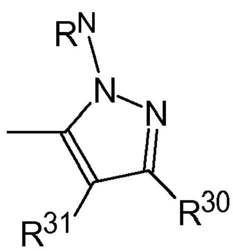 ,
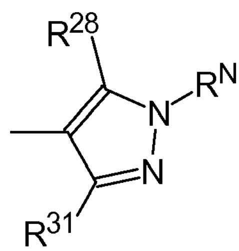 ,

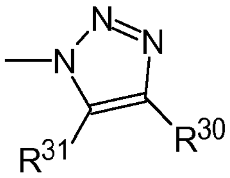,
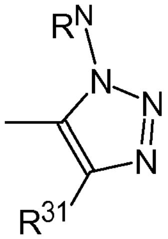,
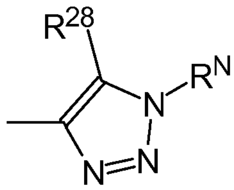,
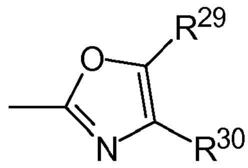,
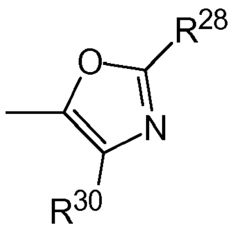,
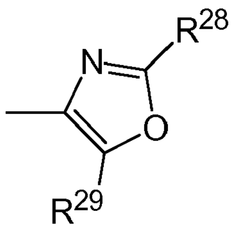,
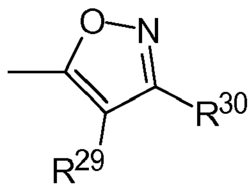,
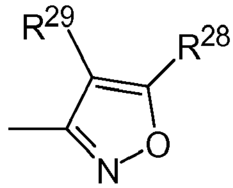,
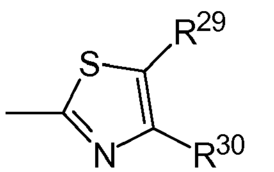,
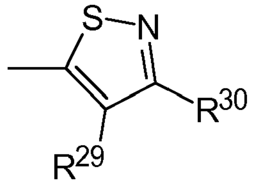,
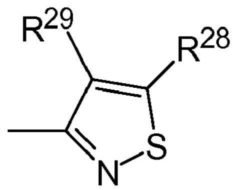,
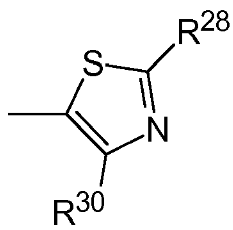,
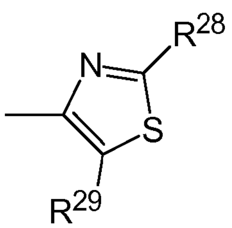,
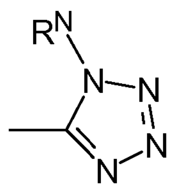,
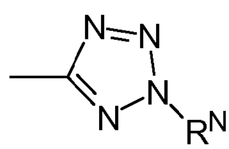, or
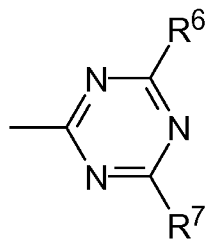;

**$R^E$** represents: **$R^{23}$** or

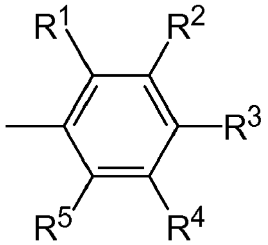,
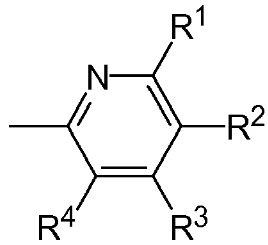,
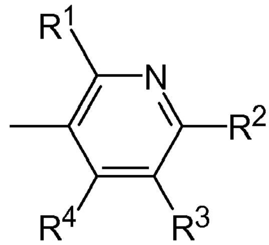,

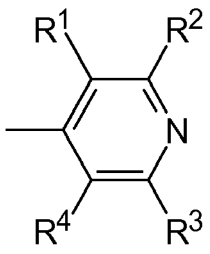,
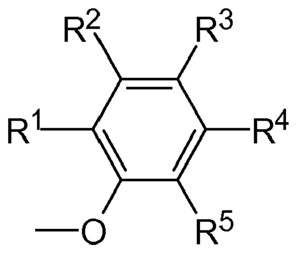,
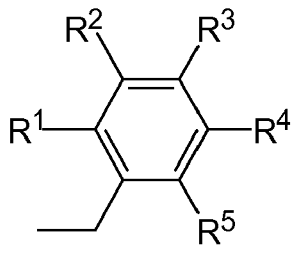,

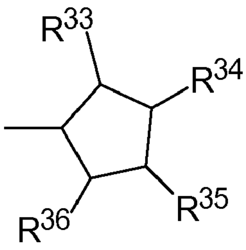,
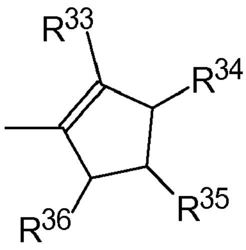,
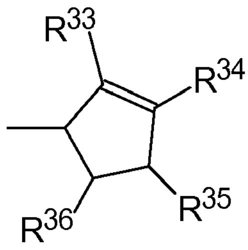,

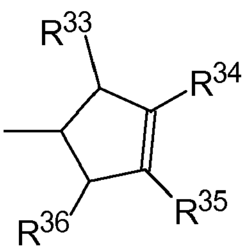,
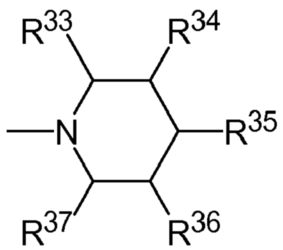,
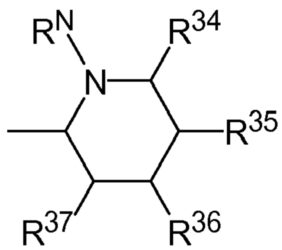,

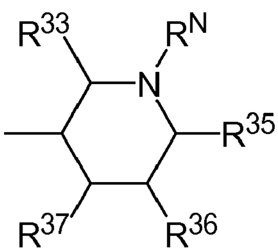,
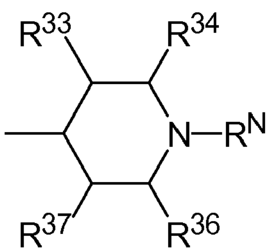,
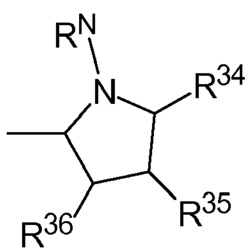,

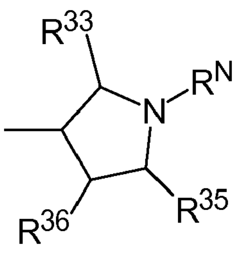,
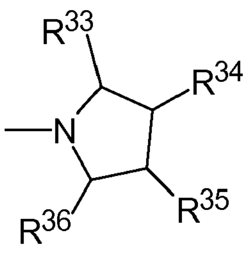,
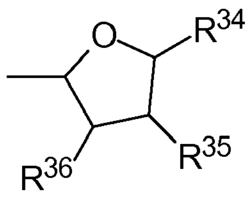,

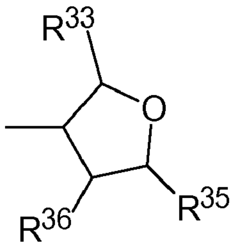 ,
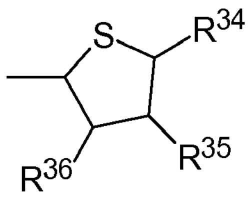 ,
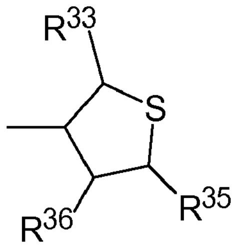 ,
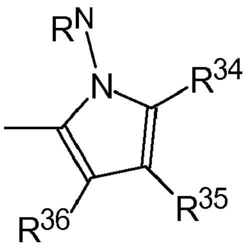 ,
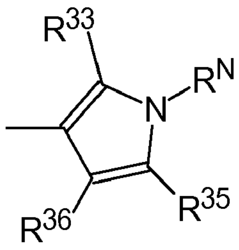 ,
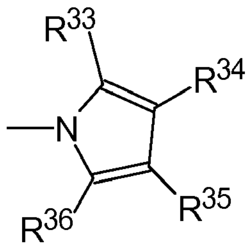 ,
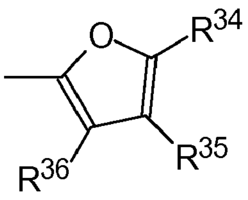 ,
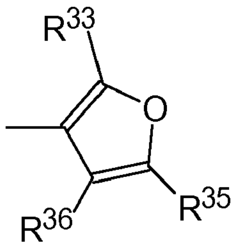 ,
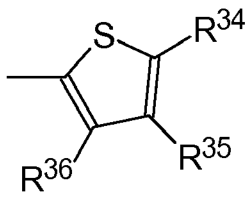 ,
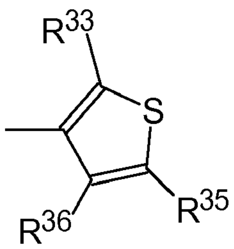 ,
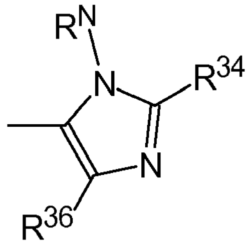 ,
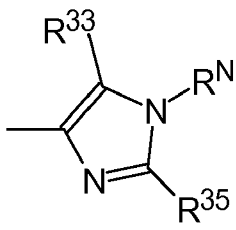 ,
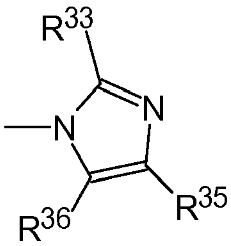 ,
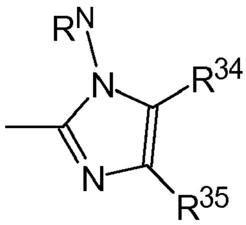 ,
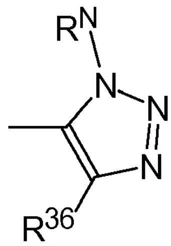 ,
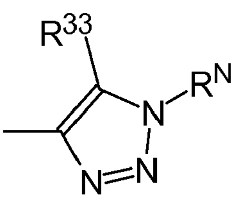 ,
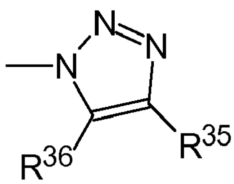 or
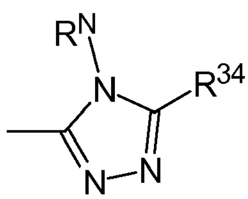 ;

**$R^F$** represents: **$R^{24}$** or

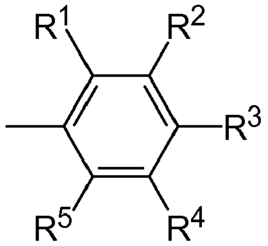
,
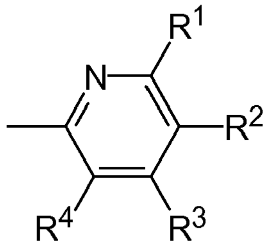
,
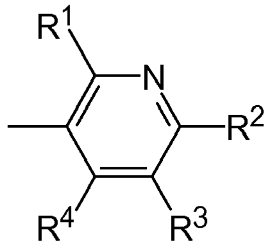
,
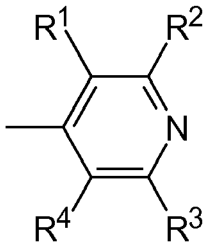
,
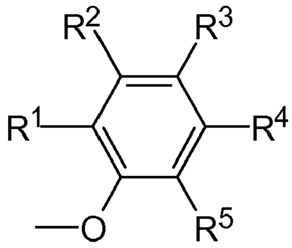
,
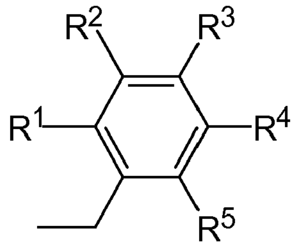
,
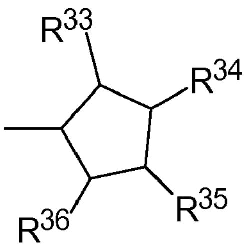
,
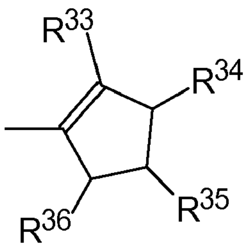
,
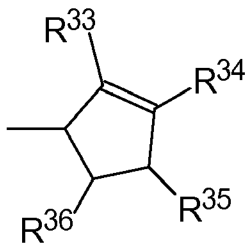
,
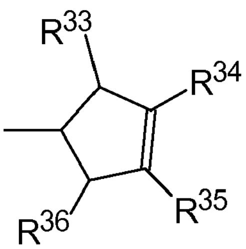
,
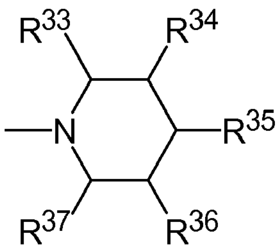
,
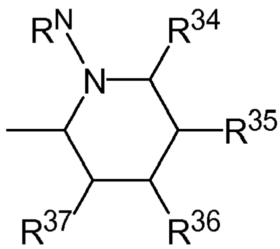
,
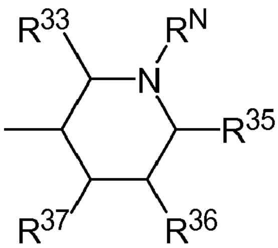
,
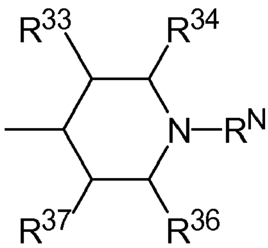
,
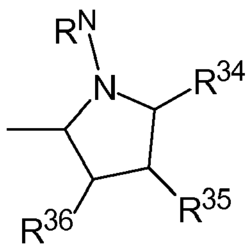
,

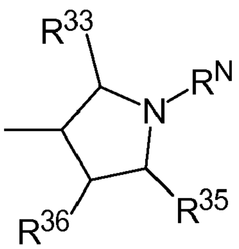 , 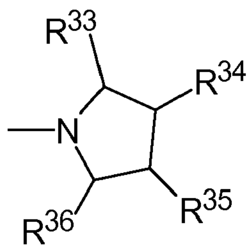 , 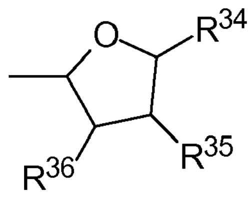 ,

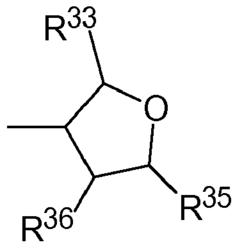 , 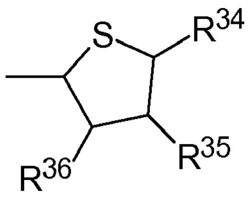 , 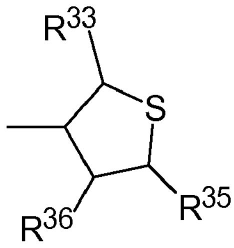 ,

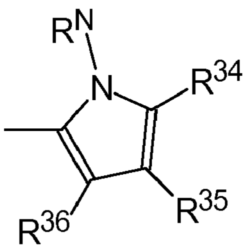 , 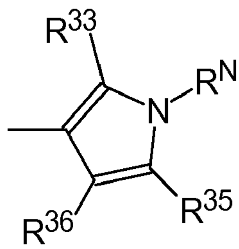 , 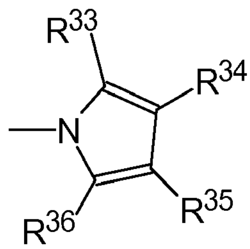 ,

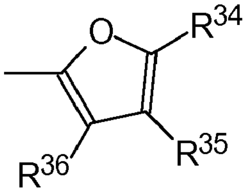 , 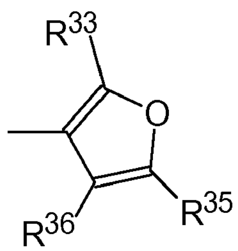 , 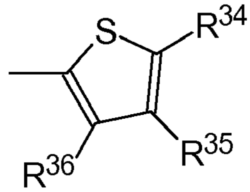 ,

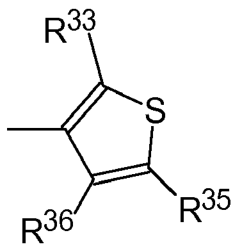 , 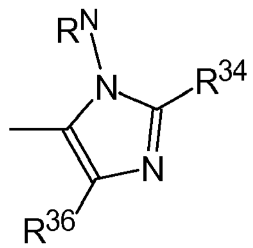 , 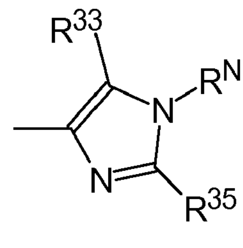 ,

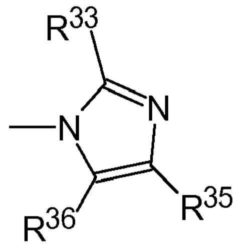 , 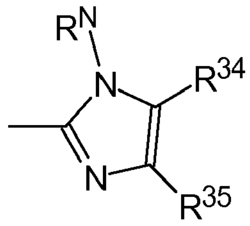 , 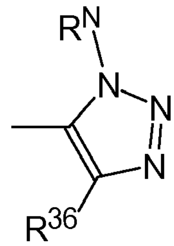 ,

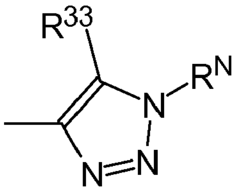 , 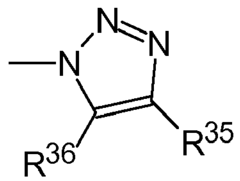 or 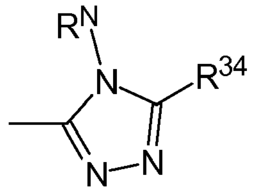 ;

$L_1$, $L_2$ and $L_3$ represent independently of each other: a bond, $-CH_2-$, $-C_2H_4-$, $-C_3H_6-$, $-C_4H_8-$, $-C_5H_{10}-$, $-C_6H_{12}-$, $-C_7H_{14}-$, $-C_8H_{16}-$, $-C_9H_{18}-$, $-C_{10}H_{20}-$, $-CH(CH_3)-$, $-C[(CH_3)_2]-$, $-CH_2-CH(CH_3)-$, $-CH(CH_3)-CH_2-$, $-CH(CH_3)-C_2H_4-$, $-CH_2-CH(CH_3)-CH_2-$, $-C_2H_4-CH(CH_3)-$, $-CH_2-C[(CH_3)_2]-$, $-C[(CH_3)_2]-CH_2-$, $-CH(CH_3)-CH(CH_3)-$, $-C[(C_2H_5)(CH_3)]-$, $-CH(C_3H_7)-$, $-(CH_2)_mO-$, $-(CH_2)_pOCH_2-$, $-(CH_2-CH_2-O)_n-CH_2-CH_2-$, $-CH=CH-$, $-C(CH_3)=CH-$, $-CH=C(CH_3)-$, $-CH_2-CH=CH-$, $-CH_2-C(CH_3)=CH-$, $-CH_2-CH=C(CH_3)-$, $-CH=CH-CH_2-$, $-C(CH_3)=CH-CH_2-$, $-CH=C(CH_3)-CH_2-$, $-C(CH_3)=CH-C(CH_3)=CH-$, $-C_2H_4-CH=CH-CH=CH-$, $-CH_2-CH=CH-CH_2-CH=CH-$, $-C_3H_6-C\equiv C-CH_2-$, $-CH_2-CH=CH-CH=CH-CH_2-$, $-CH=CH-CH=CH-C_2H_4-$, $-CH_2-CH=CH-C(CH_3)=CH-$, $-CH_2-CH=C(CH_3)-CH=CH-$, $-CH_2-C(CH_3)=CH-CH=CH-$, $-CH(CH_3)-CH=CH-CH=CH-$, $-CH=CH-CH_2-C(CH_3)=CH-$, $-CH(CH_3)-C\equiv C-CH_2-$, $-CONH-$, $-NHCO-$, $-CH_2-CONH-$ $-CONH-CH_2-$, $-NHCO-CH_2-$, $-CH_2-NHCO-$; wherein n, m, p are independently an integer from 1 to 10; or

$L_1$-$R^D$ and $L_2$-$R^E$ or $L_1$-$R^D$ and $L_3$-$R^F$ or $L_2$-$R^E$ and $L_3$-$R^F$ can form together a cyclic ring selected from the group consisting of:

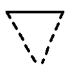 , 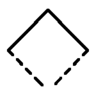 , 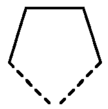 , 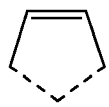 , 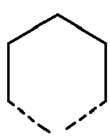 , 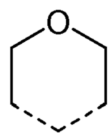 , 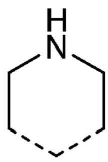 or 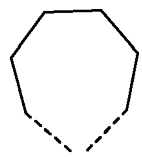 ;

$R^N$ represents -H, $-CH_2-OCH_3$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2OC_3H_7$, $-C_2H_4-OC_3H_7$, $-C_3H_6-OC_3H_7$, $-CH_2-O$-cyclo-$C_3H_5$, $-C_2H_4-O$-cyclo-$C_3H_5$, $-C_3H_6-O$-cyclo-$C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, -CHO, $-COCH_3$, $-COC_2H_5$, $-COC_3H_7$, -CO-cyclo-$C_3H_5$, $-COCH(CH_3)_2$, $-COC(CH_3)_3$, -COCN, $-COOCH_3$, $-COOC_2H_5$, $-COOC_3H_7$, -COO-cyclo-$C_3H_5$, $-COOCH(CH_3)_2$, $-COOC(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5$)$_2$, $-CON[CH(CH_3)_2]_2$, $-CON[C(CH_3)_3]_2$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-CH_2-OCF_3$, $-C_2H_4-OCF_3$, $-C_3H_6-OCF_3$, $-OC_2F_5$, $-CH_2-OC_2F_5$, $-C_2H_4-OC_2F_5$, $-C_3H_6-OC_2F_5$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, -cyclo-$C_8H_{15}$, -Ph, $-CH_2-Ph$, $-CH_2-CH_2-Ph$, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$,

$-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$,- $CH_2-CH=CH-C_3H_7$, $-CH_2-CH(CH_3)-CH_2-CH=CH_2$, $-CH=CH-C_4H_9$, $-C_3H_6-C(CH_3)=CH_2$, $-C_2H_4-CH(CH_3)-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH_2-CH(CH_3)-C(CH_3)=CH_2$, $-CH=C(CH_3)-C_3H_7$, $-C(CH_3)=CH-C_3H_7$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C\equiv CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-C_2H_5$, $-CH_2-C=C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-CH_2-CH(C\equiv CH)_2$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C(C\equiv CH)_2-CH_3$,- $C\equiv C-CH(CH_3)-C\equiv CH$, $-CH(CH_3)-C\equiv C-C\equiv CH$, $-CH(C\equiv CH)-CH_2-C\equiv CH$, or $-CH(C\equiv CH)-C\equiv C-CH_3$;

**$R^B$** represents

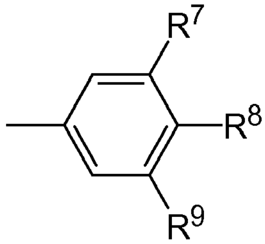
,
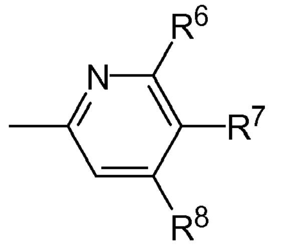
,
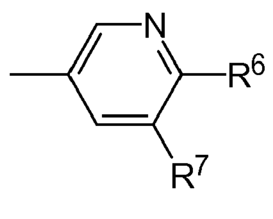
,
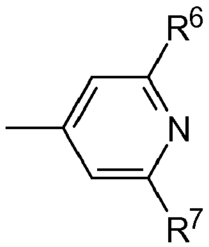
,
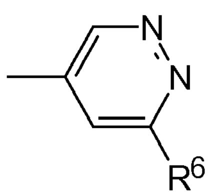
,
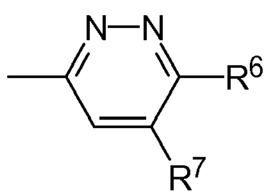
,

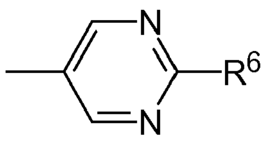
,
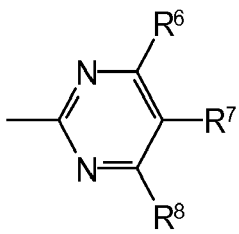
,
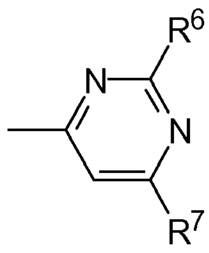
,

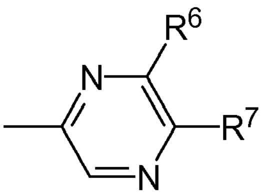
,
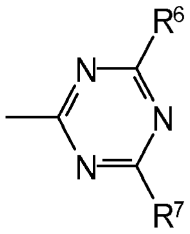
,
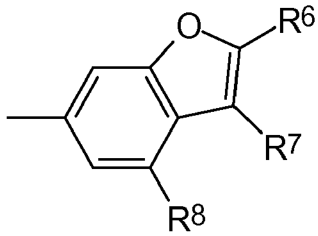
,

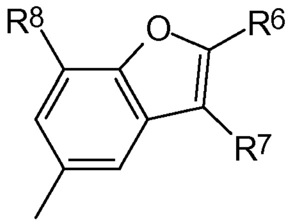
,
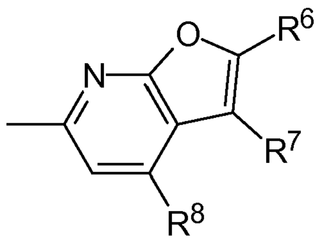
,
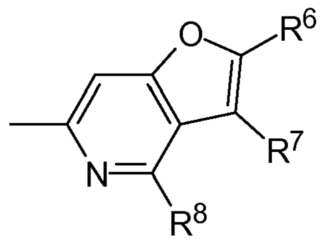
,

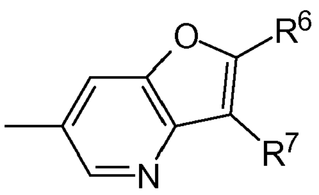
,
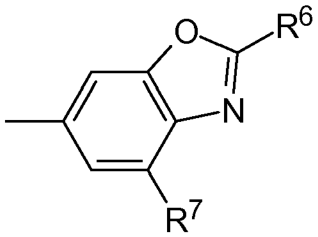
,
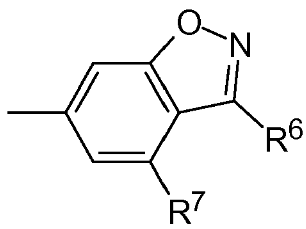
,

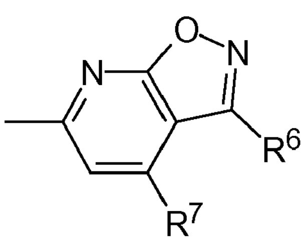
,
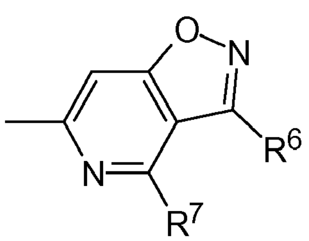
,
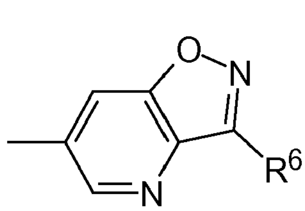
,

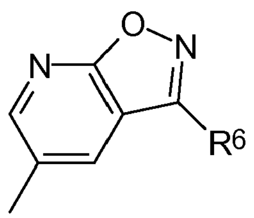

,

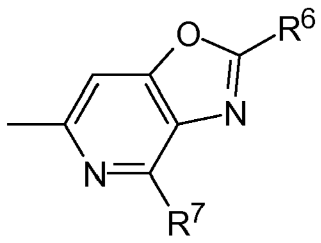

,

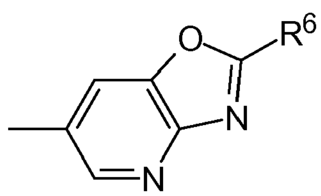

,

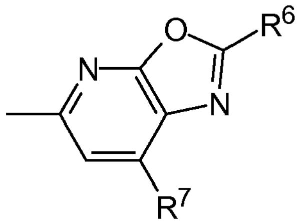

,

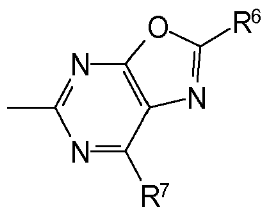

,

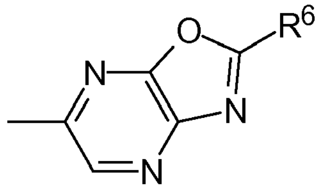

,

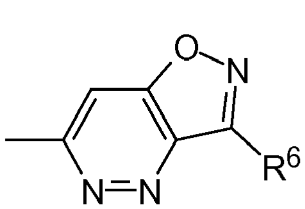

,

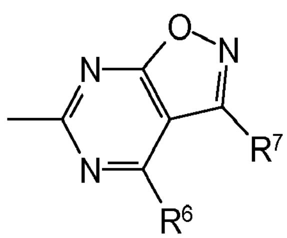

,

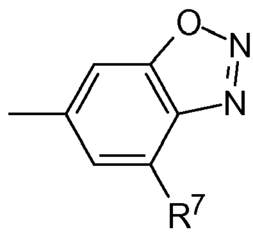

,

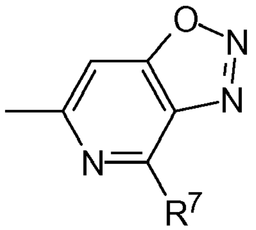

,

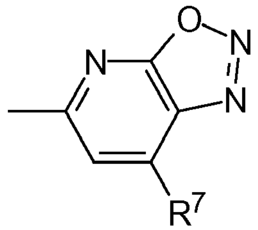

,

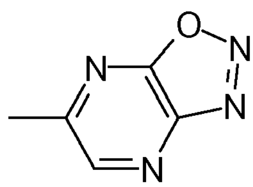

,

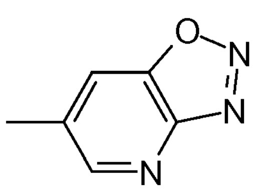

,

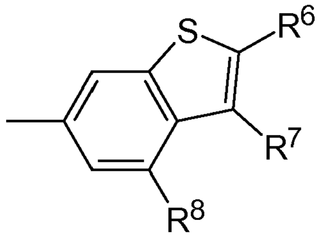

,

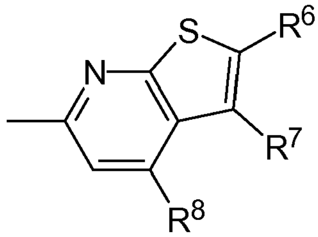

,

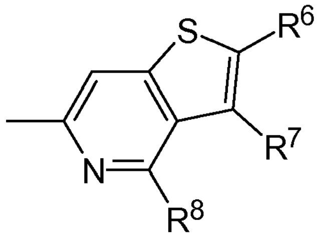

,

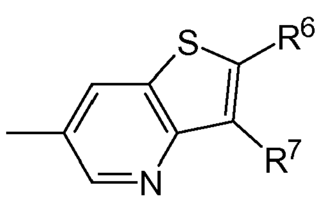

,

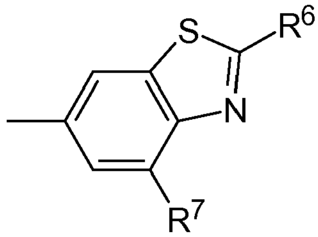

,

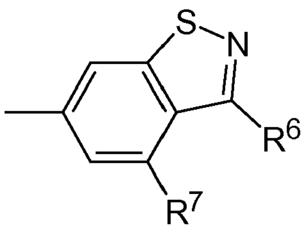
,
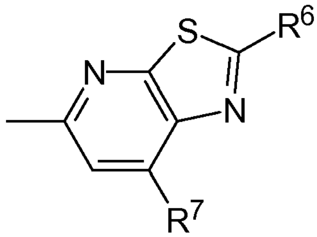
,
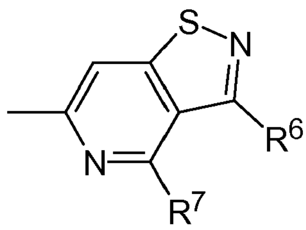
,

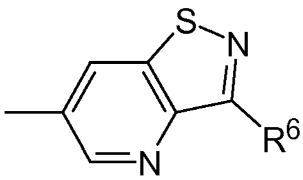
,
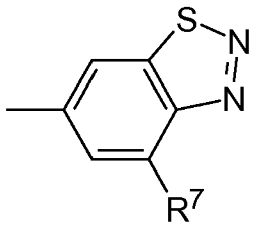
,
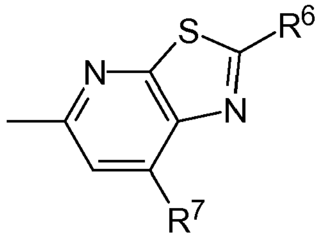
,

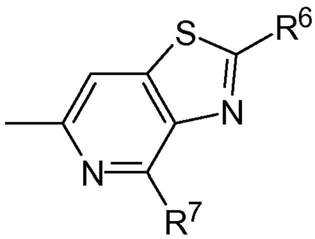
,
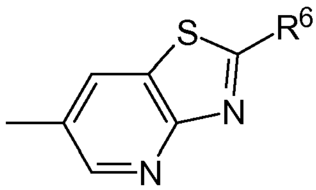
,
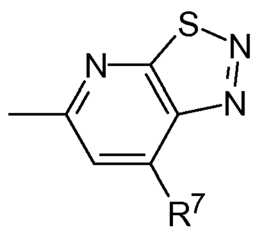
,

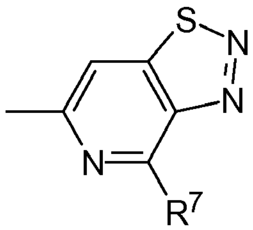
,
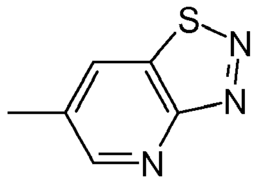
,
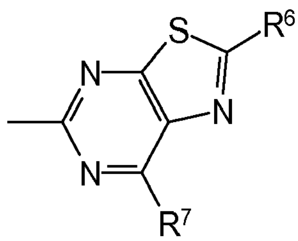
,

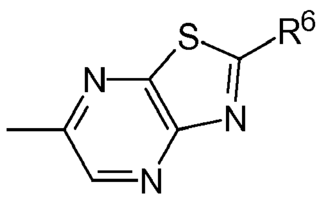
,
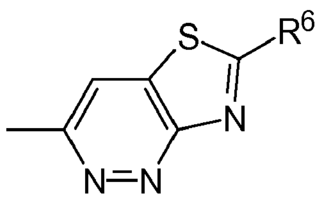
,
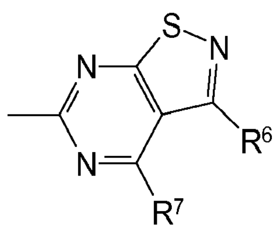
,

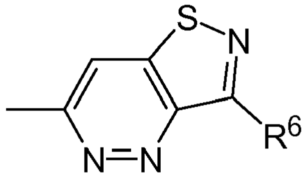
,
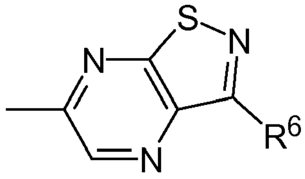
,
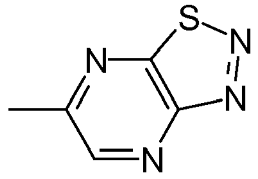
,

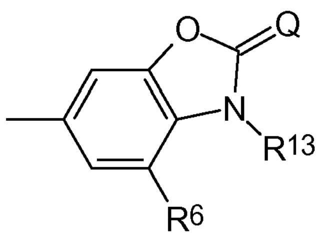
,
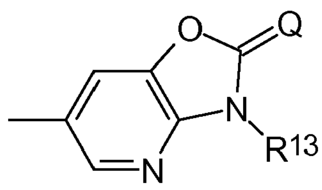
,
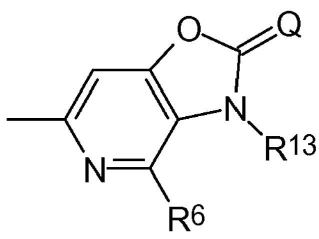
,

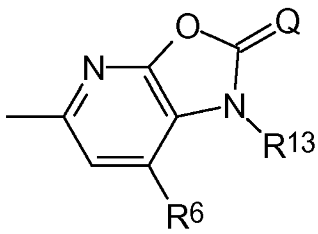
,
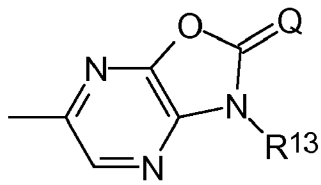
,
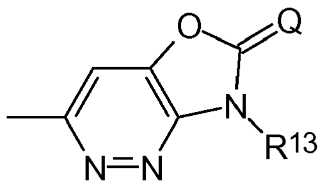
,

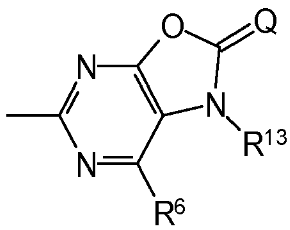
,
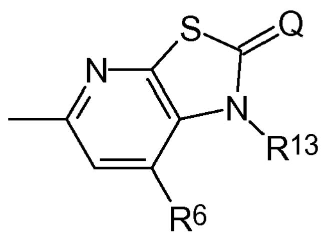
,
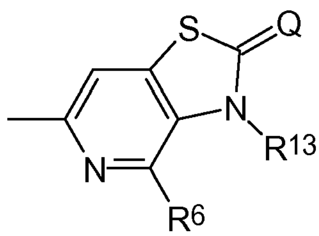
,

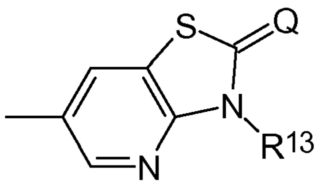
,
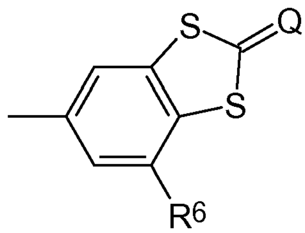
,
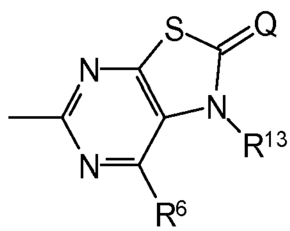
,

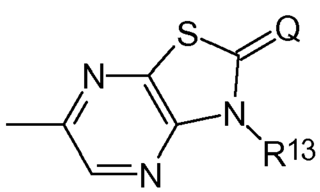
,
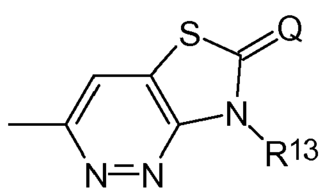
,
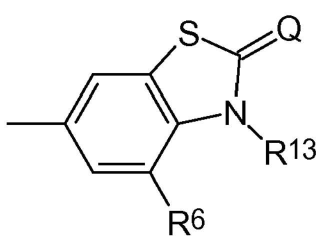
,

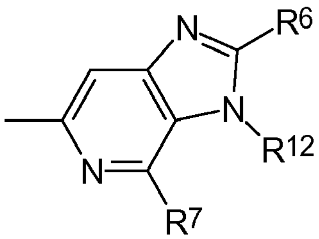,
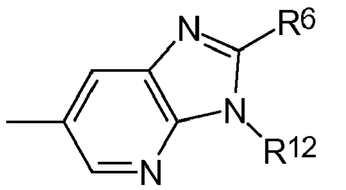,
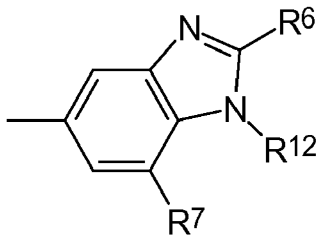,
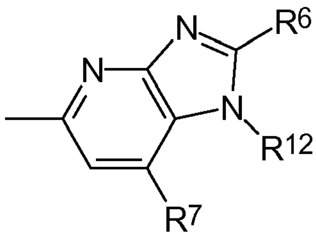,
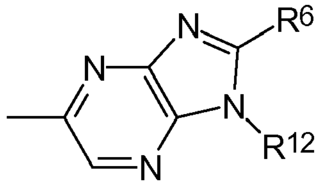,
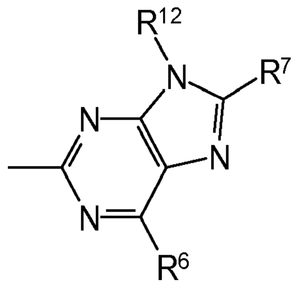,
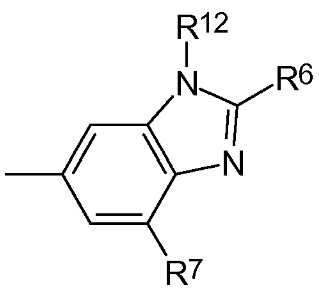,
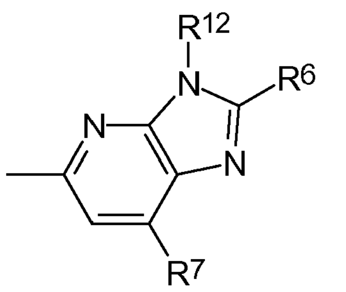,
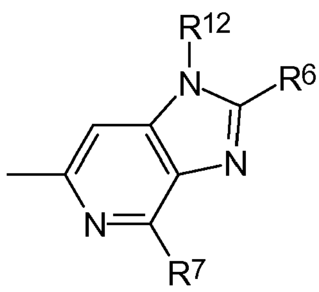,
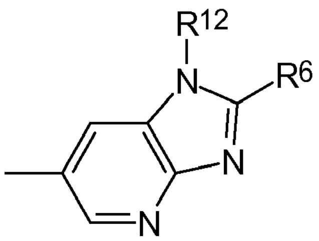,
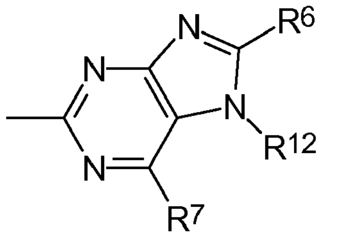,
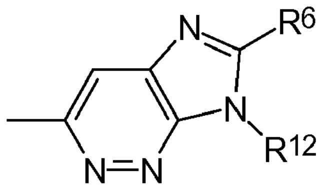,
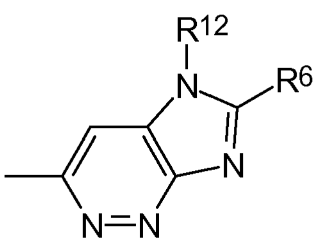,
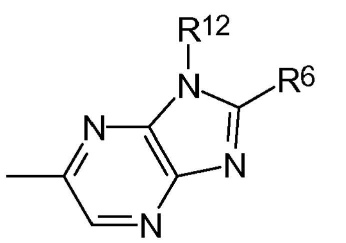,
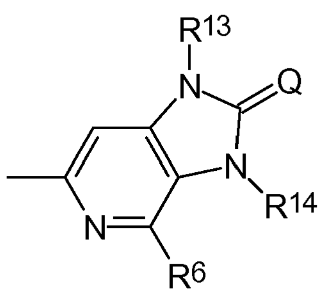,

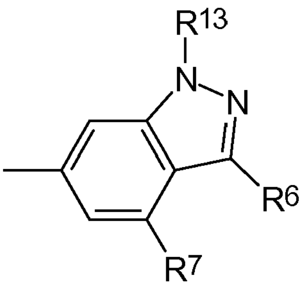
,
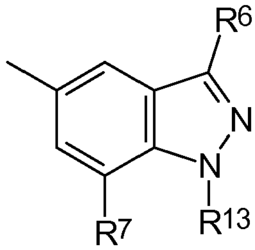
,
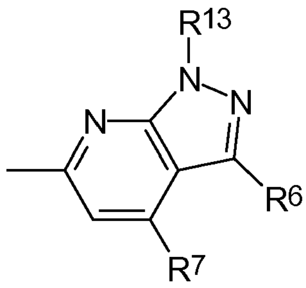
,

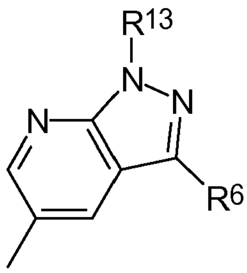
,
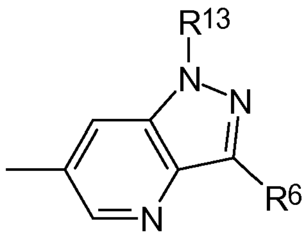
,
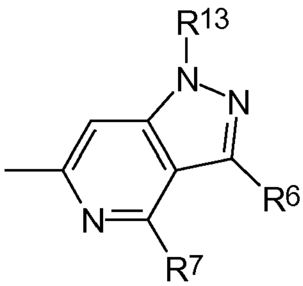
,

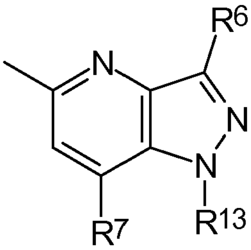
,
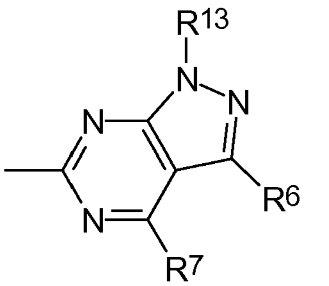
,
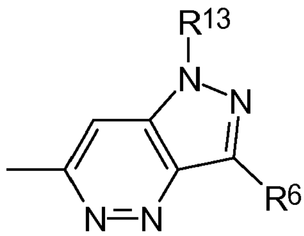
,

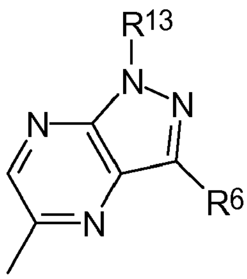
,
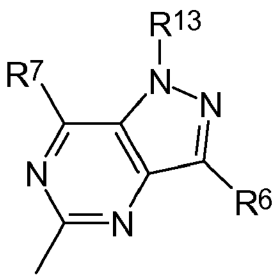
,
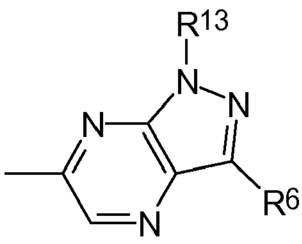
,

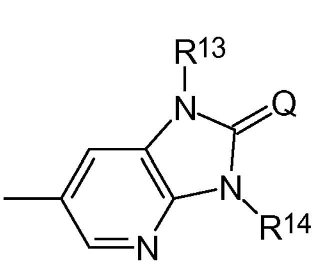
,
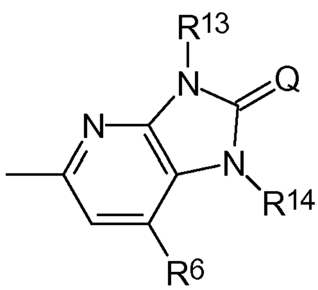
,
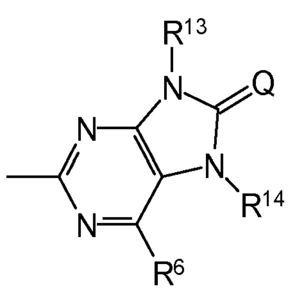
,

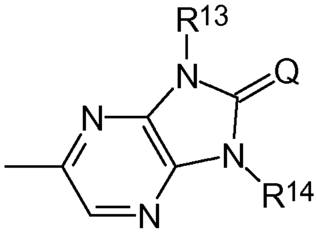 , 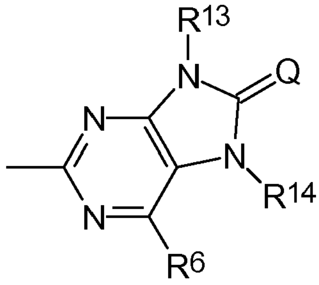 , 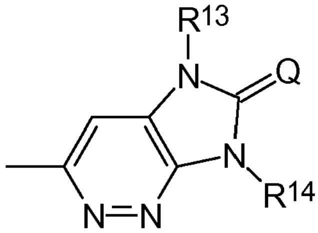 ,

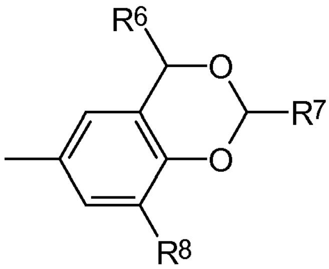 , 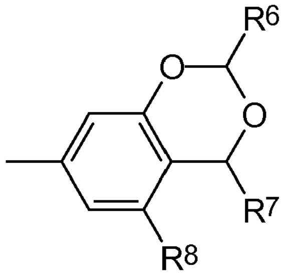 , 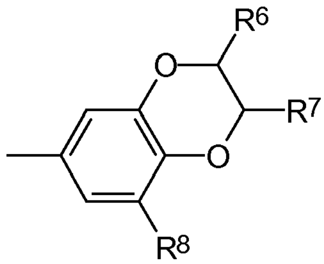 ,

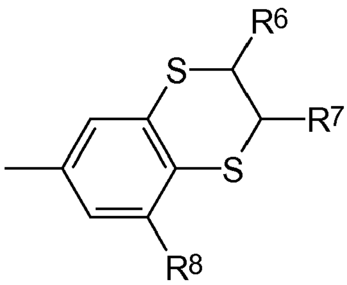 , 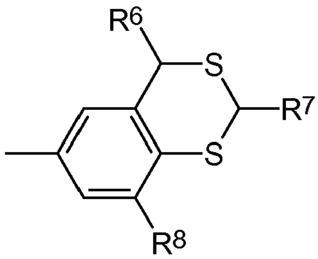 , 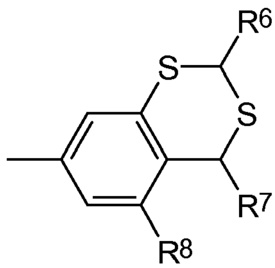 ,

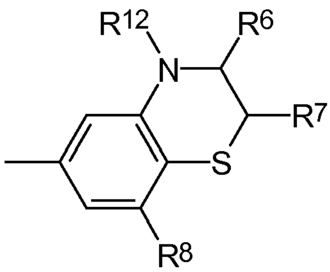 , 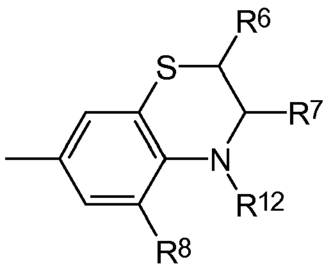 , 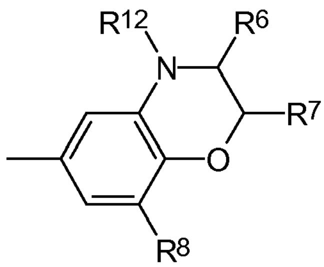 ,

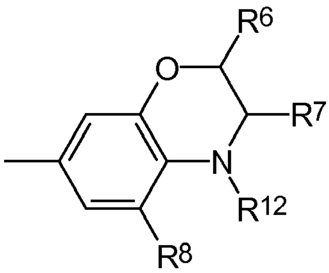 , 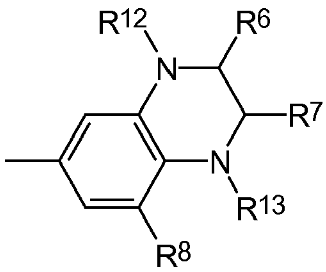 , 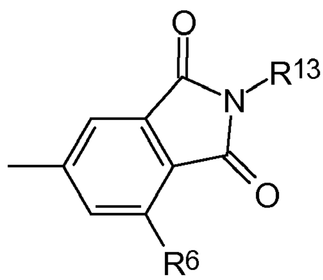 ,

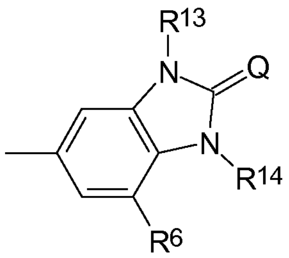 , 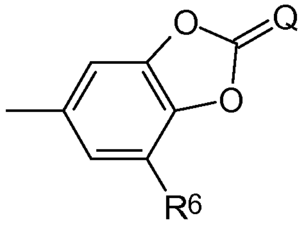 , 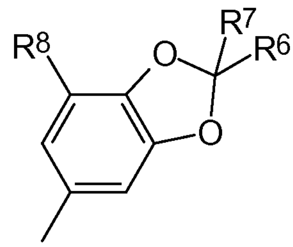 ,

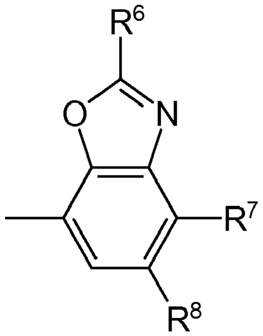 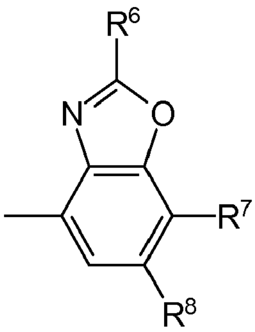 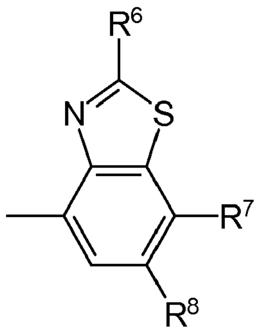 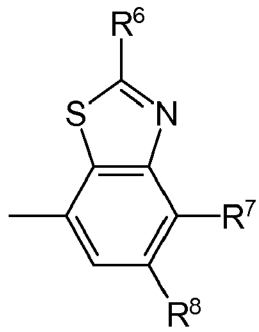

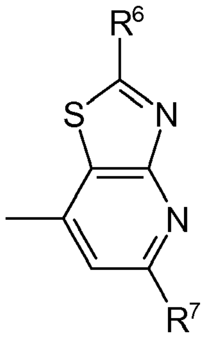 , 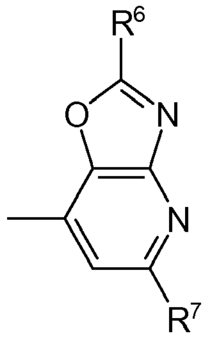 , 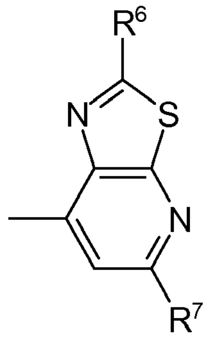 or 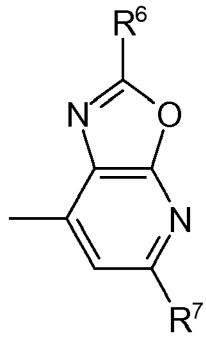 ;

**Q** represents =O, =S, or =N-$R^{12}$;

**$R^C$** represents -OH, $-CH_2-OH$, -CHO, $-C_2H_4-OH$, $-C_3H_6-OH$, $-O-CH_3$, $-O-C_2H_5$, $-O-CH_2-OH$, $-O-CH(CH_3)_2$, $-O-CH_2-O-CH_3$, $-O-C_2H_4-O-CH_3$, $-CH_2-O-CH_3$, $-CH_2-O-CH_2-OH$, $-CH_2O-C_2H_5$, $-CH_2-O-CH(CH_3)_2$, $-CH_2-O-C_3H_7$, $-CO-CH_3$, $-CH_2-CO-CH_3$, $-CO-CH_2-OH$, $-CH(OH)-CH_3$, $-C(OH)(CH_3)_2$, $-CH(CH_3)CH_2OH$, $-CH(OH)-CH_2-OH$, $-CH_2-CH(OH)-CH_3$, $-CH_2-CH(OH)-CH_2-OH$, $-CH(OCH_3)-CH_2OH$, $-CH(OC_2H_5)-CH_2OH$, $-CH(OCH_3)-CH_2OCH_3$, $-CH(OC_2H_5)-CH_2OCH_3$, $-CH(OC_2H_5)-CH_2OC_2H_5$, $-CH(OAc)-CH_2OH$, $-CH(OAc)-CH_2OAc$, $-CH(OH)-CH_2OAc$, $-CH(OH)-CH_2-NH_2$, $-CH_2-CH(OH)-CH_2-NH_2$,- $CH(OCH_3)-CH_2-NH_2$, $-CH(OC_2H_5)-CH_2-NH_2$, $-CH_2-CH(OCH_3)-CH_2-NH_2$, $-CH_2-CH(OC_2H_5)-CH_2-NH_2$, $-CH(OH)-CH_2-NHCH_3$, $-CH(OH)-CH_2-NHC_2H_5$, $-CH_2-CH(OH)-CH_2-NHCH_3$, $-CH_2-CH(OH)-CH_2-NHC_2H_5$, $-CH(OCH_3)-CH_2NHCH_3$, $-CH(OC_2H_5)-CH_2NHCH_3$, $-CH_2-CH(OCH_3)-CH_2-NHCH_3$, $-CH_2-CH(OC_2H_5)-CH_2-NHCH_3$, $-CH(OCH_3)-CH_2NHC_2H_5$, $-CH(OC_2H_5)-CH_2NHC_2H_5$, $-CH(OCH_3)-CH_2N(CH_3)_2$, $-CH(OC_2H_5)-CH_2N(CH_3)_2$, $-NH_2$, $-NHCH_3$, $-N(CH_3)_2$, $-CH_2-NH_2$, $-CH_2-NHCH_3$, $-CH_2-N(CH_3)_2$, $-C_2H_4-NH_2$, $-C_2H_4-NHCH_3$, $-C_2H_4-N(CH_3)_2$, $-CH(NHCH_3)CH_3$, $-CH(NHC_2H_5)CH_3$, $-CH(N(CH_3)_2)CH_3$, $-CH(N(C_2H_5)_2)CH_3$, $-CH(NH_2)CH_2OH$, $-CH(NHCH_3)CH_2OH$, $-CH(NHC_2H_5)CH_2OH$, $-CH(N(CH_3)_2)CH_2OH$, $-CH(N(C_2H_5)_2)CH_2OH$, $-CH(NH_2)CH_2OCH_3$, $-CH(NHCH_3)CH_2OCH_3$, $-CH(NHC_2H_5)CH_2OCH_3$, $-CH(N(CH_3)_2)CH_2OCH_3$, $-CH(N(C_2H_5)_2)CH_2OCH_3$, $-CH(NH_2)CH_2OC_2H_5$, $-CH(NHCH_3)CH_2OC_2H_5$, $-CH(NHC_2H_5)CH_2OC_2H_5$, $-CH(N(CH_3)_2)CH_2OC_2H_5$, $-CH(N(C_2H_5)_2)CH_2OC_2H_5$, $-CH(NH_2)CH_2OAc$, $-CH(NHCH_3)CH_2OAc$, $-CH(NHC_2H_5)CH_2OAc$, $-CH(N(CH_3)_2)CH_2OAc$, $-CH(N(C_2H_5)_2)CH_2OAc$, $-CH_2-CH(NHAc)CH_2OH$, $-CH_2-CH(NHAc)CH_2OCH_3$, $-CH_2-CH(NHAc)CH_2OC_2H_5$, $-CH_2-CH(NHCH_3)CH_3$, $-CH_2-CH(NHC_2H_5)CH_3$, $-CH_2-CH(N(CH_3)_2)CH_3$, $-CH_2-CH(N(C_2H_5)_2)CH_3$, $-CH_2-CH(NH_2)CH_2OH$, $-CH_2-CH(NHCH_3)CH_2OH$, $-CH_2-CH(NHC_2H_5)CH_2OH$, $-CH_2-CH(N(CH_3)_2)CH_2OH$, $-CH_2-CH(N(C_2H_5)_2)CH_2OH$, $-CH_2-CH(NH_2)CH_2OCH_3$, $-CH_2-CH(NHCH_3)CH_2OCH_3$, $-CH_2-CH(NHC_2H_5)CH_2OCH_3$, $-CH_2-CH(N(CH_3)_2)CH_2OCH_3$, $-CH_2-CH(N(C_2H_5)_2)CH_2OCH_3$, $-CH_2-CH(NH_2)CH_2OC_2H_5$,

$-CH_2-CH(NHCH_3)CH_2OC_2H_5$, $-CH_2-CH(NHC_2H_5)CH_2OC_2H_5$, $-CH_2-CH(N(CH_3)_2)CH_2OC_2H_5$, $-CH_2-CH(N(C_2H_5)_2)CH_2OC_2H_5$, $-CH_2-CH(NH_2)CH_2OAc$, $-CH_2-CH(NHCH_3)CH_2OAc$, $-CH_2-CH(NHC_2H_5)CH_2OAc$, $-CH_2-CH(N(CH_3)_2)CH_2OAc$, $-CH_2-CH(N(C_2H_5)_2)CH_2OAc$, $-CH_2-CH(NHAc)CH_2OH$, $-CH_2-CH(NHAc)CH_2OCH_3$, $-CH_2-CH(NHAc)CH_2OC_2H_5$, -NHCOCH$_3$, $-CH_2-NHCOCH_3$, $-C_2H_4-NHCOCH_3$, -NHCHO, $-CH_2-NHCHO$, $-C_2H_4-NH-CHO$, $-NHSO_2CH_3$, $-NHSO_2CF_3$, $-NHSO_2CH_2CF_3$, $-CH_2-NHSO_2CH_3$, $-CH_2-NHSO_2CF_3$, $-CH_2-NHSO_2CH_2CF_3$, $-C_2H_4-NHSO_2CH_3$, $-C_2H_4-NHSO_2CF_3$, $-C_2H_4-NHSO_2CH_2CF_3$, $-NO_2$, $-CH_2-NO_2$, $-C_2H_4-NO_2$, $-CH(OH)-NO_2$, $-CH(NO_2)-OH$, $-CO_2H$, $-CH_2-CO_2H$, $-C_2H_4-CO_2H$, $-CH=CH-CO_2H$, $-CO_2CH_3$, $-CO_2C_2H_5$, $-CO_2CH(CH_3)_2$, $-CH_2-CO_2CH_3$, $-CH_2-CO_2C_2H_5$, $-CH_2-CO_2CH(CH_3)_2$, $-C_2H_4-CO_2CH_3$, $-C_2H_4-CO_2C_2H_5$, $-C_2H_4-CO_2CH(CH_3)_2$, $-CO_2NH_2$, $-CO_2NHCH_3$, $-CO_2N(CH_3)_2$, $-CH_2-CO_2NH_2$, $-CH_2-CO_2NHCH_3$, $-CH_2-CO_2N(CH_3)_2$, $-C_2H_4-CO_2NH_2$, $-C_2H_4-CO_2NHCH_3$, $-C_2H_4-CO_2N(CH_3)_2$, $-O-Si(CH_3)_3$, $-O-Si(C_2H_5)_3$, -CO-CHO, $-CO-CO-CH_3$,- $C(OH)-CO-CH_3$, $-CO-C(OH)-CH_3$, $-CO-CH_2-CO-CH_3$, $-C(OH)-CH_2-CO-CH_3$, $-CO-CH_2-C(OH)-CH_3$, $-C(OH)-CH_2-C(OH)-CH_3$, -F, -Cl, -Br, $-CH_2-F$, $-CHF_2$, $-CF_3$, $-C_2H_4-F$, $-CH_2-CF_3$, $-CF_2-CF_3$, $-O-CHF_2$, $-O-CF_3$, $-O-CH_2-CF_3$, $-O-C_2F_5$, $-CH_3$, $-CH_2CH_3$, $-C_3CH_7$, $-CH(CH_3)_2$, $-CH=CH_2$, $-C \equiv CH$, $-CH_2-CH=CH_2$, or $-CH_2-C \equiv CH$;

**R**$^1$ - **R**$^{10}$ represent independently of each other -H, -OH, $-OCH_3$, $-OC_2H_5$, $-OC_3H_7$, $-O-cyclo-C_3H_5$, $-OCH(CH_3)_2$, $-OC(CH_3)_3$, $-OC_4H_9$, $-OCH_2-COOH$, -OPh, $-OCH_2-Ph$, $-OCPh_3$, $-CH_2-OCH_3$, $-CH_2-OH$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2-O-cyclo-C_3H_5$, $-C_2H_4-O-cyclo-C_3H_5$, $-C_3H_6-O-cyclo-C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, -SH, $-SCH_3$, $-SC_2H_5$, $-SC_3H_7$, $-S-cyclo-C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, $-CO-cyclo-C_3H_5$, $-COCH(CH_3)_2$, $-COC(CH_3)_3$, -COOH, -COCN, $-COOCH_3$, $-COOC_2H_5$, $-COOC_3H_7$, $-COO-cyclo-C_3H_5$, $-COOCH(CH_3)_2$, $-COOC(CH_3)_3$, $-OOC-CH_3$, $-OOC-C_2H_5$, $-OOC-C_3H_7$, $-OOC-cyclo-C_3H_5$, $-OOC-CH(CH_3)_2$, $-OOC-C(CH_3)_3$, $-CONH_2$, $-CONHCH_3$, $-CONHC_2H_5$, $-CONHC_3H_7$, $-CONH-cyclo-C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, $-CON(cyclo-C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, $-NHCO-cyclo-C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, $-NHCO-O-cyclo-C_3H_5$, $-NHCO-OCH(CH_3)_2$, $-NHCO-OC(CH_3)_3$, $-NH_2$, $-NHCH_3$, $-NHC_2H_5$, $-NHC_3H_7$, $-NH-cyclo-C_3H_5$, $-NHCH(CH_3)_2$, $-NHC(CH_3)_3$, $-N(CH_3)_2$, $-N(C_2H_5)_2$, $-N(C_3H_7)_2$, $-N(cyclo-C_3H_5)_2$, $-N[CH(CH_3)_2]_2$, $-N[C(CH_3)_3]_2$, $-SOCH_3$, $-SOC_2H_5$, $-SOC_3H_7$, $-SO-cyclo-C_3H_5$, $-SOCH(CH_3)_2$, $-SOC(CH_3)_3$, $-SO_2CH_3$, $-SO_2C_2H_5$, $-SO_2C_3H_7$, $-SO_2-cyclo-C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3-cyclo-C_3H_5$, $-SO_3CH(CH_3)_2$, $-SO_3C(CH_3)_3$, $-SO_2NH_2$, $-SO_2NHCH_3$, $-SO_2NHC_2H_5$, $-SO_2NHC_3H_7$, $-SO_2NH-cyclo-C_3H_5$, $-SO_2NHCH(CH_3)_2$, $-SO_2NHC(CH_3)_3$, $-SO_2N(CH_3)_2$, $-SO_2N(C_2H_5)_2$, $-SO_2N(C_3H_7)_2$, $-SO_2N(cyclo-C_3H_5)_2$, $-SO_2N[CH(CH_3)_2]_2$, $-SO_2N[C(CH_3)_3]_2$, $-O-S(=O)CH_3$,- $O-S(=O)C_2H_5$, $-O-S(=O)C_3H_7$, $-O-S(=O)-cyclo-C_3H_5$, $-O-S(=O)CH(CH_3)_2$, $-O-S(=O)C(CH_3)_3$, $-S(=O)(=NH)CH_3$, $-S(=O)(=NH)C_2H_5$, $-S(=O)(=NH)C_3H_7$, $-S(=O)(=NH)-cyclo-C_3H_5$, $-S(=O)(=NH)CH(CH_3)_2$, $-S(=O)(=NH)C(CH_3)_3$, $-NH-SO_2-CH_3$, $-NH-SO_2-C_2H_5$, $-NH-SO_2-C_3H_7$, $-NH-SO_2-cyclo-C_3H_5$, $-NH-SO_2-CH(CH_3)_2$, $-NH-SO_2-C(CH_3)_3$, $-O-SO_2-CH_3$, $-O-SO_2-C_2H_5$, $-O-SO_2-C_3H_7$, $-O-SO_2-cyclo-C_3H_5$, $-O-SO_2-CH(CH_3)_2$, $-O-SO_2-C(CH_3)_3$, $-OCF_3$, $-CH_2-OCF_3$, $-C_2H_4-OCF_3$, $-C_3H_6-OCF_3$, $-OC_2F_5$, $-CH_2-OC_2F_5$, $-C_2H_4-OC_2F_5$, $-C_3H_6-OC_2F_5$, $-O-COOCH_3$, $-O-COOC_2H_5$, $-O-COOC_3H_7$, $-O-COO-cyclo-C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CS-N(C_3H_7)_2$, $-NH-CO-NHC_3H_7$, $-NH-CO-N(C_3H_7)_2$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-NH-cyclo-C_3H_5$, $-NH-CO-N(cyclo-C_3H_5)_2$, $-NH-CO-N[CH(CH_3)_2]_2$, $-NH-CS-N(C_2H_5)_2$, $-NH-CO-N[C(CH_3)_3]_2$, $-NH-CS-NH_2$, $-NH-CS-NHCH_3$, $-NH-CS-N(CH_3)_2$, $-NH-CS-NHC_2H_5$, $-NH-CS-NHC_3H_7$, $-NH-CS-NH-cyclo-C_3H_5$, $-NH-CS-NH[CH(CH_3)_2]$, $-NH-CS-NH[C(CH_3)_3]$, $-NH-CS-N(cyclo-C_3H_5)_2$, $-NH-CS-N[CH(CH_3)_2]_2$, $-NH-CS-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, $-O-CO-NH-cyclo-C_3H_5$, $-NH-C(=NH)-NH-cyclo-C_3H_5$, $-NH-C(=NH)-NH[CH(CH_3)_2]$, $-O-CO-NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$, $-NH-C(=NH)-N(C_3H_7)_2$, $-NH-C(=NH)-N(cyclo-C_3H_5)_2$, $-O-CO-NHC_3H_7$, $-NH-C(=NH)-N[CH(CH_3)_2]_2$, $-NH-C(=NH)-N[C(CH_3)_3]_2$, $-O-CO-NH_2$, $-O-CO-NHCH_3$, $-O-CO-NHC_2H_5$, $-O-CO-NH[C(CH_3)_3]$, $-O-CO-N(CH_3)_2$, $-O-CO-N(C_2H_5)_2$, $-O-CO-N(C_3H_7)_2$, $-O-CO-N(cyclo-C_3H_5)_2$, $-O-CO-N[CH(CH_3)_2]_2$, $-O-CO-N[C(CH_3)_3]_2$, $-O-CO-OCH_3$, $-O-CO-OC_2H_5$, $-O-CO-OC_3H_7$, $-O-CO-O-cyclo-C_3H_5$, $-O-CO-OCH(CH_3)_2$, $-O-CO-OC(CH_3)_3$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, $-cyclo-C_3H_5$, $-cyclo-C_4H_7$, $-cyclo-C_5H_9$, $-cyclo-C_6H_{11}$, $-cyclo-C_7H_{13}$, $-cyclo-C_8H_{15}$, -Ph, $-CH_2-Ph$, $-CH_2-CH_2-Ph$, $-CH=CH-Ph$, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$,

-CH(CH$_3$)-C(CH$_3$)$_3$, -CH=CH$_2$, -CH$_2$-CH=CH$_2$, -C(CH$_3$)=CH$_2$, -CH=CH-CH$_3$, -C$_2$H$_4$-CH=CH$_2$, -CH$_2$-CH=CH-CH$_3$, -CH=CH-C$_2$H$_5$,- CH$_2$-C(CH$_3$)=CH$_2$, -CH(CH$_3$)-CH=CH, -CH=C(CH$_3$)$_2$, -C(CH$_3$)=CH-CH$_3$, -CH=CH-CH=CH$_2$, -C$_3$H$_6$CH=CH$_2$, -C$_2$H$_4$-CH=CH-CH$_3$, -CH$_2$-CH=CH-C$_2$H$_5$, -CH=CH-C$_3$H$_7$, -CH$_2$-CH=CH-CH=CH$_2$, -CH=CH-CH=CH-CH$_3$, -CH=CH-CH$_2$-CH=CH$_2$, -C(CH$_3$)=CH-CH=CH$_2$, -CH=C(CH$_3$)-CH=CH$_2$, -CH=CH-C(CH$_3$)=CH$_2$, -C$_2$H$_4$-C(CH$_3$)=CH$_2$, -CH$_2$-CH(CH$_3$)-CH=CH$_2$, -CH(CH$_3$)-CH$_2$-CH=CH$_2$, -CH$_2$-CH=C(CH$_3$)$_2$, -CH$_2$-C(CH$_3$)=CH-CH$_3$, -CH(CH$_3$)-CH=CH-CH$_3$, -CH=CH-CH(CH$_3$)$_2$, -CH=C(CH$_3$)-C$_2$H$_5$, -C(CH$_3$)=CH-C$_2$H$_5$, -C(CH$_3$)=C(CH$_3$)$_2$, -C(CH$_3$)$_2$-CH=CH$_2$, -CH(CH$_3$)-C(CH$_3$)=CH$_2$, -C(CH$_3$)=CH-CH=CH$_2$, -CH=C(CH$_3$)-CH=CH$_2$, -CH=CH-C(CH$_3$)=CH$_2$, -C$_4$H$_8$-CH=CH$_2$, -C$_3$H$_6$-CH=CH-CH$_3$, -C$_2$H$_4$-CH=CH-C$_2$H$_5$, -CH$_2$-CH=CH-C$_3$H$_7$, -CH=CH-C$_4$H$_9$, -C$_3$H$_6$C(CH$_3$)=CH$_2$, -C$_2$H$_4$-CH(CH$_3$)-CH=CH$_2$, -CH$_2$-CH(CH$_3$)-CH$_2$-CH=CH$_2$, -C$_2$H$_4$-CH=C(CH$_3$)$_2$, -CH(CH$_3$)-C$_2$H$_4$-CH=CH$_2$, -C$_2$H$_4$-C(CH$_3$)=CH-CH$_3$, -CH$_2$-CH(CH$_3$)-CH=CH-CH$_3$, -CH(CH$_3$)-CH$_2$-CH=CH-CH$_3$, -CH$_2$-CH=CH-CH(CH$_3$)$_2$, -CH$_2$-CH=C(CH$_3$)-C$_2$H$_5$, -CH$_2$-C(CH$_3$)=CH-C$_2$H$_5$, -CH(CH$_3$)-CH=CH-C$_2$H$_5$, -CH=CH-CH$_2$-CH(CH$_3$)$_2$, -CH=CH-CH(CH$_3$)-C$_2$H$_5$, -CH=C(CH$_3$)-C$_3$H$_7$, -C(CH$_3$)=CH-C$_3$H$_7$, -CH$_2$-CH(CH$_3$)-C(CH$_3$)=CH$_2$, -C[C(CH$_3$)$_3$]=CH$_2$, -CH(CH$_3$)-CH$_2$-C(CH$_3$)=CH$_2$, -CH(CH$_3$)-CH(CH$_3$)-CH=CH$_2$, -CH$_2$-C(CH$_3$)$_2$-CH=CH$_2$, -C(CH$_3$)$_2$-CH$_2$-CH=CH$_2$, -CH$_2$-C(CH$_3$)=C(CH$_3$)$_2$, -CH(CH$_3$)-CH=C(CH$_3$)$_2$, -C(CH$_3$)$_2$-CH=CH-CH$_3$, -CH(CH$_3$)-C(CH$_3$)=CH-CH$_3$, -CH=C(CH$_3$)-CH(CH$_3$)$_2$, -C(CH$_3$)=CH-CH(CH$_3$)$_2$, -C(CH$_3$)=C(CH$_3$)-C$_2$H$_5$, -CH=CH-C(CH$_3$)$_3$, -C(CH$_3$)$_2$-C(CH$_3$)=CH$_2$, -CH(C$_2$H$_5$)-C(CH$_3$)=CH$_2$, -C(CH$_3$)(C$_2$H$_5$)-CH=CH$_2$, -CH(CH$_3$)-C(C$_2$H$_5$)=CH$_2$, -CH$_2$-C(C$_3$H$_7$)=CH$_2$, -CH$_2$-C(C$_2$H$_5$)=CH-CH$_3$, -CH(C$_2$H$_5$)-CH=CH-CH$_3$, -C(C$_4$H$_9$)=CH$_2$, -C(C$_3$H$_7$)=CH-CH$_3$, -C(C$_2$H$_5$)=CH-C$_2$H$_5$, -C(C$_2$H$_5$)=C(CH$_3$)$_2$, -C[CH(CH$_3$)(C$_2$H$_5$)]=CH$_2$, -C[CH$_2$-CH(CH$_3$)$_2$]=CH$_2$, -C$_3$H$_6$C≡C-CH$_3$, -CH(CH$_3$)-CH$_2$-C≡CH, -CH(CH$_3$)-C≡C-CH$_3$, -C$_2$H$_4$-CH(CH$_3$)-C≡CH, -CH$_2$-CH(CH$_3$)-CH$_2$-C≡CH, -CH$_2$-CH(CH$_3$)-C≡CH, -C≡CH, -C≡C-CH$_3$, -CH$_2$-C≡CH, -C$_2$H$_4$-C≡CH, -CH$_2$-C≡C-CH$_3$, -C≡C-C$_2$H$_5$, -C$_3$H$_6$-C≡CH, -C$_2$H$_4$-C≡C-CH$_3$, -CH$_2$-C≡C-C$_2$H$_5$, -C≡C-C$_3$H$_7$, -CH(CH$_3$)-C≡CH, -C$_4$H$_8$-C≡CH, -C$_2$H$_4$-C≡C-C$_2$H$_5$, -CH$_2$-C≡C-C$_3$H$_7$, -C≡C-C$_4$H$_9$, -C≡C-C(CH$_3$)$_3$, -CH(CH$_3$)-C$_2$H$_4$-C≡CH, -CH$_2$-CH(CH$_3$)-C≡C-CH$_3$, -CH(CH$_3$)-CH$_2$-C≡C-CH$_3$, -CH(CH$_3$)-C≡C-C$_2$H$_5$, -CH$_2$-C≡C-CH(CH$_3$)$_2$, -C≡C-CH(CH$_3$)-C$_2$H$_5$, -C≡C-CH$_2$-CH(CH$_3$)$_2$, -CH(C$_2$H$_5$)-C≡C-CH$_3$, -C(CH$_3$)$_2$-C≡C-CH$_3$, -CH(C$_2$H$_5$)-CH$_2$-C≡CH, -CH$_2$-CH(C$_2$H$_5$)-C≡CH, -C(CH$_3$)$_2$-CH$_2$-C≡CH, -CH$_2$-C(CH$_3$)$_2$-C≡CH, -CH(CH$_3$)-CH(CH$_3$)-C≡CH, -CH(C$_3$H$_7$)-C≡CH, -C(CH$_3$)(C$_2$H$_5$)-C≡CH, or -CH$_2$-CH(C≡CH)$_2$;

**$R^{15}$** represents **-$R^{20}$,** -CN, -CH$_2$-CN, -CH$_2$-OR$^{17}$, -CH$_2$-CH$_2$-OR$^{17}$, -CH$_2$-NR$^{17}$R$^{18}$, -CH$_2$-NR$^{17}$COR$^{19}$, -CH$_2$-CH$_2$-NR$^{17}$R$^{18}$, -CH$_2$-CH$_2$-NR$^{17}$COR$^{19}$, -CO$_2$R$^{17}$, -CO-NR$^{17}$R$^{18}$, -CH$_2$-CO$_2$R$^{17}$, or -CH$_2$-CO-NR$^{17}$R$^{18}$;

**$R^{16}$** represents **-$R^{21}$,** -H, -CH$_3$, -C$_2$H$_5$, -C$_3$H$_7$, -CH(CH$_3$)$_2$, -C$_4$H$_9$, -CH$_2$-CH(CH$_3$)$_2$, -CH(CH$_3$)-C$_2$H$_5$, -C(CH$_3$)$_3$, -C$_5$H$_{11}$, -CH(CH$_3$)-C$_3$H$_7$, -CH$_2$-CH(CH$_3$)-C$_2$H$_5$, -CH(CH$_3$)-CH(CH$_3$)$_2$, -C(CH$_3$)$_2$-C$_2$H$_5$, -CH$_2$-C(CH$_3$)$_3$, -CH(C$_2$H$_5$)$_2$, -C$_2$H$_4$-CH(CH$_3$)$_2$, -C$_6$H$_{13}$, -C$_7$H$_{15}$, -C$_8$H$_{17}$, -C$_3$H$_6$-CH(CH$_3$)$_2$, -C$_2$H$_4$-CH(CH$_3$)-C$_2$H$_5$, -CH(CH$_3$)-C$_4$H$_9$, -CH$_2$-CH(CH$_3$)-C$_3$H$_7$, -CH(CH$_3$)-CH$_2$-CH(CH$_3$)$_2$, -CH(CH$_3$)-CH(CH$_3$)-C$_2$H$_5$, -CH$_2$-CH(CH$_3$)-CH(CH$_3$)$_2$, -CH$_2$-C(CH$_3$)$_2$-C$_2$H$_5$, -C(CH$_3$)$_2$-C$_3$H$_7$, -C(CH$_3$)$_2$-CH(CH$_3$)$_2$, -C$_2$H$_4$-C(CH$_3$)$_3$, -CH(CH$_3$)-C(CH$_3$)$_3$, -CH$_2$OH, -CH$_2$-SH, -CH(OH)CH$_3$, -C$_2$H$_4$OH, -C$_3$H$_6$OH, -C$_4$H$_8$OH, -CH(CH$_3$)-C$_2$H$_4$OH, -C$_5$H$_{10}$OH, -CH$_2$-S-CH$_3$, -CH$_2$-CH$_2$-S-CH$_3$, -C$_3$H$_6$-S-CH$_3$, -CH$_2$OCH$_3$, -C$_2$H$_4$OCH$_3$, -C$_3$H$_6$OCH$_3$, -C$_4$H$_8$OCH$_3$, -CH(CH$_3$)-C$_2$H$_4$OCH$_3$, -C$_5$H$_{10}$OCH$_3$, -CH$_2$NH$_2$, -C$_2$H$_4$NH$_2$, -C$_3$H$_6$NH$_2$, -C$_4$H$_8$NH$_2$, -CH(CH$_3$)-C$_2$H$_4$NH$_2$, -C$_5$H$_{10}$NH$_2$, -CH$_2$-CH$_2$-CH$_2$-NH-C(NH)NH$_2$, -CH$_2$-CO$_2$H, -CH$_2$-CONH$_2$, -CH$_2$-CH$_2$-CO$_2$H, -CH$_2$-CH$_2$-CONH$_2$, -CH$_2$-CO$_2$CH$_3$, -CH$_2$-CONHCH$_3$, -CH$_2$-CON(CH$_3$)$_2$, -CH$_2$-CH$_2$-CO$_2$CH$_3$, -CH$_2$-CH$_2$-CONHCH$_3$, -CH$_2$-CH$_2$-CONH(CH$_3$)$_2$, -CH=CH-CO$_2$H, -CH=CH-CO$_2$CH$_3$, -CH=CH-CONHCH$_3$, -CH=CH-CONHC$_2$H$_5$, -CH=CH-CON(CH$_3$)$_2$, -CH=CH-CON(C$_2$H$_5$)$_2$, -CH$_2$-CH=CH-CO$_2$H, -CH$_2$-CH=CH-CO$_2$CH$_3$, -CH$_2$-CH=CH-CONHCH$_3$, -CH$_2$-CH=CH-CON(CH$_3$)$_2$, -CH$_2$-CH=CH-CONHC$_2$H$_5$, -CH$_2$-CH=CH-CON(C$_2$H$_5$)$_2$, -CH=CH$_2$, -CH$_2$-CH=CH$_2$, -C(CH$_3$)=CH$_2$, -CH=CH-CH$_3$, -C$_2$H$_4$-CH=CH$_2$, -CH$_2$-CH=CH-CH$_3$, -CH=CH-C$_2$H$_5$, -CH$_2$-C(CH$_3$)=CH$_2$, -CH(CH$_3$)-CH=CH, -CH=C(CH$_3$)$_2$, -C(CH$_3$)=CH-CH$_3$, -CH=CH-CH=CH$_2$, -C$_3$H$_6$-CH=CH$_2$, -C$_2$H$_4$-CH=CH-CH$_3$, -CH$_2$-CH=CH-C$_2$H$_5$, -CH=CH-C$_3$H$_7$, -CH$_2$-CH=CH-CH=CH$_2$, -CH=CH-CH=CH-CH$_3$, -CH=CH-CH$_2$-CH=CH$_2$, -C(CH$_3$)=CH-CH=CH$_2$, -CH=C(CH$_3$)-CH=CH$_2$, -CH=CH-C(CH$_3$)=CH$_2$, -C$_2$H$_4$-C(CH$_3$)=CH$_2$, -CH$_2$-CH(CH$_3$)-CH=CH$_2$, -CH(CH$_3$)-CH$_2$-CH=CH$_2$, -CH$_2$-CH=C(CH$_3$)$_2$, -CH$_2$-C(CH$_3$)=CH-CH$_3$, -CH(CH$_3$)-CH=CH-CH$_3$, -CH=CH-CH(CH$_3$)$_2$, -CH=C(CH$_3$)-C$_2$H$_5$, -C(CH$_3$)=CH-C$_2$H$_5$, -C(CH$_3$)=C(CH$_3$)$_2$, -C(CH$_3$)$_2$-CH=CH$_2$, -CH(CH$_3$)-C(CH$_3$)=CH$_2$, -C(CH$_3$)=CH-CH=CH$_2$, -CH=C(CH$_3$)-CH=CH$_2$, -CH=CH-C(CH$_3$)=CH$_2$, -C$_4$H$_8$-CH=CH$_2$, -C$_3$H$_6$-CH=CH-CH$_3$, -C$_2$H$_4$-CH=CH-C$_2$H$_5$, -CH$_2$-CH=CH-C$_3$H$_7$, -CH=CH-C$_4$H$_9$, -C$_3$H$_6$-C(CH$_3$)=CH$_2$, -C$_2$H$_4$-CH(CH$_3$)-CH=CH$_2$, -CH$_2$-CH(CH$_3$)-CH$_2$-CH=CH$_2$, -C$_2$H$_4$-CH=C(CH$_3$)$_2$, -CH(CH$_3$)-C$_2$H$_4$-CH=CH$_2$, -C$_2$H$_4$-C(CH$_3$)=CH-CH$_3$, -CH$_2$-CH(CH$_3$)-CH=CH-CH$_3$, -CH(CH$_3$)-CH$_2$-CH=CH-CH$_3$, -CH$_2$-CH=CH-CH(CH$_3$)$_2$, -CH$_2$-CH=C(CH$_3$)-C$_2$H$_5$, -CH$_2$-C(CH$_3$)=CH-C$_2$H$_5$, -CH(CH$_3$)-CH=CH-C$_2$H$_5$, -CH=CH-CH$_2$-CH(CH$_3$)$_2$, -CH=CH-CH(CH$_3$)-C$_2$H$_5$, -CH=C(CH$_3$)-C$_3$H$_7$, -C(CH$_3$)=CH-C$_3$H$_7$, -CH$_2$-CH(CH$_3$)-C(CH$_3$)=CH$_2$, -C[C(CH$_3$)$_3$]=CH$_2$, -CH(CH$_3$)-CH$_2$-C(CH$_3$)=CH$_2$, -CH(CH$_3$)-CH(CH$_3$)-CH=CH$_2$,- CH=CH-C$_2$H$_4$-CH=CH$_2$, -CH$_2$-C(CH$_3$)$_2$-CH=CH$_2$, -C(CH$_3$)$_2$-CH$_2$-CH=CH$_2$, -CH$_2$-C(CH$_3$)=C(CH$_3$)$_2$, -CH(CH$_3$)-CH=C(CH$_3$)$_2$, -C(CH$_3$)$_2$-CH=CH-CH$_3$, -CH=CH-CH$_2$-CH=CH-CH$_3$, -CH(CH$_3$)-C(CH$_3$)=CH-CH$_3$, -CH=C(CH$_3$)-CH(CH$_3$)$_2$, -C(CH$_3$)=CH-CH(CH$_3$)$_2$, -C(CH$_3$)=C(CH$_3$)-C$_2$H$_5$, -CH=CH-C(CH$_3$)$_3$, -C(CH$_3$)$_2$-C(CH$_3$)=CH$_2$, -CH(C$_2$H$_5$)-C(CH$_3$)=CH$_2$, -C(CH$_3$)(C$_2$H$_5$)-CH=CH$_2$, -CH(CH$_3$)-C(C$_2$H$_5$)=CH$_2$, -CH$_2$-C(C$_3$H$_7$)=CH$_2$,

-CH$_2$-C(C$_2$H$_5$)=CH-CH$_3$, -CH(C$_2$H$_5$)-CH=CH-CH$_3$, -C(C$_4$H$_9$)=CH$_2$, -C(C$_3$H$_7$)=CH-CH$_3$, -C(C$_2$H$_5$)=CH-C$_2$H$_5$, -C(C$_2$H$_5$)=C(CH$_3$)$_2$, -C[CH(CH$_3$)(C$_2$H$_5$)]=CH$_2$, -C[CH$_2$-CH(CH$_3$)$_2$]=CH$_2$, -C$_2$H$_4$-CH=CH-CH=CH$_2$, -CH$_2$-CH=CH-CH$_2$-CH=CH$_2$, -C$_3$H$_6$-C=C-CH$_3$, -CH$_2$-CH=CH-CH=CH-CH$_3$, -CH=CH-CH=CH-C$_2$H$_5$, -CH$_2$-CH=CH-C(CH$_3$)=CH$_2$, -CH$_2$-CH=C(CH$_3$)-CH=CH$_2$, -CH$_2$-C(CH$_3$)=CH-CH=CH$_2$, -CH(CH$_3$)-CH$_2$-C≡CH, -CH(CH$_3$)-CH=CH-CH=CH$_2$, -CH=CH-CH$_2$-C(CH$_3$)=CH$_2$, -CH(CH$_3$)-C≡C-CH$_3$, -CH=CH-CH(CH$_3$)-CH=CH$_2$, -CH=C(CH$_3$)-CH$_2$-CH=CH$_2$, -C$_2$H$_4$-CH(CH$_3$)-C≡CH, -C(CH$_3$)=CH-CH$_2$-CH=CH$_2$, -CH=CH-CH=C(CH$_3$)$_2$, -CH$_2$-CH(CH$_3$)-CH$_2$-C≡CH, -CH=CH-C(CH$_3$)=CH-CH$_3$, -CH=C(CH$_3$)-CH=CH-CH$_3$, -CH$_2$-CH(CH$_3$)-C≡CH, -C(CH$_3$)=CH-CH=CH-CH$_3$, -CH=C(CH$_3$)-C(CH$_3$)=CH$_2$, -C(CH$_3$)=CH-C(CH$_3$)=CH$_2$, -C(CH$_3$)=C(CH$_3$)-CH=CH$_2$, -CH=CH-CH=CH-CH=CH$_2$, -C≡CH, -C≡C-CH$_3$, -CH$_2$-C≡CH, -C$_2$H$_4$-C≡CH, -CH$_2$-C≡C-CH$_3$, -C≡C-C$_2$H$_5$, -C$_3$H$_6$-C≡CH, -C$_2$H$_4$-C≡C-CH$_3$, -CH$_2$-C≡C-C$_2$H$_5$, -C=C-C$_3$H$_7$, -CH(CH$_3$)-C≡CH, -C$_4$H$_8$-C≡CH, -C$_2$H$_4$-C≡C-C$_2$H$_5$, -CH$_2$-C≡C-C$_3$H$_7$, -C≡C-C$_4$H$_9$, -C≡C-C(CH$_3$)$_3$, -CH(CH$_3$)-C$_2$H$_4$-C≡CH, -CH$_2$-CH(CH$_3$)-C≡C-CH$_3$, -CH(CH$_3$)-CH$_2$-C≡C-CH$_3$, -CH(CH$_3$)-C≡C-C$_2$H$_5$, -CH$_2$-C≡C-CH(CH$_3$)$_2$, -C≡C-CH(CH$_3$)-C$_2$H$_5$, -C≡C-CH$_2$-CH(CH$_3$)$_2$, -CH(C$_2$H$_5$)-C≡C-CH$_3$, -C(CH$_3$)$_2$-C≡C-CH$_3$, -CH(C$_2$H$_5$)-CH$_2$-C≡CH, -CH$_2$-CH(C$_2$H$_5$)-C≡CH, -C(CH$_3$)$_2$-CH$_2$-C≡CH, -CH$_2$-C(CH$_3$)$_2$-C≡CH, -CH(CH$_3$)-CH(CH$_3$)-C≡CH, -CH(C$_3$H$_7$)-C≡CH, -C(CH$_3$)(C$_2$H$_5$)-C≡CH, -CH$_2$-Ph,

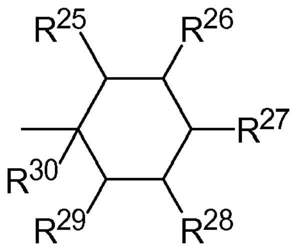
,
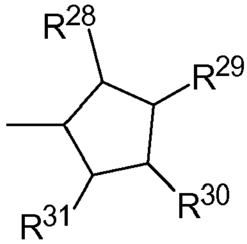
,
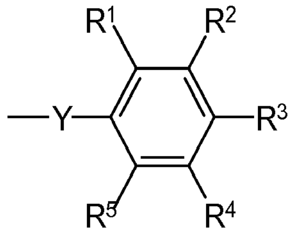
,

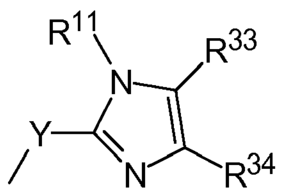
,
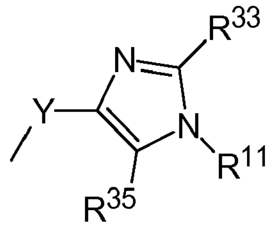
,
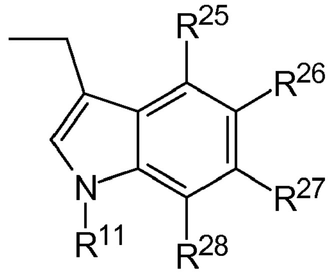
,

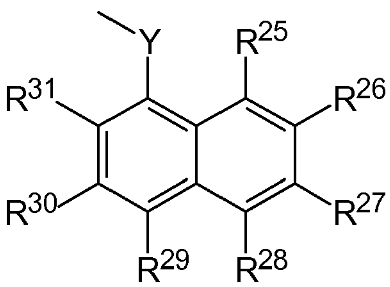
,
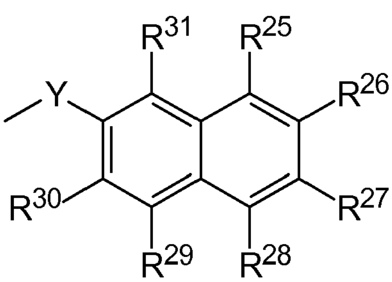
,

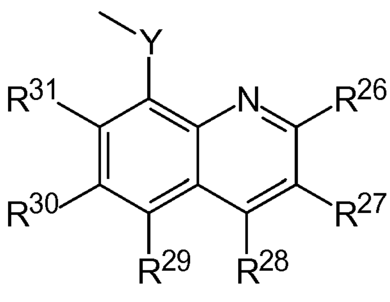
or
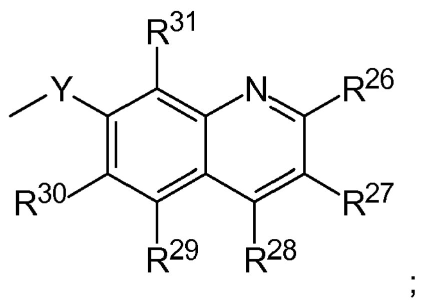
;

**$R^{11}$ - $R^{14}$** and **$R^{17}$ - $R^{21}$** represent independently of each other -H, $-CH_2F$, $-CHF_2$, $-CH_2-OCH_3$, $-CH_2-OH$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2-O-\text{cyclo}-C_3H_5$, $-C_2H_4-O-\text{cyclo}-C_3H_5$, $-C_3H_6-O-\text{cyclo}-C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, $-\text{cyclo}-C_3H_5$, $-\text{cyclo}-C_4H_7$, $-\text{cyclo}-C_5H_9$, $-\text{cyclo}-C_6H_{11}$, $-\text{cyclo}-C_7H_{13}$, $-\text{cyclo}-C_8H_{15}$, -Ph, $-CH_2-Ph$, $-CH_2-CH_2-Ph$, $-CH{=}CH-Ph$, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH{=}CH_2$, $-CH_2-CH{=}CH_2$, $-C(CH_3){=}CH_2$, $-CH{=}CH-CH_3$, $-C_2H_4-CH{=}CH_2$, $-CH_2-CH{=}CH-CH_3$, $-CH{=}CH-C_2H_5$, $-CH_2-C(CH_3){=}CH_2$, $-CH(CH_3)-CH{=}CH$, $-CH{=}C(CH_3)_2$, $-C(CH_3){=}CH-CH_3$, $-CH{=}CH-CH{=}CH_2$, $-C_3H_6-CH{=}CH_2$, $-C_2H_4-CH{=}CH-CH_3$, $-CH_2-CH{=}CH-C_2H_5$, $-CH{=}CH-C_3H_7$, $-CH_2-CH{=}CH-CH{=}CH_2$, $-CH{=}CH-CH{=}CH-CH_3$, $-CH{=}CH-CH_2-CH{=}CH_2$, $-C(CH_3){=}CH-CH{=}CH_2$, $-CH{=}C(CH_3)-CH{=}CH_2$, $-CH{=}CH-C(CH_3){=}CH_2$, $-C_2H_4-C(CH_3){=}CH_2$, $-CH_2-CH(CH_3)-CH{=}CH_2$, $-CH(CH_3)-CH_2-CH{=}CH_2$,- $CH_2-CH{=}C(CH_3)_2$, $-CH_2-C(CH_3){=}CH-CH_3$, $-CH(CH_3)-CH{=}CH-CH_3$, $-CH{=}CH-CH(CH_3)_2$, $-CH{=}C(CH_3)-C_2H_5$, $-C(CH_3){=}CH-C_2H_5$, $-C(CH_3){=}C(CH_3)_2$, $-C(CH_3)_2-CH{=}CH_2$, $-CH(CH_3)-C(CH_3){=}CH_2$, $-C(CH_3){=}CH-CH{=}CH_2$, $-CH{=}C(CH_3)-CH{=}CH_2$, $-CH{=}CH-C(CH_3){=}CH_2$, $-C_4H_8-CH{=}CH_2$, $-C_3H_6-CH{=}CH-CH_3$, $-C_2H_4-CH{=}CH-C_2H_5$, $-CH_2-CH{=}CH-C_3H_7$, $-CH{=}CH-C_4H_9$, $-C_3H_6-C(CH_3){=}CH_2$, $-C_2H_4-CH(CH_3)-CH{=}CH_2$, $-CH_2-CH(CH_3)-CH_2-CH{=}CH_2$, $-C_2H_4-CH{=}C(CH_3)_2$, $-CH(CH_3)-C_2H_4-CH{=}CH_2$, $-C_2H_4-C(CH_3){=}CH-CH_3$, $-CH_2-CH(CH_3)-CH{=}CH-CH_3$, $-CH(CH_3)-CH_2-CH{=}CH-CH_3$, $-CH_2-CH{=}CH-CH(CH_3)_2$, $-CH_2-CH{=}C(CH_3)-C_2H_5$, $-CH_2-C(CH_3){=}CH-C_2H_5$, $-CH(CH_3)-CH{=}CH-C_2H_5$, $-CH{=}CH-CH_2-CH(CH_3)_2$, $-CH{=}CH-CH(CH_3)-C_2H_5$, $-CH{=}C(CH_3)-C_3H_7$, $-C(CH_3){=}CH-C_3H_7$, $-CH_2-CH(CH_3)-C(CH_3){=}CH_2$, $-C[C(CH_3)_3]{=}CH_2$, $-CH(CH_3)-CH_2-C(CH_3){=}CH_2$, $-CH(CH_3)-CH(CH_3)-CH{=}CH_2$, $-CH_2-C(CH_3)_2-CH{=}CH_2$, $-C(CH_3)_2-CH_2-CH{=}CH_2$, $-CH_2-C(CH_3){=}C(CH_3)_2$, $-CH(CH_3)-CH{=}C(CH_3)_2$, $-C(CH_3)_2-CH{=}CH-CH_3$, $-CH(CH_3)-C(CH_3){=}CH-CH_3$, $-CH{=}C(CH_3)-CH(CH_3)_2$, $-C(CH_3){=}CH-CH(CH_3)_2$, $-C(CH_3){=}C(CH_3)-C_2H_5$, $-CH{=}CH-C(CH_3)_3$, $-C(CH_3)_2-C(CH_3){=}CH_2$, $-CH(C_2H_5)-C(CH_3){=}CH_2$, $-C(CH_3)(C_2H_5)-CH{=}CH_2$, $-CH(CH_3)-C(C_2H_5){=}CH_2$, $-CH_2-C(C_3H_7){=}CH_2$, $-CH_2-C(C_2H_5){=}CH-CH_3$, $-CH(C_2H_5)-CH{=}CH-CH_3$, $-C(C_4H_9){=}CH_2$, $-C(C_3H_7){=}CH-CH_3$, $-C(C_2H_5){=}CH-C_2H_5$, $-C(C_2H_5){=}C(CH_3)_2$, $-C[CH(CH_3)(C_2H_5)]{=}CH_2$, $-C[CH_2-CH(CH_3)_2]{=}CH_2$, $-C_3H_6C\equiv C-CH_3$, $-CH(CH_3)-CH_2-C\equiv CH$, $-CH(CH_3)-C\equiv C-CH_3$, $-C_2H_4-CH(CH_3)-C\equiv CH$, $-CH_2-CH(CH_3)-CH_2-C\equiv CH$, $-CH_2-CH(CH_3)-C\equiv CH$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C{=}C-C_3H_7$, $-CH(CH_3)-C\equiv CH$, $-C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, or $-CH_2-CH(C\equiv CH)_2$;

**$R^{22}$ - $R^{37}$** represent independently of each other -H, -OH, $-OCH_3$, $-OC_2H_5$, $-OC_3H_7$, $-O-\text{cyclo}-C_3H_5$, $-OCH(CH_3)_2$, $-OC(CH_3)_3$, $-OC_4H_9$, $-OCH_2-COOH$, -OPh, $-OCH_2-Ph$, $-OCPh_3$, $-CH_2-OH$, $-C_2H_4-OH$, $-C_3H_6-OH$, $-CH(OH)-CH_2-OH$, $-CH_2-OCH_3$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2-O-\text{cyclo}-C_3H_5$, $-C_2H_4-O-\text{cyclo}-C_3H_5$, $-C_3H_6-O-\text{cyclo}-C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$,- $CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, -SH, $-SCH_3$, $-SC_2H_5$, $-SC_3H_7$, $-S-\text{cyclo}-C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, $-CO-\text{cyclo}-C_3H_5$, $-COCH(CH_3)_2$, $-COC(CH_3)_3$, -COOH, -COCN, $-COOCH_3$, $-COOC_2H_5$, $-COOC_3H_7$, $-COO-\text{cyclo}-C_3H_5$, $-COOCH(CH_3)_2$, $-COOC(CH_3)_3$, $-OOC-CH_3$, $-OOC-C_2H_5$, $-OOC-C_3H_7$, $-OOC-\text{cyclo}-C_3H_5$, $-OOC-CH(CH_3)_2$, $-OOC-C(CH_3)_3$, $-CONH_2$, $-CH_2-CONH_2$, $-CONHCH_3$, $-CONHC_2H_5$, $-CONHC_3H_7$, $-CONH-\text{cyclo}-C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, $-CON(\text{cyclo}-C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, $-NHCO-\text{cyclo}-C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, $-NHCO-O-\text{cyclo}-C_3H_5$, $-NHCO-OCH(CH_3)_2$, $-NHCO-OC(CH_3)_3$, $-NH_2$, $-NHCH_3$, $-NHC_2H_5$, $-NHC_3H_7$, $-NH-\text{cyclo}-C_3H_5$, $-NHCH(CH_3)_2$, $-NHC(CH_3)_3$, $-N(CH_3)_2$, $-N(C_2H_5)_2$, $-N(C_3H_7)_2$, $-N(\text{cyclo}-C_3H_5)_2$, $-N[CH(CH_3)_2]_2$, $-N[C(CH_3)_3]_2$, $-SOCH_3$, $-SOC_2H_5$, $-SOC_3H_7$, $-SO-\text{cyclo}-C_3H_5$, $-SOCH(CH_3)_2$, $-SOC(CH_3)_3$, $-SO_2CH_3$, $-SO_2C_2H_5$, $-SO_2C_3H_7$, $-SO_2-\text{cyclo}-C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3-\text{cyclo}-C_3H_5$, $-SO_3CH(CH_3)_2$, $-SO_3C(CH_3)_3$, $-SO_2NH_2$, $-SO_2NHCH_3$, $-SO_2NHC_2H_5$, $-SO_2NHC_3H_7$, $-SO_2NH-\text{cyclo}-C_3H_5$, $-SO_2NHCH(CH_3)_2$, $-SO_2NHC(CH_3)_3$, $-SO_2N(CH_3)_2$, $-SO_2N(C_2H_5)_2$, $-SO_2N(C_3H_7)_2$, $-SO_2N(\text{cyclo}-C_3H_5)_2$, $-SO_2N[CH(CH_3)_2]_2$, $-SO_2N[C(CH_3)_3]_2$, $-O-S({=}O)CH_3$, -O-

$S(=O)C_2H_5$, $-O-S(=O)C_3H_7$, -O-S(=O)-cyclo-$C_3H_5$, $-O-S(=O)CH(CH_3)_2$, $-O-S(=O)C(CH_3)_3$, $-S(=O)(=NH)CH_3$, $-S(=O)(=NH)C_2H_5$, $-S(=O)(=NH)C_3H_7$, -S(=O)(=NH)-cyclo-$C_3H_5$, $-S(=O)(=NH)CH(CH_3)_2$, $-S(=O)(=NH)C(CH_3)_3$, $-NH-SO_2-CH_3$, $-NH-SO_2-C_2H_5$, $-NH-SO_2-C_3H_7$, $-NH-SO_2$-cyclo-$C_3H_5$, $-NH-SO_2-CH(CH_3)_2$, $-NH-SO_2-C(CH_3)_3$, $-O-SO_2-CH_3$, $-O-SO_2-C_2H_5$, $-O-SO_2-C_3H_7$, $-O-SO_2$-cyclo-$C_3H_5$, $-O-SO_2-CH(CH_3)_2$, $-O-SO_2-C(CH_3)_3$, $-OCF_3$, $-CH_2-OCF_3$, $-C_2H_4-OCF_3$, $-C_3H_6-OCF_3$, $-OC_2F_5$, $-CH_2-OC_2F_5$, $-C_2H_4-OC_2F_5$, $-C_3H_6-OC_2F_5$, $-O-COOCH_3$, $-O-COOC_2H_5$, $-O-COOC_3H_7$, -O-COO-cyclo-$C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CS-N(C_3H_7)_2$, $-NH-CO-NHC_3H_7$, $-NH-CO-N(C_3H_7)_2$,- $NH-CO-NH[CH(CH_3)_2]$, $-NH-CO-NH[C(CH_3)_3]$, $-NH-CO-N(CH_3)_2$, $-NH-CO-N(C_2H_5)_2$, -NH-CO-NH-cyclo-$C_3H_5$, -NH-CO-N(cyclo-$C_3H_5)_2$, $-NH-CO-N[CH(CH_3)_2]_2$, $-NH-CS-N(C_2H_5)_2$, $-NH-CO-N[C(CH_3)_3]_2$, $-NH-CS-NH_2$, $-NH-CS-NHCH_3$, $-NH-CS-N(CH_3)_2$, $-NH-CS-NHC_2H_5$, $-NH-CS-NHC_3H_7$, -NH-CS-NH-cyclo-$C_3H_5$, $-NH-CS-NH[CH(CH_3)_2]$, $-NH-CS-NH[C(CH_3)_3]$, -NH-CS-N(cyclo-$C_3H_5)_2$, $-NH-CS-N[CH(CH_3)_2]_2$, $-NH-CS-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, -O-CO-NH-cyclo-$C_3H_5$, -NH-C(=NH)-NH-cyclo-$C_3H_5$, $-NH-C(=NH)-NH[CH(CH_3)_2]$ $-O-CO-NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$, $-NH-C(=NH)-N(C_3H_7)_2$, -NH-C(=NH)-N(cyclo-$C_3H_5)_2$, $-O-CO-NHC_3H_7$, $-NH-C(=NH)-N[CH(CH_3)_2]_2$, $-NH-C(=NH)-N[C(CH_3)_3]_2$, $-O-CO-NH_2$, $-O-CO-NHCH_3$, $-O-CO-NHC_2H_5$, $-O-CO-NH[C(CH_3)_3]$, $-O-CO-N(CH_3)_2$, $-O-CO-N(C_2H_5)_2$, $-O-CO-N(C_3H_7)_2$, -O-CO-N(cyclo-$C_3H_5)_2$, $-O-CO-N[CH(CH_3)_2]_2$, $-O-CO-N[C(CH_3)_3]_2$, $-O-CO-OCH_3$, $-O-CO-OC_2H_5$, $-O-CO-OC_3H_7$, -O-CO-O-cyclo-$C_3H_5$, $-O-CO-OCH(CH_3)_2$, $-O-CO-OC(CH_3)_3$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, -cyclo-$C_5H_9$, -cyclo-$C_6H_{11}$, $-CH_2$-cyclo-$C_6H_{11}$, $-CH_2-CH_2$-cyclo-$C_6H_{11}$, -cyclo-$C_7H_{13}$, -cyclo-$C_8H_{15}$, -Ph, $-CH_2-Ph$, $-CH_2-CH_2-Ph$, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$,- $C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C\equiv CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C=C-C_3H_7$, $-CH(CH_3)-C\equiv CH$,- $C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-CH_2-CH(C\equiv CH)_2$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C(C\equiv CH)_2-CH_3$, $-C\equiv C-CH(CH_3)-C\equiv CH$, $-CH(CH_3)-C\equiv C-C\equiv CH$, $-CH(C\equiv CH)-CH_2-C\equiv CH$, $-CH(C\equiv CH)-C\equiv C-CH_3$,

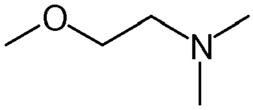 , 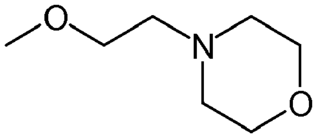 , 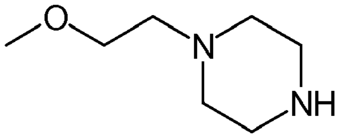 ,

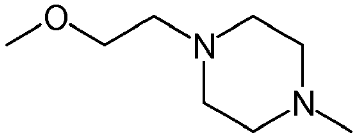 or 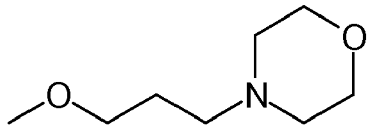 ,

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts;

with a privisio that the compound is not (1S,5S,6R)-10-(3,5-dichlorophenylsulfonyl)-3-(2-(3,4-dimethoxyphenoxy)ethyl)-5-vinyl-3,10-diazabicyclo[4.3.1]decan-2-one.

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

In regard to diazabicyclic compounds as FKBP51/52 ligands which have respectively 3,10-diazabicyclo[4.3.1]decan-2-one or 3,9-diazabicyclo[3.3.1]nonan-2-one as back bone structure, the compounds of the present invention are characterized by better affinity. These bicyclic back bones are useful for fixing the conformation of FKBP51/52 ligands. Thus, these bicyclic compounds have usually better affinity to FKBP51/52 than the monocyclic compounds which have piperidine as back bone structure. Further, it was found that the diazabicyclo[4.3.1]decan-2-one derivatives have higher affinity compared to the 3,9-diazabicyclo[3.3.1]nononan-2-one derivates, because the sulfonamide nitrogen of the diazabicyclo[4.3.1]decan-2-one derivatives can accept an unsual hydrogen bond from Tyr113 that mimics the putative FKBP transition state. However, in addition to these advantages, the compounds of the present invention are characterized by a high affintity and/or selectivity to FKBP 51 and FKBP 52.

Preferred substituents for $\mathbf{R^A}$ are

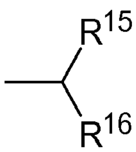 , 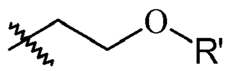 , 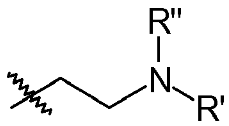 ,

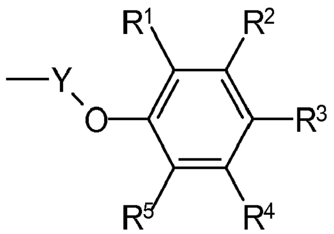 , 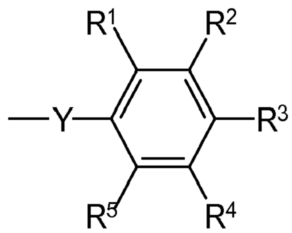 , 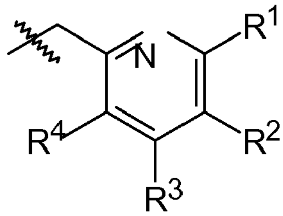 ,

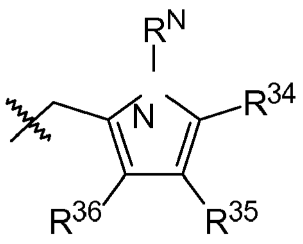,
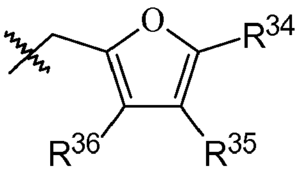,
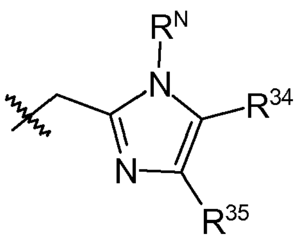 or

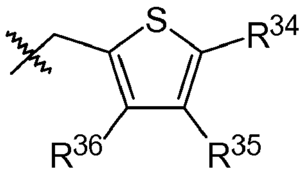.

wherein $R^1$- $R^5$, $R^{15}$- $R^{16}$, $R^{34}$- $R^{36}$, $R^N$ and Y have the meanings as defined herein and R' and R" represent independently of each other -H, $-CH_3$, $-C_2H_5$, $-CH(CH_3)_2$, $-CF_3$, $-COCH_3$.

Specifically preferred are compounds of the formula (I) having one of the following substituents $\mathbf{R^A}$:

–H, –Me, 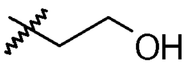, 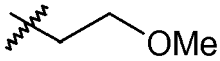, 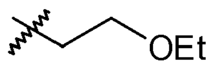,
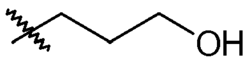, 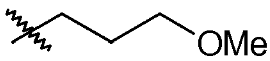, 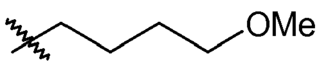,
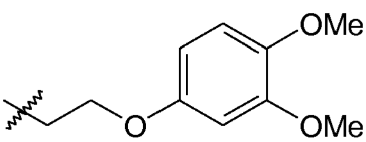 or 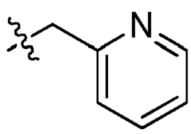.

In an alternative definition preferred are compounds of the formula (I) having a substituent $\mathbf{R^A}$ with a molecular weight of <200 g/mole, more preferable <100 g/mole, and most prefereable <50 g/mole.

Preferred substituents for $\mathbf{R^B}$ are:

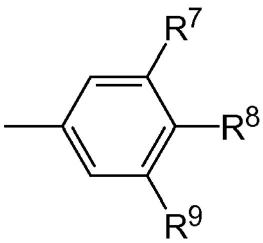, 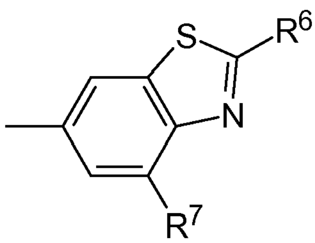 or 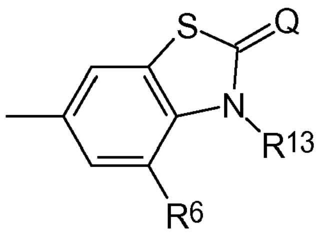.

wherein $R^6$- $R^9$, $R^{13}$ and Q have the meanings as defined herein.

Particularly preferred are compounds of the formula (I) having one of the following substituents $R^B$ :

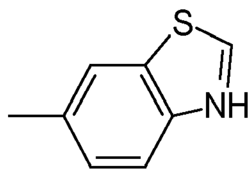,
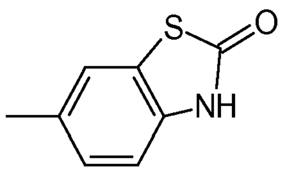,
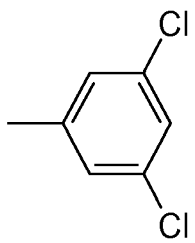,

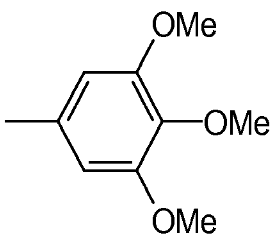

or

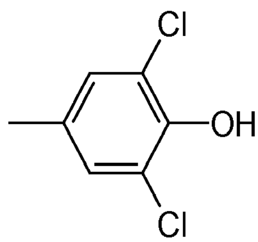.

Preferred substituents for $\mathbf{R^1}$ - $\mathbf{R^{10}}$ are: -H, -OH, $-OCH_3$, $-OC_2H_5$, $-OC_3H_7$, $-OCH(CH_3)_2$, $-OCPh_3$, $-CH_2-OCH_3$, $-CH_2-OH$, $-OC_3H_7$, $-OC(CH_3)_3$, $-OCH_2-COOH$, $-CH_3$, $-CH_2-OH$, $-C_2H_5$, $-C_3H_7$, $-CH(CH_3)_2$, -F, -Cl, -Br, -I;

In an alternative definition preferred are compounds of the formula (I) having a substituent $\mathbf{R^B}$ with a molecular weight of <300 g/mole, more preferable <200 g/mole, and most prefereable <130 g/mole.

Preferred are compounds of the formula (I) having one of the following substituents $\mathbf{R^C}$ **:** -OH, $-NH_2$, $-CH_2OH$, -CHO, $-CO_2H$, $-CONH_2$, $-COCH_3$, $-OCH_3$, $-OCH_2CH_3$, $-OCH(CH_3)_2$, $-OCH_2OCH_3$, $-OC_2H_4OCH_3$, $-CH_2OH$, $-CH_2OCH_3$, $-CH_2CH_2OH$, $-CH_2CHO$, $-CH_2CH_2CHO$, $-CH_2NH_2$, $-CH_2NHCH_3$, $-CH(OH)CH_3$, $-C(OH)(CH_3)_2$, $-CH_2CH(OH)CH_3$ or $-CH(OH)CH_2OH$.

In an alternative definition preferred are compounds of the formula (I) having a substituent $\mathbf{R^C}$ with a molecular weight of <200 g/mole, more preferable <100 g/mole, and most prefereable <50 g/mole.

In an alternative definition preferred are compounds of the formula (I) having substituents $\mathbf{R^A}$, $\mathbf{R^B}$ and $\mathbf{R^C}$ with a combined molecular weight of <400 g/mole, more preferable <300 g/mole, and most prefereable <250 g/mole.

Also preferred are compounds of the formula (II) and (III):

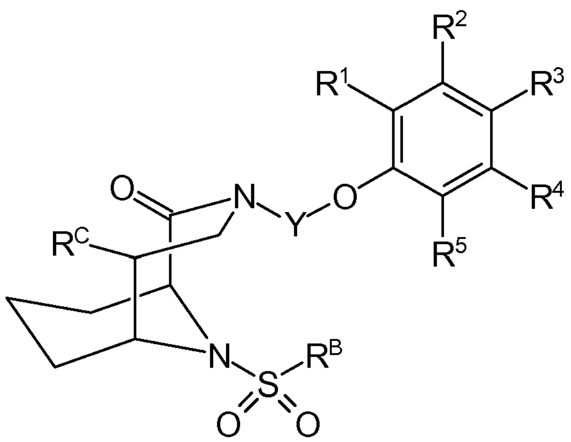

(II)

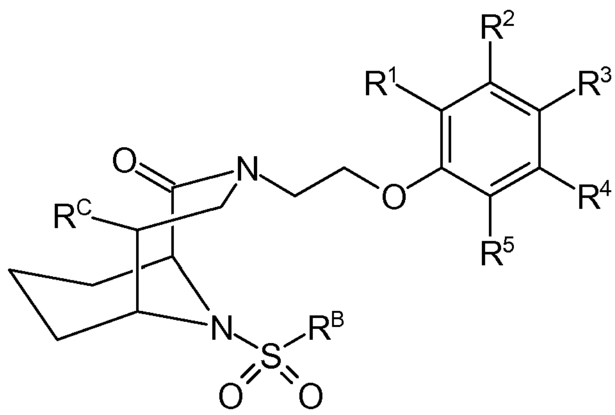

(III)

wherein Y, $R^B$, $R^C$ and the substituents $R^1$ - $R^5$ have the meanings as defined herein.

In these formulas (II) and (III) $\mathbf{R^B}$ represents preferably

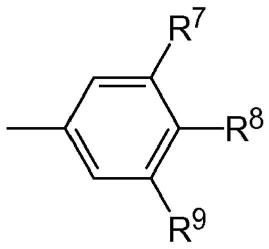 or 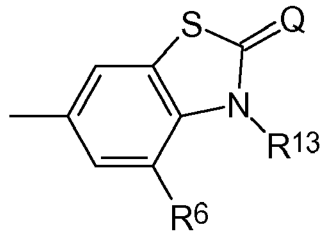 .

Further preferred are the general formula (IV), (V) and (VI):

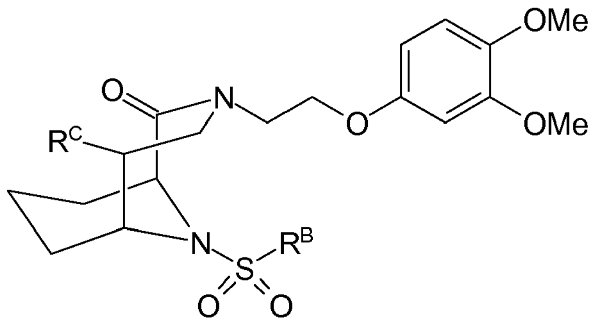

(IV)

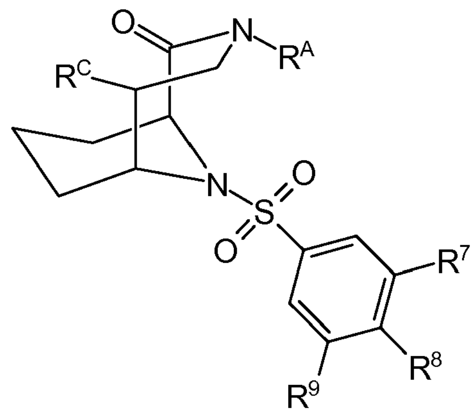

(V)

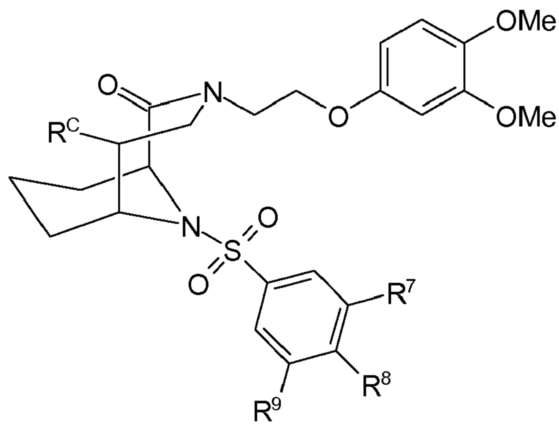

(VI)

wherein X and the substituents $\mathbf{R^B}$ and $\mathbf{R^7}$ - $\mathbf{R^9}$ have the meanings as defined herein.

Yet further preferred are the general formulas (VII) and (VIII):

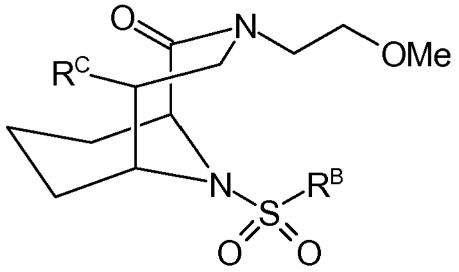

(VII)

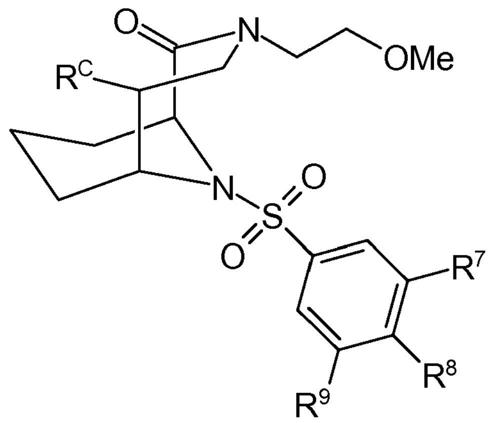

(VIII)

wherein the substituents $\mathbf{R^B}$, $\mathbf{R^C}$ and $\mathbf{R^7}$ - $\mathbf{R^9}$ have the meanings as defined herein.

Further preferred are compounds of general formula (IX):

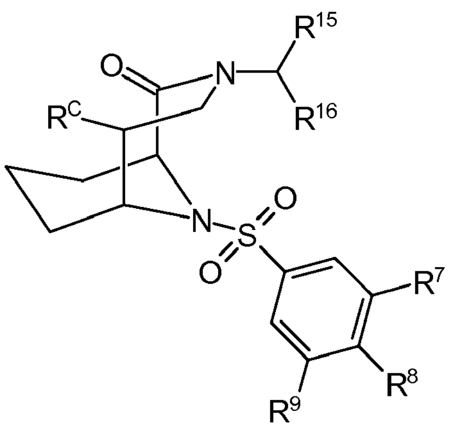

(IX)

wherein the substituents $R^C$, $R^7$ - $R^9$ and $R^{15}$ - $R^{16}$ have the meanings as defined herein.

Further preferred are compounds of general formula (X):

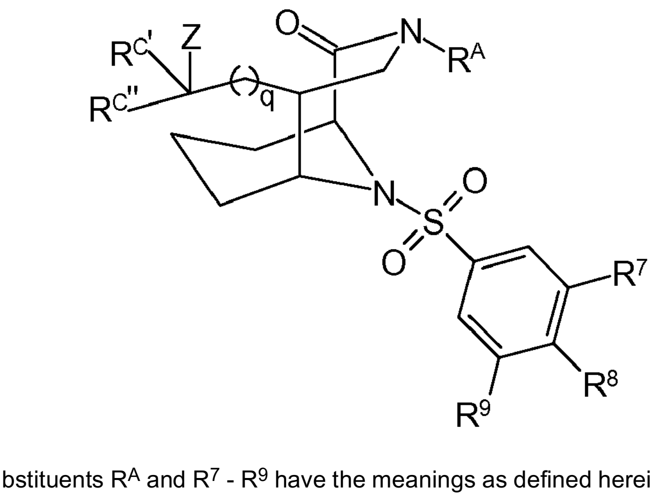

(X)

wherein the substituents $R^A$ and $R^7$ - $R^9$ have the meanings as defined herein; $R^{C'}$ and $R^{C''}$ represent independently of each other, -H, $-CH_3$, $-C_2H_5$, $-CH_2$-OH; Z represents -OH, $-OCH_3$, $-OC_2H_5$, $-OCH(CH_3)_2$, $-NH_2$, $-NH(CH_3)$, $-NH(C_2H_5)$, $-NHCH(CH_3)_2$, $-N(CH_3)_2$, or $-N(C_2H_5)_2$; and q is 0 or 1. Particularly preferred are compounds of formula (X), wherein

$\mathbf{R^A}$ represents -H, $-C_2H_4$-OH, $-C_2H_4-OCH_3$,

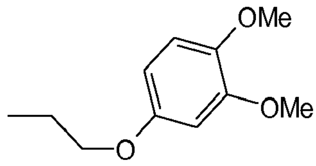

or

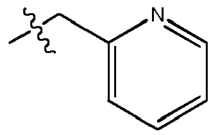

and/or wherein **$R^B$** represents

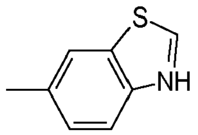

,

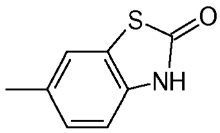

,

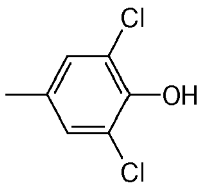

or

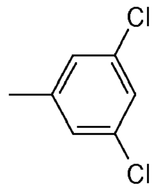

.

Most preferred are compounds of formula (XI),

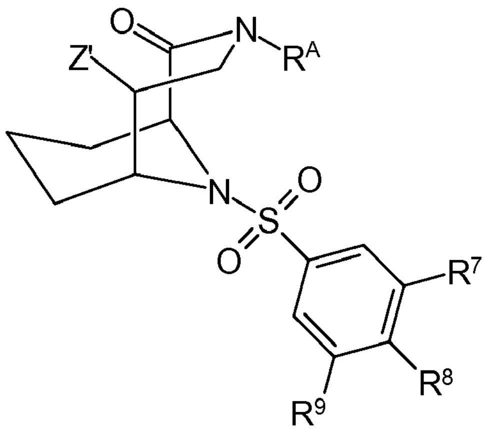

(XI)

wherein Z' represents -OH, $-OCH_3$, $-OC_2H_5$, $-OCH(CH_3)_2$, $-NH_2$, $-NH(CH_3)$, $-NH(C_2H_5)$, $-NHCH(CH_3)_2$, $-N(CH_3)_2$, or $-N(C_2H_5)_2$.

FKBP inhibitors or FKBP ligands as used herein are defined as compounds that (i) inhibit the peptidyl-prolyl isomerase activity of FKBPs (PPlase inhibitors, also referred to as rotamase inhibitors) or (ii) displace FK506 or FK506 analogs from the PPlase active site of FKBPs or (iii) bind to the FK506-binding domain of FKBPs as determined by isothermal calorimetry, surface plasmon resonance, tryptophan quenching, NMR or x-ray crystallography.

The expression prodrug is defined as a pharmacological substance, a drug, which is administered in an inactive or significantly less active form. Once administered, the prodrug is metabolized in the body *in vivo* into the active compound.

The expression tautomer is defined as an organic compound that is interconvertible by a chemical reaction called tautomerization. Tautomerization can be catalyzed preferably by bases or acids or other suitable compounds.

In yet another preferred embodiment of the present invention, the compound according to the general formula (I) is selected from the group comprising or consisting of:

| | |
|---|---|
| **B1** | (1*S*,5*S*,6*R*)-5-acetyl-10-((3,5-dichlorophenyl)sulfonyl)-3-(2-methoxyethyl)-3,10-diazabicyclo[4.3.1]decan-2-one |
| **B2** | 2-((1*S*,5*S*,6*R*)-10-((3,5-dichlorophenyl)sulfonyl)-3-(2-methoxyethyl)-2-oxo-3,10-diazabicyclo[4.3.1]decan-5-yl)acetaldehyde |
| **B3** | (1*S*,5*S*,6*R*)-10-((3,5-dichlorophenyl)sulfonyl)-5-(2-hydroxypropan-2-yl)-3-(2-methoxyethyl)-3,10-diazabicyclo[4.3.1]decan-2-one |
| **B6** | (1*S*,5*R*,6*R*)-10-((3,5-dichlorophenyl)sulfonyl)-5-((R)-1-hydroxyethyl)-3-(2-methoxyethyl)-3,10-diazabicyclo[4.3.1]decan-2-one |

(continued)

| | |
|---|---|
| **B7** | (1*S*,5*R*,6*R*)-10-((3,5-dichlorophenyl)sulfonyl)-5-((S)-1-hydroxyethyl)-3-(2-methoxyethyl)-3,10-diazabicyclo[4.3.1]decan-2-one |
| **B8** | (1*S*,5*S*,6*R*)-10-((3,5-dichlorophenyl)sulfonyl)-5-(2-hydroxyethyl)-3-(2-methoxyethyl)-3,10-diazabicyclo[4.3.1]decan-2-one |
| **B9** | (1*S*,5*S*,6*R*)-10-((3,5-dichlorophenyl)sulfonyl)-3-(2-methoxyethyl)-2-oxo-3,10-diazabicyclo[4.3.1]decane-5-carbaldehyde |
| **B10** | (1S,5S,6R)-10-((3,5-dichlorophenyl)sulfonyl)-3-(2-methoxyethyl)-2-oxo-3,10-diazabicyclo[4.3.1]decane-5-carboxylic acid |
| **B11** | (1*S*,5*S*,6*R*)-10-((3,5-dichlorophenyl)sulfonyl)-3-(2-methoxyethyl)-2-oxo-3,10-diazabicyclo[4.3.1]decane-5-carboxamide |
| **B12** | (1*S*,5*S*,6*R*)-10-((3,5-dichlorophenyl)sulfonyl)-3-(2-methoxyethyl)-5-((methylamino)methyl)-3,10-diazabicyclo[4.3.1]decan-2-one |
| **B13** | (1*S*,5*R*,6*R*)-10-((3,5-dichlorophenyl)sulfonyl)-5-(hydroxymethyl)-3-(2-methoxyethyl)-3,10-diazabicyclo[4.3.1]decan-2-one |
| **B14** | (1*S*,5*R*,6*R*)-10-((3,5-dichlorophenyl)sulfonyl)-3-(2-methoxyethyl)-5-(methoxymethyl)-3,10-diazabicyclo[4.3.1]decan-2-one |
| **B15** | (1S,5S,6R)-10-((3,5-dichlorophenyl)sulfonyl)-5-ethyl-3-(2-methoxyethyl)-3,10-diazabicyclo[4.3.1]decan-2-one |
| **C1** | (1*S*,5*R*,6*R*)-1 0-((3,5-dichlorophenyl)sulfonyl)-3-(2-hydroxyethyl)-5-(hydroxymethyl)-3,10-diazabicyclo[4.3.1]decan-2-one |
| **C2** | (1*S*,5*S*,6*R*)-10-((3,5-dichlorophenyl)sulfonyl)-5-(1,2-dihydroxyethyl)-3-(2-(3,4-dimethoxyphenoxy)ethyl)-3,10-diazabicyclo[4.3.1]decan-2-one |
| **C3** | (1*S*,5*S*,6*R*)-10-((3,5-dichlorophenyl)sulfonyl)-5-(1,2-dihydroxyethyl)-3-(2-ethoxyethyl)-3,10-diazabicyclo[4.3.1]decan-2-one |
| **D1** | (1S,5S,6R)-5-acetyl-10-((3,5-dichlorophenyl)sulfonyl)-3-(2-(3,4-dimethoxyphenoxy)ethyl)-3,10-diazabicyclo[4.3.1]decan-2-one |
| **D2** | 2-((1S,5S,6R)-10-((3,5-dichlorophenyl)sulfonyl)-3-(2-(3,4-dimethoxyphenoxy)ethyl)-2-oxo-3,10-diazabicyclo[4.3.1]decan-5-yl)acetaldehyde |
| **D3** | (1*S*,5*S*,6*R*)-10-((3,5-dichlorophenyl)sulfonyl)-3-(2-(3,4-dimethoxyphenoxy)ethyl)-5-(2-hydroxypropan-2-yl)-3,10-diazabicyclo[4.3.1]decan-2-one |
| **D4** | (1*S*,5*S*,6*R*)-10-((3,5-dichlorophenyl)sulfonyl)-3-(2-(3,4-dimethoxyphenoxy)ethyl)-5-(2-hydroxypropyl)-3,10-diazabicyclo[4.3.1]decan-2-one |
| **D5** | (1*S*,5*S*,6*R*)-10-((3,5-dichlorophenyl)sulfonyl)-3-(2-(3,4-dimethoxyphenoxy)ethyl)-5-(2-hydroxypropyl)-3,10-diazabicyclo[4.3.1]decan-2-one |
| **D6** | (1*S*,5*S*,6*R*)-10-((3,5-dichlorophenyl)sulfonyl)-3-(2-(3,4-dimethoxyphenoxy)ethyl)-5-((R)-1-hydroxyethyl)-3,10-diazabicyclo[4.3.1]decan-2-one |
| **D7** | (1*S*,5*S*,6*R*)-10-((3,5-dichlorophenyl)sulfonyl)-3-(2-(3,4-dimethoxyphenoxy)ethyl)-5-((R)-1-hydroxyethyl)-3,10-diazabicyclo[4.3.1]decan-2-one |
| **D8** | (1*S*,5*S*,6*R*)-10-((3,5-dichlorophenyl)sulfonyl)-3-(2-(3,4-dimethoxyphenoxy)ethyl)-5-(2-hydroxyethyl)-3,10-diazabicyclo[4.3.1]decan-2-one |
| **G6** | (1S,5S,6R)-10-((3,5-dichlorophenyl)sulfonyl)-5-hydroxy-3,10diazabicyclo-[4.3.1]decan-2-one |
| **H1.2** | (1S,5R,6R)-5-amino-10-((3,5-dichlorophenyl)sulfonyl)-3,10-diazabicyclo-[4.3.1]decan-2-one |
| **H1.3** | (1S,5S,6R)-10-((3,5-dichlorophenyl)sulfonyl)-5-methoxy-3-methyl-3,10-diazabicyclo[4.3.1]decan-2-one |
| **H1.4** | (1S,5R,6R)-10-((3,5-dichlorophenyl)sulfonyl)-5-hydroxy-3,10-diazabicyclo-[4.3.1]decan-2-one |

(continued)

| | |
|---|---|
| **H2.1** | (1S,5S,6R)-10-((3,5-dichlorophenyl)sulfonyl)-5-(2-methoxyethoxy)-3-(2-methoxy-ethyl)-3,10-diazabicyclo[4.3.1]decan-2-one |
| **H2.2** | (1S,5S,6R)-10-((3,5-dichlorophenyl)sulfonyl)-5-(methoxymethoxy)-3,10-diaza-bicyclo[4.3.1]decan-2-one |
| **H2.3** | (1S,5S,6R)-10-((3,5-dichlorophenyl)sulfonyl)-3-(2-(3,4-dimethoxyphenoxy)ethyl)-5-hydroxy-3,10-diazabicyclo[4.3.1]decan-2-one |
| **J2** | (1S,5R,6R)-10-((3,5-dichlorophenyl)sulfonyl)-3-(3-methoxypropyl)-5-vinyl-3,10-diazabicyclo[4.3.1]decan-2-one |
| **J3** | (1S,5R,6R)-10-((3,5-dichlorophenyl)sulfonyl)-3-(4-methoxybutyl)-5-vinyl-3,10-diazabicyclo[4.3.1]decan-2-one |
| **J4** | (1S,5R,6R)-10-((3,5-dichlorophenyl)sulfonyl)-3-(pyridin-2-ylmethyl)-5-vinyl-3,10-diazabicyclo[4.3.1]decan-2-one |
| **J5** | (1S,5R,6R)-10-((3,5-dichlorophenyl)sulfonyl)-5-(hydroxymethyl)-3-(3-hydroxypropyl)-3,10-diazabicyclo[4.3.1]decan-2-one |
| **J6** | (1S,5R,6R)-10-((3,5-dichlorophenyl)sulfonyl)-5-(hydroxymethyl)-3-(3-methoxypropyl)-3,10-diazabicyclo[4.3.1]decan-2-one |
| **J7** | (1S,5R,6R)-10-((3,5-dichlorophenyl)sulfonyl)-5-(hydroxymethyl)-3-(4-methoxybutyl)-3,10-diazabicyclo[4.3.1]decan-2-one |
| **J8** | (1S,5R,6R)-10-((3,5-dichlorophenyl)sulfonyl)-5-(hydroxymethyl)-3-(pyridin-2-ylmethyl)-3,10-diazabicyclo[4.3.1]decan-2-one |

**Synthetic Methods**

The compounds of the general formula (I) can be prepared according to the following synthetic route in scheme 1. Accordingly, intermediate compound **(I-A1)** can be prepared by providing 6-carboxy-2-piperidone and a precursor molecule for the moiety $R^A$ which has a suitable leaving group (LG) such as trimethylsilyl (TMS) and a carbon-carbon double bond in allyl position to the $R^A$ amino group. Said $R^A$ amino group is reacted with the carboxy moiety of 6-carboxy-2-piperidone. Subsequently, this compound undergoes a cyclization reaction upon which the leaving group LG is detached from the starting molecule. After deprotecting $PG_1$ from amine, 5-vinyl-3,10-diazabicyclo[4.3.1]decane-2-one derivative **(I-B1)** is formed. This intermediate can subsequently be reacted with a suitable precursor for the moiety $-SO_2-R^B$. By suitable transformation reactions of vinyl group at C-5 position of the resulting sulphonamide compound **(I-C),** the compound of the general formula (I) can be obtained. Preferred, the vinyl group could be transformed by oxidation reaction with oxygen gas or epoxidation reaction.

## Scheme 1

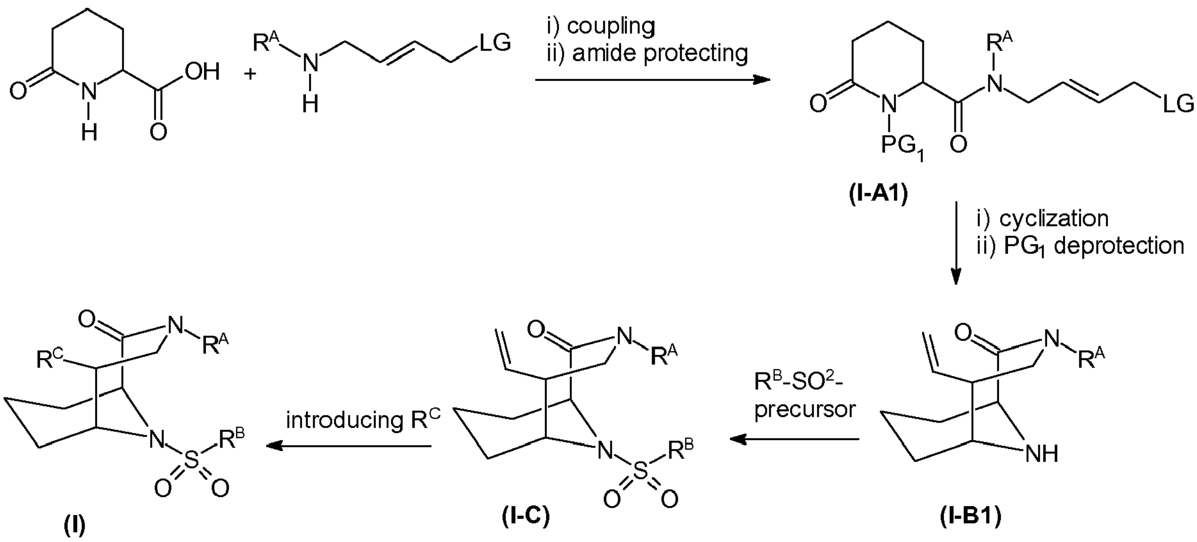


In one embodiment of the present invention, a method for preparation of compound of the general formula (I) by synthetic route 1 comprises the following steps:

(a) providing an intermediate compound of the formula **(I-A1)**

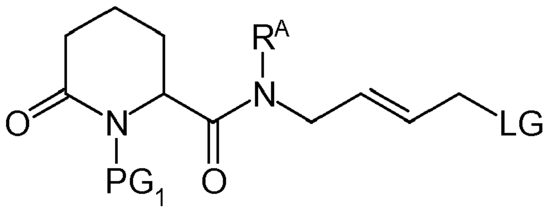

**(I-A1)**

wherein $R^A$ has the meanings as defined herein, LG refers to a leaving group, and $PG_1$ refers to a protecting group for amine;

(b) performing a cyclization reaction and deprotecting $PG_1$ to yield compound of the formula **(I-B1)**

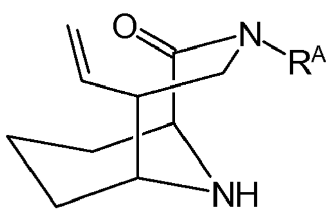

**(I-B1)**

(c) introducing the moiety $-SO_2-R^B$ to yield compound of the formula **(I-C)**

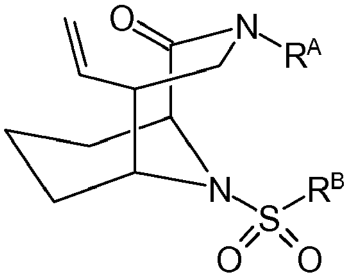

(I-C)

(d) transforming the vinyl group of compound **(I-C)** into the substituent $R^C$ to yield compound of the formula (I)

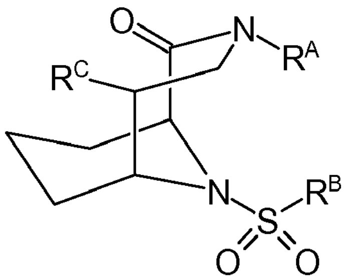

(I)

wherein $R^A$, $R^B$ and $R^C$ have the meanings as defined herein.

As synthetic route 2, intermediate compound **(I-A2)** can be prepared by providing 6-carboxy-2-piperidone and a butenyl amine protected with a protecting group $PG_6$ which has a suitable leaving group (LG) such as trimethylsilyl (TMS) and a carbon-carbon double bond in allyl position to the amino group. Said protected amino group is reacted with the carboxy moiety of 6-carboxy-2-piperidone. Subsequently, this compound undergoes a cyclization reaction upon which the leaving group LG is detached from the starting molecule. After deprotecting $PG_1$ from amide, 5-vinyl-3,10-diazabicyclo[4.3.1]decane-2-one derivative **(I-B2)** is formed. A sulphonamide intermediate **(I-B3)** can be obtained by subsequently reacting **(I-B2)** with a suitable precursor for the moiety $-SO_2R^B$. By deprotecting $PG_6$ from amide group of the intermediate **(I-B3)** and subsequently reacting with a suitable precursor for the moiety $R^{A,}$ a sulphonamide compound **(I-C)** can be produced. By suitable transformation reactions of vinyl group at C-5 position of the resulting sulphonamide compound **(I-C)**, the compound of the general formula (I) can be obtained. Preferred, the vinyl group could be transformed by oxidation reaction with oxygen gas or ozone, epoxidation reaction or dihydroxylation catalyzed by osmium (VIII) oxide.

## Scheme 2

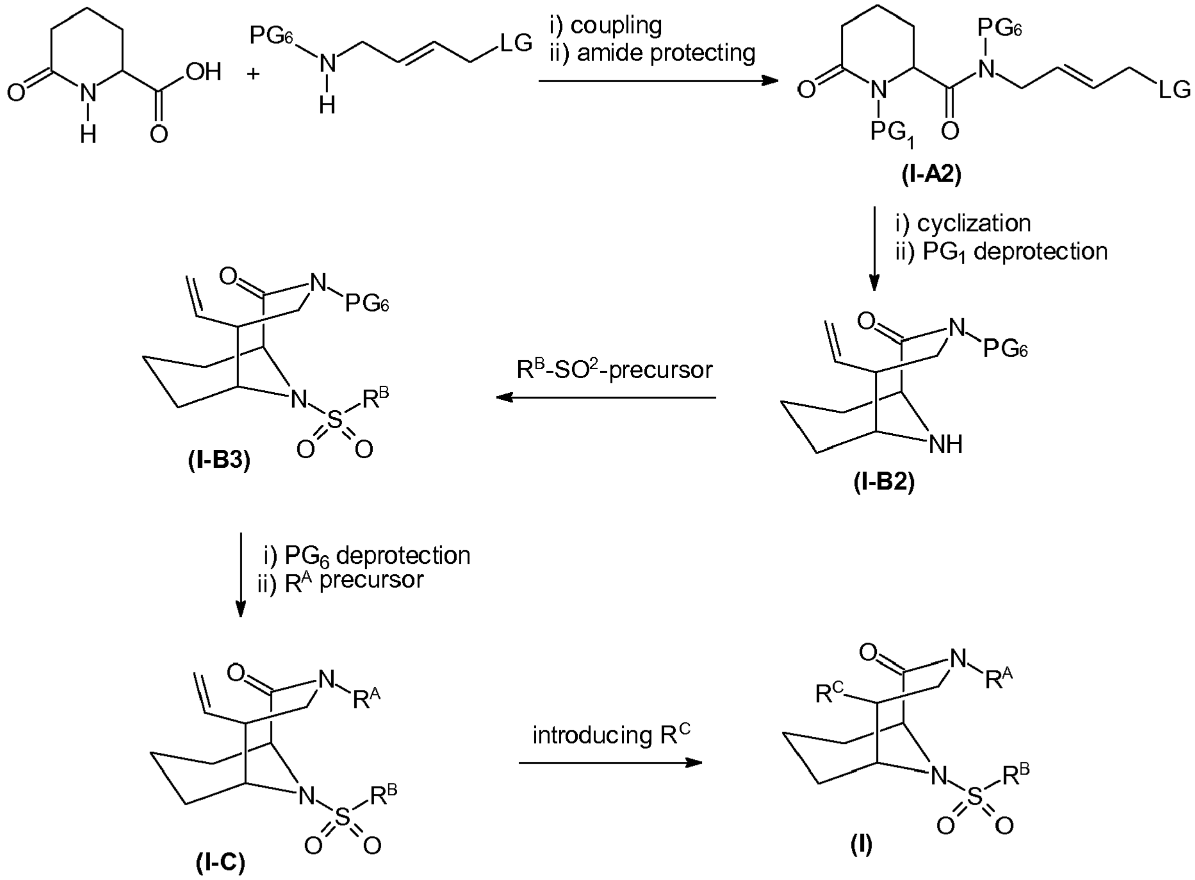


In another embodiment of the present invention, a method for preparation of compound of the general formula (I) by synthetic route 2 comprises the following steps:

(a) providing an intermediate compound of the formula **(I-A2)**

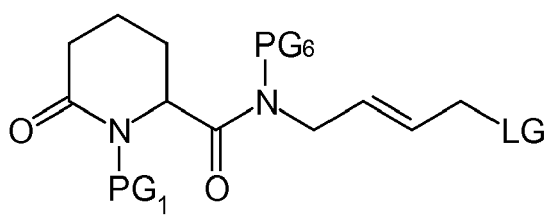

**(I-A2)**

wherein $R^A$ has the meanings as defined herein, LG refers to a leaving group, and $PG_1$ and $PG_6$ refer to protecting groups for amines;

(b) performing a cyclization reaction and deprotecting $PG_1$ to yield compound of the formula **(I-B1)**

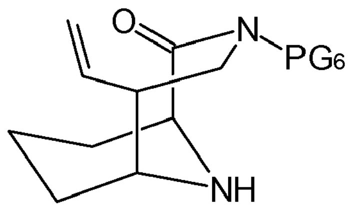

**(I-B2)**

(c) introducing the moiety $-SO_2-R^B$ to yield compound of the formula **(I-B3)**

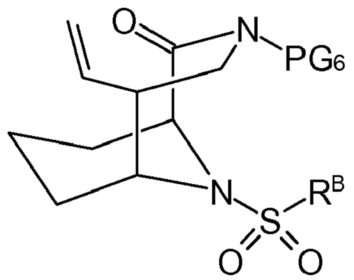

**(I-B3)**

wherein $R^B$ has the meanings as defined herein;

(d) deprotecing $PG_6$ and introducing the moiety $R^A$ to yield compound of the formula **(I-C)**

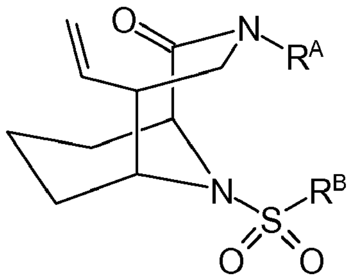

**(I-C)**

(e) transforming the vinyl group of compound **(I-C)** into the substituent $R^C$ to yield compound of the formula (I)

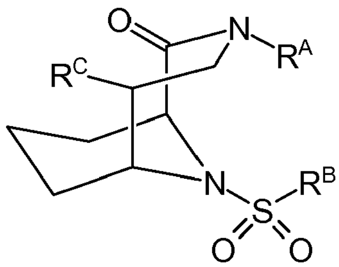

**(I)**

wherein $R^A$, $R^B$ and $R^C$ have the meanings as defined herein.

## Scheme 3

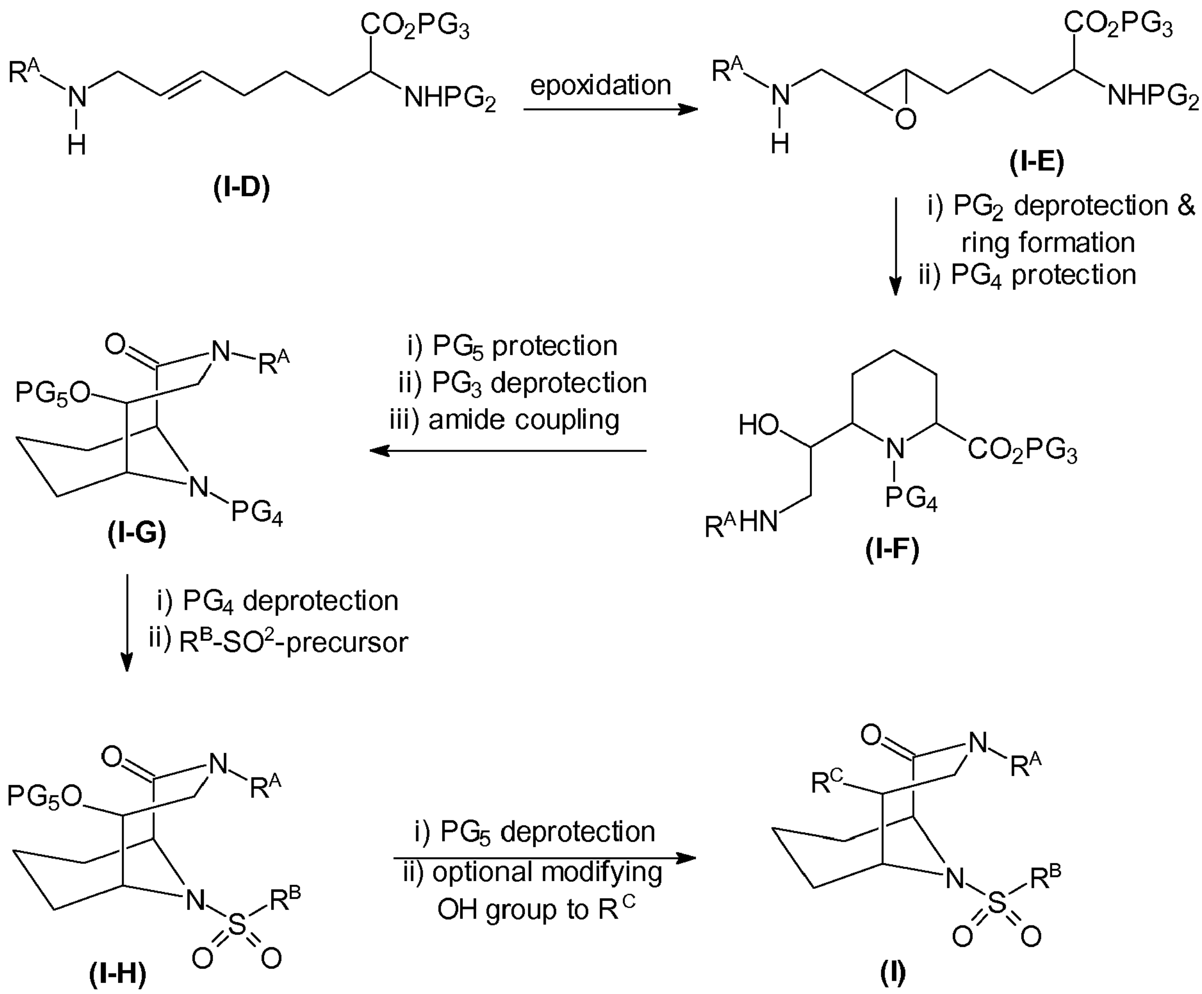


(I-D) (I-E) (I-F) (I-G) (I-H) (I)

As alternative synthetic route 3, 1-amino-hex-2-en-yl substituted amino acid derivate **(I-D)** can be used as starting material (scheme 2). Amino group of hexenyl substituent is further substituted with $R^A$. Amino group and carboxy group of said amino acid are also protected respectively with suitable protecting groups $PG_2$ and $PG_3$. After epoxidation of a double bond of starting material, piperidine intermediate **(I-F)** is formed by deprotecting $PG_2$ and spontaneous ring formation reaction. An amino group of piperidine ring and a hydroxyl group derived from epoxide ring are protected respectively with protecting groups $PG_4$ and $PG_5$. After deprotecting of carboxylic protecting group $PG_3$, 3,10-diazabicyclo[4.3.1]dencane-2-one derivative **(I-G)** is formed by an intramolecular amide coupling reaction between free carboxylic acid and $\mathbf{R^A}$ substituted amine.

After deprotecting $PG_4$, the $SO_2$-$R^B$ precursor reacts with the amino group of the above shown intermediate product **(I-G)** in order o introduce the residue -$SO_2$-$R^B$ by covalent chemical bonding into the intermediate product to obtain the intermediate compound **(I-H).** The term $SO_2$-$R^B$ precursor has the same meaning as defined in synthetic route 1. The inventive compound is prepared by deprotecting $PG_5$ or further transformation of hydroxy group obtained by deprotecing $PG_5$. Preferred, the Hydroxy group can be transformed into keto, ether, ester or amine functional group.

Thus, in another embodiment of the present invention, a method for preparation of compound of the general formula (I) by alternative synthetic route 2 comprises the following steps:

(a) providing an intermediate compound of the formula **(I-E)**

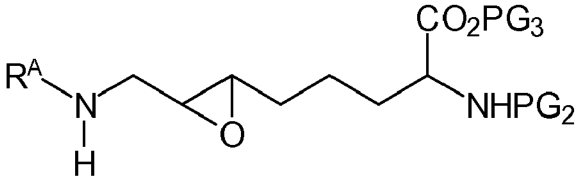

(I-E)

wherein $R^A$ has the meanings as defined herein, $PG_2$ refers to a protecting group for amine, and $PG_3$ refers to a protecting group for carboxylic acid;

(b) deprotecting $PG_2$, performing a piperindine ring formation reaction and introducing $PG_4$ to yield compound of the formula **(I-F)**

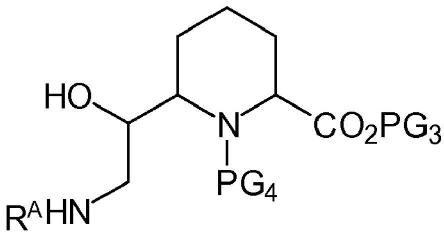

(I-F)

wherein $R^A$ has the meanings as defined herein, $PG_3$ refers to a protecting group for carboxylic acid , and $PG_4$ refers to a protecting group for amine;

(c) introducing $PG_5$, deprotecting $PG_3$ and performing a amide coupling reaction to yield compound of the formula **(I-G)**

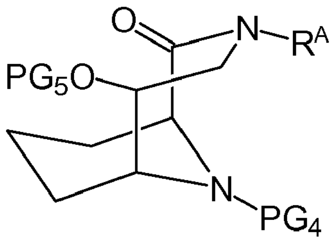

(I-G)

wherein $R^A$ has the meanings as defined herein, $PG_4$ refers to a protecting group for amine, and $PG_5$ refers to a protecting group for hydroxyl group;

(d) deprotecting $PG_4$ and introducing the moiety $-SO_2-R^B$ to yield compound of the formula **(I-H)**

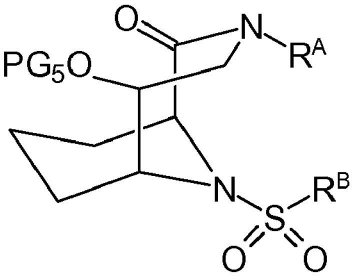

(I-H)

wherein $R^A$ and $R^B$ have the meanings as defined herein, $PG_5$ refers to a protecting group for hydroxyl group;

(e) transforming a hydroxyl group obtained by deprotecting $PG_5$ to yield compound of the formula (I)

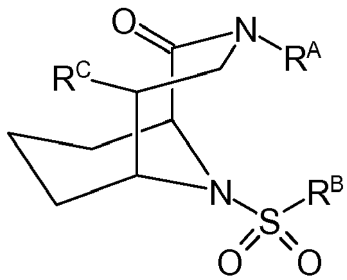

(I)

wherein $R^A$, $R^B$ and $R^C$ have the meanings as defined herein.

Suitable protecting groups $PG_1$ - $PG_4$, and $PG_6$, for amines are carbobenzyloxy (Cbz), p-methoxybenzyl carbonyl (Moz), tert-butyloxycarbonyl (Boc), 9-fluorenylmethyloxycarbonyl (Fmoc), allyloxycarbonyl (Alloc), 2,2,2-trichlorethoxycarbonyl (Troc), trifluoroacetyl, 2-(trimethylsilyl)ethoxycarbonyl (Teoc) p-methoxy-benzyl (PMB) and phtalimide.
Suitable protecting group $PG_3$ for carboxylic acid is methyl, ethyl, isopropyl, tert-butyl (tBu), allyl, and benzyl (Bn).
Suitable protecting group $PG_5$ for hydroxyl group is trimethylsilyl (TMS), triethylsilyl (TES), tert-butyldimethylsilyl (TBDMS), tert-butyldiphenylsilyl (TBDPS), allyl, benzyl, tert-butyl, methoxylmethyl (MOM), methoxyethyl (MEM), tetrahydropyranyl (THP), acetyl (Ac), benzoyl (Bz) and pivalic (Piv).

**Influence of substitutent $R^C$ of the general formula (I) to Affinity to FK506 domain of FKBP51/52**

The compounds of this invention can be distinguished from the closest prior art by having the additional substituent $R^C$.
In the present invention, the influence of substituent $\mathbf{R^C}$ at C-5 position of 3,10-diazabicyclo[4.3.1]decan-2-one derivative is proved. In the present invention, *in vitro* fluorescence polarization assays with the compounds of the general formula (I) were performed to determine the binding affinities to FKBP51 and FKBP52 or to the isolated FK506-binding domains of FKBP51 and FKBP52 (Example 8). Binding to the isolated FK506-binding domains is reliably indicative of binding to the full length proteins. The affinities for the isolated FK506-binding domains and for full-length FKBP51 or FKBP52 are the same for all compounds tested so far (Kozany et al, ChemBioChem 2009, 10, 1402-1410).
Surprisingly, it was found by these assays that substitutent $\mathbf{R^C}$ of the compounds of the general formula (I) consistently increases affintity to FKBP51 and/or FKBP 52. In Table 1 the binding affinity data of exemplary compounds of the general formula (I) are summarized.
The superiority of the compounds of this invention over the closest prior art is best demonstrated by the direct comparison between **Ref. 1** and **C2, D3-D8** and **H2.3.** These compounds share the exact same scaffold and differ only in the $\mathbf{R^C}$ position, where **Ref. 1** has a simple hydrogen while all inventive compounds have larger substituents. These additional $\mathbf{R^C}$ substituentes impart a gain in affinity for FKBP51 and FKBP52 by at least 20 fold.
Molecular modelling suggests that the increase in affinity can be caused by additional Van-der-Waals contacts of the inventive $\mathbf{R^C}$ substituent with Tyr57, Asp68, Arg73 or Phe77 of FKBP51 or FKBP52 or conserved equivalent residues in other FKBPs. Preferred compounds of this invention have at least one polar heteroatom (N, O, S, F, Cl) in the inventive $\mathbf{R^C}$ substituent and thus have the potential to engage in hydrogen bonds with Tyr57, Asp68, or Arg73 of FKBP51 or FKBP52 or conserved equivalent residues in other FKBPs. This is further supported by a direct comparison between compounds **J2** and **J6,** between **J3** and **J7** and between **J4** and **J8.** These compounds share the exact same scaffold and differ only in the $\mathbf{R^C}$ position, where **J2-4** have a vinyl group while **J6-8** have a $CH_2OH$ group. In each case the latter have the better affinities when pairwise compared with their $\mathbf{R^C}$ = vinyl counterparts.
As abovementioned, the compounds of the general formula (I) are characterized in that the substituent $\mathbf{R^C}$ significantly increases affinity to FKBP51/52. Compared to the compounds (Ref.1 - 5) of the prior art, the inventive compounds of the general formula (I) have $\mathbf{R^C}$ as hydrophilic substituents containing polar functional groups or hetero atoms which can form hydrogen bond(s) with amino acid(s) of FK506 domain.

**N2a cellular assay**

FK506 analogs have repeatedly been described as neuroprotective and neuroregenerative agents in the prior arts. Using a neurite outgrowth assay with N2a neuroblastoma cells, which are a recognized model for neuronal differentiation, compounds **C2a** and **C2b** potently enhanced neurite outgrowth (Fig.1).

**Pharmaceutical Composition**

The present invention also comprises pharmaceutically acceptable salts of the compounds according to the general formula (I) and the subformulas (II-XI), all stereoisomeric forms of the compounds according to the general formula (I) and the subformulas (II- XI) as well as solvates, especially hydrates or prodrugs thereof.

In case, the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, $NH_4OH$, tetraalkylammonium hydroxide, lysine or arginine and the like. Salts may be prepared in a conventional manner using methods well known in the art, for example by treatment of a solution of the compound of the general formula (I) and the subformulas (II-XI) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula (I) and the subformulas (II-XI) may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1% w/w of the other isomers.

Another aspect of the present invention relates to the use of the inventive FKBP51/52 ligand derivatives as drugs, i.e. as pharmaceutically active agents applicable in medicine.

Therefore one aspect of the present invention is that the compounds according to the general formula (I) and the subformulas (II-XI) are suitable for use as inhibitor of FK506-binding proteins (FKBP). It is preferred if said compound is suitable for use as inhibitor of the FK506-binding protein 51 (FKBP51) and/or the FK506-binding protein 52 (FKBP52).

FKBP51 has been implicated in numerous in human diseases (Schmidt et al., ChemMedChem 2012, 7, 1351-1359; Gaali et al, Curr Med Chem 2011, 18, 5355-5379; Galigniana et al, J. Neurochem 2012, 122, 4-18; Erlejman et al, Futue Med Chem 2013, 5,591-607; Sanchez, biochim Biophys Acta Mol Cell Res 2012r, 1823, 722-729; Zannas, A. S. & Binder, E. B. Gene-environment interactions at the FKBP5 locus: sensitive periods, mechanisms and pleiotropism. Genes Brain Behav, doi: 10.1111/gbb.12104 (2013)). Consequently, FKBP51 is a target which is addressed in order to prevent and/or treat the diseases disclosed in the afore-mentioned literature.

Thus, FKBP51 and/or FKBP 52 ligand compounds of the present invention can be used as pharmaceutically active agent in medicine.

Preferred, the FKBP51/52 ligand compounds of the present invention can be used for treatment, or for the preparation of a pharmaceutical formulation for prophylaxis and treatment of these FKBP51/52-associated diseases. These include psychiatric and neurodegenerative diseases, disorders and conditions, for metabolic diseases such as localized adiposity or obesity, for sleep disorders, neuroprotection or neuroregeneration, for the treatment of neurological disorders, for the treatment of diseases relating to neurodegeneration, for the treatment of cancers such as malignant melanoma or acute lymphoblastic leukemia and especially steroid-hormone dependent cancers such as prostate cancer, for the treatment of glucocorticoid hyposensitivity syndromes and for peripheral glucocorticoid resistance, for asthma, especially steroid-resistant asthma, and for the treatment of infectious diseases, for the treatment of alopecia and promoting hair growth, for the treatment or prevention of multi-drug resistance, for stimulating neurite growth or neuroregeneration, for neuroprotection, for the use as wound healing agents for treating wounds resulting from injury or surgery; for the use in antiglaucomatous medications for treating abnormally elevated intraocular pressure; for the use in limiting or preventing hemorrhage or neovascularization for treating macular degeneration, and for treating oxidative

damage to eye tissues, for treating a vision disorder, for improving vision, for treating memory impairment or enhancing memory performance.

The FKBP51 and/or FKBP52 ligand compounds of the present invention are preferably suitable for treatment, or for the preparation of a pharmaceutical formulation for prophylaxis and treatment of psychiatric diseases. It is especially preferred if this psychiatric diseases is an affective disorder (ICD-10 classification: F30-F39) or an anxiety disorder.

Affective disorder is a mental disorder characterized by dramatic changes or extremes of mood. The affective disorder according to the invention is selected from the group comprising or consisting of depression, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given only to a handful, including Alzheimer's Disease (Blair et al, J Clin Invest 2013, DOI: 10.1172/JCI69003), Parkinson's Disease, and amyotrophic lateral sclerosis.

Among the glucocorticoid hyposensitivity syndromes, the attention has been given to the group of related diseases enclosing resistant asthma (Tajiri et al, PLOS One 2013, 8, e65284), eosinophilic esophagitis (Caldwell et al, J Allerg Clin Immunol 2010, 125, 879-888), AIDS, rheumatoid arthritis, hypertension and diabetes, metabolic syndrome or obesity (Warrier, PhD Thesis 2008, University of Toledo, ProQuest LLC, "Role of FKBP51 and FKBP52 in Glucocorticoid Receptor Regulated Metabolism").

Among the cancers, the attention has been given to malignant melanoma (Romano et al, Cell Death Dis 2013, 4, e578), acute lymphoblastic leukemia (Li at al, Br J Cancer, 2013, DOI: 10.1038/bjc.2013.562), gliomas (Jiang et al, Neoplasia 2013, 10, 235-243), idiopathic myelofibrosis (Komura et al, Cancer Res 2005, 65, 3281-3289), pancreatic and breast cancers (Hou & Wang, PLOS One 2012, 7, e36252), steroid-hormone dependent cancers or prostate cancer.

Among the hundreds of infectious diseases, the attention has been given to malaria and the Legionnaires' disease (Gaali et al, Curr Med Chem 2011, 18, 5355-5379).

Among the vision disorders, the attention has been given to visual impairments; orbital disorders; disorders of the lacrimal apparatus; disorders of the eyelids; disorders of the conjunctiva; disorders of the Cornea; cataract; disorders of the uveal tract; disorders of the retina; disorders of the optic nerve or visual pathways; free radical induced eye disorders and diseases; immunologically-mediated eye disorders and diseases; eye injuries; and symptoms and complications of eye disease, eye disorder, or eye injury.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be

included, where appropriate. The disintegrants, diluents, lubricants, binders etc. are discussed in more detail below.

Moreover, the pharmaceutical compositions of the present invention may comprise an additional pharmaceutically active compound or drug. The pharmaceutically active compound or drug may belong to the group of glucocorticoids. Thus an embodiment of the current invention comprises the administration of a compound of the current invention in addition to a co-administration of glucocorticoids.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions may further comprise at least one FKBP51/52 ligand of the general formulas (I) and subformulas (II-XI).

The pharmaceutical compositions may further comprise at least one further active agent. It is preferred if this active agent is selected from the group consisting of anti-depressant and other psychotropic drugs. It is further preferred if the anti-depressant is selected from amitriptyline, amioxide clomipramine, doxepine, duloxetine, imipramine trimipramine, mirtazapine, reboxetine, citaloprame, fluoxetine, moclobemide and sertraline.

## Description of the Figures

**Figure 1:** Effects of FKBP51/52 inhibitors **C2a (a)** and **C2b (b)** of the present invention and of **FK506** (c) on neurite outgrowth of N2a cells.

## Examples

### Abbreviations:

AIBN (azobisisobutyronitrile), Boc (tert-butyloxycarbonyl), CAN (ceric ammonium nitrate), CDI (1,1'-carbonyldiimidazole), DCM (dichloromethane), dba (dibenzylideneacetone), DIBAL-H (diisobutylaluminium hydride), DIPEA (N,N-diisopropylethylamine), DMAP (4-dimethyl-aminopyridine), DMF (dimethylformamide), DMM (dimethoxymethane), DMSO (dimethyl sulfoxide), EDC (1-ethyl-3-(3-dimethylaminopropyl)carbodiimide), EDTA (ethylenediaminetetraacetic acid), EtOAc (ethylacetate), Fmoc (fluorenylmethyloxycarbonyl), HATU (2-(7-aza-1 H-benzotriazole-1-yl)-1,1,3,3-tetramethyluronium hexafluorophosphate), HMDS (hexamethyldisilazane), HOBt (hydroxybenzotriazole), NMO (N-methylmorpholine *N*-oxide), MOM (methoxymethyl), oNs (2-nitrobenzenesulfonyl), OTf (triflate), *o*Tol (ortho-tolyl), PMB (p-methoxybenzyl), TBAF (tetra-*n*-butylammonium chloride), tBu (tert-butyl), TEA (triethylamine), TES (triethylsilyl), TFA (trifluoroacetic acid), THF (tetrahydrofuran), TMS (trimethylsilyl).

### Synthetic Procedures

The compound of the general formula (I) can be prepared according to the following synthetic procedures.

### I. Preparation of compound A7 as starting meterial

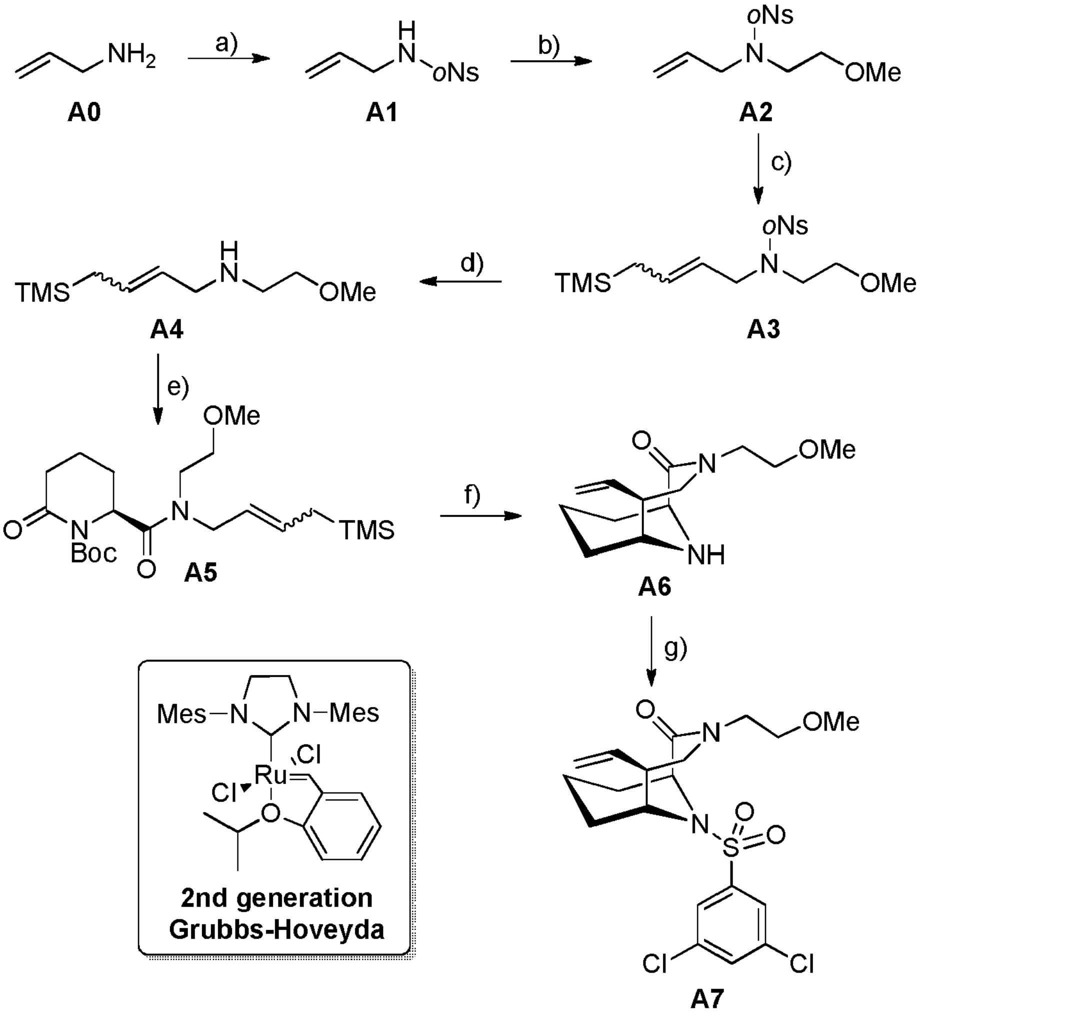


**Scheme A.** Reagents and conditions: a) 2-Nitrobenzene-sulfonylchloride, $CH_2Cl_2$, rt, 1 h, 96 %; b) $K_2CO_3$, 1-Bromo-2-methoxyethane, DMF, 60 °C, 2 h, 78 %; c) AllylTMS, Grubbs-Hoveyda II, $CH_2Cl_2$, 60 °C, 4 h, 78 %; d) $K_2CO_3$, PhSH, DMF, rt, 14 h, 82 %; e) 1.) (*S*)-6-Oxopiperidine-2-carboxylic acid, HATU, DIPEA, rt, 4 h; 2.) $Boc_2O$, DIPEA, DMAP, $CH_2Cl_2$, 6 h, 50 % (2 steps); f) 1.) DIBAL, THF, −78 °C,15 min; 2.) HF•pyridine, $CH_2Cl_2$, −78 °C, 1 h, 49 % (2 steps); g) 3,5-dichlorobenzene-1-sulfonyl chloride, DIPEA, DMAP, rt, 24 h, 60 %

**Example 1-1:** Preparation of N-allyl-2-nitrobenzenesulfonamide A1

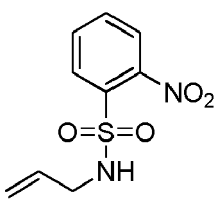

**A1**

To a solution of Allylamine (7.63 g, 10 mL, 134 mmol) in $CH_2Cl_2$ (500 mL) was added 2-nitrobenzene-1-sulfonyl chloride (10.0 g, 45.1 mmol). After 1 h stirring at room temperature saturated aqueous NaCl (100 mL) was added to the

reaction and washed with $CH_2Cl_2$ (3 x 200 mL). The solvent was removed under reduced pressure affording the title compound (10.5 g, 43.3 mmol, 96.1 %) as a slightly yellow solid, which was used for the next step without further purification.

**$R_f$:** 0.24 (Cyclohexane/EtOAc = 4:1)

**$^1$H-NMR** (300 MHz, $CDCl_3$): $\delta$ = 3.72-3.79 (m, 2 H), 5.09 (d, J = 10.5 Hz, 1 H), 5.19 (d, J = 17.1, 1 H), 5.40 ($S_{br}$, 1 H), 5.65-5.80 (m, 1 H), 7.70-7.78 (m, 2 H), 7.81-7.88 (m, 1 H), 8.08-8.14 (m, 1 H).

**$^{13}$C-NMR** (75.5 MHz, $CDCl_3$): $\delta$ = 46.28, 118.0, 125.3, 131.0, 132.5, 132.8, 133.6, 133.9, 147.9.

**Example 1-2:** Preparation of N-allyl-N-(2-methoxyethyl)-2-nitrobenzenesulfonamide **A2**

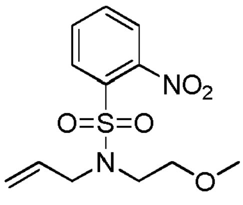

**A2**

To a solution of N-allyl-2-nitrobenzenesulfonamide (100 mg, 0.413 mmol) in DMF (4 mL) were added $K_2CO_3$ (114 mg, 0.826 mmol) and 1-Bromo-2-methoxyethane (63.1 mg, 43 $\mu$L, 0.454 mmol). The reaction was heated to 60 °C. After two hours the reaction was cooled to room temperature, $Et_2O$ (90 mL) was added and washed with sat. aq. NaCl solution (3 x 10 mL). The organic layer was dried over $MgSO_4$ and the solvent removed under reduced pressure. Flash column chromatography (10 - 40 % EtOAc in Cyclohexane) afforded the title compound (97.0 mg, 0.323 mmol, 78.2 %).

**$R_f$:** 0.175 (Cyclohexane/EtOAc = 4:1)

**$^1$H-NMR** (400 MHz, $CDCl_3$): $\delta$ = 3.23 (s, 3 H), 3.47 - 3.48 (m, 4 H), 4.02 (dt, J = 8.0, 1.6 Hz, 2 H), 5.16-5.24 (m, 2 H), 5.64-5.74 (m, 1 H), 7.26-7.68 (m, 3 H), 8.04-8.07 (m, 1 H).

**$^{13}$C-NMR** (101 MHz, $CDCl_3$): $\delta$ = 46.18, 51.08, 53.43, 58.71, 70.85, 119.2, 124.1, 130.9, 131.6, 132.7, 133.3, 133.9.

**MS** (ESI): m/z (%) = 301.00 $[M + H]^+$

**Example 1-3:** Preparation of N-(2-methoxyethyl)-2-nitro-N-(4-(trimethylsilyl)but-2-en-1-yl)benzenesulfonamide **A3**

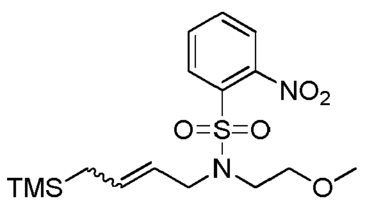

**A3**

To a solution of N-allyl-N-(2-methoxyethyl)-2-nitrobenzenesulfonamide (10.0 g, 33.3 mmol) in $CH_2Cl_2$ (350 mL) were added sequentially Allyltrimethylsilane (38.0 g, 52.9 mL, 333 mmol) and Grubbs Hoveyda II generation catalyst (1.46 g, 2.33 mmol). The reaction was stirred at 60 °C for 4 h. Sat. aq. NaCl solution (100 mL) was added to the reaction and the organic phase was separated. The aqueous phase was extracted with $CH_2Cl_2$ (3 x 200 mL) . The combined organic layers were dried over $MgSO_4$ and the solvent was removed under reduced pressure. Flash column chromatography over $SiO_2$ (5-10 % EtOAc in Cyclohexane) afforded the title compound (10.1 g, 26.1 mmol, 78.4 %).

**$R_f$:** 0.37 (Cyclohexane/EtOAc = 4:1)

**$^1$H-NMR** (300 MHz, $CDCl_3$) $\delta$ = -0.06 - 0.08 (m, 9 H), 1.40 - 1.53 (m, 2 H), 3.16 - 3.56 (m, 7 H), 3.86 - 4.10 (m, 2 H), 5.07 - 5.21 (m, 1 H), 5.56 - 5.70 (m, 1 H), 7.59 - 7.70 (m, 3 H), 8.02 - 8.09 (m, 1 H).

**$^{13}$C-NMR** (75 MHz, $CDCl_3$) $\delta$ = -1.99, 22.91, 45.45, 50.66, 58.69, 70.79, 121.2, 122.0, 124.0, 130.8, 131.4, 133.1, 133.4, 134.2.

**Example 1-4:** Preparation of *N*-(2-methoxyethyl)-4-(trimethylsilyl)but-2-en-1-amine **A4**

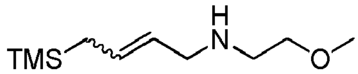

**A4**

To a solution of N-(2-methoxyethyl)-2-nitro-N-(4-(trimethylsilyl)but-2-en-1-yl) benzenesulfonamide (10.0 g, 25.9 mmol) in DMF (100 mL) was added $K_2CO_3$ (10.7 g, 78.0 mmol) and thiophenol (2.85 g, 2.65 mL, 25.9 mmol). After 14 hours stirring at room temperature $Et_2O$ (500 mL) was added to the reaction and washed with sat. aq. $NaHCO_3$ (3 x 100 mL). The organic phase was dried over $MgSO_4$ and the solvent was removed under reduced pressure. Column chromatography over $SiO_2$ (2 % TEA and 2 % MeOH in EtOAc) afforded the title compound (4.30 g, 21.4 mmol, 82.4 %) as a yellow oil.
**$R_f$:** 0.25 (EtOAc + 2 % TEA)
**MS** (ESI): m/z (%) = 201.92 $[M + H]^+$

**Example 1-5:** Preparation of (S)-N-(2-methoxyethyl)-6-oxo-N-(4-(trimethylsilyl)but-2-en-1-yl)piperidine-2-carboxamide **A5.1**

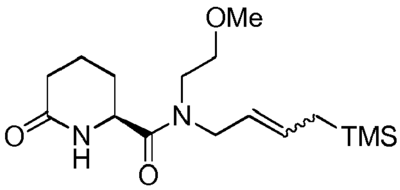

**A5.1**

To a solution of (S)-6-oxopiperidine-2-carboxylic acid (2.70 g, 18.9 mmol) in $CH_2Cl_2$ (80 mL) were added sequentially HATU (7.89 g, 20.8 mmol), DIPEA (2.68 g, 3.63 mL, 20.8 mmol) and N-(2-methoxyethyl)-4-(trimethylsilyl)but-2-en-1-amine (3.80 g, 18.9 mmol). The reaction was stirred for 4h and then saturated sat. aq. $NH_4Cl$ solution (25 mL) was added to the reaction and the organic phase was separated. The aqueous phase was extracted with $CH_2Cl_2$ (3 x 100 mL). The combined organic layers were dried over $MgSO_4$ and the solvent was removed under reduced pressure. The crude product was used for the next step without further purification.
**$R_f$:** 0.44 (EtOAc + 3 % MeOH + 1 %TEA)
MS (ESI): m/z (%) = 327.17 $[M + H]^+$, 653.02 $[2 x M + H]^+$, 675.06 $[2 x M + Na]^+$

Example **1-6:** Preparation of (S)-tert-butyl 2-((2-methoxyethyl)(4-(trimethylsilyl)but-2-en-1-yl)carbamoyl)-6-oxopiperidine-1-carboxylate **A5.2**

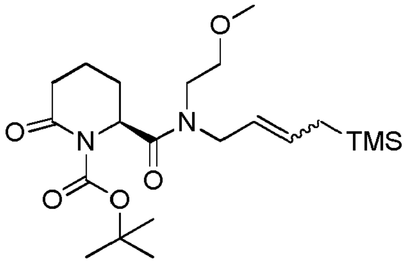

**A5.2**

Crude mixture of **A5.1** was dissolved in $CH_2Cl_2$ (150 mL). TEA (3.82 g, 5.23 mL, 38.0 mmol), $Boc_2O$ (8.29 g, 38 mmol) and DMAP (3.46 g, 28 mmol) were added. After 6 h stirring at room temperature sat. aq. NaCl solution (25 mL) was added to the reaction and the organic phase was separated. The aqueous phase was extracted with $CH_2Cl_2$ (3 x 100 mL) and the combined organic layers were dried over $MgSO_4$ and the solvent was removed under reduced pressure.

Flash column chromatography (10 - 40 % EtOAc in Cyclohexane) afforded the title compound (4.05 g, 9.40 mmol, 50 % over two steps) as a yellow resin.
**$R_f$:** 0.56 (Cyclohexane/EtOAc = 1:1)
**MS** (ESI): m/z (%) = 327.12 [M - Boc + H]$^+$, 875.12 [2 x M + Na]$^+$

**Example 1-6:** Preparation of (1*S*,5*R*,6*R*)-3-(2-methoxyethyl)-5-vinyl-3,10-diazabicyclo[4.3.1]decan-2-one A6

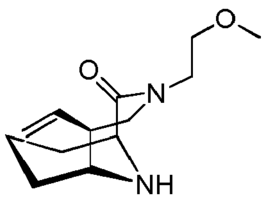

**A6**

A solution of (6*S*)-tert-butyl 2-hydroxy-6-((2-methoxyethyl)(4-(trimethylsilyl)but-2-en-1-yl)carbamoyl)piperidine-1-carboxylate (220 mg, 0.516 mmol) in THF (5 mL) was cooled to -78 °C and then DIBAL-H (1 M solution in $CH_2Cl_2$, 1.03 mL, 1.03 mmol) was added. After 1 h th solvent was removed under reduced pressure.

The resin resulted from the first step was dissolved in $CH_2Cl_2$ (25 mL) and cooled to - 78 °C. HF (70 % in pyridine, 1.25 mL) was added and the reaction was stirred for 1 h. Then Sat. aq. $NaHCO_3$ solution was added. The reaction was extracted three times with $CH_2Cl_2$, the combined organic layers were dried over $MgSO_4$ and the solvent removed under reduced pressure. Column chromatography (5 % MeOH and 2 % TEA in EtOAc) afforded the title compound (60.0 mg, 0.251 mmol, 49.1 % over two steps) as an orange resin.
**$^1$H-NMR** (599 MHz, $CDCl_3$) $\delta$ = 1.56 - 1.80 (m, 5 H), 2.22 - 2.38 (m, 1 H), 2.58 - 2.76 (m, 1 H), 2.79 - 2.90 (m, 1 H), 3.07 (dd, J = 13.9, 2.0 Hz, 1 H), 3.31 (m, 1 H), 3.34 (s, 3 H), 3.50 - 3.56 (m, 3 H), 3.65 - 3.74 (m, 1 H), 3.71 - 3.86 (m, 1 H), 4.08 (dd, J = 13.9, 10.7 Hz, 1 H), 4.94 - 5.04 (m, 2 H), 5.65 (ddd, J = 17.0, 10.2, 8.4 Hz, 1 H).
**$^{13}$C-NMR** (151 MHz, $CDCl_3$) $\delta$ = 28.04, 29.36, 29.67, 49.61, 51.03, 52.40, 52.57, 57.59, 58.80, 71.32, 114.9, 139.2, 174.2.
**MS** (ESI): m/z (%) = 239.23 [M + H]$^+$, 477.02 [2 x M + H]$^+$

**Example 1-6:** Preparation of (1*S*,5*R*,6*R*)-10-((3,5-dichlorophenyl)sulfonyl)-3-(2-methoxyethyl)-5-vinyl-3,10-diazabicyclo[4.3.1]decan-2-one **A7**

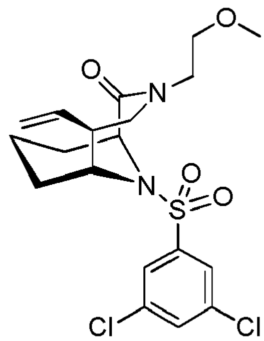

**A7**

To a solution of (1*S*,5*R*,6*R*)-3-(2-methoxyethyl)-5-vinyl-3,10-diazabicyclo [4.3.1] decan-2-one (600 mg, 2.52 mmol) in $CH_2Cl_2$ (25 mL) were added 3,5-dichlorobenzene-1-sulfonyl chloride (618 mg, 2.52 mmol), DMAP (308 mg, 2.52 mmol) and DIPEA (0.879 mL, 5.04 mmol) and the reaction was stirred for 24 hours at room temperature. Sat. aq. $NaHCO_3$ solution (25 mL) was added to the mixture and extracted with $CH_2Cl_2$ (250 mL). Flash column chromatography on $SiO_2$ (5 - 20 % EtOAc in Cyclohexane) afforded the title compound (680 mg, 1.52 mmol, 60.3 %) as a colorless solid.
**MS** (ESI): m/z (%) = 447.17 [M + H]$^+$, 894.44 [2 x M + H]$^+$

**II. Preparation of compounds B1 - B15**

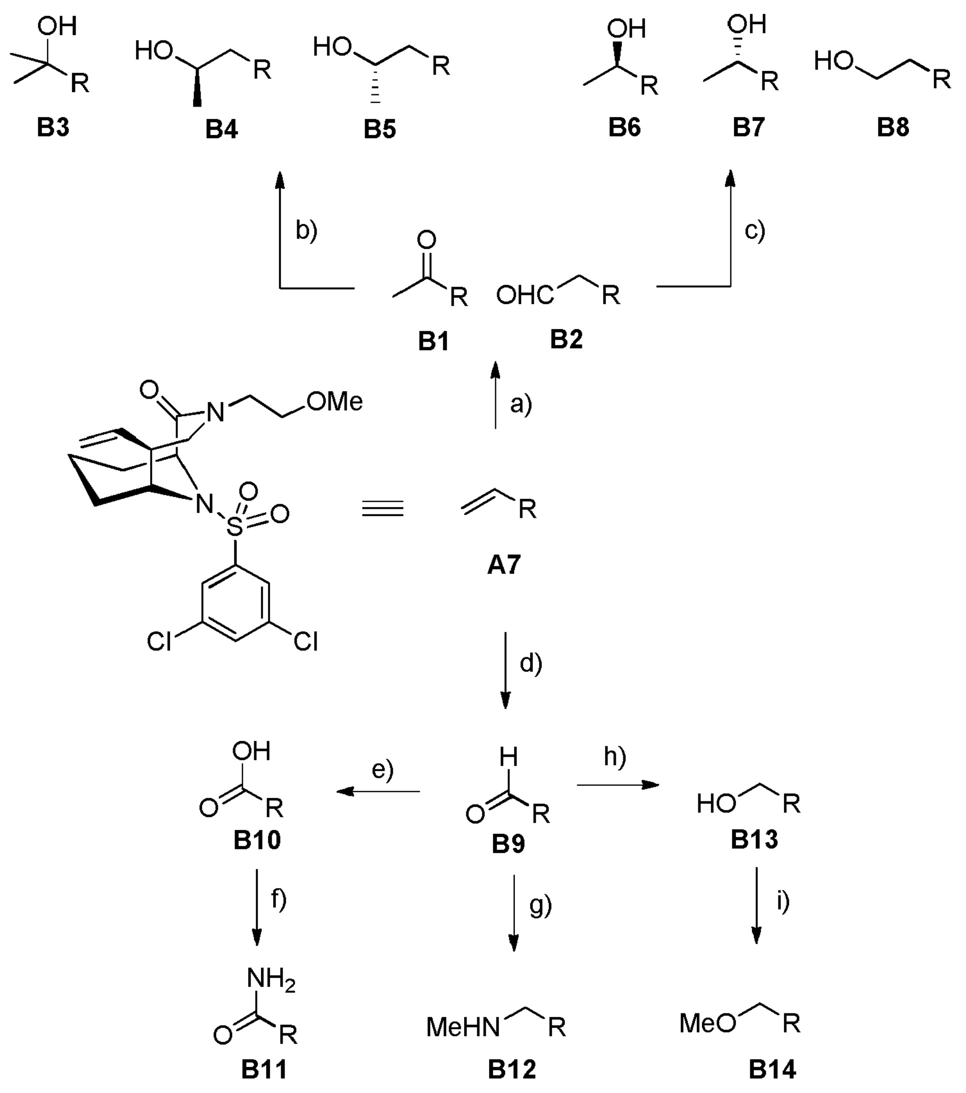

**B3** **B4** **B5** **B6** **B7** **B8**
**B1** **B2**
**A7**
**B10** **B9** **B13**
**B11** **B12** **B14**

**Scheme B.** Reagents and conditions: a) $PdCl_2$, CuCl, $O_2$, DMF/$H_2O$, rt, 3 d, 39 %; b) MeMgBr, THF, −78 °C, 1 h, 62 %; c) $NaBH_4$, EtOH/$CH_2Cl_2$, rt, 1 h, 36 %d) $NaIO_4$, $OsO_4$, 2,6-lutidine, dioxane/$H_2O$, 20 h, rt, 70 %; e) 2-methylbutene, $NaClO_2$, $NaH_2PO_4$, $CHCl_3$/*t*-BuOH/$H_2O$, rt, 16 h, 93 %; f) CDI, $NH_3$ aq., EtOAc, rt, 2 h, 41 % g) $NH_2Me\cdot HCl$, $NaBH(OAc)_3$, MeOH/AcOH, rt, 4 h, 33%; h) $NaBH_4$, EtOH, rt, 1 h, 65 %; i) NaH, MeI, THF, rt, 5 h, 50 %.

Example **2-1:** Preparation of (1*S*,5*S*,6*R*)-5-acetyl-10-((3,5-dichlorophenyl)sulfonyl)-3-(2-methoxyethyl)-3,10-diazabicyclo[4.3.1]decan-2-one **B1** and 2-((1*S*,5*S*,6*R*)-10-((3,5-dichlorophenyl)sulfonyl)-3-(2-methoxyethyl)-2-oxo-3,10-diazabicyclo[4.3.1] decan-5-yl)acetaldehyde **B2**

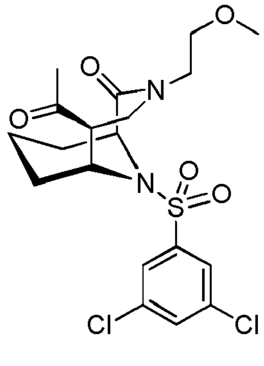 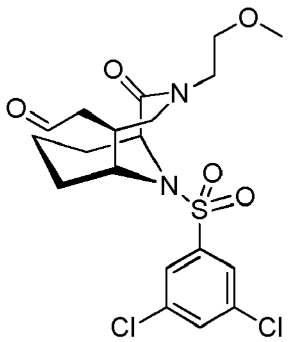

**B1** **B2**

To a solution of (1*S*,5*R*,6*R*)-10-((3,5-dichlorophenyl)sulfonyl)-3-(2-methoxyethyl)-5-vinyl-3,10-diazabicyclo[4.3.1]decan-2-one (450 mg, 1.01 mmol) in DMF/$H_2O$ (0.571 mL, 7:1) was added $PdCl_2$ (35.7 mg, 0.201 mmol) and CuCl (100 mg, 1.01 mmol). After three days stirring $Et_2O$ was added to the reaction and extracted three times with saturated $Na_2S_2O_3$. The organic layer was dried over $MgSO_4$ and the solvent removed under reduced pressure. Column chromatography on $SiO_2$ (EtOAc/Cyclohexane = 1:1) afforded an inseparable mixture of the title compounds (180 mg, 0.388 mmol, 38.6 %) as a slightly yellow solid.
**$R_f$:** 0.6 (Cyclohexan/EtOAc = 15:85)
**MS** (ESI): m/z (%) = 463.13 $[M + H]^+$

**Example 2-2:** Preparation of (1*S*,5*S*,6*R*)-10-((3,5-dichlorophenyl)sulfonyl)-5-(2-hydroxypropan-2-yl)-3-(2-methoxyethyl)-3,10-diazabicyclo[4.3.1]decan-2-one **B3**

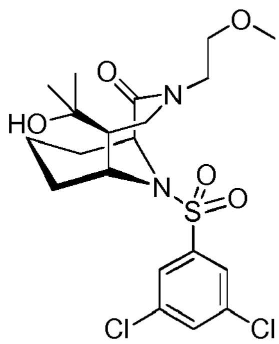

**B3**

A solution of a mixture of 2-((1*S*,5*S*,6*R*)-10-((3,5-dichlorophenyl)sulfonyl)-3-(2-methoxyethyl)-2-oxo-3,10-diazabicyclo[4.3.1]decan-5-yl)acetaldehyde and (5S)-5-acetyl-10-((3,5-dichlorophenyl)sulfonyl)-3-(2-methoxyethyl)-3,10-diazabicyclo[4.3.1] decan-2-one (58.0 mg, 0.125 mmol) in THF (1.5 mL) was cooled to -78 °C. $CH_3MgBr$ (3 M in $Et_2O$, 0.189 mmol, 63.0 μL) was added and the reaction was stirred for 1 h. Aq. Sat. $NH_4Cl$ solution (10 mL) was added to the reaction and extracted with $CH_2Cl_2$ (2 x 90 mL). The organic layer was dried over $MgSO_4$ and the solvent was evaporated under reduced pressure. Normal phase preparative chromatography (10 - 30 % EtOAc in *n*-Hexane) afforded the title compound (37.0 mg, 0.0772 mmol, 61.8%) as a colorless solid.
**$^1$H-NMR** (600 MHz, Chloroform-*d*) δ = 1.11 (m, 1 H), 1.26 (s, 3 H), 1.31 (s, 3 H), 1.38 - 1.47 (m, 1 H), 1.46 - 1.57 (m, 3 H), 1.65 (d, *J* = 13.4 Hz, 1 H), 2.14 (ddd, *J* = 10.3, 6.8, 1.4 Hz, 1 H), 2.23 (d, *J* = 13.6 Hz, 1 H), 3.27 (dd, *J* = 14.1, 1.6 Hz, 2 H), 3.35 (s, 3 H), 3.50 - 3.61 (m, 2 H), 3.95 (dd, *J* = 14.3, 10.5 Hz, 1 H), 4.06 (ddd, *J* = 14.1, 5.2, 3.6 Hz, 1 H), 4.25 - 4.30 (m, 1 H), 4.68 (d, *J* = 5.7 Hz, 1 H), 7.56 (t, *J* = 1.8 Hz, 1 H), 7.72(d, *J* = 1.9Hz,2H).
**$^{13}$C-NMR** (150MHz, Chloroform-*d*) δ = 15.32, 27.10, 27.88, 28.10, 29.72, 49.94, 51.45, 52.01, 53.96, 57.15, 58.80, 71.38, 72.49, 125.0, 132.6, 136.2, 143.9, 169.4.
**MS** (ESI): *m*/*z* (%) = 479.11 $[M + H]^+$

**Example 2-3:** Preparation of (1*S*,5*S*,6*R*)-10-((3,5-dichlorophenyl)sulfonyl)-5-((R)-1-hydroxyethyl)-3-(2-methoxyethyl)-3,10-diazabicyclo[4.3.1]decan-2-one **B6,** (1*S*,5*S*,6*R*)-10-((3,5-dichlorophenyl)sulfonyl)-5-((S)-1-hydroxyethyl)-3-(2-methoxy ethyl)-3,10-diazabicyclo[4.3.1]decan-2-one **B7,** and (1*S*,5*S*,6*R*)-10-((3,5-dichlorophenyl)sulfonyl)-5-(2-hydroxyethyl)-3-(2-methoxyethyl)-3,1 0-diazabicyclo[4.3.1]decan-2-one **B8**

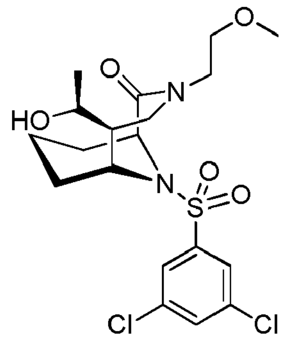

**B6**

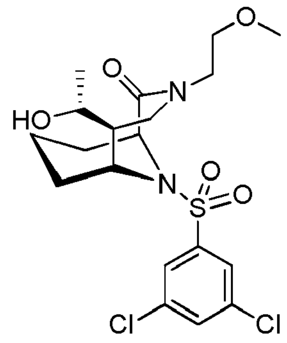

**B7**

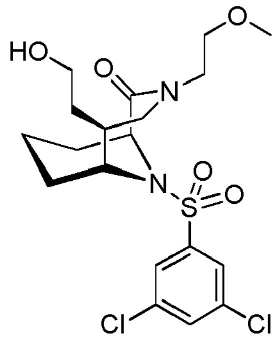

**B8**

To a solution of a mixture of (1*S*,5*S*,6*R*)-5-acetyl-10-((3,5-dichlorophenyl)sulfonyl)-3-(2-methoxyethyl)-3,10-diazabicyclo[4.3.1]decan-2-one and (2-((1*S*,5*S*,6*R*)-10-((3,5-dichlorophenyl)sulfonyl)-3-(2-methoxyethyl)-2-oxo-3,10-diazabicyclo[4.3.1]decan-5-yl)acetaldehyde (60.0 mg, 0.129 mmol) in EtOH/$CH_2Cl_2$ (2 mL, 3:1) was added $NaBH_4$ (7.35 mg, 0.194 mmol) and then stirred for 30 minutes at room temperature. Sat. aq. NaCl solution (10 mL) was added to the reaction and extracted with $CH_2Cl_2$ (3 x 50 mL). The organic layer was dried over $MgSO_4$ and the solvent was evaporated under reduced pressure. Normal phase preparative chromatography on $SiO_2$ (10-30 % EtOAc in *n*-Hexane) afforded the title compounds **(B6:** 5.0 mg, 0.011 mmol, **B7:** 4.2 mg, 0.0090 mmol, **B8:** 13.0 mg, 0.0279 mmol, 36.9 %).

**B6**

**$R_f$:** 0.5 (Cyclohexane/EtOAc = 15:85)

**$^1$H-NMR:** (600 MHz, $CDCl_3$) $\delta$ = 1.28 - 1.29 (m, 5 H), 1.47 - 1.58 (m, 4 H), 2.06 - 2.13 (m, 1 H), 2.21 - 2.29 (m, 1 H), 3.29 - 3.33 (m, 2 H), 3.34 (s, 3 H), 3.49 - 3.60 (m, 2 H), 3.78 - 3.90 (m, 2 H), 3.96 - 4.02 (m, 1 H), 4.12 - 4.17 (m, 1 H), 4.66 - 4.70 (m, 1 H), 7.56 (t, J = 1.8 Hz, 1 H), 7.71 (d, J = 1.8 Hz, 2 H).

**$^{13}$C-NMR:** (150 MHz, $CDCl_3$) $\delta$ = 20.92, 28.15, 28.51, 29.66, 48.96, 50.55, 51.33, 52.61, 56.96, 58.83, 68.89, 71.24, 124.9, 124.9, 132.6, 132.6, 136.2, 143.9, 169.6.

**MS** (ESI): *m/z* (%) = 465.14 $[M + H]^+$

**B7**

**$R_f$:** 0.5 (Cyclohexane/EtOAc = 15:85)

**$^1$H-NMR:** (600 MHz, $CDCl_3$) $\delta$ = 1.28 - 1.29 (m, 5 H), 1.47 - 1.58 (m, 4 H), 2.06-2.13 (m, 1 H), 2.21 - 2.29 (m, 1 H), 3.29 - 3.33 (m, 2 H), 3.34 (s, 3 H), 3.49 - 3.60 (m, 2 H), 3.78 - 3.90 (m, 2 H), 3.96 - 4.02 (m, 1 H), 4.12 - 4.17 (m, 1 H), 4.66 - 4.70 (m, 1 H), 7.56 (t, J = 1.8 Hz, 1 H), 7.71 (d, J = 1.8 Hz, 2 H).

**$^{13}$C-NMR:** (150 MHz, $CDCl_3$) $\delta$ = 20.92, 28.15, 28.51, 29.66, 48.96, 50.55, 51.33, 52.61, 56.96, 58.83, 68.89, 71.24, 124.9, 124.9, 132.6, 132.6, 136.2, 143.9, 169.6.

**MS** (ESI): *m/z* (%) = 465.14 $[M + H]^+$

**B8**

**$R_f$:** 0.34 (Cyclohexane/EtOAc = 15:85)

**$^1$H-NMR:** (600 MHz, $CDCl_3$) $\delta$ = 1.34 - 1.41 (m, 2 H), 1.47 - 1.69 (m, 3 H), 2.22 - 2.33 (m, 2 H), 3.18 - 3.25 (m, 1 H), 3.37 (s, 3 H), 3.46 - 3.55 (m, 2 H), 3.65 - 3.70 (m, 1 H), 3.71 - 3.75 (m, 2 H), 3.83 - 3.89 (m, 1 H), 3.94 (dd, J = 14.4, 10.5 Hz, 1 H), 4.04 - 4.11 (m, 1 H), 4.68 (d, J = 5.8 Hz, 1 H), 7.56 (t, J = 1.8 Hz, 1 H), 7.69 (d, J = 1.9Hz,2H).

**$^{13}$C-NMR:** (150 MHz, $CDCl_3$) $\delta$ = 26.83, 29.66, 35.59, 40.84, 55.79, 56.72, 59.02, 60.09, 72.05, 124.8, 132.6, 136.3, 144.0, 170.0.

**MS** (ESI): m/z (%) = 465.14 $[M + H]^+$

**Example 2-4:** Preparation of (1*S*,5*S*,6*R*)-10-((3,5-dichlorophenyl)sulfonyl)-3-(2-methoxyethyl)-2-oxo-3,10-diazabicyclo[4.3.1]decane-5-carbaldehyde B9

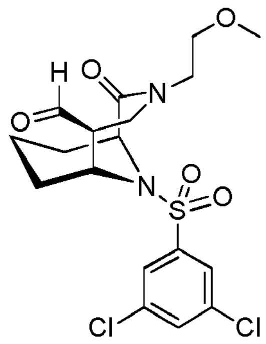

**B9**

To a solution of (1*S*,5*R*,6*R*)-10-((3,5-dichlorophenyl)sulfonyl)-3-(2-methoxyethyl)-5-vinyl-3,10-diazabicyclo[4.3.1]decan-2-one (250 mg, 0.559 mmol) in Dioxane/$H_2O$ (5.6 mL, 3:1) was added $NaIO_4$ (478 mg, 2.23 mmol), $OsO_4$ (2.5 % Solution in *tert*-Butanol, 0.011 mmol, 140 mL) and 2,6-Lutidine. The solution was stirred for 20 h at room temperature, then $Et_2O$ (90 mL) was added to the reaction and washed with sat. aq. NaCl solution (3 x 10 mL). Column chromatography over $SiO_2$ (EtOAc/Cyclohexane 1:1) afforded the title compound (175 mg, 0.389 mmol, 70.0%) as a slightly yellow solid.
**$R_f$:** 0.21 (Cyclohexane/EtOAc = 1:1)
**$^1$H-NMR** (600 MHz, $CDCl_3$) $\delta$ 1.21 - 1.29 (m, 1 H), 1.44 - 1.50 (m, 3 H), 1.53 - 1.59 (m, 1 H), 2.29 (dd, J = 13.5, 2.1 Hz, 1 H), 3.03 (ddd, J = 10.5, 7.0, 1.7 Hz, 1 H), 3.36 (s, 3 H), 3.37 - 3.42 (m, 1 H), 3.50 - 3.56 (m, 1 H), 3.61 - 3.66 (m, 1 H), 3.68 - 3.72 (m, 1 H), 4.01 - 4.08 (m, 2 H), 4.70 (d, J = 7.0 Hz, 1 H), 4.72 (d, J = 5.9 Hz, 1 H), 7.57 (t, J = 1.9 Hz, 1 H), 7.72 (d, J = 1.8 Hz, 2 H), 9.65 (s, 1 H).
**$^{13}$C-NMR** (150 MHz, $cdcl_3$) $\delta$ = 15.20, 28.06, 28.38, 47.66, 48.81, 51.73, 56.97, 57.22, 59.02, 71.53, 124.9, 132.8, 136.4, 143.7, 169.6, 197.7.
**MS** (ESI): m/z (%) = 449.05 $[M + H]^+$

**Example 2-5:** Preparation of (1S,5S,6R)-10-((3,5-dichlorophenyl)sulfonyl)-3-(2-methoxyethyl)-2-oxo-3,10-diazabicyclo[4.3.1]decane-5-carboxylic acid **B10**

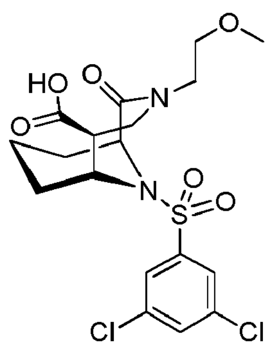

**B10**

To a solution of (1S,5S,6R)-5-acetyl-10-((3,5-dichlorophenyl)sulfonyl)-3-(2-methoxyethyl)-3,10-diazabicyclo[4.3.1]decan-2-one (25.0 mg, 0.0560 mmol) in $CHCl_3$/*t*-BuOH/$H_2O$ (0.9 ml, 6:2:1) were metylbutene (60 $\mu$L), sodium chlorite (19.5 mg, 0.216 mmol) and sodium dihydrogenphosphate (25.9 mg). After 16 h a room temperature the solvent was removed under reduced pressure and the crude mix loaded directly on $SiO_2$. Flash column chromatography (20 - 60 % EtOAc in Cyclohexane) afforded the title compound (24.0 mg, 0.0516 mmol, 92.7 %) as a white solid.
**MS** (ESI): m/z (%) = 465.07

**Example 2-5:** Preparation of (1*S*,5*S*,6*R*)-10-((3,5-dichlorophenyl)sulfonyl)-3-(2-methoxyethyl)-2-oxo-3,10-diazabicyclo[4.3.1]decane-5-carboxamide **B11**

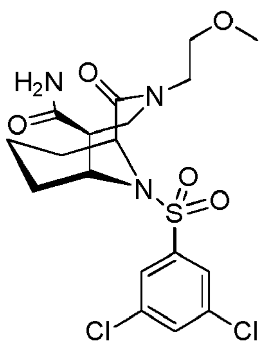
B11

To a solution of (1*S*,5*S*,6*R*)-10-((3,5-dichlorophenyl)sulfonyl)-3-(2-methoxyethyl)-2-oxo-3,10-diazabicyclo[4.3.1]decane-5-carboxylic acid (30.0 mg, 0.0645 mmol) in EtOAc (0.5 ml) was added CDI (15.7 mg, 0.0970 mmol). After one hour stirring at room temperature aqueous ammonia was added (7 M, 184 μL, 1.29 mmol) and the reaction was stirred for two hours. Solvent was removed under reduced pressure and flash column chromatography afforded the title compound (12 mg, 0.026 mmol, 41 %).
**$R_f$:** 0.125 (Cyclohexane/EtOAc = 3:7 + 2 % MeOH)
**$^1$H-NMR** (600 MHz, Chloroform-*d*) δ = 1.42 (s, 2 H), 1.45 - 1.52 (m, 2 H), 1.52 - 1.56 (m, 1 H), 2.29 (d, *J* = 13.1 Hz, 1 H), 2.98 (ddd, *J* = 10.2, 7.3, 1.5 Hz, 1 H), 3.20 (ddd, *J* = 14.2, 9.5, 3.1 Hz, 1 H), 3.34 - 3.38 (m, 1 H), 3.40 (s, 3 H), 3.51 - 3.56 (m, 1 H), 3.70 (td, *J* = 9.7, 2.5 Hz, 1 H), 4.12 - 4.20 (m, 2 H), 4.74 (d, *J* = 5.8 Hz, 1 H), 4.77-4.81 (m, 1 H), 5.90 (s, 1 H), 6.14 (s, 1 H), 7.56 - 7.58 (m, 1 H), 7.73 - 7.75 (m, 2 H). **$^{13}$C-NMR** (150 MHz, Chloroform-*d*) δ = 15.29, 27.82, 28.03, 51.22, 51.23, 51.58, 51.80, 56.88, 59.10, 72.36, 124.9, 132.8, 136.3, 143.7, 169.7, 172.3.
**MS** (ESI): *m*/*z* (%) = 464.07 $[M + H]^+$

**Example 2-6:** Preparation of (1*S*,5*S*,6*R*)-10-((3,5-dichlorophenyl)sulfonyl)-3-(2-methoxyethyl)-5-((methylamino)methyl)-3,10-diazabicyclo[4.3.1]decan-2-one B12

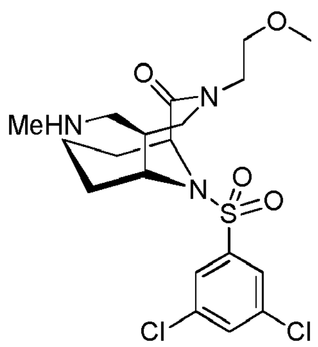
B12

To a solution of (1S,5R,6R)-10-((3,5-dichlorophenyl)sulfonyl)-3-(2-methoxyethyl)-2-oxo-3,10-diazabicyclo[4.3.1]decane-5-carbaldehyde (15.0 mg, 0.0330 mmol) in MeOH/AcOH (0.5 mL, 98:2) were added Methanamine Hydrochlorid (6.80 mg, 0.100 mmol) and $NaBH(OAc)_3$ (14.2 mg, 0.0660 mmol) and the reaction was stirred at room temperature for 4 hours. The solvent was removed under reduced pressure and the crude was loaded directly on $SiO_2$. Column chromatography over $SiO_2$ (EtOAc + 2 % TEA + 2 % MeOH) afforded the title compound (5.00 mg, 0.0107 mmol, 32.6 %).
**$R_f$:** 0.16 (EtOAc + 2 % TEA + 2 % MeOH)
**MS** (ESI): m/z (%) = 464.36 $[M + H]^+$

**Example 2-7:** Preparation of (1*S*,5*R*,6*R*)-10-((3,5-dichlorophenyl)sulfonyl)-5-(hydroxymethyl)-3-(2-methoxyethyl)-3,10-diazabicyclo[4.3.1]decan-2-one **B13**

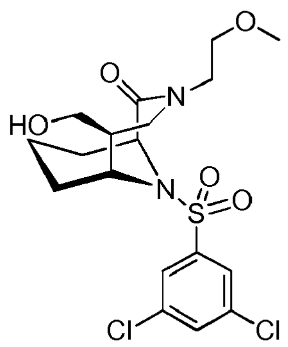

**B13**

To solution of (1*S*,5*R*,6*R*)-10-((3,5-dichlorophenyl)sulfonyl)-3-(2-methoxyethyl)-2-oxo-3,10-diazabicyclo[4.3.1]decane-5-carbaldehyde (17.0 mg, 0.0378 mmol) in EtOH (1 mL) was added $NaBH_4$ (2.20 mg, 0.0588 mmol). After 1 h stirring at room temperature sat. aq. NaCl solution (10 mL) was added to the reaction and extracted with $CH_2Cl_2$ (3 x 50 mL). The combined organic phases were dried over $MgSO_4$ and the solvent was removed under reduced pressure. Column chromatography on $SiO_2$ (Cyclohexane/EtOAc = 1:4) afforded the title compound (11.0 mg, 0.0244 mmol, 64.6 %).

**$R_f$:** 0.2 (Cyclohexane/EtOAc = 1:4)

**$^1$H-NMR** (600 MHz, $CDCl_3$) $\delta$ = 1.38 - 1.44 (m, 2 H), 1.50 - 1.57 (m, 3 H), 2.24 - 2.32 (m, 2 H), 3.09 - 3.14 (m, 1 H), 3.30 (dd, *J* = 14.4, 1.7 Hz, 1 H), 3.35 (s, 3 H), 3.41 (dd, *J* = 14.0, 4.0 Hz, 1 H), 3.50 - 3.54 (m, 1 H), 3.57 (dd, *J* = 7.2, 3.9 Hz, 1 H), 3.59 (dd, *J* = 7.1, 3.5 Hz, 2 H), 3.85 - 3.97 (m, 3 H), 4.69 - 4.72 (m, 1 H), 7.56 (t, *J* = 1.8 Hz, 1 H), 7.70 (d, *J* = 1.9 Hz, 2 H).

**$^{13}$C-NMR** (150 MHz, $CDCl_3$) $\delta$ = 15.43, 27.98, 29.66, 46.94, 50.23, 51.19, 52.35, 56.99, 58.90, 63.53, 71.21, 124.8, 132.6, 136.2, 144.0, 169.8.

**MS** (ESI): m/z (%) = 451.15 $[M + H]^+$

**Example 2-8:** Preparation of (1*S*,5*R*,6*R*)-10-((3,5-dichlorophenyl)sulfonyl)-3-(2-methoxyethyl)-5-(methoxymethyl)-3,10-diazabicyclo[4.3.1]decan-2-one **B14**

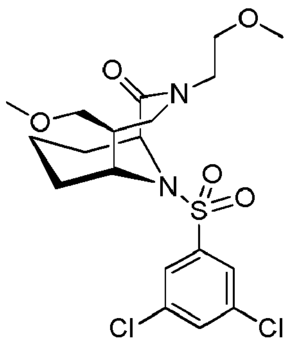

**B14**

To a solution of (1*S*,5*R*,6*R*)-10-((3,5-dichlorophenyl)sulfonyl)-5-(hydroxymethyl)-3-(2-methoxyethyl)-3,10-diazabicyclo[4.3.1]decan-2-one (25.0 mg, 0.0554 mmol) in THF (1 mL) was added NaH (60 % dispersion in mineral oil, 4.7 mg, 0.12 mmol) and stirred for 20 minutes. $CH_3I$ (16.8 mg, 7.4 µL, 0.118 mmol) was added. After 5 h the solvent was removed under reduced pressure and column chromatography over $SiO_2$ (EtOAc/Cyclohexane = 1:1) afforded the title compound (13.0 mg, 0.0279 mmol, 50.4%) as a yellow oil.

**$^1$H-NMR** (600MHz, $CDCl_3$) $\delta$ = 1.27 - 1.33 (m, 2 H), 1.55 - 1.65 (m, 3 H), 2.27 (d, *J* = 13.1 Hz, 1 H), 2.30 - 2.37 (m, 1 H), 3.18 (dd, J = 14.3, 1.7 Hz, 1 H), 3.23 - 3.34 (m, 2 H), 3.34 (s, 3 H), 3.35 (s, 3 H), 3.40 - 3.47 (m, 1 H), 3.47 - 3.57 (m, 2 H), 3.75 (dd, J = 14.3, 10.6 Hz, 1 H), 3.79 - 3.86 (m, 1 H), 3.89 - 3.95 (m, 1 H), 4.70 (d, J = 5.9 Hz, 1 H), 7.55 (t, J = 1.8 Hz, 1 H), 7.70 (d, J = 1.8 Hz, 2 H).

**$^{13}$C-NMR** (150 MHz, $CDCl_3$) $\delta$ 15.45, 22.66, 28.11, 28.16, 29.67, 44.73, 50.31, 51.18, 52.64, 56.99, 70.81, 73.44, 124.9, 132.5, 136.2, 144.0, 169.7.

**MS** (ESI): m/z (%) = 465.12 $[M + H]^+$

**Example 2-9:** Preparation of (1S,5S,6R)-10-((3,5-dichlorophenyl)sulfonyl)-5-ethyl-3-(2-methoxyethyl)-3,10-diazabicyclo[4.3.1]decan-2-one **B15**

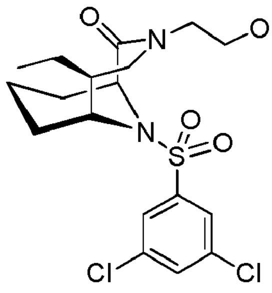

**B15**

To a solution of (1*S*,5*R*,6*R*)-10-((3,5-dichlorophenyl)sulfonyl)-3-(2-methoxyethyl)-5-vinyl-3,10-diazabicyclo[4.3.1]decan-2-one (20.0 mg, 0.0447 mmol) in MeOH (1.0 mL) was added Pd/C (10 % wt, 4,76 mg, 4,47 μmol) and the reaction was stirred for 1 h at room temperature. The mixture was then filtered through celite and washed with MeOH. $CH_2Cl_2$ was added to the solution and washed three times with sat. aq. NaCl solution. The organic phase was separated and the solvent was removed under reduced pressure affording the title compound (20.0 mg, 0.0447 mmol, quant.) as a colorless solid.

**$^1$H NMR** (600 MHz, $CDCl_3$) δ = 0.95 - 0.99 (m, 2H), 1.22 - 1.26 (m, 3H), 1.30 - 1.56 (m, 5H), 1.92 - 1.99 (m, 1 H), 2.23 (d, J = 13.5 Hz, 1 H), 3.07 - 3.13 (m, 1H), 3.31-3.38 (m, 4H), 3.47 - 3.56 (m, 2H), 3.79 - 3.91 (m, 3H), 4.63 - 4.68 (m, 1 H), 7.52-7.56 (m, 1 H), 7.67 - 7.70 (m, 2 H).

**III. Preparation of compounds C1 - C3**

**Example 3-1:** Preparation of (1*S*,5*R*,6*R*)-10-((3,5-dichlorophenyl)sulfonyl)-3-(2-hydroxyethyl)-5-(hydroxymethyl)-3,10-diazabicyclo[4.3.1]decan-2-one **C1**

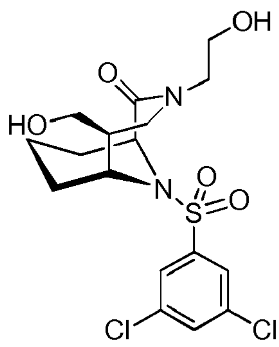

**C1**

(1*S*,5*R*,6*R*)-10-((3,5-dichlorophenyl)sulfonyl)-3-(2-hydroxyethyl)-5-vinyl-3,10-diazabicyclo[4.3.1]decan-2-one in $CH_2Cl_2$/MeOH (5 mL, 1:1) was cooled to -78 °C. $N_2$ was bubbled trough the solution for 15 minutes. Then $O_3$ was bubbled through the solution for 5 minutes, until the solution turned blue. The residual $O_3$ was removed with more $N_2$ flux. The reaction was allowed to warm to room temperature, quenched with dimethyl sulfide (13 μL) and then 3.6 mg of $NaBH_4$ (0,096 mmol) were added. After 30 minutes solvent was removed under reduced pressure and column chromatography over $SiO_2$ (ethyl acetate/cyclohexane, 1:1) afforded the title compound (25.0 mg, 0,0569 mmol, 64.9 %) as a white solid.

**$^1$H-NMR** (600 MHz, Chloroform-*d*) δ = 1.37 (s, 2 H), 1.51 - 1.54 (m, 3 H), 2.25 (d, *J* = 13.2 Hz, 1 H), 2.33 (dt, J = 10.3, 5.1 Hz, 1 H), 3.33 (dd, J = 14.4, 1.7 Hz, 1 H), 3.39 (d, J = 14.4 Hz, 1 H), 3.55 - 3.62 (m, 2 H), 3.78 - 3.82 (m, 2 H), 3.86 - 3.89 (m, 1 H), 3.90 - 3.95 (m, 2 H), 4.69 (d, J = 5.9 Hz, 1 H), 7.56 (t, J = 1.8 Hz, 1 H), 7.70 (d, J = 1.9Hz,2H).

**$^{13}$C-NMR** (150 MHz, Chloroform-*d*) δ = 15.44, 27.71, 29.67, 30.29, 47.10, 50.07, 52.28, 54.08, 57.04, 61.21, 63.17, 124.9, 132.8, 136.3, 143.9, 171.4.

**Example 3-2:** Preparation of (1*S*,5*S*,6*R*)-10-((3,5-dichlorophenyl)sulfonyl)-5-(1,2-dihydroxyethyl)-3-(2-(3,4-dimethoxy-phenoxy)ethyl)-3,10-diazabicyclo[4.3.1]decan-2-one **C2/C2b**

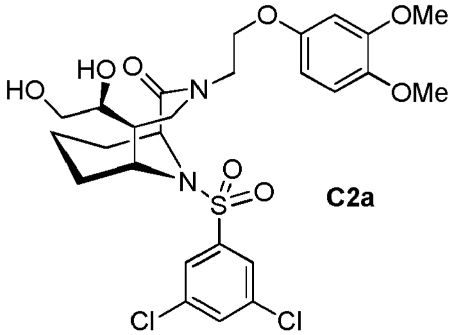
**C2a**

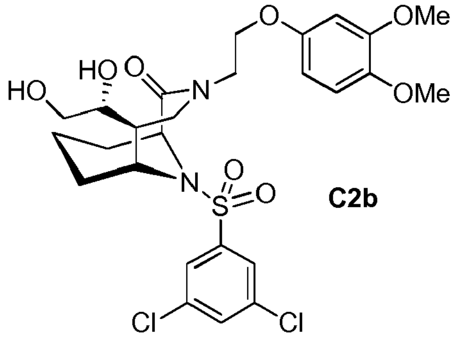
**C2b**

To a solution of (1*S*,5*R*,6*R*)-10-((3,5-dichlorophenyl)sulfonyl)-3-(2-(3,4-dimethoxy-phenoxy)ethyl)-5-vinyl-3,10-diazabicyclo[4.3.1]decan-2-one (30.0 mg, 0.527 mmol) in *t*-BuOH/$H_2O$ (1 mL, 1:1) was added AD-MIX-ß (73.8 mg) and the reaction was stirred at room temperature for 24 h. Saturated NaCl was added to the mixture and extracted three times with $CH_2Cl_2$. The combined organic layers were dried over $MgSO_4$ and the solvent was removed under reduced pressure. Column chromatography on $SiO_2$ (Cyclohexane/EtOAc = 3:7 + 1 % AcOH) afforded the title compound (24.0 mg, 0.0398 mmol, 75.1 %), which were separated by preparative HPLC in pure diastereoisomers.
**$R_f$** (C2a): 0.3 (Cyclohexane/EtOAc = 3:7 + 1 % AcOH)
**$R_f$** (C2b): 0.3 (Cyclohexane/EtOAc = 3:7 + 1 % AcOH)
**MS** (C2a) (ESI): *m/z* (%) = 603.02 $[M + H]^+$
**MS** (C2b) (ESI): m/z (%) = 603.11 $[M + H]^+$

**Example 3-3:** Preparation of (1*S*,5*S*,6*R*)-10-((3,5-dichlorophenyl)sulfonyl)-5-(1,2-dihydroxyethyl)-3-(2-ethoxyethyl)-3,10-diazabicyclo[4.3.1]decan-2-one **C3**

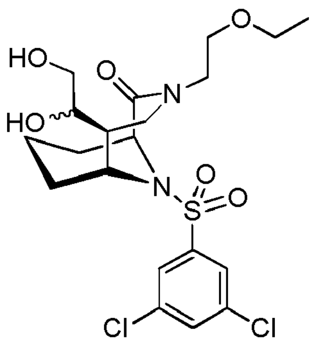
**C3**

To a solution of ((1*S*,5*R*,6*R*)-10-((3,5-dichlorophenyl)sulfonyl)-3-(2-ethoxyethyl)-5-vinyl-3,10-diazabicyclo[4.3.1]decan-2-one (30.0 mg, 0.0650 mmol) in THF/$H_2O$ (1.5 mL, 2:1) were added NMO (30.5 mg, 0,260 mmol) and $OsO_4$ (2.5 % solution in *tert*-butanol, 2.60 μmol, 33μL). After 1 h stirring at room temperature saturated sat. aq. NaCl solution (10 mL) was added to the reaction and extracted with $CH_2Cl_2$ (3 x 40 mL). The organic layer was dried over $MgSO_4$ and the solvent removed under reduced pressure. Column chromatography over $SiO_2$ (1 % AcOH in cyclohexane/EtOAc = 1:4) afforded the title compound (15.0 mg, 0.0303 mmol, 46.6 %).
**$R_f$:** 0.22 (Cyclohexane/EtOAc = 1:4 + 1 % AcOH)
**MS** (ESI): m/z (%) = 495.29 $[M + H]^+$

**IV. Preparation of compounds D1 - D8**

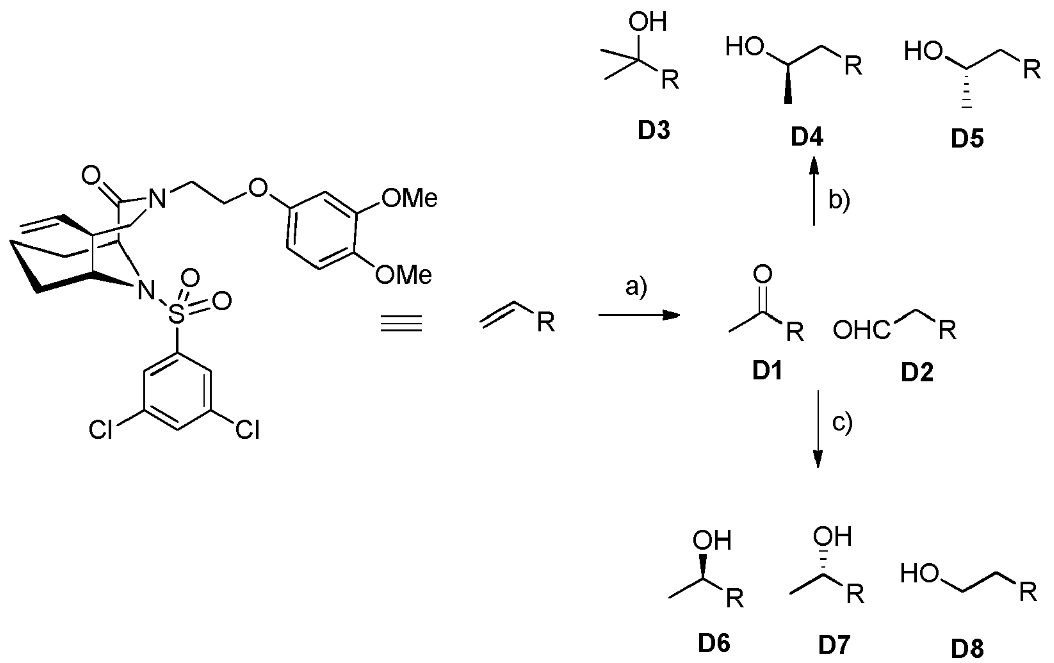

D3 D4 D5 D1 D2 D6 D7 D8

**Scheme D.** Reagents and conditions: a) $PdCl_2$, CuCl, $O_2$, DMF/$H_2O$, rt, 24 h, 72 %; b) MeMgBr, THF, −78 °C, 1 h, 75 %; c) $NaBH_4$, EtOH/$CH_2Cl_2$, rt, 1 h, 68 %.

**Example 4-1:** Preparation of (1S,5S,6R)-5-acetyl-10-((3,5-dichlorophenyl)sulfonyl)-3-(2-(3,4-dimethoxyphenoxy)ethyl)-3,10-diazabicyclo[4.3.1]decan-2-one D1 and 2-((1S,5S,6R)-10-((3,5-dichlorophenyl)sulfonyl)-3-(2-(3,4-dimethoxyphenoxy)ethyl)-2-oxo-3,10-diazabicyclo[4.3.1]decan-5-yl)acetaldehyde **D2**

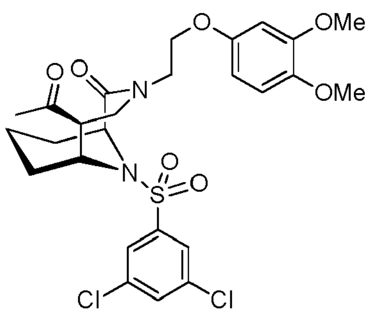

**D1**

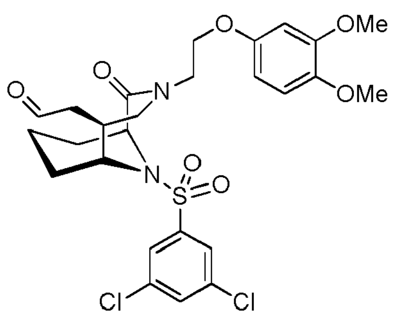

**D2**

To a solution of (1S,5R,6R)-10-((3,5-dichlorophenyl)sulfonyl)-3-(2-(3,4-dimethoxy-phenoxy)ethyl)-5-vinyl-3,10-diazabicyclo[4.3.1]decan-2-one (25.0 mg, 0.0440 mmol) in DMF/$H_2O$ (0.229 mL, 7:1) was added $PdCl_2$ (1.57 mg, 8.78 μmol) and CuCl (4.35 mg, 0.440 mmol). After 24 h days stirring $Et_2O$ (90 mL) was added to the reaction and washed three times with saturated $Na_2S_2O_3$ (3 x 10 mL). The organic layer was dried over $MgSO_4$ and the solvent removed under reduced pressure. Column chromatography on $SiO_2$ (EtOAc/Cyclohexane = 1:1) afforded an inseparable mixture of the title compounds (18.0 mg, 0.0307 mmol, 69.8 %) as a white solid.

$\mathbf{R_f}$: 0.36 (EtOAc/Cyclohexane = 1:1)

**MS D1:** (ESI): *m/z* (%) = 585.09 $[M + H]^+$

**MS D2:** (ESI): *m/z* (%) = 585.10 $[M + H]^+$

**Example 4-2:** Preparation of (1*S*,5*S*,6*R*)-10-((3,5-dichlorophenyl)sulfonyl)-3-(2-(3,4-dimethoxyphenoxy)ethyl)-5-(2-hydroxypropan-2-yl)-3,10-diazabicyclo[4.3.1]decan-2-one **D3,** (1*S*,5*S*,6*R*)-10-((3,5-dichlorophenyl)sulfonyl)-3-(2-(3,4-dimethoxyphenoxy) ethyl)-5-(2-hydroxypropyl)-3,10-diazabicyclo[4.3.1]decan-2-one D4, and (1S,5S,6R)-10-((3,5-dichlorophenyl)sulfonyl)-3-(2-(3,4-dimethoxyphenoxy)ethyl)-5-(2-hydroxypropyl)-3,10-diazabicyclo[4.3.1]decan-2-one D5

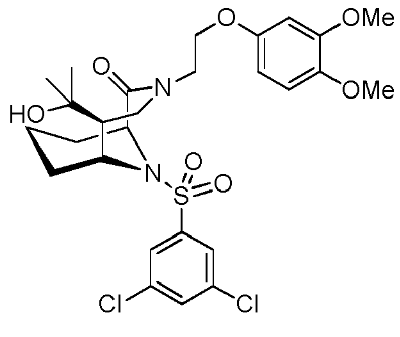

**D3**

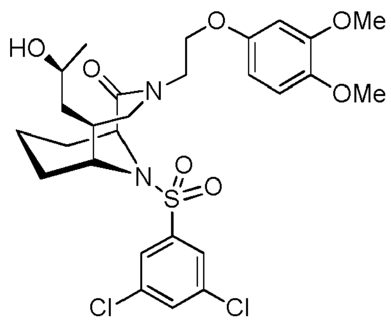

**D4**

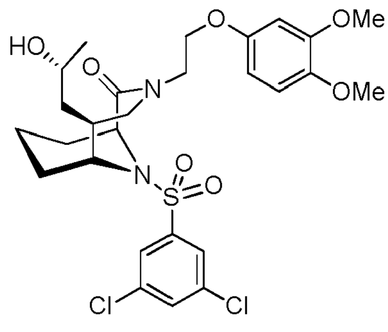

**D5**

A solution of an inseparable mixture of 2-((1*S*,5*S*,6*R*)-10-((3,5-dichlorophenyl)sulfonyl)-3-(2-(3,4-dimethoxyphenoxy)ethyl)-2-oxo-3,10-diazabicyclo [4.3.1]decan-5-yl)acetaldehyde and (1*S*,5*S*,6*R*)-5-acetyl-10-((3,5-dichlorophenyl) sulfonyl)-3-(2-(3,4-dimethoxyphenoxy)ethyl)-3,10-diazabicyclo[4.3.1] decan-2-one (48.0 mg, 0.0820 mmol) in THF (1 mL) was cooled to -78 °C. MeMgBr (3 M in $Et_2O$, 0.123mmol, 41.0 μL) was added and the reaction was stirred for 1 h. Sat. aq. $NH_4Cl$ (15 mL) solution was added to the reaction and extracted with $CH_2Cl_2$ (3 × 90 mL). The organic layer was dried over $MgSO_4$ and the solvent was evaporated under reduced pressure. Flash column chromatography on $SiO_2$ (20-50% EtOAc in cyclohexane) afforded the title compound **(D3:** 23.0 mg, 0.0382 mmol, **D4:** 6 mg, 0.00998 mmol, **D5:** 8 mg, 0.0133 mmol, 75.0%).

**D3:**

**$^1$H-NMR:** (600MHz, Chloroform-*d*) δ = 1.25 (s, 3 H), 1.27 (s, 3 H), 1.44 - 1.52 (m, 2 H), 1.57 - 1.62 (m, 3 H), 2.15 (ddd, J = 10.5, 6.7, 1.5 Hz, 1 H), 2.23 (d, J = 13.4 Hz, 1 H), 3.41 (dd, J = 14.2, 1.4 Hz, 1 H), 3.51 - 3.59 (m, 1 H), 3.83 (s, 3 H), 3.84 (s, 3 H), 3.98 - 4.07 (m, 2 H), 4.13 - 4.18 (m, 2 H), 4.25 - 4.30 (m, 1 H), 4.69 (d, J = 5.7 Hz, 1 H), 6.40 (dd, J = 8.8, 2.8 Hz, 1 H), 6.54 (d, J = 2.8 Hz, 1 H), 6.77 (d, J = 8.8 Hz, 1 H), 7.54 (t, J = 1.9 Hz, 1 H), 7.72 (d, J = 1.9 Hz, 2 H).

**$^{13}$C-NMR:** (150 MHz, Chloroform-*d*) δ = 15.33, 27.42, 27.90, 28.11, 29.68, 50.22, 51.73, 51.96, 54.16, 55.84, 56.40, 57.19, 67.13, 72.50, 100.8, 104.2, 111.9, 125.0, 132.7, 136.3, 143.7, 143.9, 149.8, 153.1, 169.8.

**MS** (ESI): m/z (%) = 601.28 $[M + H]^+$

**D4:**

**$^1$H-NMR:** (600 MHz, Chloroform-*d*) δ = 1.23 - 1.29 (m, 5 H), 1.49 - 1.66 (m, 5 H), 2.25 (d, J = 12.3 Hz, 1 H), 2.33 - 2.41 (m, 1 H), 3.31 (dd, J = 14.4, 1.7 Hz, 1 H), 3.52 - 3.60 (m, 1 H), 3.82 - 3.87 (m, 7 H), 3.93 - 4.09 (m, 4 H), 4.14 - 4.20 (m, 1 H), 4.68 (d, J = 5.9 Hz, 1 H), 6.42 (dd, J = 8.7, 2.8 Hz, 1 H), 6.56 (d, J = 2.8 Hz, 1 H), 6.77 (d, J = 8.8 Hz, 1 H), 7.54 (t, J = 1.8 Hz, 1 H), 7.69 (d, J = 1.9 Hz, 2 H).

**$^{13}$C-NMR:** (151 MHz, Chloroform-*d*) δ = 15.46, 24.98, 29.68, 41.55, 43.10, 51.77, 54.00, 55.58, 55.89, 56.39, 56.91, 65.69, 67.31, 100.8, 104.1, 111.8, 124.8, 132.6, 136.3, 143.8, 144.1, 149.8, 153.0, 170.0.

**MS** (ESI): m/z (%) = 601.28 $[M + H]^+$

**D5:**

**$^1$H-NMR:** (600 MHz, Chloroform-*d*) δ = 1.21 - 1.27 (m, 5 H), 1.45 - 1.62 (m, 5 H), 2.22 - 2.29 (m, 1 H), 2.35 - 2.41 (m, 1 H), 3.29 (dd, J = 14.4, 1.8 Hz, 1 H), 3.49 - 3.56 (m, 1 H), 3.81 - 3.88 (m, 7 H), 3.93 - 4.02 (m, 2 H), 4.03 - 4.08 (m, 1 H), 4.13 - 4.24 (m, 2 H), 4.69 (dt, J = 6.3, 1.9 Hz, 1 H), 6.43 (dd, J = 8.7, 2.8 Hz, 1 H), 6.57 (d, J = 2.8 Hz, 1 H), 6.78 (d, J = 8.8 Hz, 1 H), 7.54 (t, J = 1.8 Hz, 1 H), 7.69 (dd, J = 1.9, 0.4 Hz, 2 H).

**$^{13}$C-NMR:** (150 MHz, Chloroform-*d*) δ = 15.50, 24.49, 26.71, 26.90, 27.71, 29.68, 40.96, 42.63, 51.73, 52.46, 55.88, 56.38, 56.80, 64.95, 67.97, 100.8, 104.4, 111.9, 124.8, 132.6, 136.3, 143.9, 144.1, 149.9, 153.1, 170.3.

**MS** (ESI): m/z (%) = 601.32 $[M + H]^+$

**Example 4-3:** Preparation of (1*S*,5*S*,6*R*)-10-((3,5-dichlorophenyl)suifonyl)-3-(2-(3,4-dimethoxyphenoxy)ethyl)-5-((R)-1-hydroxyethyl)-3,10-diazabicyclo[4.3.1]decan-2-one **D6,** (1*S*,5*S*,6*R*)-10-((3,5-dichlorophenyl)sulfonyl)-3-(2-(3,4-dimethoxyphenoxy)ethyl)-5-((R)-1-hydroxyethyl)-3,10-diazabicyclo[4.3.1]decan-2-one **D7,** and (1S,5S,6R)-10-((3,5-dichlorophenyl)sulfonyl)-3-(2-(3,4-dimethoxyphenoxy)ethyl)-5-(2-hydroxyethyl)-3,10-diazabicyclo[4.3.1]decan-2-one **D8**

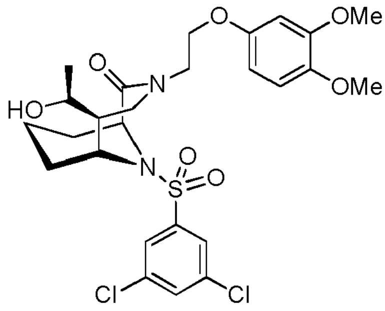

**D6**

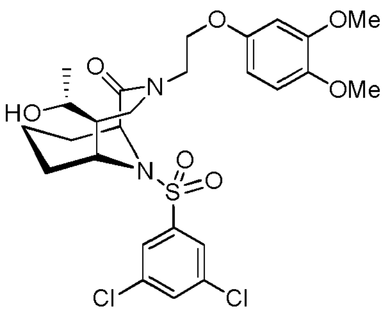

**D7**

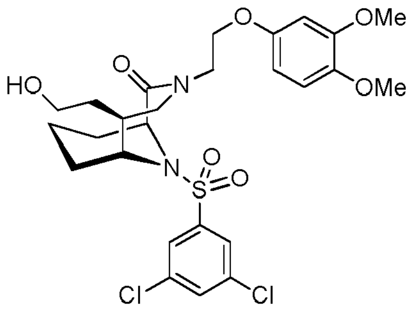

**D8**

To a solution of an inseparable mixture of 2-((1*S*,5*S*,6*R*)-10-((3,5-dichlorophenyl)sulfonyl)-3-(2-(3,4-dimethoxyphenoxy)ethyl)-2-oxo-3,10 diazabicyclo [4.3.1]decan-5-yl)acetaldehyde and (1*S*,5*S*,6*R*)-5-acetyl-10-((3,5-dichlorophenyl) sulfonyl)-3-(2-(3,4-dimethoxyphenoxy)ethyl)-3,10 diazabicyclo[4.3.1]decan-2-one (90.0 mg, 0.154 mmol) in EtOH (1.5 mL) were added of $NaBH_4$ (5.82 mg, 0.154 mmol) and then stirred for 15 minutes at room temperature. EtOAc (90 mL) was added to the reaction and washed with sat. aq. NaCl solution (3 $\times$ 10 mL). The organic layer was dried over $MgSO_4$ and the solvent was evaporated under reduced pressure. Flash column chromatography on $SiO_2$ (20-50% EtOAc in cyclohexane) afforded the title compound **(D6:** 20.0 mg, 0.0340 mmol, **D7:** 12.0 mg, 0.0204 mmol, **D8:** 29.0 mg, 0.0494 mmol, 67.7 %).

**D6:**

$\mathbf{R_f}$: 0.53 (Cyclohexane/EtOAc = 3:7)

**$^1$H-NMR:** (600MHz, Chloroform-d) $\delta$ = 1.29 (s, 3 H), 1.37 - 1.55 (m, 5 H), 2.11 - 2.18 (m, 1 H), 2.26 (d, J = 13.4 Hz, 1 H), 3.45 - 3.49 (m, 1 H), 3.52 - 3.58 (m, 1 H), 3.83 (s, 3 H), 3.84 (s, 3 H), 3.86 - 3.91 (m, 1 H), 3.95 (dd, J = 14.4, 10.6 Hz, 1 H), 4.01 - 4.06 (m, 1 H), 4.13 - 4.17 (m, 3 H), 4.70 (d, J = 5.7 Hz, 1 H), 6.39 (dd, J = 8.7, 2.8 Hz, 1 H), 6.54 (d, J = 2.8 Hz, 1 H), 6.77 (d, J = 8.8 Hz, 1 H), 7.53 (t, J = 1.9 Hz, 1 H), 7.69 - 7.72 (m, 2 H).

**$^{13}$C-NMR:** (150 MHz, Chloroform-*d*) $\delta$ = 15.31, 21.09, 28.19, 28.67, 29.67, 49.68, 50.63, 51.67, 52.48, 55.85, 56.40, 57.00, 67.20, 68.91, 100.6, 103.9, 111.8, 124.9, 132.7, 136.3, 143.7, 143.9, 149.8, 153.1, 169.9.

**MS** (ESI): m/z (%) = 587.16 $[M + H]^+$

**D7:**

$\mathbf{R_f}$: 0.5 (Cyclohexane/EtOAc = 3:7)

**$^1$H-NMR:** (600 MHz, Chloroform-*d*) $\delta$ = 1.28 (s, 3 H), 1.34 - 1.54 (m, 5 H), 2.21 (s, 1 H), 2.26 (d, J = 13.7 Hz, 1 H), 3.33 - 3.39 (m, 1 H), 3.51 - 3.58 (m, 1 H), 3.83 (s, 3 H), 3.85 (s, 3 H), 3.84 - 3.90 (m, 1 H), 4.04 (dd, J = 14.1, 3.3 Hz, 2 H), 4.11 - 4.20 (m, 3 H), 4.70 (d, J = 5.7 Hz, 1 H), 6.40 (dd, J = 8.7, 2.8 Hz, 1 H), 6.54 (d, J = 2.8 Hz, 1 H), 6.78 (d, J = 8.8 Hz, 1 H), 7.53 (t, J = 1.9 Hz, 1 H), 7.71 (d, J = 1.8 Hz, 2 H).

**$^{13}$C-NMR:** (150 MHz, Chloroform-*d*) $\delta$ = 15.40, 20.25, 28.13, 28.82, 29.68, 50.44, 50.74, 51.56, 51.66, 55.85, 56.41, 57.01, 67.23, 69.12, 100.5, 103.9, 111.9, 124.9, 124.9, 132.7, 136.3, 143.7, 143.9, 149.9, 153.1, 169.9.

**MS** (ESI): *m/z* (%) = 587.16 $[M + H]^+$

**D8:**

$\mathbf{R_f}$: 0.37 (Cyclohexane/EtOAc = 3:7)

**$^1$H-NMR:** (600 MHz, Chloroform-*d*) $\delta$ = 1.42 - 1.56 (m, 7 H), 2.26 (d, J = 13.6 Hz, 1 H), 2.32 - 2.36 (m, 1 H), 3.29 (d, J = 12.6 Hz, 1 H), 3.50 - 3.56 (m, 1 H), 3.73 - 3.78 (m, 2 H), 3.83 (s, 3 H), 3.86 (s, 3 H), 3.94 (s, 1 H), 4.02 - 4.08 (m, 1 H), 4.13 - 4.21 (m, 3 H), 4.69 (d, J = 5.9 Hz, 1 H), 6.42 (dd, J = 8.8, 2.8 Hz, 1 H), 6.55 (d, J = 2.8 Hz, 1 H), 6.78 (d, J = 8.8 Hz, 1 H), 7.55 (t, J = 1.8 Hz, 1 H), 7.69 (dd, J = 1.9, 0.6 Hz, 2 H).

**$^{13}$C-NMR:** (75 MHz, Chloroform-*d*) $\delta$ = 15.44, 27.35, 27.84, 29.67, 36.10, 41.09, 51.73, 53.19, 55.42, 55.89, 56.40, 56.85, 59.86, 67.62, 100.7, 104.2, 111.9, 124.8, 132.6, 136.3, 143.8, 144.1, 149.9, 153.1, 170.1.

**MS** (ESI): m/z (%) = 587.15 $[M + H]^+$

**V. Preparation of Compounds G4-G6**

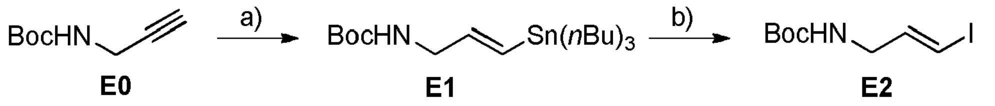


**Scheme E.** Reagents and conditions: a) $(nBu)_3SnH$, AIBN, toluene, 120 °C, 2.5 h, 63 %; b) $I_2$, $CH_2Cl_2$, rt, 1 h, 97 %.

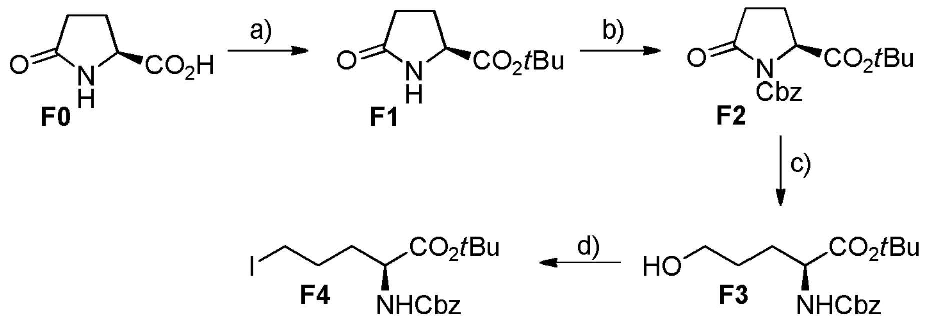


**Scheme F.** Reagents and conditions: a) *t*BuOAc, $HClO_4$, rt, 24 h, 74 %; b) NaH, CbzCl, THF, rt, 20 h, 90 %; c) $NaBH_4$, $KH_2PO_4$, $MeOH/H_2O$, rt, 2 h, 66 %; d) $I_2$, $PPh_3$, imidazole, $CH_2Cl_2$, rt, 1 h, 86 %.

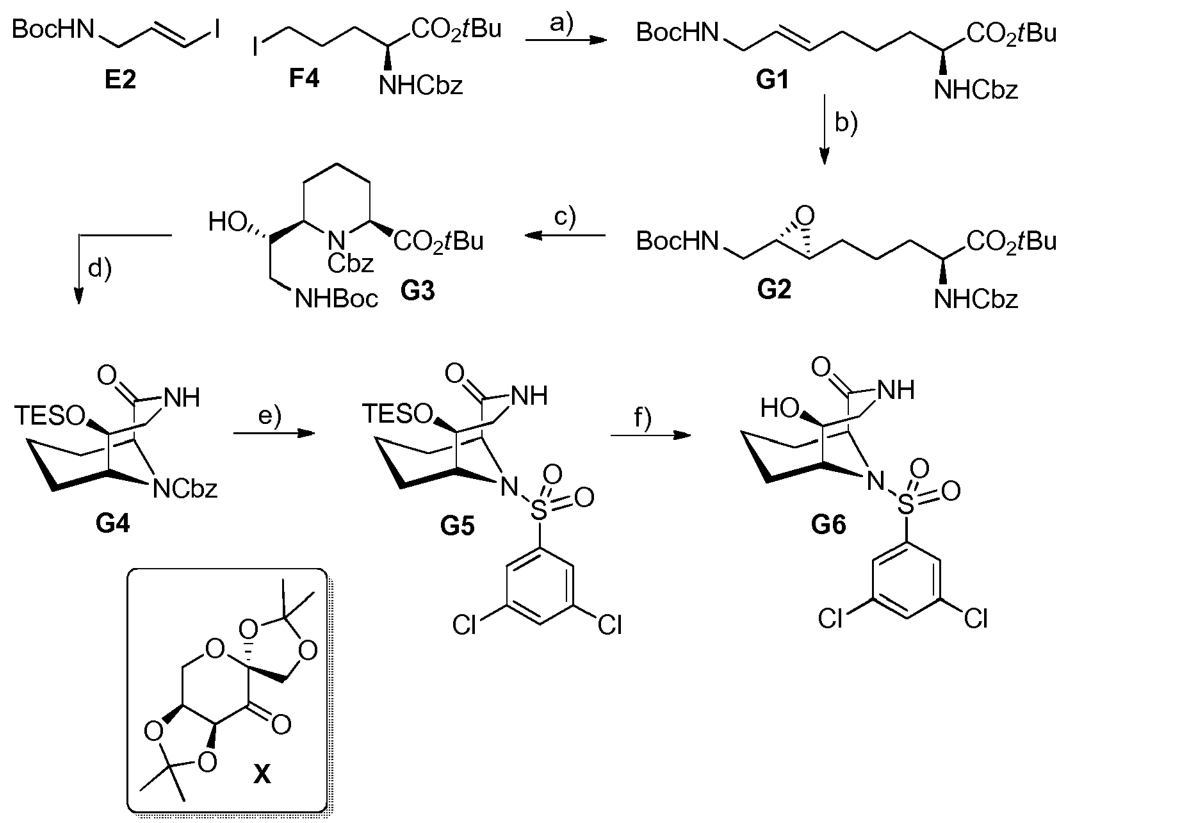


**Scheme G.** Reagents and conditions: a) 1.) Zn*, DMF, 35 °C, 1 h; 2.) $Pd_2(dba)_3$, $P(otol)_3$, rt, 16 h, 90 %; b) **X**, Oxone, $K_2CO_3$, $n Bu_4NHSO_4$, DMM/MeCN, aq. $Na_2(EDTA)$, aq. $Na_2B_4O_7$, rt, 18 h, 71 %, *dr* 8:1; c) 1.) Pd/C, $H_2$, EtOH, rt, 2 h; 2.) CbzCl, $Na_2CO_3$, dioxane/$H_2O$, rt, 18 h, 83 % (2 steps); d) 1.) TESOTf, 2,6-lutidine, $CH_2Cl_2$, 0 °C to rt, 20 h; 2.) HATU, $(iPr)_2NEt$, $CH_2Cl_2$ ($c = 5 \cdot 10^{-3}$ M), rt, 16 h, 50 % (2 steps); e) 1.) Pd/C, $H_2$, EtOAc, rt, 2 h; 2.) 3,5-Dichlorobenzenesulfonyl chloride, DMAP, $(iPr)_2NEt$, $CH_2Cl_2$, rt, 20 h, 73 % (2 steps); f) TBAF, THF, rt, 1.5 h, 96 %.

**Example 5-1:** Preparation of (E)-N-Boc-3-tributylstannyl-2-propen-1-amine **E1**

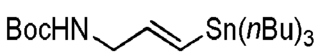

**E1**

To a solution of N-Boc-propargylamine (818 mg, 5.27 mmol) in toluene (26 mL) was added AIBN (0.2 M in toluene, 1.32 mL, $2.64 \cdot 10^{-5}$ mol, 0.05 equiv.) and tributyltin hydride (1.56 mL, 5.80 mmol, 1.1 equiv.) and it was stirred for 2.5 h at 120 °C (preheated oil bath). After cooling to room temperature, the solvent was evaporated under reduced pressure. Column chromatography on $SiO_2$ (cyclohexane/EtOAc 60:1) afforded the title compound (1.48 g, 3.32 mmol, 63 %) as colorless liquid.

**$R_f$:** 0.42 (Cyclohexane/EtOAc 20:1, $KMnO_4$)

**$^1$H-NMR** (600 MHz, $CDCl_3$): $\delta$ = 0.85-0.90 (m, 15 H, 3 $\times$ $CH_2$, 3 $\times$ $CH_3$), 1.25-1.33 (m, 6 H, 3 $\times$ $CH_2$), 1.45 (s, 9 H, 3 $\times$ $CH_3$), 1.46-1.51 (m, 6 H, 3 $\times$ $CH_2$), 3.78 ($s_{br}$, 1 H, $CH_2$), 4.60 ($s_{br}$, 1 H, NH), 5.95 (dt, $J$ = 19.2, 4.8 Hz, 1 H, CH), 6.08 (dt, $J$ = 19.2, 1.8 Hz, 1 H, CH).

**$^{13}$C-NMR** (150 MHz, $CDCl_3$): $\delta$ = 9.65, 13.90, 27.48, 28.62, 29.27, 46.21, 79.45, 129.3, 144.5, 156.0.

**Example 5-2:** Preparation of (*E*)-N-Boc-3-iodo-2-propen-1-amine E2

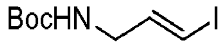

**E2**

To a solution of **E1** (6.96 g, 15.6 mmol) in $CH_2Cl_2$ (160 mL) was added Iodine (4.16 g, 16.4 mmol, 1.05 equiv.) and the resulting suspension was stirred for 2 h at room temperature. The reaction mixture was washed with 1 M $Na_2S_2O_3$ solution (150 mL), dried over $MgSO_4$, filtered and the solvent was evaporated under reduced pressure. Column chromatography on $SiO_2$ (cyclohexane/EtOAc 10:1) afforded the title compound (4.28 g, 15.1 mmol, 97 %) as slightly yellow liquid, which solidified upon cooling to 4 °C.
**$R_f$:** 0.17 (Cyclohexane/EtOAc 20:1, $KMnO_4$)
**$^1$H-NMR** (400 MHz, $CDCl_3$): $\delta$ = 1.44 (s, 9 H, 3 $\times$ $CH_3$), 3.65-3.73 (m, 2 H, $CH_2$), 4.67 ($s_{br}$, 1 H, NH), 6.27 (dt, *J* = 14.4, 1.6 Hz, 1 H, CH), 6.53 (dt, *J* = 14.4, 4.0 Hz, 1 H, CH).
**$^{13}$C-NMR** (100 MHz, $CDCl_3$): $\delta$ = 28.56, 44.97, 77.95, 79.99, 142.6, 155.6.

**Example 5-3:** Preparation of (2*S*)-*tert*-Butyl-pyroglutamate F1

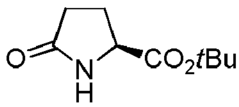

**F1**

To a suspension of L-Pyroglutamic acid (6.46 g, 50.0 mmol) and tBuOAc (100 mL) in a pressure flask was added $HClO_4$ (70 %, 1.51 mL 0.35 equiv.) and the solution was stirred for 24 h at room temperature. It was poured into a sat. aq. $NaHCO_3$ solution (200 mL), the organic phase was separated and the aqueous phase was extracted with $CH_2Cl_2$ (2 $\times$ 100 mL). The combined organic phases were dried over $MgSO_4$, filtered and the solvent was evaporated under reduced pressure to afford the title compound (6.87 g, 37.1 mmol, 74 %) as a colorless solid.
**$R_f$:** 0.41 (EtOAc, $KMnO_4$)
**$^1$H-NMR** (300 MHz, $CDCl_3$): $\delta$ = 1.44 (s, 9 H, 3 $\times$ $CH_3$), 2.12-2.15 (m, 4 H, 2 $\times$ $CH_2$), 4.08-4.16 (m, 1 H, CH), 6.11 ($s_{br}$, 1 H, NH).
**$^{13}$C-NMR** (75.5 MHz, $CDCl_3$): $\delta$ = 25.08, 28.18, 29.58, 56.25, 82.62, 171.2, 177.9.

**Example 5-4:** Preparation of (2*S*)-*N*-Cbz-*tert*-Butyl-pyroglutamate **F2**

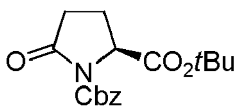

**F2**

To a solution of **F1** (2.29 g, 12.4 mmol) in THF (62 mL) was added NaH (60 % oil dispersion, 546 mg, 13.6 mmol, 1.1 equiv.) and it was stirred for 30 min at room temperature. Subsequently Benzyl chloroformate (1.94 mL, 13.6 mmol, 1.1 equiv.) was added and stirring was continued for further 20 h. The solvent was evaporated under reduced pressure, sat. aq. $NH_4Cl$ solution (100 mL) was added and extrated with EtOAc (2 $\times$ 100 mL). The organic phase was dried over $MgSO_4$, filtered and the the solvent was evaporated under reduced pressure. Column chromatography on $SiO_2$ (cyclohexane/EtOAc 3:1) afforded the title compound (3.56 g, 11.1 mmol, 90 %) as colorless oil.
**$R_f$:** 0.18 (Cyclohexane/EtOAc 3:1, $KMnO_4$)
**$^1$H-NMR** (300 MHz, $CDCl_3$): $\delta$ = 1.39 (s, 9 H, 3 $\times$ $CH_3$), 1.97-2.70 (m, 4 H, 2 $\times$ $CH_2$), 4.54 (dd, J = 9.3, 2.7 Hz, 1 H, CH), 5.27 (dd, J = 16.2, 12.3 Hz, 2 H, $CH_2$), 7.27-7.43 (m, 5 H, 5 $\times$ Ar-H).
**$^{13}$C-NMR** (75.5 MHz, $CDCl_3$): $\delta$ = 25.11, 27.12, 31.23, 59.60, 68.43, 82.76, 128.4, 128.6, 128.7, 135.3, 151.1, 170.3, 173.3.

**Example 5-5:** Preparation of *tert*-Butyl-(S)-2-(benzyloxycarbonylamino)-5-hydroxypentanoate F3

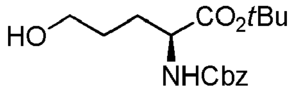

**F3**

To a suspension of **F2** (2.15 g, 6.73 mmol) and $KH_2PO_4$ (6.87 g, 50.5 mmol, 7.5 equiv.) in MeOH/$H_2O$ (5:1, 67 mL) was added $NaBH_4$ (1.91 g, 50.5 mmol, 7.5 equiv.) in portions. After stirring for 1 h the mixture was filtered and the solvent was evaporated under reduced pressure. Column chromatography on $SiO_2$ (cyclohexane/EtOAc 2:1) afforded the title compound (1.43 g, 4.42 mmol, 66 %) as colorless oil.
**$R_f$:** 0.29 (Cyclohexane/EtOAc 2:1, $KMnO_4$)
**$^1$H-NMR** (300 MHz, DMSO-$d_6$): $\delta$ = 1.39 (s, 9 H, 3 $\times$ $CH_3$), 1.41-1.91 (m, 4 H, 2 $\times$ $CH_2$), 3.38 (dd, *J* = 6.3, 1.8 Hz, 2 H, $CH_2$), 3.82-3.92 (m, 1 H, CH), 5.04 (d, *J* = 2.4 Hz, 2 H, $CH_2$), 7.28-7.40 (m, 5 H, 5 $\times$ Ar-H), 7.58 ($d_{br}$, *J* = 7.8 Hz, 1 H, NH). **$^{13}$C-NMR** (75.5 MHz, DMSO-$d_6$): $\delta$ = 27.62, 28.83, 54.48, 60.10, 65.32, 80.32, 127.7, 127.8, 128.3, 156.1, 171.6. **MS** (ESI): *m*/*z* (%) = 267.9 (16) $[M-tBu+H]^+$, 324.0 (36) $[M+H]^+$, 346.2 (10) $[M+Na]^+$, 646.9 (100) $[2M+H]^+$, 669.0 (28) $[2M+Na]^+$.

**Example 5-6:** Preparation of *tert*-Butyl-(S)-2-(benzyloxycarbonylamino)-5-iodopentanoate **F4**

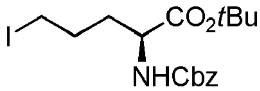

**F4**

To a suspension of $PPh_3$ (2.30 g, 8.78 mmol, 2.0 equiv.), $I_2$ (2.23 g, 8.78 mmol, 2.0 equiv.) and imidazole (897 mg, 13.2 mmol, 3.0 equiv.) in $CH_2Cl_2$ (44 mL) was added **F3** (1.42 g, 4.39 mmol) and it was stirred for 1 h at room temperautre. $H_2O$ was added (30 mL), the organic phase separated, washed with aq. 1 M $Na_2S_2O_3$ solution (30 mL), dried over $MgSO_4$ and the solvent was evaporated under reduced pressure. Column chromatography on $SiO_2$ ($CH_2Cl_2$) afforded the title compound (1.64 g, 3.79 mmol, 86 %) as slightly yellow oil, which solidified upon cooling to 4 °C.
**$R_f$:** 0.35 (Cyclohexane/EtOAc 8:1, $KMnO_4$)
**$^1$H-NMR** (300 MHz, $CDCl_3$): $\delta$ = 1.47 (s, 9 H, 3 $\times$ $CH_3$), 1.69-2.00 (m, 4 H, 2 $\times$ $CH_2$), 3.08-3.28 (m, 2 H, $CH_2$), 4.24-4.33 (m, 1 H, CH), 5.11 (s, 2 H, $CH_2$), 5.33 ($d_{br}$, *J* = 7.5 Hz, 1 H, NH), 7.28-7.38 (m, 5 H, 5 $\times$ Ar-H).
**$^{13}$C-NMR** (75.5 MHz, $CDCl_3$): $\delta$ = 5.82, 28.22, 29.20, 34.00, 53.68, 67.18, 82.70, 128.3, 128.4, 128.8, 136.5, 156.0, 171.3.
***MS*** (ESI): m/z (%) = 377.8 (74) $[M-tBu+H]^+$, 433.7 (74) $[M+H]^+$, 456.1 (12) $[M+Na]^+$, 810.6 (12) $[2M-tBu+H]^+$, 866.6 (12) $[2M+H]^+$.

***Example* 5-7:** Preparation of (S,E)-tert-Butyl 2-(((benzyloxy)carbonyl)amino)-8-((tert-butoxycarbonyl)amino)oct-6-enoate ***G1***

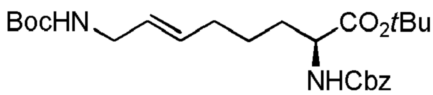

**G1**

To a suspension of Zn powder (1.57 g, 24.0 mmol, 6.0 equiv.) in DMF (1.8 mL) was added 1,2-Dibromomethane (103 $\mu$L, 1.20 mmol, 0.3 equiv.) and it was stirred for 0.5 h at 60 °C. After cooling to room temperature, TMSCl (30 $\mu$L, 0.240 mmol, 0.06 equiv.) was added and stirring was continued for further 0.5 h. Then **F4** (1.73 g, 4.00 mmol, 1.0 equiv.) was added and the reaction mixture was stirred for 0.5 h. $Pd_2(dba)_3$ (73 mg, 0.080 mmol, 0.02 equiv), P(*o*tol)$_3$ (97 mg, 0.320 mmol, 0.08 equiv.), and **E2** (849 mg, 3.00 mmol, 0.75 equiv.) in DMF (1.8 mL), were added and it was stirred for 16 h at room temperature. The resulting green suspension was poured into EtOAc (150 mL) and the organic phase was washed with sat. aq. NaCl solution (3 $\times$ 50 mL), dried over $MgSO_4$ and the solvent was evaporated under reduced

pressure. Column chromatography on $SiO_2$ (cyclohexane/EtOAc 8:1 → 4:1) afforded the title compound (1.25 g, 2.70 mmol, 90 %) as yellow oil.
**$R_f$:** 0.23 (Cyclohexane/EtOAc 4:1, $KMnO_4$)
**$^1$H-NMR** (400 MHz, $CDCl_3$): δ = 1.44 (s, 9 H, 3 × $CH_3$), 1.45 (s, 9 H, 3 × $CH_3$), 1.54-1.86 (m, 4 H, 2 × $CH_2$), 1.95-2.10 (m, 2 H, $CH_2$), 3.62-3.72 (m, 2 H, $CH_2$), 4.20-4.28 ($m_c$, 1 H, CH), 4.57 ($s_{br}$, 1 H, NH), 5.10 (s, 2 H, $CH_2$), 5.29 ($d_{br}$, *J* = 7.6 Hz, 1 H, NH), 5.39-5.48 (m, 1 H, CH), 5.49-5.58 (m, 1 H, CH), 7.28-7.38 (m, 5 H, 5 × Ar-H).
**$^{13}$C-NMR** (100 MHz, $CDCl_3$): δ = 24.67, 28.20, 28.61, 31.80, 32.49, 42.74, 54.34, 67.04, 82.21, 127.4, 128.3, 128.3, 128.7, 128.7, 132.1, 136.6, 155.9, 156.0, 171.7.
**MS** (ESI): m/z (%) = 307.3 (28) $[M\text{-}Boc\text{-}tBu+H]^+$, 363.2 (100) $[M\text{-}Boc+H]^+$, 463.0 (64) $[M+H]^+$, 485.2 (16) $[M+Na]^+$, 825.0 (34) $[2M\text{-}Boc+H]^+$.

**Example 5-8:** Preparation of (S)-tert-Butyl 2-(((benzyloxy)carbonyl)amino)-5-((2S,3S)-3-(((tert-butoxycarbonyl)amino)methyl)oxiran-2-yl)pentanoate **G2**

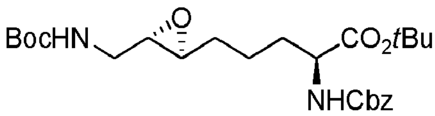

**G2**

To a solution of G1 (2.00 g, 4.32 mmol) in DMM/MeCN (60 mL, 2:1) was added buffer (40 mL, 0.05 M solution of $Na_2B_4O_7 \cdot 10\ H_2O$ in $4 \cdot 10^{-4}$ M aq. $Na_2(EDTA)$), $nBu_4NHSO_4$ (148 mg, 0.432 mmol, 0.1 equiv.) and L-fructose derived Shi ketone (X) (1.68 g, 2.16 mmol, 0.5 equiv.). Then, a solution of Oxone (3.98 g, 6.48 mmol, 1.5 equiv.) in aq. $Na_2(EDTA)$ (40 mL, $4 \cdot 10^{-4}$ M) and a solution of $K_2CO_3$ (2.39 g, 17.3 mmol, 4.0 equiv.) in $H_2O$ (40 mL) were added dropwise separately over a period of 1.5 h via addition funnels. The reaction was stirred for further 16 h at room temperature. The organic phase was separated and the aqueous phase was extracted with $CH_2Cl_2$ (2 × 200 mL). The combined organic phases were washed with 1 M aq. $Na_2S_2O_3$ solution (100 mL), dried over $MgSO_4$, and the solvent was removed under reduced pressure. Column chromatography on $SiO_2$ (cyclohexane/EtOAc 5:1 → 3:1 + 0.1 $NEt_3$) afforded the title compound (1.46 g, 3.05 mmol, 71 %) as colorless oil.
**$R_f$:** 0.38 (Cyclohexane/EtOAc 2:1, $KMnO_4$)
**$^1$H-NMR** (300 MHz, $CDCl_3$): δ = 1.42 (s, 9 H, 3 × $CH_3$), 1.45 (s, 9 H, 3 × $CH_3$), 1.48-1.92 (m, 6 H, 3 × $CH_2$), 2.70-2.79 (m, 1 H, $CH_A$), 2.79-2.85 (m, 1 H, $CH_B$), 3.17 (ddd, J = 14.8, 6.4, 5.2 Hz, 1 H, CH), 3.39-3.53 (m, 1 H, CH), 4.24 ($m_c$, 1 H, CH), 4.77 ($s_{br}$, 1 H, NH), 5.09 (s, 2 H, $CH_2$), 5.37 ($d_{br}$, *J* = 8.1 Hz, 1 H, NH), 7.28-7.38 (m, 5 H, 5 × Ar-H).
**$^{13}$C-NMR** (75.5 MHz, $CDCl_3$): δ = 21.78, 28.17, 28.52, 31.18, 32.64, 41.59, 54.39, 56.51, 57.05, 67.05, 79.77, 82.31, 128.3, 128.3, 128.7, 128.7, 136.5, 156.0, 171.6.
**MS** (ESI): m/z (%) = 323.2 (28) $[M\text{-}Boc\text{-}tBu+H]^+$, 367.1 (58) $[M\text{-}Boc\text{-}tBu\text{-}H+2Na]^+$, 423.0 (100) $[M\text{-}tBu+H]^+$, 479.0 (20) $[M+H]^+$, 501.1 (14) $[M+Na]^+$, 957.0 (12) $[2M+H]^+$.

**Example 5-9:** Preparation of (2S,6R)-1-benzyl 2-tert-butyl 6-((S)-2-((tert-butoxy-carbonyl)amino)-1-hydroxyethyl)piperidine-1,2-dicarboxylate **G3**

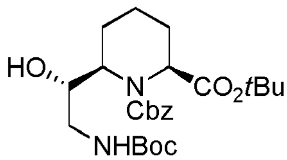

**G3**

A suspension of **G2** (1.45 g, 3.03 mmol) and Pd/C (145 mg, 10 wt. %) in EtOH (30 mL) was stirred for 2 h at room temperature under a $H_2$-atmosphere. The reaction mixture was filtered over Celite, washed with EtOAc and the solvent was evaporated under reduced pressure to give a colorless, crystalline solid.
This solid was dissolved in dioxane/$H_2O$ (30 mL, 2:1), $Na_2CO_3$ (963 mg, 9.09 mmol, 3.0 equiv.) and benzyl chloroformate (646 μL, 4.55 mmol, 1.5 equiv.) were added and it was stirred for 18 h at room temperature. $H_2O$ (50 mL) was added and it was extracted with $CH_2Cl_2$ (2 × 50 mL), dried over $MgSO_4$, and the solvent was removed under reduced pressure. Column chromatography on $SiO_2$ (cyclohexane/EtOAc 4:1) afforded the title compound (1.20 g, 2.51

mmol, 83 % over 2 steps) as colorless oil.
**$R_f$:** 0.50 (Cyclohexane/EtOAc 2:1, $KMnO_4$)
**$^1$H-NMR** (400 MHz, DMSO-$d_6$): $\delta$ = 1.35 ($s_{br}$, 9 H, 3 × $CH_3$), 1.37 (s, 9 H, 3 × $CH_3$), 1.40-1.68 (m, 4 H, 2 × $CH_2$), 1.96-2.12 (m, 2 H, $CH_2$), 2.82-2.92 (m, 1 H, $CH_A$), 3.28-3.36 (m, 1 H, $CH_B$), 3.55-3.63 (m, 1 H, CH), 3.96-4.04 (m, 1 H, CH), 4.61-4.67 (m, 1 H, CH), 4.76 ($d_{br}$, J = 5.2 Hz, 1 H, OH), 5.03 (d, J = 12.4 Hz, 1 H, $CH_A$), 5.15 (d, J = 12.4 Hz, 1 H, $CH_B$), 6.13 ($s_{br}$, 1 H, NH), 7.28-7.40 (m, 5 H, 5 × Ar-H).
**$^{13}$C-NMR** (100 MHz, DMSO-$d_6$): $\delta$ = 15.76, 22.96, 25.18, 27.35, 28.19, 53.51, 54.87, 66.64, 68.64, 77.53, 81.00, 126.4, 126.6, 127.3, 136.6, 142.5, 155.6, 171.3.
**MS** (ESI): m/z (%) = 323.2 (70) [M-Boc-*t*Bu+H]$^+$, 379.1 (74) [M-Boc+H]$^+$, 478.9 (26) [M+H]$^+$, 501.2 (42) [M+Na]$^+$, 801.0 (92) [2M-Boc-*t*Bu+H]$^+$, 856.9 (100) [2M-Boc+H]$^+$, 956.5 (6) [2M+H]$^+$, 978.8 (36) [2M+Na]$^+$.

**Example 5-10:** Preparation of (1S,5S,6R)-benzyl 2-oxo-5-((triethylsilyl)oxy)-3,10-diazabicyclo[4.3.1]decane-10-carboxylate **G4**

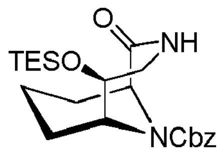

**G4**

To a solution of **G3** (500 mg, 1.04 mmol) and 2,6-lutidine (1.21 mL, 10.4 mmol, 10 equiv.) in $CH_2Cl_2$ (20 mL) at 0 °C was added TESOTf (1.17 mL, 5.20 mmol, 5.0 equiv.) dropwise. After 30 minutes the cooling bath was removed and the reaction was stirred for 20 h at room temperature. Sat. aq. $NH_4Cl$ solution (20 mL) was added and stirring was continued for 1 h. The organic phase was separated, the aqueous phase was extracted with $CH_2Cl_2$ (2 × 50 mL), the combined organic phases were dried over $MgSO_4$, and the solvent was evaporated under reduced pressure. The residue was purified by column chromatography on $SiO_2$ ($CH_2Cl_2$/MeOH 20:1 to 10:1).

The obtained amino acid was dissolved in $CH_2Cl_2$ (25 mL) and the solution was added dropwise at room temperature over 1 h to a solution of HATU (593 mg, 1.56 mmol, 1.5 equiv.) and (*i*Pr)$_2$NEt (400 $\mu$L, 2.23 mmol, 2.2 equiv.) in $CH_2Cl_2$ (200 mL). Stirring was continued for further 16 h, following evaporation of the solvent under reduced pressure. The residue was taken up in $CH_2Cl_2$ (100 mL), washed with $CuSO_4$ solution (10 wt. %, 3 × 100 mL), dried over $MgSO_4$, and the solvent was evaporated under reduced pressure. Column chromatography on $SiO_2$ (cyclohexane/EtOAc 2:1) afforded the title compound (218 mg, 0.521 mmol, 50 % over 2 steps) as a slightly yellow oil.

The compound consists of two carbamate rotamers in a 1:0.7 ratio.
**$R_f$:** 0.31 (Cyclohexane/EtOAc 1:1, $KMnO_4$) **$^1$H-NMR** (400 MHz, DMSO-$d_6$): $\delta$ = 0.58 (q, J = 7.6 Hz, 6 H, 3 × $CH_2$), 0.90 (t, J = 7.6 Hz, 5.4 H, 3 × $CH_3$), 0.91 (t, J = 7.6 Hz, 3.6 H, 3 × $CH_3$), 1.37-1.70 (m, 5 H, 2 × $CH_2$, CH), 2.00-2.11 (m, 1 H, CH), 2.70-2.79 (m, 1 H, $CH_A$), 2.95-3.07 (m, 1 H, $CH_B$), 3.83-3.93 (m, 1 H, CH), 4.24-4.32 (m, 1 H, CH), 4.61-4.66 (m, 1 H, CH), 5.05 (d, J = 12.8 Hz, 0.6 H, $CH_A$), 5.09 (d, J = 12.8 Hz, 0.4 H, $CH_A$), 5.17 (d, J = 12.8 Hz, 0.4 H, $CH_B$), 5.26 (d, J = 12.8 Hz, 0.6 H, $CH_B$), 7.29-7.41 (m, 5 H, 3 × Ar-H), 7.81-7.86 (m, 0.6 H, NH), 7.88-7.93 (m, 0.4 H, NH).
**$^{13}$C-NMR** (100 MHz, DMSO-$d_6$): $\delta$ = 4.20 (0.4 C), 4.24 (0.6 C), 6.62 (0.6 C), 6.64 (0.4 C), 15.95 (0.4 C), 16.01 (0.6 C), 26.30, 26.81 (0.4 C), 27.14 (0.6 C), 44.84 (0.4 C), 44.88 (0.6 C), 54.64 (0.6 C), 54.88 (0.4 C), 56.41 (0.4 C), 57.27 (0.6 C), 66.74 (0.6 C), 66.79 (0.4 C), 74.45, (0.4 C), 74.84 (0.6 C), 127.2 (0.6 C), 127.4 (0.4 C), 127.9 (0.6 C), 127.9 (0.4 C), 128.3 (0.6 C), 128.5 (0.4 C), 136.6 (0.6 C), 136.7 (0.4 C), 155.1 (0.4 C), 155.3 (0.6 C), 172.2 (0.4 C), 172.3 (0.6 C).
**MS** (ESI): m/z (%) = 419.2 (34) [M+H]$^+$, 837.1 (100) [2M+H]$^+$.

**Example 5-10:** Preparation of (1S,5S,6R)-10-((3,5-dichlorophenyl)sulfonyl)-5-((triethylsilyl)oxy)-3,10-diazabicyclo[4.3.1]decan-2-one **G5**

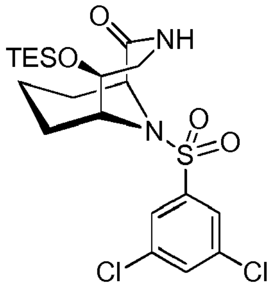
**G5**

A suspension of **G4** (500 mg, 1.19 mmol) and Pd/C (50.0 mg, 10 wt. %) in EtOAc (6 mL) was stirred for 3 h at room temperature under a $H_2$-atmosphere. The reaction mixture was filtered over Celite, washed with EtOAc and the solvent was evaporated under reduced pressure to give a colorless, crystalline solid.

The solid was dissolved in $CH_2Cl_2$ (6 mL), ($i$Pr)$_2$NEt (624 μL, 3.57 mmol, 3.0 equiv.), DMAP (145 mg, 1.19 mmol, 1.0 equiv.) and 3,5-Dichlorobenzenesulfonyl chloride (584 mg, 2.38 mmol, 2.0 equiv.) were added and the solution was stirred for 20 h at room temperature. Sat. aq. $NH_4Cl$ solution was added (25 mL), the aqueous phase was extracted with $CH_2Cl_2$ (2 × 25 mL), dried over $MgSO_4$, and the solvent evaporated under reduced pressure. Column chromatography on $SiO_2$ (cyclohexane/EtOAc 3:1) afforded the title compound (420 mg, 0.851 mmol, 72 % over 2 steps) as colorless, crystalline solid.
$R_f$**:** 0.31 (Cyclohexane/EtOAc 2:1)
**$^1$H-NMR** (600 MHz, $CDCl_3$): δ = 0.61 (q, $J$ = 7.8 Hz, 6 H, 3 × $CH_2$), 0.96 (t, $J$ = 7.8 Hz, 9 H, 3 × $CH_3$), 1.25-1.70 (m, 5 H, 2 × $CH_2$, CH), 2.16-2.21 (m, 1 H, CH), 2.83-2.91 (m, 1 H, $CH_A$), 2.83-2.91 (m, 1 H, $CH_A$), 3.59-3.64 (m, 1 H, $CH_B$), 3.93-3.97 (m, 1 H, CH), 4.12-4.15 (m, 1 H, CH), 4.64-4.67 (m, 1 H, CH), 6.04-6.09 (m, 1 H, NH), 7.57 (t, $J$ = 1.8 Hz, 1 H, Ar-H), 7.72 (d, $J$ = 1.8 Hz, 2 H, 2 × Ar-H).
**$^{13}$C-NMR** (75.5 MHz, $CDCl_3$): δ = 4.99, 6.95, 16.26, 26.59, 27.19, 46.88, 56.42, 60.20, 74.89, 125.3, 133.0, 136.5, 144.0, 172.8.
**MS** (ESI): $m/z$ (%) = 493.4 (44) $[M+H]^+$, 987.2 (100) $[2M+H]^+$.

**Example 5-10:** Preparation of (1S,5S,6R)-10-((3,5-dichlorophenyl)sulfonyl)-5-hydroxy-3,10-diazabicyclo[4.3.1]decan-2-one G6

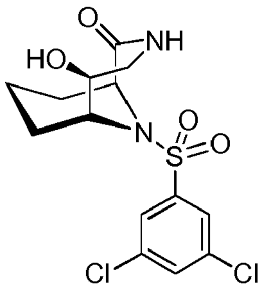
**G6**

To a solution of **G5** (400 mg, 0.811 mmol) in THF (4 mL) was added TBAF (1 M in THF, 892 μL, 0.892 mmol, 1.1 equiv.) and the solution was stirred for 1.5 h at room temperature. Removal of the solvent under reduced pressure and column chromatography on $SiO_2$ ($CH_2Cl_2$/MeOH 20:1) afforded the title compound (294 mg, 0.775 mmol, 96 %) as a colorless solid.
$R_f$**:** 0.30 (EtOAc)
**$^1$H-NMR** (400 MHz, MeOH-$d_4$): δ = 1.27-1.37 (m, 2 H, $CH_2$), 1.51-1.59 (m, 2 H, $CH_2$), 1.69-1.79 (m, 1 H, CH), 2.04-2.11 (m, 1 H, CH), 2.99 (dd, J = 13.2, 2.8 Hz, 1 H, $CH_A$), 3.58 (dd, J = 13.2, 10.4 Hz, 1 H, $CH_B$), 3.94 (ddd, J = 10.4, 5.2, 2.8 Hz, 1 H, CH), 4.07 ($m_c$, 1 H, CH), 4.66 (dt, J = 6.0, 2.0 Hz, 1 H, CH), 7.81 (t, J = 2.0 Hz, 1 H, Ar-H), 7.89 (d, J = 2.0 Hz, 2 H, 2 × Ar-H).
**$^{13}$C-NMR** (100 MHz, MeOH-$d_4$): δ = 16.92, 27.51, 27.94, 47.04, 57.80, 60.52, 74.75, 126.5, 134.1, 137.8, 145.7, 175.0.
**MS** (ESI): m/z (%) = 379.0 (100) $[M+H]^+$, 758.7 (52) $[2M+H]^+$.

**VI. Preparation of compounds H1.1 - H1.4 and H2.1 - H2.3**

Further examples for the compound of the general formula (I) are outlined in scheme H1 and scheme H2:

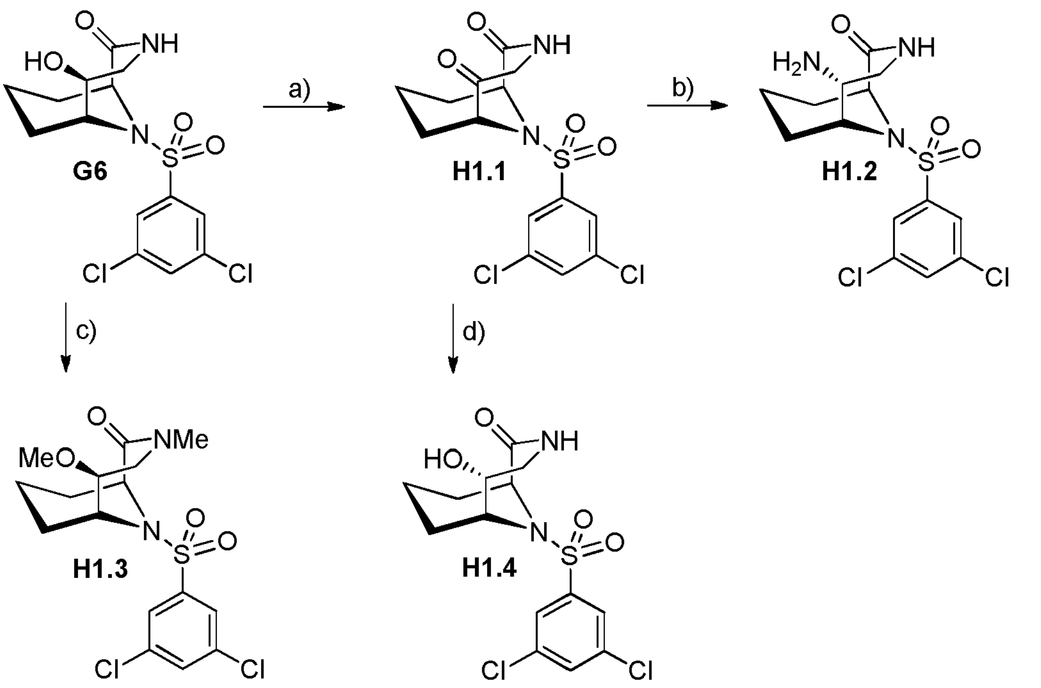


**Scheme H1.** Reagents and conditions: a) Dess-Martin periodinane, $CH_2Cl_2$, rt, 18 h, quant.; b) $NH_4OTFA$, $NaBH(OAc)_3$, THF, rt, 10 h, *dr* 10:1, quant.; c) NaHMDS, MeI, 0 °C to rt, 16 h, 26 %; d) L-Selectride, THF, −78 °C, 1 h, quant.

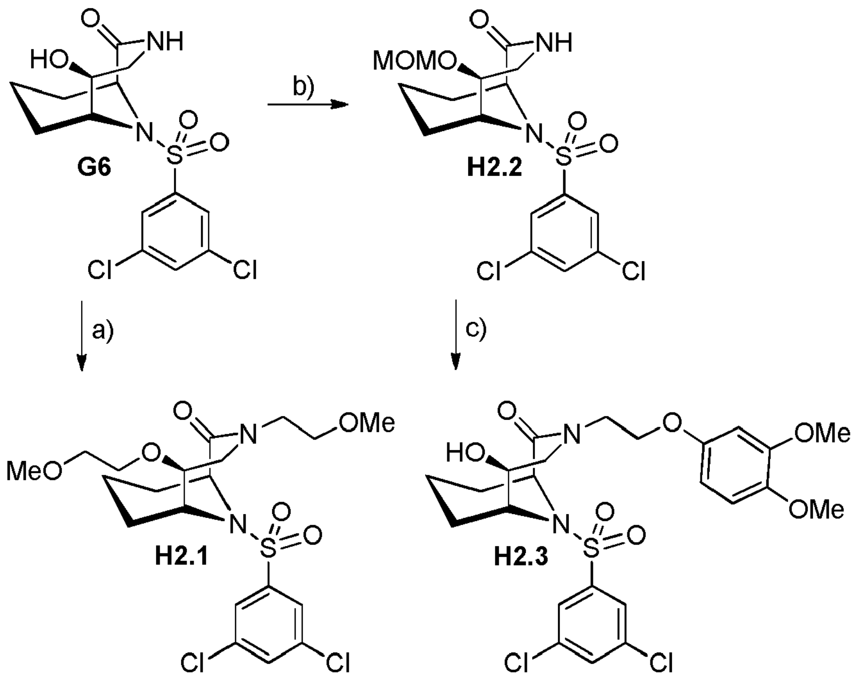


G6, H2.2, H2.1, H2.3

**Scheme H2.** Reagents and conditions: a) NaH, 2-Bromoethyl methyl ether, DMF, 80 °C, 6 h, 23 %, b) MOMCl, ($i$Pr)$_2$NEt, DMF, 80 °C, 3 h, 39 %; c) 1.) NaH, 4-(2-Bromoethoxy)-1,2-dimethoxy benzene, DMF, 80 °C, 2 h; 2.) conc. HCl, EtOH, rt, 2 d, 20 % (2 steps).

**Example 6-1:** Preparation of (1S,6R)-10-((3,5-dichlorophenyl)sulfonyl)-3,10-diazabicyclo[4.3.1]decane-2,5-dione **H1.1**

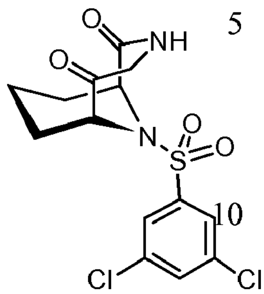

**H1.1**

To a solution of **G6** (100 mg, 0.264 mmol) in $CH_2Cl_2$ (6 mL) was added Dess-Martin periodinane (134 mg, 0.317 mmol, 1.2 equiv.) and the solution was stirred for 18 h at room temperature. Removal of the solvent under reduced pressure and column chromatography on $SiO_2$ ($CH_2Cl_2$/EtOAc 10:1 $\rightarrow$ 5:1) afforded the title compound (100 mg, 0.264 mmol, quant.) as a colorless solid.

**$R_f$:** 0.28 (Cyclohexane/EtOAc 2:1)

**$^1$H-NMR** (400 MHz, $CDCl_3$): $\delta$ =1.19-1.73 (m, 5 H, 2 $\times$ $CH_2$, CH), 2.20-2.30 (m, 1 H, CH), 3.50 (dd, J = 13.2, 8.4 Hz, 1 H, $CH_A$), 4.64-4.68 (m, 1 H, CH), 4.76 (dd, J = 13.2, 1.6 Hz, 1 H, $CH_B$), 4.78-4.81 (m, 1 H, CH), 6.36 ($d_{br}$, $J$ = 8.0 Hz, 1 H, NH), 7.63 (d, J = 2.0 Hz, 1 H, Ar-H), 7.72 (d, J = 2.0 Hz, 1 H, 2 $\times$ Ar-H).

**$^{13}$C-NMR** (100 MHz, $CDCl_3$): $\delta$ = 17.70, 24.74, 27.24, 51.13, 57.14, 62.57, 125.2, 133.7, 137.0, 143.0, 171.5, 205.6.

**MS** (ESI): m/z (%) = 377.0 (100) $[M+H]^+$.

**Example 6-2:** Preparation of (1S,5R,6R)-5-amino-10-((3,5-dichlorophenyl)sulfonyl)-3,10-diazabicyclo[4.3.1]decan-2-one **H1.2**

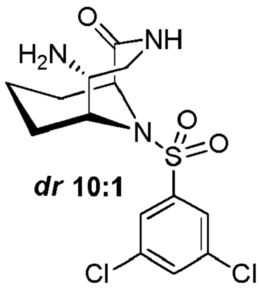

**H1.2**

To a solution of **H1.1** (10.0 mg, $2.65\cdot10^{-5}$ mol) in THF (0.5 mL) was added ammonium trifluoroacetate (34.7 mg, 0.264 mmol, 10 equiv.) and $NaBH(OAc)_3$ (16.8 mg, $7.95\cdot10^{-5}$ mol, 3.0 equiv.) and the solution was stirred for 10 h at room temperature. 1 M aq. NaOH (15 mL) was added, the aqueous phase was extracted with EtOAc (2 × 15 mL), dried over $MgSO_4$, and the solvent evaporated under reduced pressure. Column chromatography on $SiO_2$ (cyclohexane/EtOAc 1:2 to 0:1) afforded the title compound (10.0 mg, $2.65\cdot10^{-5}$ mol, quant.) as a colorless solid.
**$R_f$:** 0.52 (EtOAc)
**$^1$H-NMR** (400 MHz, MeOH-$d_4$): δ = 1.32-1.52 (m, 3 H, $CH_2$, CH), 1.65-1.77 (m, 1 H, CH), 2.02-2.12 (m, 1 H, CH), 3.28 (dd, J = 14.4, 8.0 Hz, 1 H, $CH_A$), 3.47 (dd, J = 14.4, 3.6 Hz, 1 H, $CH_B$), 4.14 ($m_c$, 1 H, CH), 4.37 ($m_c$, 1 H, CH), 4.62-4.67 (m, 1 H, CH), 7.78 (t, J = 1.6 Hz, 1 H, Ar-H), 7.89 (d, J = 1.6 Hz, 2 H, 2 × Ar-H).
**MS** (ESI): m/z (%) = 379.1 (100) $[M+H]^+$. 758.8 (82) $[2M+H]^+$.

**Example 6-3:** Preparation of (1S,5S,6R)-10-((3,5-dichlorophenyl)sulfonyl)-5-methoxy-3-methyl-3,10-diazabicyclo[4.3.1]decan-2-one **H1.3**

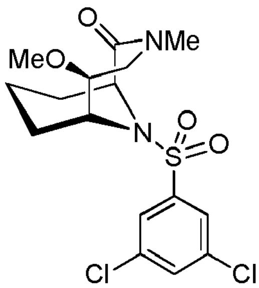

**H1.3**

To a solution of **G6** (40.0 mg, 0.105 mmol) in THF (1 mL) at 0 °C was added NaHMDS (1 M in THF, 126 μL, 0.126 mmol, 1.2 equiv.). After 5 min MeI (7.2 μL, 0.116 mmol, 1.1 equiv.) was added, the cooling bath was removed and the solution was stirred for 16 h at room temperature. Sat. aq. $NH_4Cl$ solution (20 mL) was added, the aqueous phase was extracted with EtOAc (2 × 20 mL), dried over $MgSO_4$, and the solvent removed under reduced pressure. Column chromatography on $SiO_2$ (cyclohexane/EtOAc 1:1 → 1:2) afforded the title compound (11.0 mg, $2.70\cdot10^{-5}$ mol, 26 %) as a colorless solid.
**$R_f$:** 0.34 (Cyclohexane/EtOAc 1:1)
**$^1$H-NMR** (400 MHz, $CDCl_3$): δ = 1.12-1.67 (m, 5 H, 2 × $CH_2$, CH), 2.19-2.26 (m, 1 H, CH), 3.11 (s, 3 H, $NCH_3$), 3.15 (dd, J = 13.6, 2.4 Hz, 1 H, $CH_A$), 3.43 (s, 3 H, $OCH_3$), 3.49 (ddd, J = 10.4, 4.8, 2.4 Hz, 1 H, CH), 3.97 (dd, J = 13.6, 10.4 Hz, 1 H, $CH_B$), 4.08-4.13 (m, 1 H, CH), 4.68 ($m_c$, 1 H, CH), 7.56 (t, J = 2.0 Hz, 1 H, Ar-H), 7.70 (t, J = 2.0 Hz, 2 H, 2 × Ar-H).
**$^{13}$C-NMR** (100 MHz, $CDCl_3$): δ = 16.34, 27.18, 27.50, 50.56, 56.92, 57.08, 58.39, 83.17, 125.3, 133.0, 136.6, 144.1, 170.1.
**MS** (ESI): m/z (%) = 407.0 (100) $[M+H]^+$, 814.7 (8) $[2M+H]^+$.

**Example** 6-4: Preparation of (1S,5R,6R)-10-((3,5-dichlorophenyl)sulfonyl)-5-hydroxy-3,10-diazabicyclo[4.3.1]decan-2-one **H1.4**

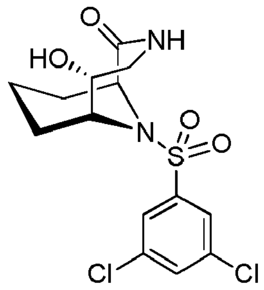

**H1.4**

To a solution of **H1.1** (60.0 mg, 0.159 mmol) in THF (3 mL) at -78 °C was added L-Selectride (1 M in THF, 191 μL, 0.191 mmol, 1.2 equiv.) and the solution was stirred for 1 h at -78 °C. The cooling bath was removed, $H_2O$ (1 mL) was added and the reaction was allowed to reach room temperature. Sat. aq. $NH_4Cl$ solution (20 mL) was added, the aqueous phase was extracted with EtOAc (2 × 20 mL), dried over $MgSO_4$, and the solvent was evaporated under reduced pressure. Column chromatography on $SiO_2$ (EtOAc) afforded the title compound (59.0 mg, 0.156 mmol, quant.) as a colorless solid.
**$R_f$:** 0.30 (EtOAc)
**$^1$H-NMR** (400 MHz, MeOH-$d_4$): δ = 1.21-1.52 (m, 5 H, 2 × $CH_2$, CH), 2.02-2.11 (m, 1 H, CH), 3.28 (dd, J = 14.4, 8.0 Hz, 1 H, $CH_A$), 3.47 (dd, J = 14.4, 3.2 Hz, 1 H, $CH_B$), 4.12-4.17 (m, 1 H, CH), 4.37 ($m_c$, 1 H, CH), 4.63-4.67 (m, 1 H, CH), 7.79 (t, J = 2.0 Hz, 1 H, Ar-H), 7.86 (d, J = 2.0 Hz, 2 H, 2 × Ar-H).
**$^{13}$C-NMR** (100 MHz, MeOH-$d_4$): δ = 19.23, 22.80, 28.42, 46.74, 55.25, 58.59, 71.91, 126.5, 134.0, 137.7, 145.6, 178.1.
**MS** (ESI): m/z (%) = 379.1 (100) $[M+H]^+$, 758.7 (94) $[2M+H]^+$.

**Example 6-5:** Preparation of (1S,5S,6R)-10-((3,5-dichlorophenyl)sulfonyl)-5-(2-methoxyethoxy)-3-(2-methoxyethyl)-3,10-diazabicyclo[4.3.1]decan-2-one **H2.1**

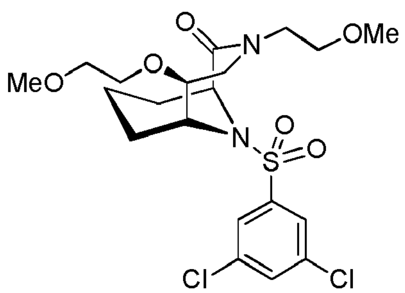

**H2.1**

To a solution of **G6** (10.0 mg, $2.64 \cdot 10^{-5}$ mol) in DMF (0.25 mL), was added NaH (2.6 mg, $6.60 \cdot 10^{-5}$ mol, 2.5 equiv.). After stirring for 5 min, 2-Bromoethyl methyl ether (5.40 μL, $5.81 \cdot 10^{-5}$ mol, 2.2 equiv.) was added and the suspension was stirred at 80 °C for 6 h. After cooling to room temperature, $H_2O$ (15 mL) was added and the aqueous phase was extracted with $Et_2O$ (2 × 15 mL), dried over $MgSO_4$, and the solvent was evaporated under reduced pressure. Column chromatography on $SiO_2$ (cyclohexane/EtOAc 1:1 → 1:2) afforded the title compound (3.0 mg, $6.06 \cdot 10^{-6}$ mol, 23 %) as colorless solid.
**$R_f$:** 0.26 (Cyclohexane/EtOAc 1:2)
**$^1$H-NMR** (600 MHz, $CDCl_3$): δ = 1.25-1.60 (m, 5 H, CH, 2 × $CH_2$), 2.22 ($m_c$, 1 H, CH), 3.25-3.30 (m, 1 H, CH). 3.35 (s, 3 H, OMe), 3.38 (s, 3 H, OMe), 3.49-3.55 (m, 4 H, 2 × $CH_2$), 3.57-3.65 (m, 2 H, $CH_2$), 3.72-3.77 (m, 2 H, $CH_2$), 3.94 (dd, J = 14.4, 10.2 Hz, 1 H, CH), 4.01 (dt, J = 14.4, 3.6 Hz, 1 H, CH), 4.13 ($m_c$, 1 H, CH), 4.67 ($m_c$, 1 H, CH), 7.56 (t, J = 1.8 Hz, 1 H, Ar-H), 7.71 (d, J = 1.8 Hz, 2 H, 2 × Ar-H).
**MS** (ESI): m/z (%) = 495.23 (100) $[M+H]^+$, 990.32 (28) $[2M+H]^+$.

**Example 6-6:** Preparation of (1S,5S,6R)-10-((3,5-dichlorophenyl)sulfonyl)-5-(methoxymethoxy)-3,10-diazabicyclo[4.3.1]decan-2-one H2.2

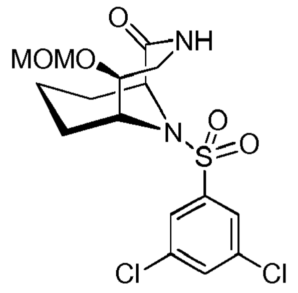

**H2.2**

To a solution of **G6** (25.0 mg, $6.59 \cdot 10^{-5}$ mol) in DMF (0.5 mL) was added $(i\text{Pr})_2\text{NEt}$ (138 μL, 0.791 mmol, 12 equiv.) and MOMCl (50.0 μL, 0.659 mmol, 10 equiv.) and it was stirred at 80 °C for 3 h. After cooling to room temperature, $H_2O$ (15 mL) was added and the aqueous phase was extracted with $Et_2O$ (2 × 15 mL), dried over $MgSO_4$, and the solvent was evaporated under reduced pressure. Column chromatography on $SiO_2$ (cyclohexane/EtOAc 1:1 → 1:2) afforded the title compound (11.0 mg, $2.60 \cdot 10^{-5}$ mol, 39 %) as colorless solid.
$\mathbf{R_f}$: 0.10 (Cyclohexane/EtOAc 1:1)
**$^1$H-NMR** (400 MHz, $CDCl_3$): δ = 1.20-1.75 (m, 5 H, 2 × $CH_2$, CH), 2.15-2.22 (m, 1 H, CH), 3.17 (ddd, J = 13.6, 8.8, 2.8 Hz, 1 H, $CH_A$), 3.39 (s, 3 H, $OCH_3$), 3.68-3.76 (m, 1 H, $CH_B$), 3.85 (ddd, J = 10.4, 4.8, 2.8 Hz, 1 H, CH), 4.22 ($m_c$, 1 H, CH), 4.65 (d, J = 7.2 Hz, $CH_A$), 4.66 ($m_c$, 1 H, CH), 4.69 (d, J = 7.2 Hz, $CH_B$), 6.32 ($d_{br}$, *J* = 7.2 Hz, 1 H, NH), 7.58 (d, J = 2.0 Hz, 1 H, Ar-H), 7.72 (d, J = 2.0 Hz, 2 H, 2 × Ar-H).
**$^{13}$C-NMR** (100 MHz, $CDCl_3$): δ = 16.19, 26.87, 27.26, 43.99, 56.00, 56.50, 57.85, 80.53, 96.69, 125.3, 133.1, 136.6, 144.0, 172.6.
**MS** (ESI): m/z (%) = 423.0 (100) $[M+H]^+$, 846.8 (94) $[2M+H]^+$.

**Example 6-7:** Preparation of (1S,5S,6R)-10-((3,5-dichlorophenyl)sulfonyl)-3-(2-(3,4-dimethoxyphenoxy)ethyl)-5-hydroxy-3,10-diazabicyclo[4.3.1]decan-2-one **H2.3**

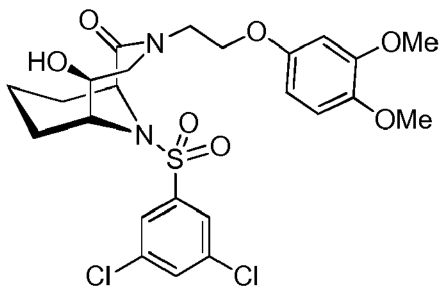

**H2.3**

To a solution of **H2.2** (11.0 mg, $2.60 \cdot 10^{-5}$ mol) in DMF (0.2 mL) was added NaH (1.6 mg, $3.9 \cdot 10^{-5}$ mol, 1.5 equiv.). After stirring for 10 min at room temperature, 4-(2-Bromoethoxy)-1,2-dimethoxy benzene (7.50 mg, $2.86 \cdot 10^{-5}$ mol, 1.1 equiv.) was added and it was stirred at 80 °C for 2 h. After cooling to room temperature, sat aq. $NH_4Cl$ solution (15 mL) was added and the aqueous phase was extracted with $Et_2O$ (2 × 15 mL), dried over $MgSO_4$, and the solvent was evaporated under reduced pressure. Column chromatography on $SiO_2$ (cyclohexane/EtOAc 2:1 → 1:1) afforded a colorless solid (6.0 mg, $9.94 \cdot 10^{-6}$ mol, 38 %).

This solid was dissolved in EtOH (0.5 mL), conc. aq. HCl (0.1 mL) was added and it was stirred for 2 d at room temperature. Evaporation of the solvent under reduced pressure and column chromatography on $SiO_2$ (cyclohexane/EtOAc 1:2) afforded the title compound (3.0 mg, $5.36 \cdot 10^{-6}$ mol, 54 %) as colorless solid.
$\mathbf{R_f}$: 0.19 (Cyclohexane/EtOAc 1:1)
**$^1$H-NMR** (600 MHz, $CDCl_3$): δ = 1.27-1.67 (m, 5 H, CH, 2 × $CH_2$), 2.19-2.24 (m, 1 H, CH), 3.47 (dd, *J* = 13.8, 2.4 Hz, 1 H, CH), 3.60-3.67 (m, 1 H, CH), 3.84 (s, 3 H, OMe), 3.86 (s, 3 H, OMe), 4.01-4.09 (m, 4 H, 2 × CH, $CH_2$), 4.10-4.17 (m,

2 H, $CH_2$), 4.70 ($m_c$, 1 H, CH), 6.39 (dd, *J* = 9.0, 2.4 Hz, 1 H, Ar-H), 6.51 (d, *J* = 2.4 Hz, 1 H, Ar-H), 6.78 (d, *J* = 9.0 Hz, 1 H, Ar-H), 7.51-7.53 (m, 1 H, Ar-H), 7.69-7.71 (m, 2 H, 2 × Ar-H).
**MS** (ESI): *m*/*z* (%) = 559.0 (100) $[M+H]^+$.

## V. Preparation of compounds J1-8

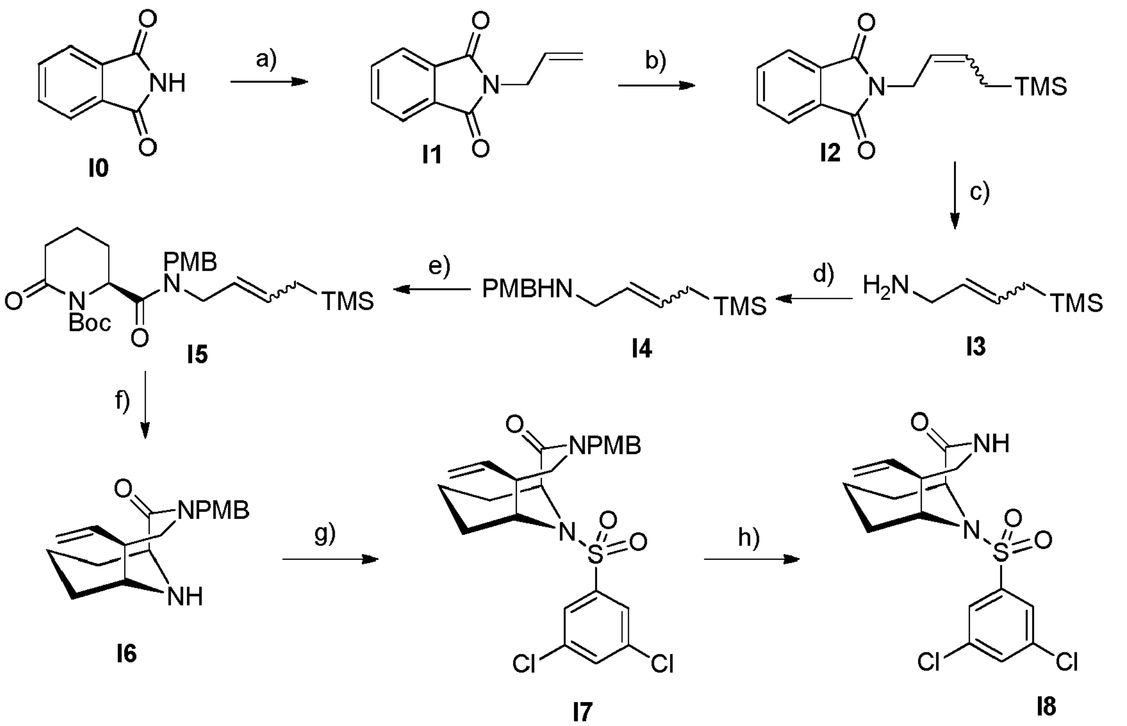


**Scheme I.** Reagents and conditions: a) $K_2CO_3$, allylbromide, DMF, rt, 3 h, quant.; b) AllylTMS, Grubbs I, $CH_2Cl_2$, 60 °C, 4 h, 79 %; c) $NH_2NH_2$, MeOH, 70 °C, 24 h; d) 4-Methoxybenzaldehyde, $NaBH_4$, EtOH, rt, 4 h, 64 % (2 steps); e) 1.) (*S*)-6-Oxopiperidine-2-carboxylic acid, HOBt, EDC, rt, DMF, 2 h; 2.) $Boc_2O$, DIPEA, DMAP, $CH_2Cl_2$, 48 h, 59 % (2 steps); f) 1.) DIBAL, THF, −78 °C,15 min; 2.) HF•pyridine, $CH_2Cl_2$, −78 °C, 1 h, 60 % (2 steps); g) 3,5-dichlorobenzene-1-sulfonyl chloride, DIPEA, DMAP, rt, 24 h, 60 %; h) CAN, MeCN/$H_2O$, rt, 4 h, 90 %;

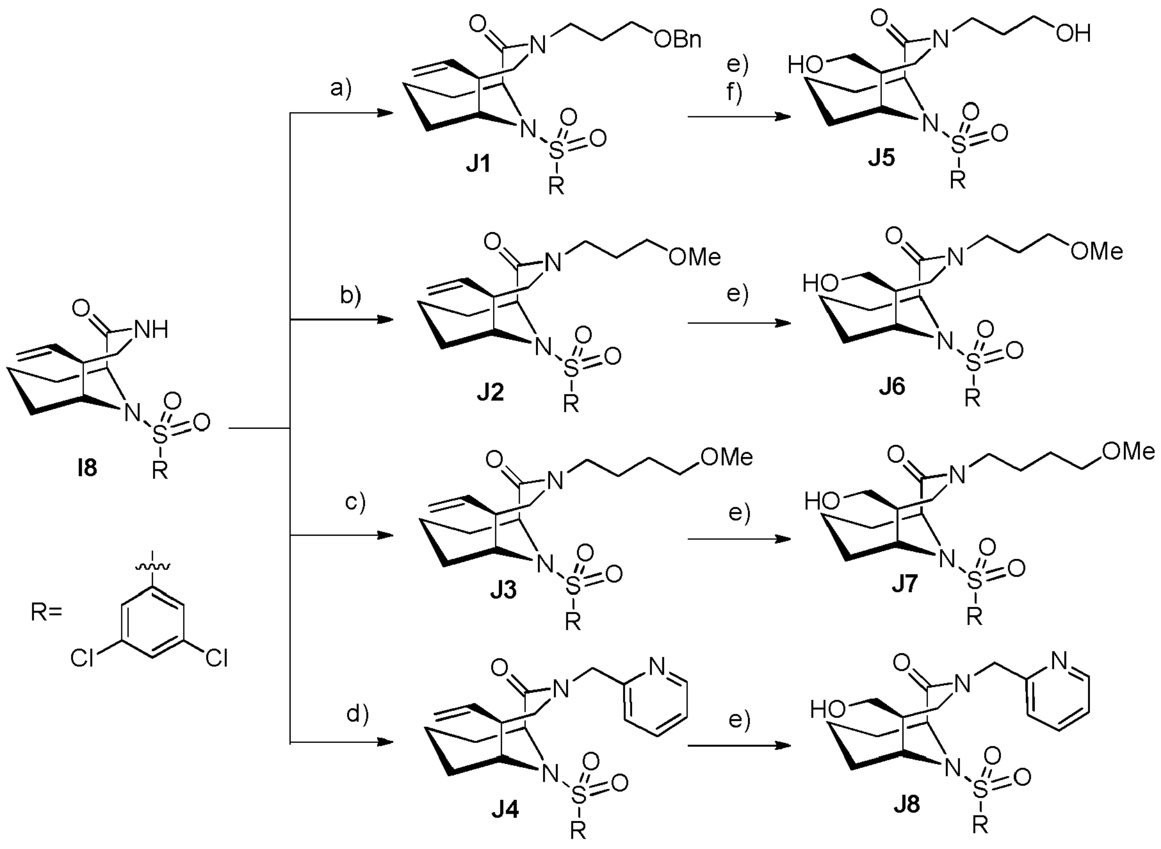


**Scheme J.** Reagents and conditions: a) NaH,((3-bromopropoxy)methyl)benzene, DMF, 1h, 80 °C, 64 %; b) NaH,1-bromo-4-methoxypropane, DMF, 1h, 80 °C, 48 % c) NaH,1-bromo-4-methoxybutane, DMF, 1h, 80 °C,65 %; d) NaH, 2-(bromomethyl)pyridine•HBr, DMF, 1h, 80 °, 100%; e) 1.) $NaIO_4$, $OsO_4$, 2,6-lutidine, dioxane/$H_2O$, 20 h, rt; 2.) $NaBH_4$, EtOH, rt, 1 h ; f) $BCl_3$ $SMe_2$, $CH_2Cl_2$, rt, 30 min.

**Example 7-1:** Preparation of 2-allylisoindoline-1,3-dione 11

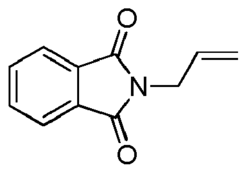

**I1**

To a solution of Phtalimide (15.0 g, 102 mmol) in DMF (100 mL) was added Allylbromide (12.3 g, 8.82 mL, 102 mmol). After 3 h stirring at room temperature $EtO_2$ (300 mL) was added to the reaction and washed with sat. aq. NaCl solution (3 × 75 mL). The solvent was removed under reduced pressure affording the title compound (19.0 g, 101 mmol, 99.0 %) as a colourless solid, which was used for the next step without further purification.

**$R_f$:** 0.43 (Cyclohexane/EtOAc = 85:15)

**$^1$H NMR** (300 MHz, $CDCl_3$) δ = 4.29 (dt, J = 5.7, 1.5 Hz, 2 H), 5.15 - 5.31 (m, 2 H), 5.79 - 5.97 (m, 1 H), 7.68 - 7.76 (m, 2 H), 7.80 - 7.89 (m, 2 H).

**$^{13}$C-NMR** (75.5 MHz, $CDCl_3$) δ = 40.03, 117.72, 123.27, 131.50, 132.10, 133.94, 167.88.

**Example 7-2:** Preparation of 2-(4-(trimethylsilyl)but-2-en-1-yl)isoindoline-1,3-dione **12**

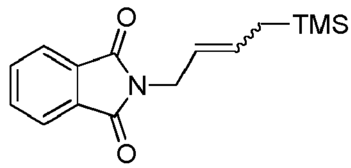

**I2**

To a solution of 2-allylisoindoline-1,3-dione 11 (13.0 g, 69.4 mmol) in $CH_2Cl_2$ (500 mL) was added AllylTMS (79.0 g, 110 mL, 694 mmol) and Grubbs I generation catalyst. The reaction was heated to 60 °C and stirred under reflux for 4 h. Tris(hydrodxymethyl)phosphine (1 M solution in *i*-PrOH, 58 mL) was added and stirred under reflux for 12 h, while the color of the reaction turned from black to orange. Sat. aq. NaCl solution (100 mL) was added to the reaction and the organic phase was separated. The aqueous phase was extracted with $CH_2Cl_2$ (3 × 200 mL). The combined organic layers were dried over $MgSO_4$ and the solvent was removed under reduced pressure. Column chromatography over $SiO_2$ (Cyclohexane/ EtOAc = 85:15) afforded the title compound (15.0 g, 54.9 mmol, 78.9 %) as a yellow resin.
$\mathbf{R_f}$: 0.57 (Cyclohexane/EtOAc = 85:15)
**$^1$H NMR** (300 MHz, $CDCl_3$) δ = -0.10 - 0.09 (m, 9H), 1.44 (d, J = 8.2 Hz, 1.6 H), 1.72 (d, J = 8.8 Hz, 0.4 H), 4.21 (d, J = 6.5 Hz, 1.6 H), 4.29 (dd, J = 14.5, 5.9 Hz, 0.4 H), 5.26 - 5.47 (m, 0.8 H), 5.56 - 5.70 (m, 0.2 H), 5.70 - 5.87 (m, 0.8 H), 5.94 - 6.09 (m, 0.2 H), 7.65 - 7.74 (m, 2H), 7.79 - 7.88 (m, 2H).
**$^{13}$C NMR** (75 MHz, $cdcl_3$) δ = -2.05, -1.83, -1.43, 18.92, 22.73, 34.72, 39.87, 120.51, 121.30, 123.12, 130.78, 132.24, 133.76, 133.90, 167.99.

**Example 7-3:** Preparation of 4-(trimethylsilyl)but-2-en-1-amine **13**

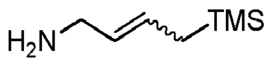

**I3**

To a solution of 2-(4-(trimethylsilyl)but-2-en-1-yl)isoindoline-1,3-dione **12** (150 mg, 0.549 mmol) in MeOH (5 mL) was added Hydrazine (35.2 mg, 0.034 mL, 1.10 mmol) and the reaction was heated to 75 °C and stirred under reflux for 24 h. $CH_2Cl_2$ (90 mL) was added to the solution and washed with NaOH (1 M solution, 3 x 10 mL). The organic layer was dried over $MgSO_4$ and the solvent was removed under reduced pressure (at room temperature at 150 mbar) affording the title compound as a colourless liquid, which was stored at - 20 °C.
$\mathbf{R_f}$: 0.24 (EtOAc + 2 % MeOH + 2% TEA)
**$^1$H NMR** (300 MHz, $CDCl_3$) δ = -0.04 - 0.03 (m, 9 H), 1.40 - 1.50 (m, 2 H), 1.69 (s, 2 H), 3.14 - 3.34 (m, 2 H), 5.30 - 5.61 (m, 2 H).
**$^{13}$C NMR** (75 MHz, $CDCl_3$) δ = -2.02, 22.50, 44.36, 127.19, 129.72

**Example 7-4:** Preparation of N-(4-methoxybenzyl)-4-(trimethylsilyl)but-2-en-1-amine **14**

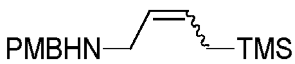

**I4**

To a solution of 4-(trimethylsilyl)but-2-en-1-amine 13 (7.86 g, 54.8 mmol) in EtOH (250 mL) was added 4-methoxybenzaldehyde (7.47 g, 54.8 mL, 1.10 mmol). After 2h stirring at room temperature $NaBH_4$ (3.11 g, 82 mmol) was added to the solution, which was stirred until no more gas evolution was observed. NaOH (1 M solution, 100 mL) was added to the reaction and extracted with $CH_2Cl_2$ (300 mL). The organic layer was dried over $MgSO_4$ and the solvent was removed under reduced pressure. Column chromatography (EtOAc + 2 % MeOH + 2% TEA) afforded the title compound (11.2 g, 42.5 mmol, 77.6 % over 2 steps) as a slightly yellow oil.
$\mathbf{R_f}$: 0.35 (EtOAc + 2 % MeOH + 2% TEA)

**MS** (ESI): m/z (%) = 263.98 $[M + H]^+$, 526.24 $[2M + H]^+$

**Example 7-5:** Preparation of (S)-N-(4-methoxybenzyl)-6-oxo-N-(4-(trimethylsilyl)but-2-en-1-yl)piperidine-2-carboxamide **I5.1** and (S)-tert-butyl 2-((4-methoxybenzyl)(4-(trimethylsilyl)but-2-en-1-yl)carbamoyl)-6-oxopiperidine-1-carboxylate **15.2**

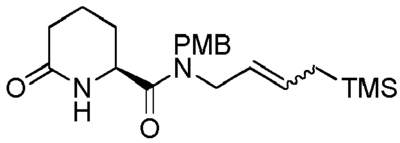

**I5.1**

To a solution of (S)-6-oxopiperidine-2-carboxylic acid (1.74 g, 12.15 mmol) in DMF (120 mL) were added N-(4-methoxybenzyl)-4-(trimethylsilyl)but-2-en-1-amine **14** (3.20 g, 12.2 mmol), EDC (2.79 g, 14.6 mmol) and HOBt (2.23 g, 14.6 mmol). After 2h stirring at room temperature $EtO_2$ (300 mL) was added to the reaction and washed with sat. aq. NaCl solution (3 × 75 mL). The organic layer was dried over $MgSO_4$ and the solvent was removed under reduced pressure and the crude compound **I5.1** was used for the next step without further purification.
**MS** (ESI): m/z (%) = 389.29 $[M + H]^+$, 777.25 $[2M + H]^+$

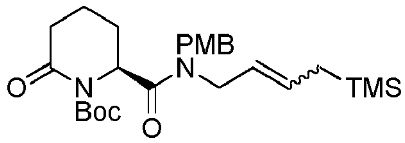

**I5.2**

Crude compound **I5.1** was dissolved in $CH_2Cl_2$ (120 mL). DIPEA (3.14 g, 4.24 mL, 24.2 mmol), $Boc_2O$ (7.95 g, 36.4 mmol) and DMAP (1.48 g, 12.2 mmol) were added. After 24 h stirring at room temperature sat. aq. NaCl solution (25 mL) was added to the reaction and the organic phase was separated. The aqueous phase was extracted with $CH_2Cl_2$ (3 × 100 mL) and the combined organic layers were dried over $MgSO_4$ and the solvent was removed under reduced pressure. Flash column chromatography (10 - 30 % EtOAc in Cyclohexane) afforded the compound **15.2** (3.50 g, 7.16 mmol, 59 % over two steps) as a yellow resin.
$\mathbf{R_f}$: 0.14 (Cyclohexane/EtOAc = 1:1)
**MS** (ESI): m/z (%) = 389.15 $[M + H - Boc]^+$, 999.09 $[2M + H]^+$

**Example 7-6:** Preparation of (1S,5R,6R)-3-(4-methoxybenzyl)-5-vinyl-3,10-diazabicyclo[4.3.1]decan-2-one **16**

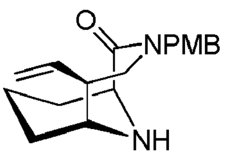

**I6**

A solution of (6S)-tert-butyl 2-hydroxy-6-((4-methoxybenzyl)((Z)-4-(trimethylsilyl)but-2-en-1-yl)carbamoyl)piperidine-1-carboxylate (3.5 mg, 7.16 mmol) in THF (75 mL) was cooled to -78 °C and then DIBAL-H (1 M solution in $CH_2Cl_2$, 10.7 mL, 10.7 mmol) was added. After 15 minutes an excess of Glauber's salt ($Na_2SO_4$ 10 $H_2O$) was added to the reaction. The solution was allowed to warm to room temperature and more Glaubers salt was added and stirred for 15 minutes. The solution was filtered through celite and the solvent was removed under reduced pressure, affording a yellow resin.

The resin resulted from the first step was dissolved in $CH_2Cl_2$ (300 mL) in a teflon flask and cooled to -78 °C. HF (70 % in pyridine, 15 mL) was added and the reaction flask was putted in an ice bath at 0 °C. After one hour sat. aq. $CaCO_3$ solution and NaOH (10 M solution) was added to the solution to neutralize the acid and precipitate the fluoride ions as $CaF_2$. The reaction was extracted three times with $CH_2Cl_2$, the combined organic layers were dried over $MgSO_4$ and the solvent removed under reduced pressure. Column chromatography (5 % MeOH and 2 % TEA in EtOAc) afforded the title compound (1.30 g, 4.33 mmol, 60.6 % over two steps) as an orange resin.

**$^1$H NMR** (400 MHz, Chloroform-*d*) δ = 1.39 - 1.55 (m, 2 H), 1.60 - 1.65 (m, 3 H), 2.29 - 2.36 (m, 1 H), 2.40 - 2.50 (m, 1 H), 2.76 - 2.81 (m, 1 H), 2.91 (dd, J = 13.8, 2.0 Hz, 1 H), 3.79 (s, 3 H), 3.81 - 3.85 (m, 1 H), 3.89 (dd, J = 13.8, 10.8 Hz, 1 H), 4.44 (d, J = 14.3 Hz, 1 H), 4.73 (d, J = 14.3 Hz, 1 H), 4.77 - 4.91 (m, 2 H), 5.54 (ddd, J = 17.0, 10.2, 8.4 Hz, 1 H), 6.83 - 6.88 (m, 2 H), 7.19 - 7.25 (m, 2 H).

**Example 7-7:** Preparation of (1S,5R,6R)-10-((3,5-dichlorophenyl)sulfonyl)-3-(4-methoxybenzyl)-5-vinyl-3,10-diazabicyclo[4.3.1]decan-2-one 17

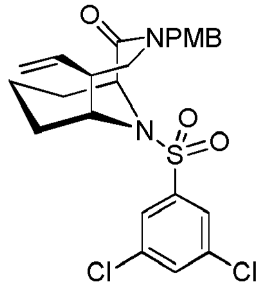

I7

To a solution of (1S,5R,6R)-3-(4-methoxybenzyl)-5-vinyl-3,10-diazabicyclo[4.3.1]decan-2-one (650 mg, 2.16 mmol) in $CH_2Cl_2$ (20 mL) were added 3,5-dichlorobenzene-1-sulfonyl chloride (1.06 g, 4.33 mmol), DMAP (264 mg, 2.16 mmol) and DIPEA (1.13 mL, 6.49 mmol) and the reaction was stirred for 16 hours at room temperature. Sat. aq. $NaHCO_3$ solution (25 mL) was added to the mixture and extracted with $CH_2Cl_2$ (250 mL). The organic layer was dried over $MgSO_4$ and the solvent removed under reduced pressure. Flash column chromatography on $SiO_2$ (5- 30 % EtOAc in Cyclohexane) afforded the title compound (680 mg, 1.33 mmol, 61.7 %) as a colorless solid.
**$^1$H NMR** (400 MHz, $CDCl_3$) δ = 1.26 (d, *J* = 7.1 Hz, 2 H), 1.46 - 1.55 (m, 3 H), 2.28 - 2.41 (m, 2 H), 2.91 (dd, *J* = 14.3, 1.9 Hz, 1 H), 3.80 (s, 3 H), 3.90 (dd, *J* = 14.4, 10.9 Hz, 1 H), 3.94 - 3.98 (m, 1 H), 4.38 (d, *J* = 14.4 Hz, 1 H), 4.77 (dd, *J* = 4.2, 1.8 Hz, 1 H), 4.79 - 4.90 (m, 2 H), 5.00 (dd, *J* = 10.1, 1.3 Hz, 1 H), 5.66 (ddd, *J* = 17.0, 10.1, 8.9 Hz, 1 H), 6.86 (d, *J* = 8.8 Hz, 2 H), 7.20 (d, *J* = 8.8 Hz, 2 H), 7.54 - 7.57 (m, 1 H), 7.69 - 7.72 (m, 2 H).

**Example 7-8:** Preparation of (1S,5R,6R)-10-((3,5-dichlorophenyl)sulfonyl)-5-vinyl-3,10-diazabicyclo[4.3.1]decan-2-one **18**

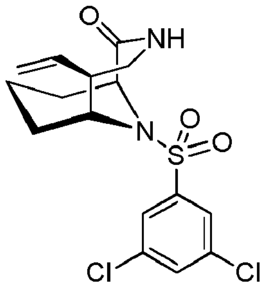

I8

To a solution of (1S,5R,6R)-10-((3,5-dichlorophenyl)sulfonyl)-3-(4-methoxybenzyl)-5-vinyl-3,10-diazabicyclo[4.3.1]decan-2-one (25.0 mg, 0.0491 mmol) in $MeCN/H_2O$ (1.5 mL, 2:1) was added CAN (81.0 mg, 0.147 mmol). After 4h stirring at room temperature sat. aq. NaCl solution (10 mL) was added to the mixture and extracted with $CH_2Cl_2$ (90 mL). The organic layer was dried over $MgSO_4$ and the solvent removed under reduced pressure. Flash column chromatography on $SiO_2$ (10 - 40 % EtOAc in Cyclohexane) afforded the title compound (17 mg, 0.0437 mmol, 89.0 %) as a colorless solid.
**$^1$H NMR** (300 MHz, $CDCl_3$) δ = 1.29 - 1.36 (m, 2 H), 1.54 - 1.64 (m, 3 H), 2.23 (d, *J* = 13.0 Hz, 2 H), 2.68 (d, *J* = 7.8 Hz, 1 H), 2.90 (d, *J* = 12.5 Hz, 1 H), 3.69 - 3.85 (m, 1 H), 4.04 (s, 1 H), 4.66 (d, *J* = 5.1 Hz, 1 H), 5.06 - 5.21 (m, 2 H), 5.72 - 5.87 (m, 1 H), 7.57 (t, *J* = 1.8 Hz, 1 H), 7.70 (d, *J* = 1.8 Hz, 2 H).
**$^{13}$C NMR** (75 MHz, $CDCl_3$) δ = 15.36, 26.48, 26.89, 26.96, 29.67, 45.07, 50.09, 55.47, 117.07, 124.89, 132.75, 136.37,

136.96, 143.97.

***Example 7.9:*** Preparation of (1S,5R,6R)-3-(3-(benzyloxy)propyl)-10-((3,5-dichlorophenyl)sulfonyl)-5-vinyl-3,10-diazabicyclo[4.3.1]decan-2-one **J1**

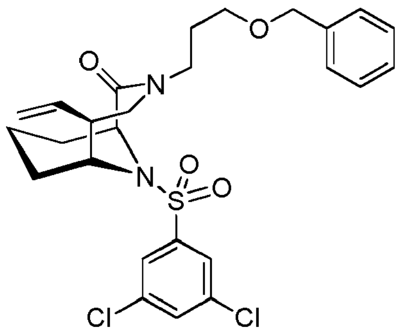

To a solution of (1S,5R,6R)-10-((3,5-dichlorophenyl)sulfonyl)-5-vinyl-3,10 - diazabicyclo [4.3.1] decan-2-one (35.0 mg, 0.0899 mmol) in DMF (1.0 mL) was added NaH (5.39 mg, 0.135 mmol). After 20 minutes ((3-bromopropoxy)methyl)benzene (20.6 mg, 0.0899 mmol) was added, the solution was heated to 80 °C and stirred for 1 h. $Et_2O$ (90 mL) was added to the solution and washed with sat. aq. NaCl solution (3 × 10 mL). The organic layer was dried over $MgSO_4$ and the solvent removed under reduced pressure. Column chromatography on $SiO_2$ (Cyclohexane/EtOAc = 1:1) afforded the title compound (31.0 mg, 0.0577 mmol, 64.2 %) as a colorless solid.
$\mathbf{R_f}$: 0.25 (Cyclohexane/EtOAc = 7:3)
**$^1$H *NMR*** (600 MHz, $CDCl_3$) $\delta$ = 1.28 (s, 2 H), 1.47 - 1.54 (m, 3 H), 1.83 - 1.92 (m, 2 H), 2.23 - 2.29 (m, 1 H), 2.58 - 2.64 (m, 1 H), 2.95 - 3.00 (m, 1 H), 3.49 - 3.59 (m, 4 H), 3.95 - 4.04 (m, 2 H), 4.50 (s, 2 H), 4.65 (d, J = 6.2 Hz, 1 H), 5.09 - 5.15 (m, 2 H), 5.74 - 5.82 (m, 1 H), 7.28 (s, 1 H), 7.33 (d, J = 1.3 Hz, 4 H), 7.56 (s, 1 H), 7.69 (d, J =1.8Hz,2H).
**$^{13}$C *NMR*** (150 MHz, $CDCl_3$) $\delta$ = 15.42, 26.29, 26.89, 27.36, 28.14, 29.68, 49.16, 49.53, 51.85, 54.83, 56.84, 67.64, 73.08, 116.8, 124.9, 127.6, 127.7, 128.4, 132.6, 136.3, 137.2, 138.2, 144.1, 169.7.

***Example 7.10:*** Preparation of (1S,5R,6R)-10-((3,5-dichlorophenyl)sulfonyl)-3-(3-methoxypropyl)-5-vinyl-3,10-diazabicyclo[4.3.1]decan-2-one ***J2***

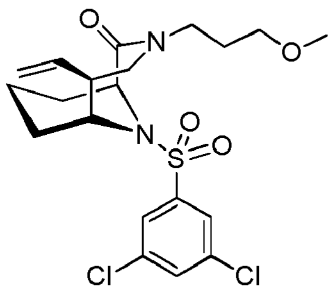

**J2**

To a solution of (1S,5R,6R)-10-((3,5-dichlorophenyl)sulfonyl)-5-vinyl-3,10-diazabicyclo [4.3.1]decan-2-one (35.0 mg, 0.0899 mmol) in DMF (1.0 mL) was added NaH (5.39 mg, 0.135 mmol). After 20 minutes 1-bromo-4-methoxypropane (13.8 mg, 0.0102 mL, 0.0899 mmol) was added, the solution was heated to 80 °C and stirred for 1 h. $Et_2O$ (90 mL) was added to the solution and washed with sat. aq. NaCl solution (3 x 10 mL). The organic layer was dried over $MgSO_4$ and the solvent removed under reduced pressure. Column chromatography on $SiO_2$ (Cyclohexane/EtOAc = 3:7) afforded the title compound (20.0 mg, 0.0433 mmol, 48.2 %) as a colorless solid.
$\mathbf{R_f}$: 0.57 (Cyclohexane/EtOAc = 1:1)
**$^1$H NMR** (600 MHz, $CDCl_3$) $\delta$ = 1.27 - 1.34 (m, 2 H), 1.50 - 1.56 (m, 3 H), 1.83 (q, *J* = 6.9 Hz, 2 H), 2.26 (d, *J* = 13.4 Hz, 1 H), 2.63 (qd, *J* = 8.3, 4.1 Hz, 1 H), 2.94 - 3.00 (m, 1 H), 3.33 (s, 3 H), 3.41 (t, *J* = 6.2 Hz, 2 H), 3.47 - 3.59 (m, 2 H), 3.96 - 4.04 (m, 2 H), 4.65 (d, *J* = 5.7 Hz, 1 H), 5.11 (s, 1 H), 5.14 (d, *J* = 6.0 Hz, 1 H), 5.79 (dt, *J* = 16.7, 9.4 Hz, 1 H),

7.56 (s, 1 H), 7.69 (s, 2 H).
**$^{13}$C NMR** (150 MHz, $CDCl_3$) $\delta$ = 15.42, 26.29, 27.36, 28.03, 49.21, 49.53, 51.88, 54.83, 56.84, 58.68, 70.04, 116.8, 124.9, 132.6, 136.3, 137.3, 144.1, 169.7.

**Example 7.11:** Preparation of (1S,5R,6R)-10-((3,5-dichlorophenyl)sulfonyl)-3-(4-methoxybutyl)-5-vinyl-3,10-diazabicyclo[4.3.1]decan-2-one **J3**

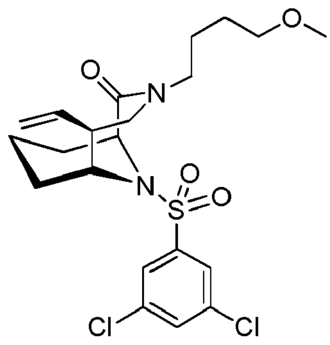

**J3**

To a solution of (1S,5R,6R)-10-((3,5-dichlorophenyl)sulfonyl)-5-vinyl-3,10-diazabicyclo [4.3.1] decan-2-one (35.0 mg, 0.0899 mmol) in DMF (1.0 mL) was added NaH (5.39 mg, 0.135 mmol). After 20 minutes 1-bromo-4-methoxybutane (15.0 mg, 0.0118 mL, 0.0899 mmol) was added, the solution was heated to 80 °C and stirred for 1 h. $Et_2O$ (90 mL) was added to the solution and washed with sat. aq. NaCl solution (3 x 10 mL). The organic layer was dried over $MgSO_4$ and the solvent removed under reduced pressure. Column chromatography on $SiO_2$ (Cyclohexane/EtOAc = 3:7) afforded the title compound (27.8 mg, 0.0584 mmol, 65.1 %) as a colorless solid.
**$R_f$:** 0.16 (Cyclohexane/EtOAc = 1:1)
**$^1$H NMR** (600 MHz, $CDCl_3$) $\delta$ =1.24 - 1.26 (m, 2 H), 1.51 - 1.55 (m, 3 H), 1.55 - 1.64 (m, 4 H), 2.26 (dt, *J* = 14.3, 2.4 Hz, 1 H), 2.59 - 2.65 (m, 1 H), 2.90 - 2.95 (m, 1 H), 3.33 (d, *J* = 1.0 Hz, 3 H), 3.38 - 3.42 (m, 2 H), 3.42 - 3.50 (m, 2 H), 3.96 - 4.02 (m, 2 H), 4.66 (dt, *J* = 5.8, 1.9 Hz, 1 H), 5.11 - 5.16 (m, 2 H), 5.76 - 5.84 (m, 1 H), 7.55 - 7.57 (m, 1 H), 7.68 - 7.69 (m, 2 H).
**$^{13}$C NMR** (150 MHz, $CDCl_3$) $\delta$ = 15.45, 24.43, 26.31, 26.78, 27.39, 29.67, 49.55, 51.18, 51.43, 54.83, 56.86, 58.53, 72.20, 116.8, 124.9, 132.6, 136.3, 137.3, 144.1, 169.6.

**Example 7.12:** Preparation of (1S,5R,6R)-10-((3,5-dichlorophenyl)sulfonyl)-3-(pyridin-2-ylmethyl)-5-vinyl-3,10-diazabicyclo[4.3.1]decan-2-one **J4**

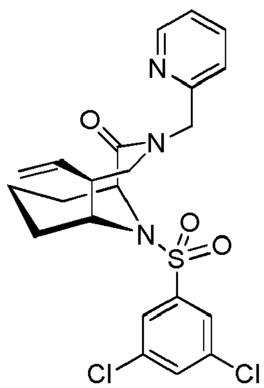

**J4**

To a solution of (1S,5R,6R)-10-((3,5-dichlorophenyl)sulfonyl)-5-vinyl-3,10-diazabicyclo [4.3.1] decan-2-one (35.0 mg, 0.0899 mmol) in DMF (1.0 mL) was added NaH (5.39 mg, 0.135 mmol). At the same time 2-(bromomethyl)pyridine-hydrobromide (22.7 mg, 0.0899 mmo) in DMF (1 mL) was neutralized with NaH (2.16 mg, 0.0899 mmol). After 20 minutes the solution were united and heated to 80 °C and stirred for 1 h. $Et_2O$ (90 mL) was added to the solution

and washed with sat. aq. NaCl solution (3 x 10 mL). The organic layer was dried over $MgSO_4$ and the solvent removed under reduced pressure. Column chromatography on $SiO_2$ (Cyclohexane/EtOAc = 1:1) afforded the title compound (43 mg, 0.0899 mmol, 100 %) as a colorless solid.

**$R_f$:** 0.2 (Cyclohexane/EtOAc = 1:1)

**$^1$H NMR** (600MHz, $CDCl_3$) $\delta$ = 1.30 - 1.39 (m, 2 H), 1.52 - 1.63 (m, 3 H), 2.26 - 2.33 (m, 1 H), 2.73 (q, *J* = 8.3, 7.9 Hz, 1 H), 3.14 (dd, *J* = 14.3, 2.0 Hz, 1 H), 4.00 - 4.06 (m, 2 H), 4.74 (dt, *J* = 6.1, 1.7 Hz, 1 H), 4.86 (d, *J* = 15.4 Hz, 1 H), 4.90 - 4.98 (m, 1 H), 4.98 - 5.08 (m, 2 H), 5.72 (dddd, *J* = 17.0, 10.0, 8.8, 1.2 Hz, 1 H), 7.29 (t, *J* = 6.3 Hz, 1 H), 7.41 (d, *J* = 7.9 Hz, 1 H), 7.57 (td, *J* = 1.8, 1.0 Hz, 1 H), 7.70 (dd, *J* = 1.9, 1.0 Hz, 2 H), 7.79 (t, *J* = 7.7 Hz, 1 H), 8.49 - 8.61 (m, 1 H).

**$^{13}$C NMR** (150 MHz, $CDCl_3$) $\delta$ = 15.46, 26.39, 27.49, 29.67, 49.06, 52.20, 54.89, 55.51, 56.85, 117.02, 122.55, 122.85, 124.84, 132.72, 136.34, 136.93, 143.95, 156.31, 170.56.

**Example 7.13:** Preparation of (1S,5R,6R)-10-((3,5-dichlorophenyl)sulfonyl)-5-(hydroxymethyl)-3-(3-hydroxypropyl)-3,10-diazabicyclo[4.3.1]decan-2-one **J5**

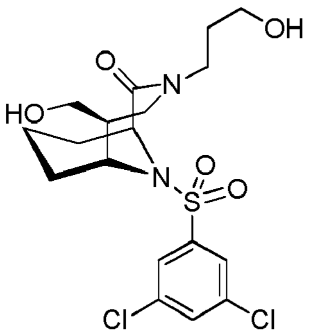

**J5**

To a solution of (1S,5R,6R)-3-(3-(benzyloxy)propyl)-10-((3,5-dichlorophenyl)sulfonyl)-5-vinyl-3,10-diazabicyclo[4.3.1]decan-2-one (31 mg, 0.0576 mmol) in Dioxane/$H_2O$ (0.8 mL, 3:1) was added $NaIO_4$ (52.3 mg, 0.245 mmol), $OsO_4$ (2.5 % Solution in *tert*-Butanol, 0.00122 mmol, 0.015 mL) and 2,6-Lutidine (0.014 mL, 0.122 mmol). The solution was stirred for 18 h at room temperature, then $Et_2O$ (90 mL) was added to the reaction and washed with sat. aq. NaCl solution (3 x 10 mL). The obtained crude product was dissolved in EtOH (1 mL) and $NaBH_4$ (3.47 mg, 0.0917 mmol) was added and stired for 1 h at room temperature. $Et_2O$ (90 mL) was added to the reaction and washed with sat. aq. NaCl solution (3 x 10 mL). The solvent was removed under reduced pressure and the crude mixture was dissolved in $CH_2Cl_2$ (0.5 mL). $BCl_3$ $SMe_2$ (2 M solution in $CH_2Cl_2$, 0.144 mL, 0.288 mmol) and the reaction was stirred for 30 minutes. $Et_2O$ (90 mL) was added to the reaction and washed with sat. aq. NaCl solution (3 x 10 mL). The solvent was removed under reduced pressure and flash column chromatography over $SiO_2$ (50 - 90 % EtOAc in Cyclohexane) afforded the title compound (18 mg, 0.0398 mmol, 68.8 %) as a colorless wax.

**$^1$H NMR** (600 MHz, $CDCl_3$) $\delta$ = 1.36 - 1.42 (m, 2 H), 1.52 - 1.58 (m, 3 H), 1.71-1.80 (m, 2 H), 2.19 - 2.29 (m, 2 H), 2.52 - 2.61 (m, 2 H), 3.20 - 3.24 (m, 1 H), 3.49 - 3.55 (m, 2 H), 3.56 - 3.64 (m, 3 H), 3.73 (s, 1 H), 3.84 (dd, *J* = 14.3, 10.7 Hz, 1 H), 3.88 - 3.92 (m, 1 H), 4.71 (d, *J* = 6.1 Hz, 1 H), 7.57 (s, 1 H), 7.69 (d, *J* = 1.9 Hz, 2 H).

**MS** (ESI): *m/z (%)* = 451.14 $[M+H]^+$

**Example 7.14:** Preparation of (1S,5R,6R)-10-((3,5-dichlorophenyl)sulfonyl)-5-(hydroxymethyl)-3-(3-methoxypropyl)-3,10-diazabicyclo[4.3.1]decan-2-one **J6**

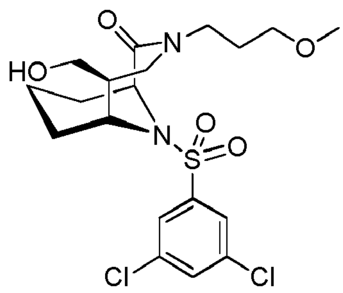

**J6**

To a solution of (1S,5R,6R)-10-((3,5-dichlorophenyl)sulfonyl)-3-(3-methoxypropyl)-5-vinyl-3,10-diazabicyclo[4.3.1]decan-2-one (14 mg, 0.0299 mmol) in Dioxane/$H_2O$ (0.4 mL, 3:1) was added $NaIO_4$ (26.0 mg, 0.121 mmol), $OsO_4$ (2.5 % Solution in *tert*-Butanol, 0.00607 mmol, 0.00761 mL) and 2,6-Lutidine (0.00707 mL, 0.061 mmol). The solution was stirred for 24 h at room temperature, then $Et_2O$ (90 mL) was added to the reaction and washed with sat. aq. NaCl solution (3 x 10 mL). The obtained crude product was dissolved in EtOH (1 mL) and $NaBH_4$ (1.72 mg, 0.046 mmol) was added and stirred for 1 h at room temperature. $Et_2O$ (90 mL) was added to the reaction and washed with sat. aq. NaCl solution (3 x 10 mL). The solvent was removed under reduced pressure and flash column chromatography over $SiO_2$ (50 - 70 % EtOAc in Cyclohexane) afforded the title compound (13.0 mg, 0.0279 mmol, 93 %) as a colorless solid.
**$R_f$:** 0.18 (Cyclohexane/EtOAc = 1:4)
**MS** (ESI): m/z (%) = 465.14 $[M + H]^+$

**Example 7.15:** Preparation of (1S,5R,6R)-10-((3,5-dichlorophenyl)sulfonyl)-5-(hydroxymethyl)-3-(4-methoxybutyl)-3,10-diazabicyclo[4.3.1]decan-2-one J7

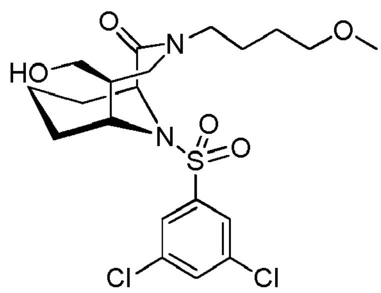

**J7**

To a solution of (1S,5R,6R)-10-((3,5-dichlorophenyl)sulfonyl)-3-(4-methoxybutyl)-5-vinyl-3,10-diazabicyclo[4.3.1]decan-2-one (24 mg, 0.0499 mmol) in Dioxane/$H_2O$ (0.8 mL, 3:1) was added $NaIO_4$ (43.2 mg, 0.202 mmol), $OsO_4$ (2.5 % Solution in *tert*-Butanol, 0.0101 mmol, 0.013 mL) and 2,6-Lutidine (0.012 mL, 0.101 mmol). The solution was stirred for 24 h at room temperature, then $Et_2O$ (90 mL) was added to the reaction and washed with sat. aq. NaCl solution (3 x 10 mL). The obtained crude product was dissolved in EtOH (1 mL) and $NaBH_4$ (2.86 mg, 0.076 mmol) was added and stirred for 1 h at room temperature. $Et_2O$ (90 mL) was added to the reaction and washed with sat. aq. NaCl solution (3 x 10 mL). The solvent was removed under reduced pressure and flash column chromatography over $SiO_2$ (50 - 90 % EtOAc in Cyclohexane) afforded the title compound (17.5 mg, 0.0365 mmol, 73.0 %) as a colorless solid.
**$R_f$:** 0.18 (Cyclohexane/EtOAc = 1:4)
**MS** (ESI): m/z (%) = 479.22 $[M + H]^+$

**Example 7-16:** Preparation of (1S,5R,6R)-10-((3,5-dichlorophenyl)sulfonyl)-5-(hydroxymethyl)-3-(pyridin-2-ylmethyl)-3,10-diazabicyclo[4.3.1]decan-2-one **J8**

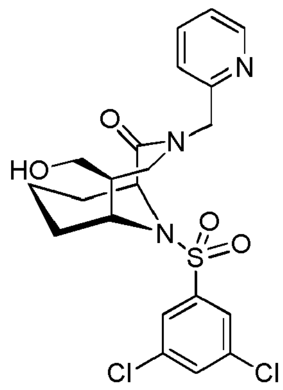

J8

To a solution of (1S,5R,6R)-10-((3,5-dichlorophenyl)sulfonyl)-3-(pyridin-2-ylmethyl)-5-vinyl-3,10-diazabicyclo[4.3.1]decan-2-one (43 mg, 0.0891 mmol) in Dioxane/$H_2O$ (0.8 mL, 3:1) was added $NaIO_4$ (76 mg, 0.357 mmol), $OsO_4$ (2.5 % Solution in *tert*-Butanol, 0.00178 mmol, 0.022 mL) and 2,6-Lutidine (0.021 mL, 0.178 mmol). The solution was stirred for 24 h at room temperature, then $Et_2O$ (90 mL) was added to the reaction and washed with sat. aq. NaCl solution (3 x 10 mL). The obtained crude product was dissolved in EtOH (1 mL) and $NaBH_4$ (5.00 mg, 0.132 mmol) was added and stirred for 1 h at room temperature. $Et_2O$ (90 mL) was added to the reaction and washed with sat. aq. NaCl solution (3 x 10 mL). Flash column chromatography over $SiO_2$ (30 - 70 % EtOAc in Cyclohexane) afforded the title compound (16 mg, 0.0330 mmol, 37.1 %) as a colorless solid.

**$^1$H NMR** (600 MHz, $CDCl_3$) $\delta$ = 1.24 - 1.30 (m, 2 H), 1.49 - 1.58 (m, 3 H), 2.25 - 2.33 (m, 2 H), 3.48 - 3.56 (m, 3 H), 3.86 (dd, *J* = 14.4, 10.6 Hz, 1 H), 3.92 - 3.96 (m, 1 H), 4.74 - 4.77 (m, 1 H), 4.87 (d, *J* = 7.9 Hz, 2 H), 7.29 - 7.34 (m, 1 H), 7.45 (s, 1 H), 7.56 (s, 1 H), 7.70 (s, 2 H), 7.78 - 7.83 (m, 1 H), 8.54 (dt, *J* = 5.2, 0.9 Hz, 1 H).

**$^{13}$C NMR** (150 MHz, $CDCl_3$) $\delta$ = 15.51, 27.80, 27.82, 46.93, 49.67, 52.32, 55.22, 57.01, 63.25, 123.10, 123.22, 124.87, 132.71, 136.31, 138.63, 143.91, 147.63, 156.12, 170.54.

**Example 8: Fluorescence polarization (FP) assays:**

In-vitro fluorescence polarization assays were performed to determine the binding affinities for FKBP51 and 52 according to a literature procedure (Kozany, C.; Marz, A.; Kress, C.; Hausch, F., Fluorescent probes to characterise FK506-binding proteins. Chembiochem 2009, 10, (8), 1402-10).

For fluorescence polarization assays the fluorescent ligand **2b** was dissolved in HEPES (20 mm, pH 8), Triton-X100 (0.01 %), at double the concentration required for the final sample. The target protein was also diluted in this assay buffer at double the highest concentration required for the final sample. This protein stock was used for a 1:1 serial dilution.

The fluorescent ligand **2b** was diluted in assay buffer to a concentration double the final concentration (20nM **2b).** The inventive compound was dissolved in DMSO to reach a 100-times concentrated stock solution. This was used for a 1:1 serial dilution in DMSO. Every sample of this serial dilution was diluted by a factor of 50 in assay buffer supplemented with ligand **2b** to achieve a 2x concentrated mixture of ligand **2b** and the inventive compound. To this competitive ligand double the protein concentration for **2b** assay: FKBP51 FK1 560 nM (2x280 nM) or FKBP52FK1 800 nM (2x400 nM) diluted in assay buffer was added.

The samples were transferred to black 384-well assay plates (No.: 3575; Corning Life Sciences). After incubation at room temperature for 30 min the fluorescence anisotropy was measured (GENios Pro, Tecan, Männedorf, Switzerland) by using an excitation filters of 485/20 nm and emission filters of 535/25 nm. The binding assays were performed in duplicates in the plate format.

The competition curves were analyzed by using SigmaPlot9. Data were fitted to a four parameter logistic curve to deduce the $IC_{50}$ values. For the analysis of $K_i$ values, data were fitted to the following equation (Z.X. Wang, FEBS Lett. 1995, 360, 111-114).

$$A = (A_{max} - A_{min}) / [L]_t \times (([L]_t \times ((2\times((K_{lig} + K_{comp} + [L]_t + [I]_t - [R]_t)^{2} - 3\times( K_{comp} \times ([L]_t - [R]_t) + K_{lig} \times ([I]_t - [R]_t) + K_{lig} \times K_{comp}))^{0.5}\times COS(ARCCOS((-2\times( K_{lig} + K_{comp} + [L]_t + [I]_t - [R]_t)^{3} + 9\times( K_{lig} + K_{comp} + [L]_t + [I]_t - [R]_t) \times ( K_{comp} \times ([L]_t - [R]_t)+ K_{lig} \times ([I]_t - [R]_t) + K_{lig} \times K_{comp}) - 27\times(-1\times K_{lig} \times K_{comp} \times [R]_t)) / (2\times (((( K_{lig} + K_{comp} + [L]_t + [I]_t - [R]_t)^{2} - 3\times ( K_{comp} \times ([L]_t - [R]_t) + K_{lig} \times ([I]_t - [R]_t) + K_{lig} \times K_{comp}))^{3})^{0.5}))) / 3)) - (K_{lig} + K_{comp} + [L]_t + [I]_t - [R]_t))) / ((3\times K_{lig}) + ((2\times(( K_{lig} + K_{comp} + [L]_t + [I]_t - [R]_t)^{2} - 3\times(K_{comp} \times ([L]_t - [R]_t) + K_{lig} \times ([I]_t - [R]_t) + K_{lig} \times K_{comp}))^{0.5}\times COS(ARCCOS((-2\times(K_{lig} + K_{comp} + [L]_t + [I]_t - [R]_t)^{3} + 9\times(K_{lig} + K_{comp} + [L]_t + [I]_t - [R]_t) \times (K_{comp} \times ([L]_t - [R]_t) + K_{lig} \times ([I]_t - [R]_t) + K_{lig} \times K_{comp}) - 27\times(-1\times K_{lig} \times K_{comp} \times [R]_t)) / (2\times(((( K_{lig} + K_{comp} + [L]_t + [I]_t - [R]_t)^{2} - 3\times(K_{comp} \times ([L]_t - [R]_t) + K_{lig} \times ([I]_t - [R]_t) + K_{lig} \times K_{comp}))^{3})^{0.5})))/3)) - (K_{lig} + K_{comp} + [L]_t + [I]_t - [R]_t)))) + A_{min}$$

In this equation $K_{lig}$ and $K_{comp}$ stand for the $K_d$ values of the used tracer or competing inhibitor, $[I]_t$ is referring to the total concentration of the titrated inhibitor.

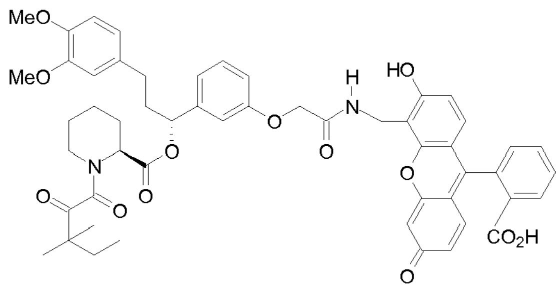

**2b**

These assays revealed that substitutents $\mathbf{R^C}$ significantly increased the affinity to FKBP51/52 compared to known FKBP51 ligands (Ref.1 - Ref.5). **Table 1** summarize the binding data of exemplary compounds.

The compounds **B3 - J8** are FKBP51/52 inhibitors of which Ki values are:

| | |
|---|---|
| $K_i \leq 0.1\ \mu M$ | ++++ |
| $0.1\ \mu M < K_i \leq 0.5\ \mu M$ | +++ |
| $0.5\ \mu M < K_i \leq 1\ \mu M$ | ++ |
| $1\ \mu M < K_i \leq 5\ \mu M$ | + |
| $5\ \mu M < K_i$ | ○ |

**Table 1:** Chemical compounds according to formula (I) tested for binding to the FK506-binding domain of FKBP51/52 using fluorescence polarization assay (Kozany et al, ChemBioChem 2009, 10, 1402)

| Compound | FKBP51 $K_i$ [$\mu$M] | FKBP52 $K_i$ [$\mu$M] |
|---|---|---|
| **B3** | ++++ | ++++ |
| **B4&B5** | +++ | ++++ |
| **B6** | ++++ | ++++ |
| **B7** | ++++ | ++++ |
| **B8** | +++ | +++ |
| **B10** | + | ○ |
| **B11** | +++ | +++ |
| **B12** | +++ | +++ |
| **B13** | ++++ | +++ |
| **B14** | ++++ | +++ |

(continued)

| Compound | FKBP51 $K_i$ [$\mu$M] | FKBP52 $K_i$ [$\mu$M] |
|---|---|---|
| B15 | ++++ | ++++ |
| C1 | ++++ | ++++ |
| C2(C2a/b) | ++++ | ++++ |
| C3 | +++ | +++ |
| D3 | ++++ | ++++ |
| D4 | ++++ | ++++ |
| D5 | ++++ | ++++ |
| D6 | ++++ | ++++ |
| D7 | ++++ | ++++ |
| D8 | ++++ | ++++ |
| G6 | ++ | +++ |
| H1.2 | + | + |
| H1.3 | ++ | ++ |
| H1.4 | + | + |
| H2.1 | +++ | +++ |
| H2.2 | ++ | ++ |
| H2.3 | ++++ | +++ |
| J2 | + | + |
| J3 | ++ | + |
| J4 | +++ | +++ |
| J5 | ++ | ++ |
| J6 | +++ | ++ |
| J7 | ++ | ++ |
| J8 | ++++ | ++++ |
| FK506 | ++++ | ++++ |
| Ref. 1 | + | + |
| Ref. 2 | + | + |
| Ref. 3 | +++ | + |
| Ref. 4 | ○ | ○ |
| Ref. 5 | + | + |

Reference compounds Ref.1 - Ref. 5 and FK506 have the following structures:

| Compound | Structure | Compound | Structure |
|---|---|---|---|
| Ref.1 | 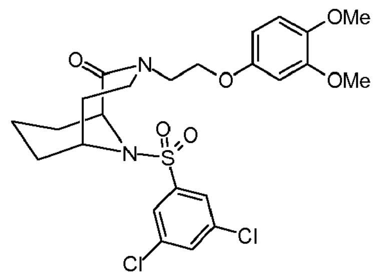 | Ref.4 | 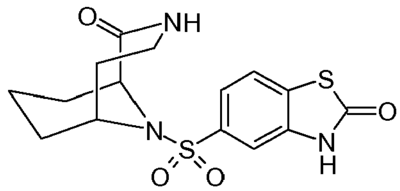 |
| Ref.2 | 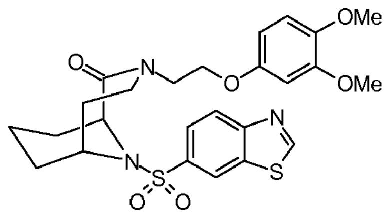 | Ref.5 | 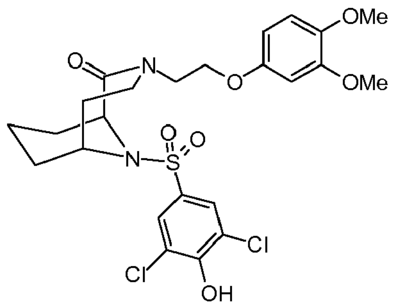 |
| Ref.3 | 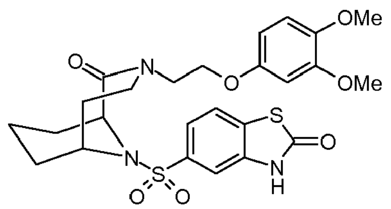 | FK506 | 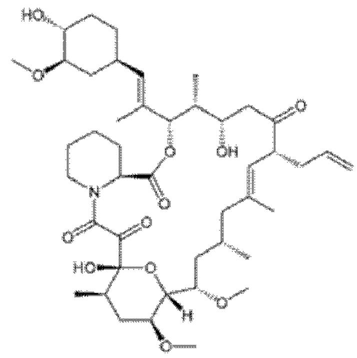 |

**Example 9: N2a Cellular assay**

At day one N2a cells were plated into 24-well plates with cover slips (pretreated with polylysine) at a density of 35,000 cells/well and cultured with DMEM (incl. FCS 10% and Pen/Strep 1 %) for 24 h. Next, cells were transfected with 80 ng expression plasmids encoding Venus as well as 720 ng pRK5 (mock transfection) in a total volume of 500 μl starvation media containing different concentrations of compounds or DMSO for 36 h (media without FCS; induction of neurite outgrowth). Therefore media was removed and replaced by 400 μl DMEM (empty). Next an equivalent volume of plasmids were given to 50 μl OPTIMEM and incubated for 5 min at RT. Additionally 1,5 μl Lipofectamine 2000 was separately dissolved in 50 μl OPTIMEM. After 5 min both solutions (plasmids and Lipofactamine 2000 containing media) were combined and incubated again for another 20 min. After that 100 μl of this mixture was given to 400 μl media per well. (See also protocol of the provider - Life Technologies).

On the next day cells were washed with PBS and incubated for 30 min with 300 μl PFA (4%) and sucrose (5%) to fix cells. After fixation cells were washed three times, mounted onto microscope slides using 4 μl Vectashield (mounting media) and analyzed by fluorescence microscopy. Each bar represents the mean of the neurite length of 30 - 50 cells after the indicated treatment **(Figure 1).**

FK506 was included (see Fig. 1 (c)). None of the Reference compounds 1-5 are good enough that a cellular N2a experiment would be warranted.

## Claims

**1.** Compound of the general formula (I):

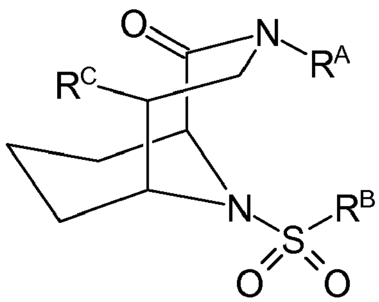

(I)

wherein

**$R^A$** represents $-H$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH_2OH$, $-C_2H_4OH$, $-C_3H_6OH$, $-C_4H_8OH$, $-CH(CH_3)-C_2H_4OH$, $-C_5H_{10}OH$, $-CH_2OCH_3$, $-C_2H_4OCH_3$, $-C_3H_6OCH_3$, $-C_4H_8OCH_3$, $-CH(CH_3)-C_2H_4OCH_3$, $-C_5H_{10}OCH_3$, $-CH_2NH_2$, $-C_2H_4NH_2$, $-C_3H_6NH_2$, $-C_4H_8NH_2$, $-CH(CH_3)-C_2H_4NH_2$, $-C_5H_{10}NH_2$, $-C_2H_4NHCH_3$, $-C_2H_4N(CH_3)_2$, $-C_2H_4N(CH_3)_2$, $-C_2H_4NHCH(CH_3)_2$, $-C_2H_4NH(CH_2CH_3)$, $-C_2H_4N(CH_2CH_3)_2$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$,- $CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C\equiv CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-CH_2-Ph$,

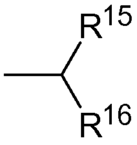 , 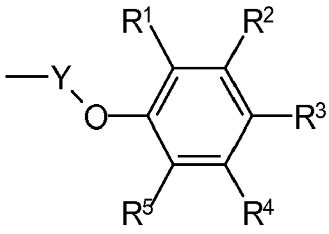 , 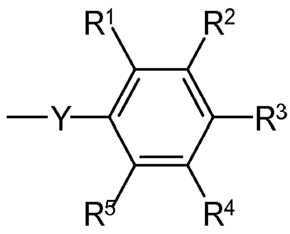 or 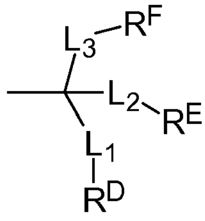 ;

Y represents $-CH_2-$, $-CH_2-CH_2-$, $-CH_2-CH_2-CH_2-$, -CH=CH-, $-CH=CH-CH_2-$, $-CH_2-CH=CH-$, $-CHCH_3-$, $-CHCH_3-CH_2-$, $-CH_2-CHCH_3-$, $-CH_2-CHCH_3-CH_2-$, or $-CH_2-O-CH_2-$;

**$R^D$** represents: **$R^{22}$** or

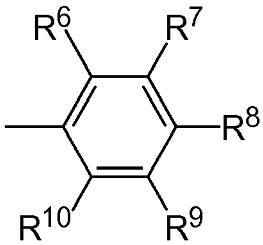 , 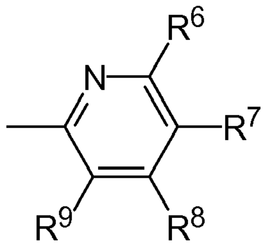 , 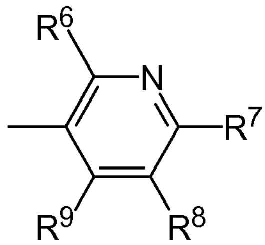 , 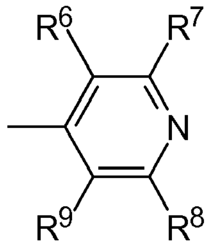 ,

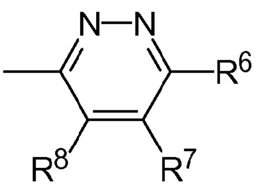 , 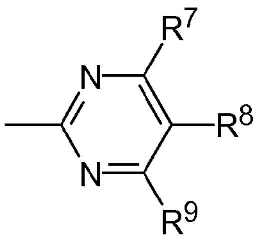 , 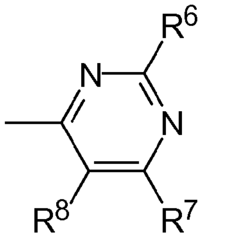 , 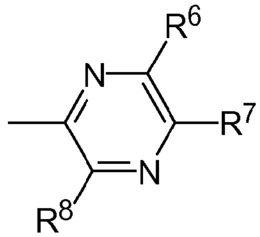 ,

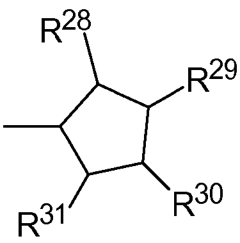 , 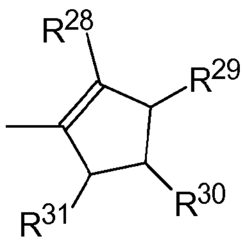 , 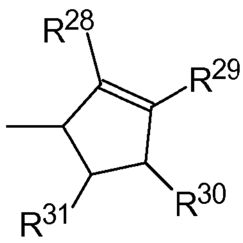 , 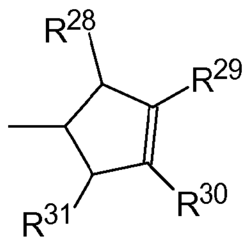 ,

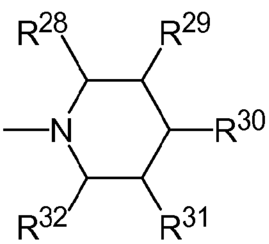 , 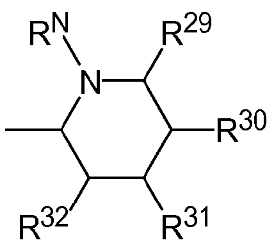 , 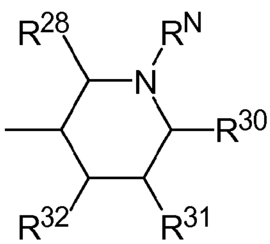 , 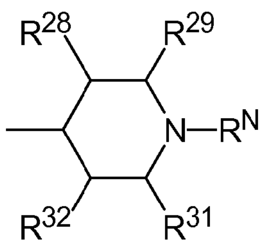 ,

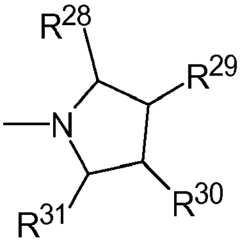,

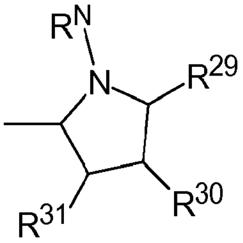,

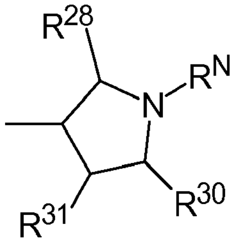,

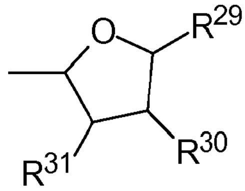,

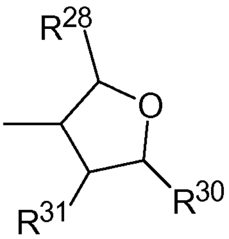,

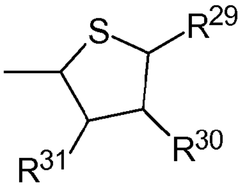,

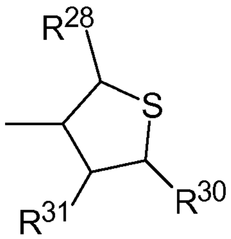,

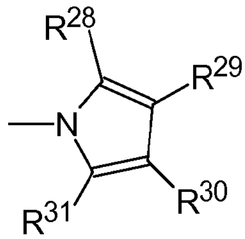,

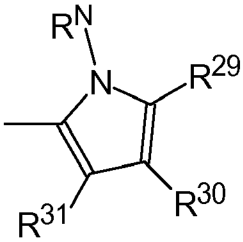,

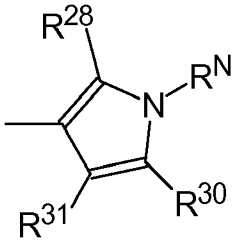,

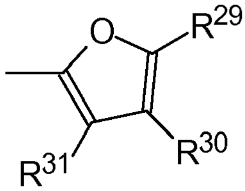,

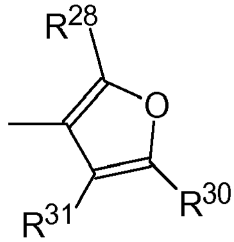,

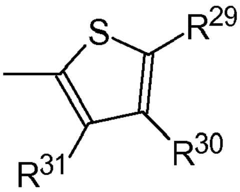,

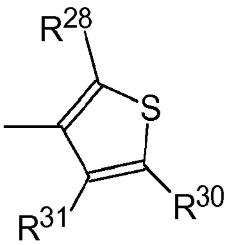,

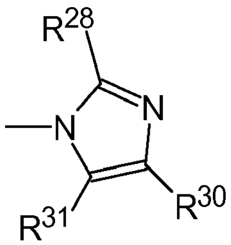,

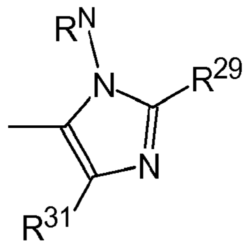,

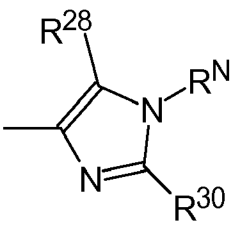,

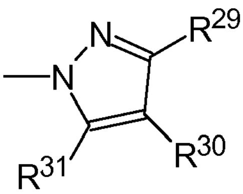,

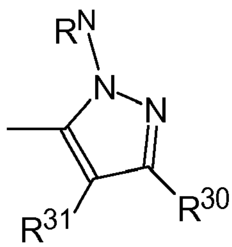,

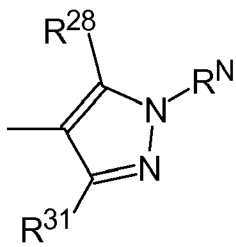,

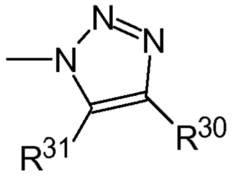,
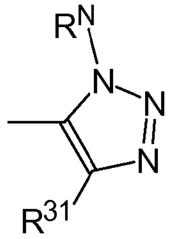,
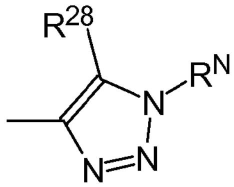,
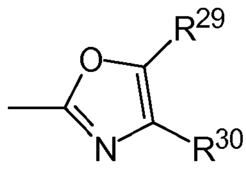,
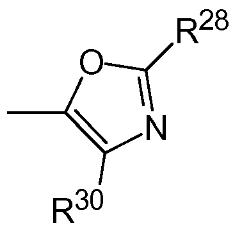,
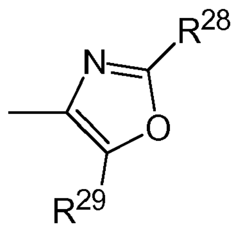,
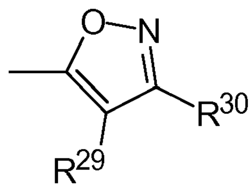,
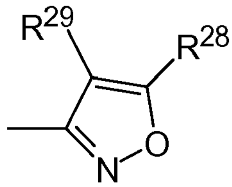,
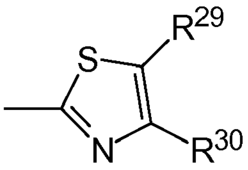,
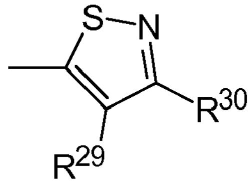,
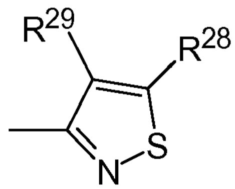,
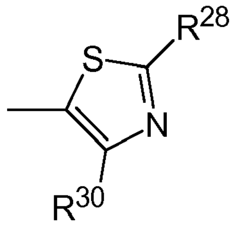,
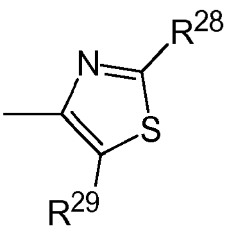,
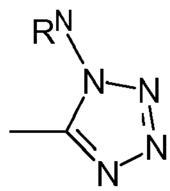,
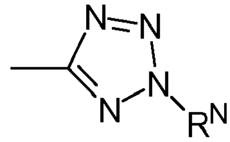
or
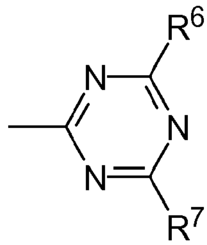;

$\mathbf{R^E}$ represents: $\mathbf{R^{23}}$ or

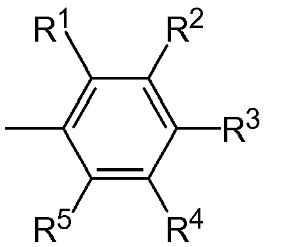,
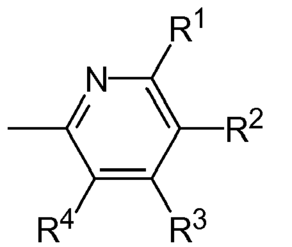,
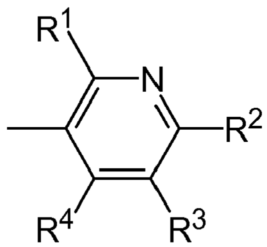,

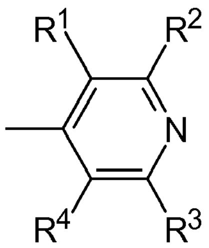,
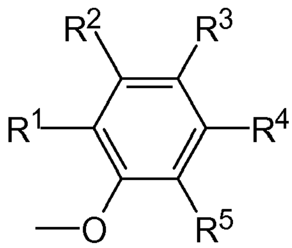,
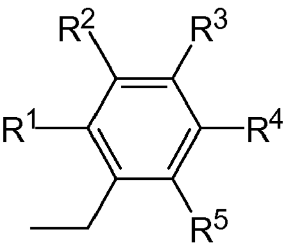,
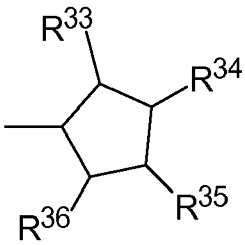,
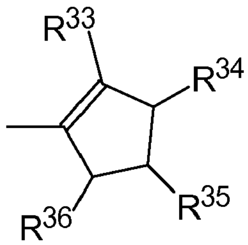,
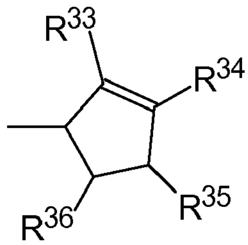,
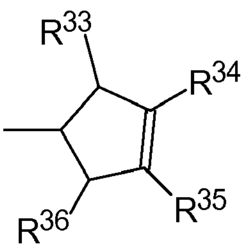,
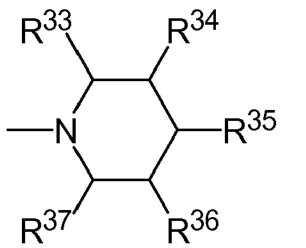,
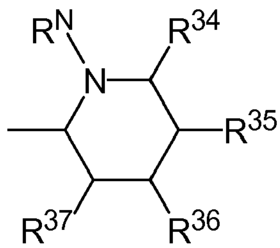,
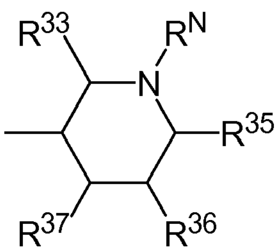,
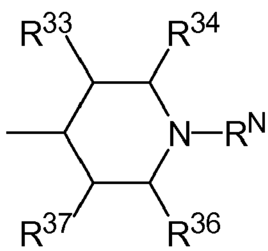,
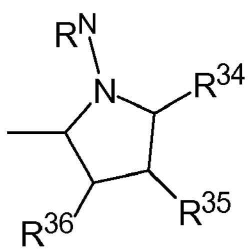,
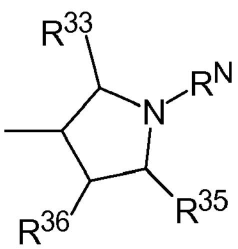,
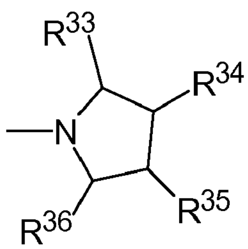,
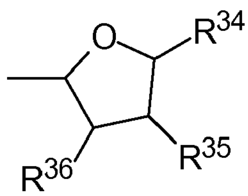,

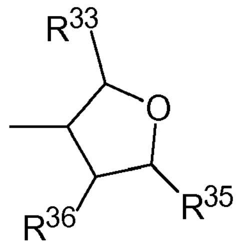
,
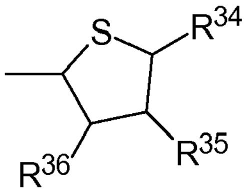
,
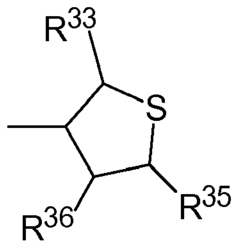
,
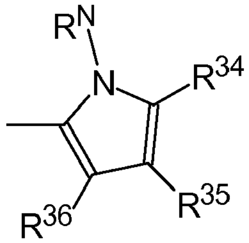
,
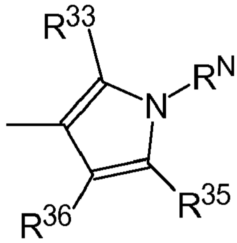
,
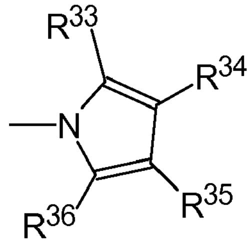
,
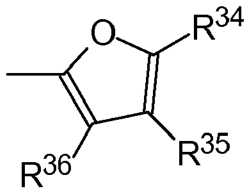
,
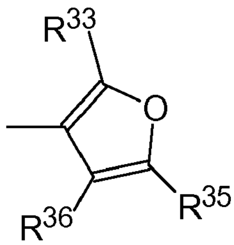
,
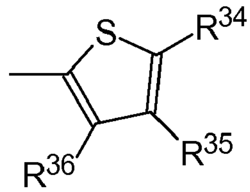
,
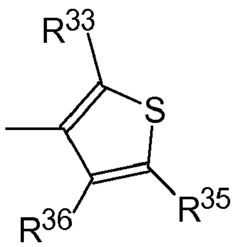
,
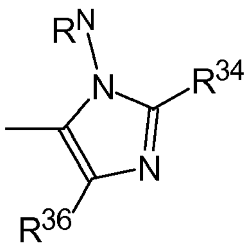
,
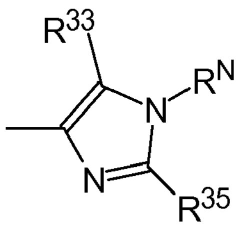
,
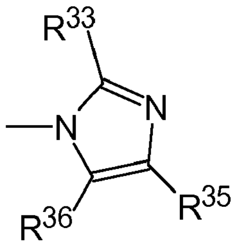
,
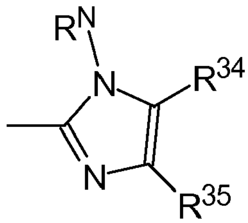
,
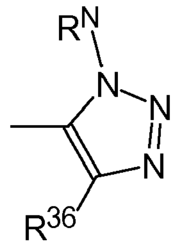
,
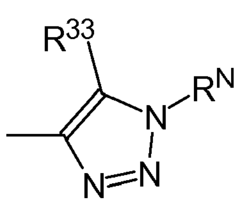
,
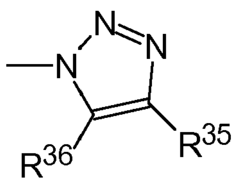
or
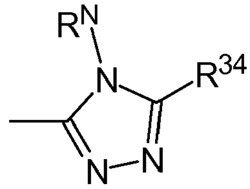
;

**$R^F$** represents: **$R^{24}$** or

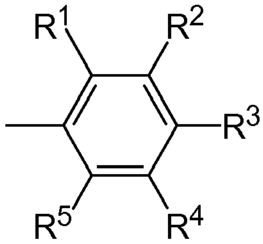
,
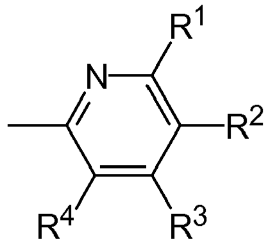
,
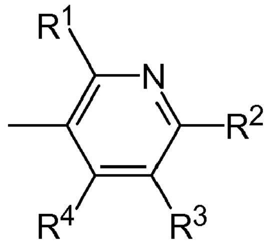
,

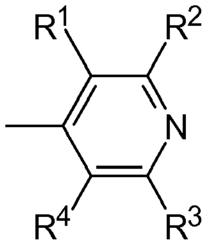
,
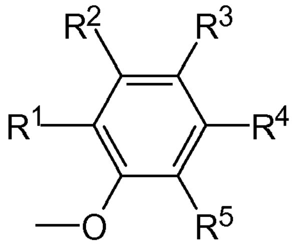
,
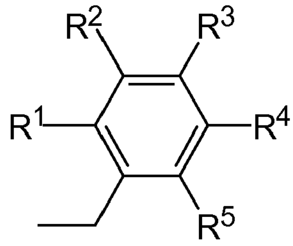
,

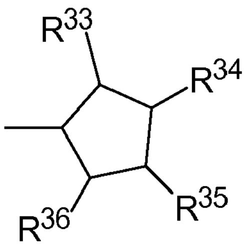
,
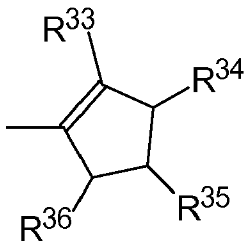
,
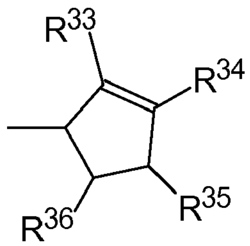
,

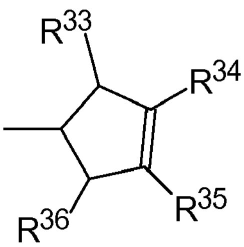
,
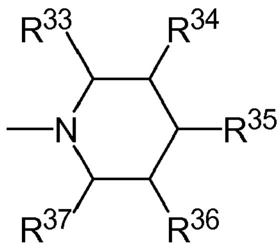
,
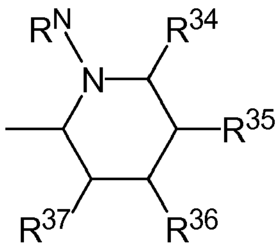
,

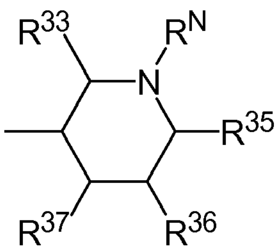
,
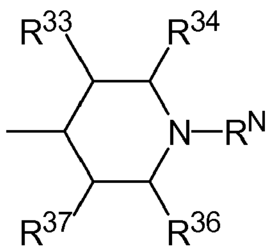
,
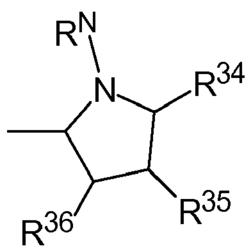
,

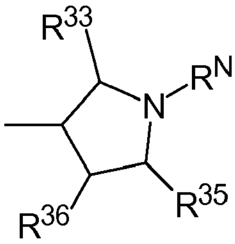
,
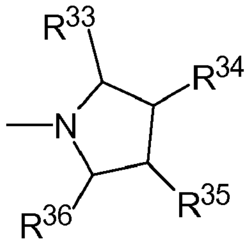
,
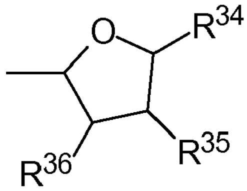
,

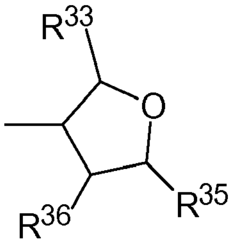
,
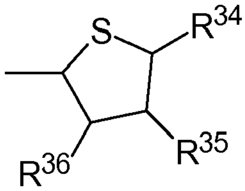
,
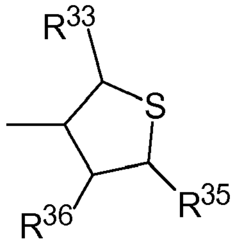
,

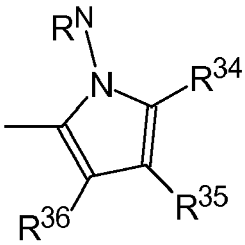
,
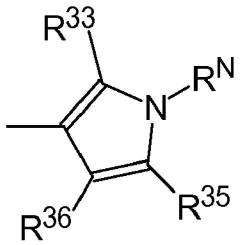
,
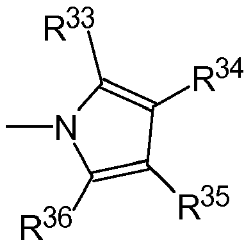
,

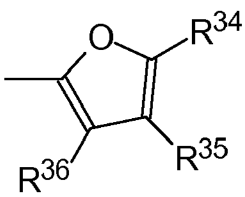
,
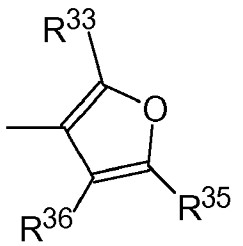
,
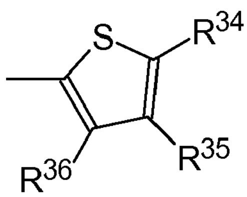
,

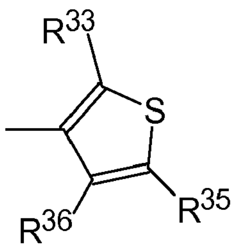
,
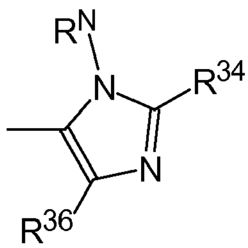
,
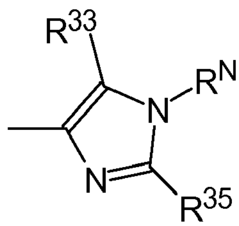
,

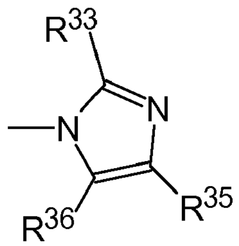 , 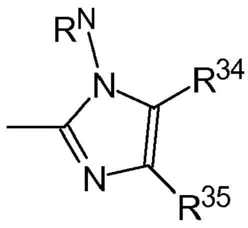 , 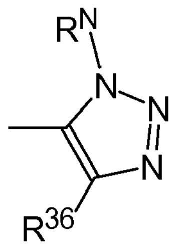 ,

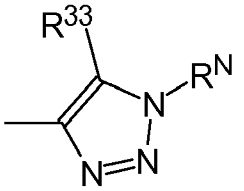 , 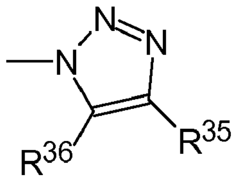 or 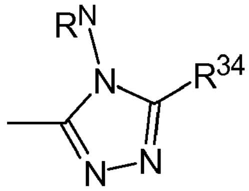 ;

**$L_1$, $L_2$** and **$L_3$** represent independently of each other:

a bond, $-CH_2-$, $-C_2H_4-$, $-C_3H_6-$, $-C_4H_8-$, $-C_5H_{10}-$, $-C_6H_{12}-$, $-C_7H_{14}-$, $-C_8H_{16}-$, $-C_9H_{18}-$, $-C_{10}H_{20}-$, $-CH(CH_3)-$, $-C[(CH_3)_2]-$, $-CH_2-CH(CH_3)-$, $-CH(CH_3)-CH_2-$, $-CH(CH_3)-C_2H_4-$, $-CH_2-CH(CH_3)-CH_2-$, $-C_2H_4-CH(CH_3)-$, $-CH_2-C[(CH_3)_2]-$, $-C[(CH_3)_2]-CH_2-$, $-CH(CH_3)-CH(CH_3)-$, $-C[(C_2H_5)(CH_3)]-$, $-CH(C_3H_7)-$, $-(CH_2)_mO-$, $-(CH_2)_pOCH_2-$, $-(CH_2-CH_2-O)_n-CH_2-CH_2-$, $-CH=CH-$, $-C(CH_3)=CH-$, $-CH=C(CH_3)-$, $-CH_2-CH=CH-$, $-CH_2-C(CH_3)=CH-$, $-CH_2-CH=C(CH_3)-$, $-CH=CH-CH_2-$, $-C(CH_3)=CH-CH_2-$, $-CH=C(CH_3)-CH_2--C(CH_3)=CH-C(CH_3)=CH-$, $-C_2H_4-CH=CH-CH=CH-$, $-CH_2-CH=CH-CH_2-CH=CH-$, $-C_3H_6-C\equiv C-CH_2-$, $-CH_2-CH=CH-CH=CH-CH_2-$, $-CH=CH-CH=CH-C_2H_4-$, $-CH_2-CH=CH-C(CH_3)=CH-$, $-CH_2-CH=C(CH_3)-CH=CH-$, $-CH_2-C(CH_3)=CH-CH=CH-$, $-CH(CH_3)-CH=CH-CH=CH-$, $-CH=CH-CH_2-C(CH_3)=CH-$, $-CH(CH_3)-C\equiv C-CH_2-$, $-CONH-$, $-NHCO-$, $-CH_2-CONH-$ $-CONH-CH_2-$, $-NHCO-CH_2-$, $-CH_2-NHCO-$;

wherein n, m, p are independently an integer from 1 to 10; or

**$L_1$-$R^D$** and **$L_2$-$R^E$** or **$L_1$-$R^D$** and **$L_3$-$R^F$** or **$L_2$-$R^E$** and **$L_3$-$R^F$** can form together a cyclic ring selected from the group consisting of:

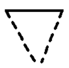 , 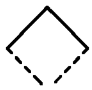 , 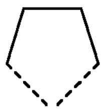 , 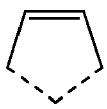 , 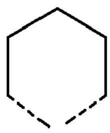 , 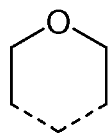 , 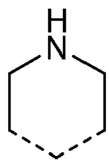 or 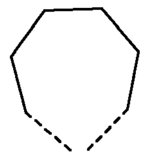 ;

**$R^N$** represents -H, $-CH_2-OCH_3$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2-O-cyclo-C_3H_5$, $-C_2H_4-O-cyclo-C_3H_5$, $-C_3H_6-O-cyclo-C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, -CHO, $-COCH_3$, $-COC_2H_5$, $-COC_3H_7$, $-CO-cyclo-C_3H_5$, $-COCH(CH_3)_2$, $-COC(CH_3)_3$, -COCN, $-COOCH_3$, $-COOC_2H_5$, $-COOC_3H_7$, $-COO-cyclo-C_3H_5$, $-COOCH(CH_3)_2$, $-COOC(CH_3)_3$, $-CONH_2$, $-CONHCH_3$, $-CONHC_2H_5$, $-CONHC_3H_7$, $-CONH-cyclo-C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, $-CON(cyclo-C_3H_5)_2$, $-CON[CH(CH_3)_2]_2$, $-CON[C(CH_3)_3]_2$, $-SO_2CH_3$, $-SO_2C_2H_5$, $-SO_2C_3H_7$, $-SO_2-cyclo-C_3H_5$, $-SO_2CH(CH_3)_2$, $-SO_2C(CH_3)_3$, $-CH_2-OCF_3$, $-C_2H_4-OCF_3$, $-C_3H_6-OCF_3$, $-OC_2F_5$, $-CH_2-OC_2F_5$, $-C_2H_4-OC_2F_5$, $-C_3H_6-OC_2F_5$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, $-cyclo-C_8H_{15}$, -Ph, $-CH_2-Ph$, $-CH_2-CH_2-Ph$, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$,

$-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$,- $C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C\equiv CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$,- $CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C3H7)-C\equiv CH$, $-C(CH_3)(C_2H_5)-C\equiv CH$, $-CH_2-CH(C\equiv CH)_2$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C(C\equiv CH)_2-CH_3$, $-C\equiv C-CH(CH_3)-C\equiv CH$, $-CH(CH_3)-C\equiv C-C\equiv CH$, $-CH(C\equiv CH)-CH_2-C\equiv CH$, $-CH(C\equiv CH)-C\equiv C-CH_3$;

$\mathbf{R^B}$ represents

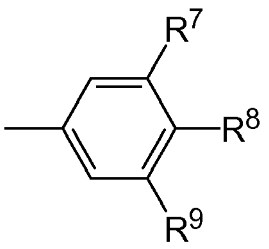
,
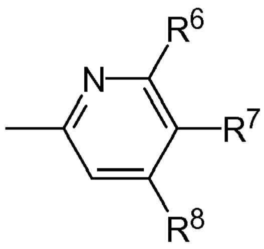
,
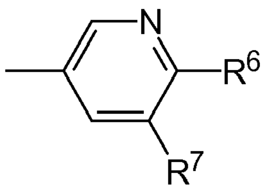
,
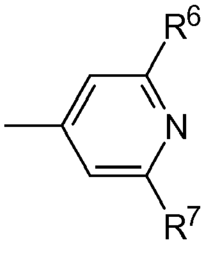
,
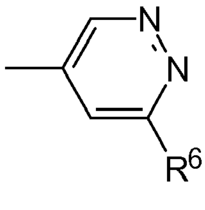
,
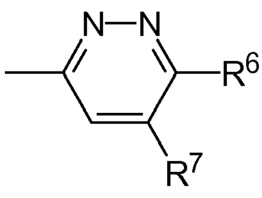

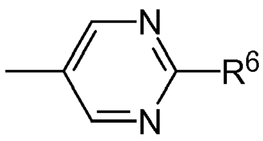

,

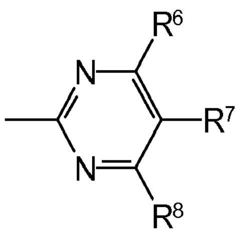

,

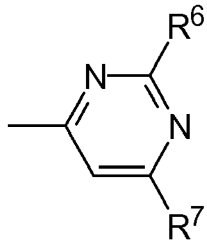

,

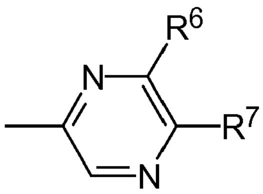

,

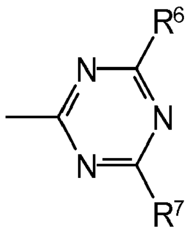

,

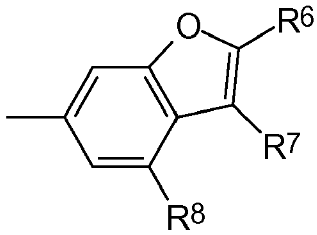

,

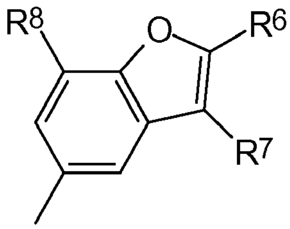

,

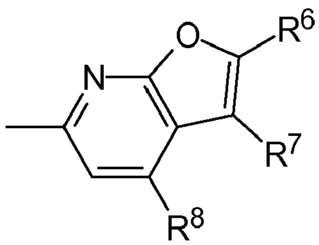

,

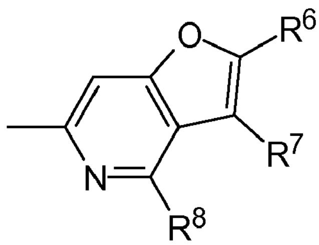

,

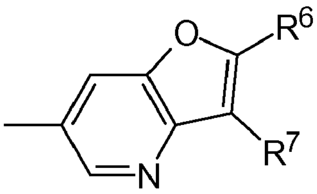

,

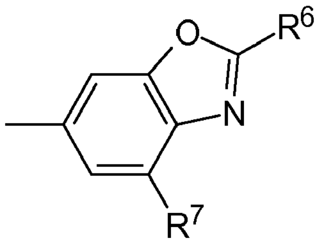

,

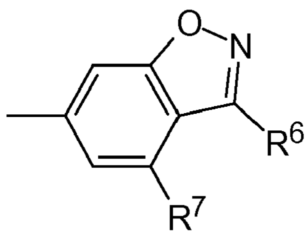

,

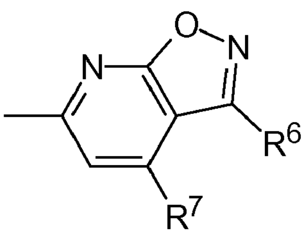

,

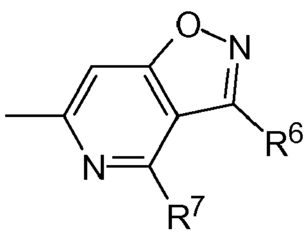

,

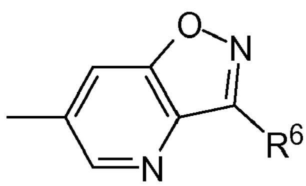

,

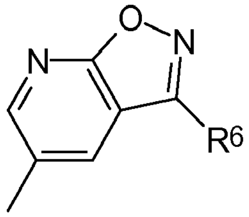
,
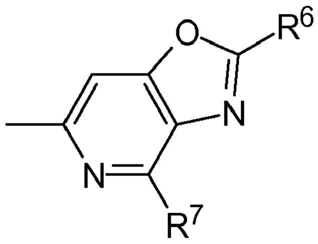
,
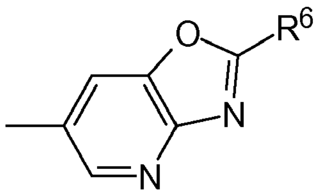
,

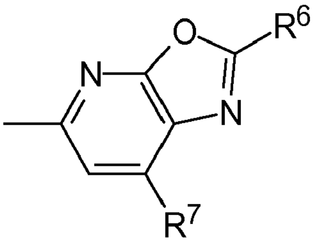
,
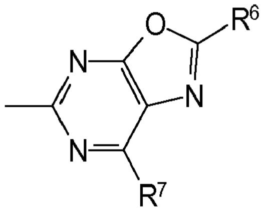
,
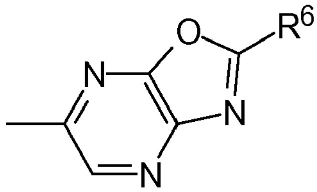
,

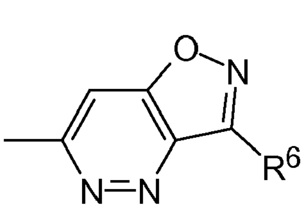
,
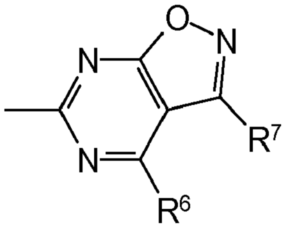
,
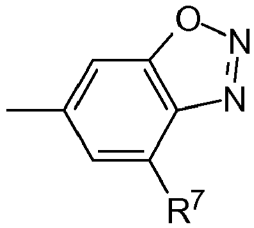
,

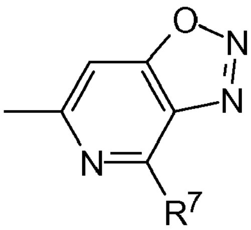
,
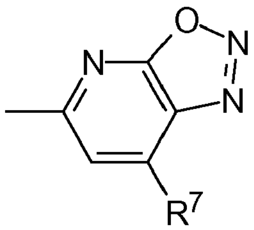
,
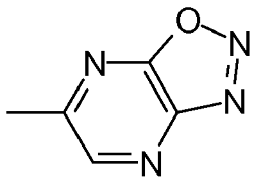
,

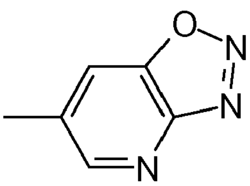
,
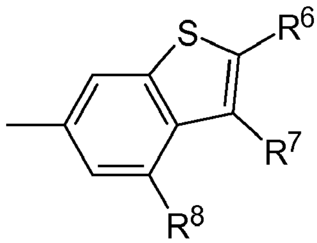
,
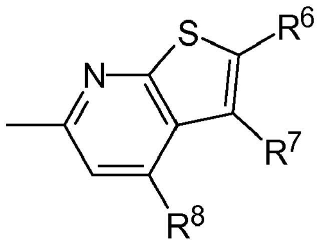
,

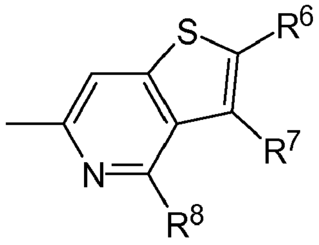,
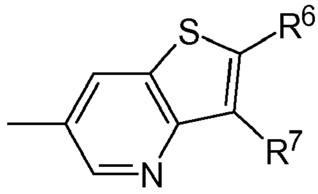,
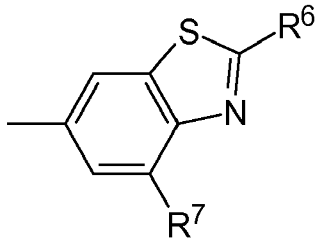,

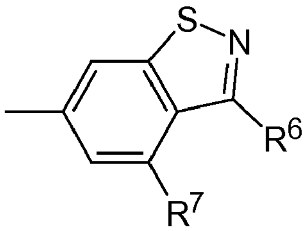,
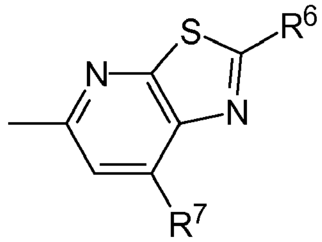,
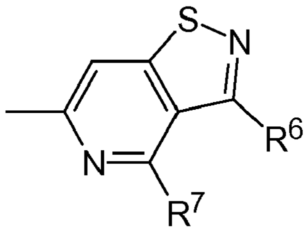,

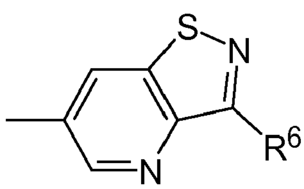,
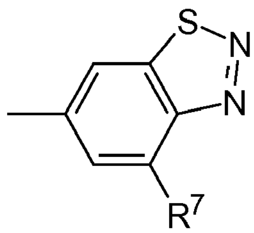,
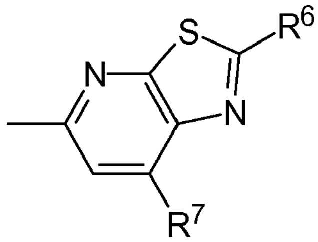,

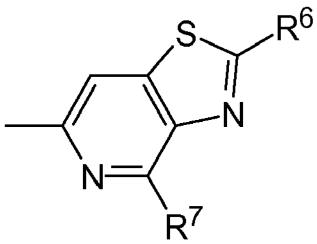,
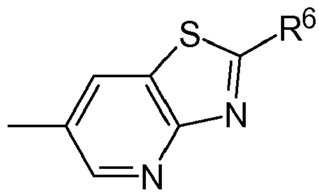,
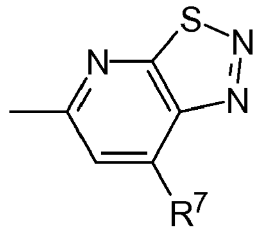,

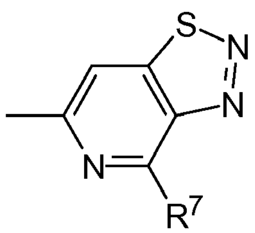,
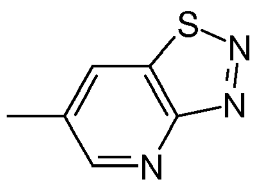,
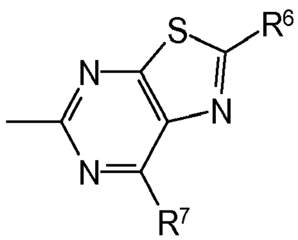,

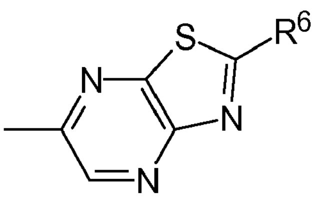 ,
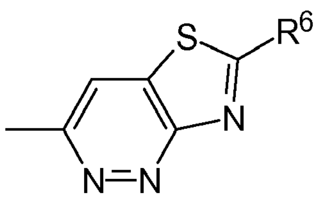 ,
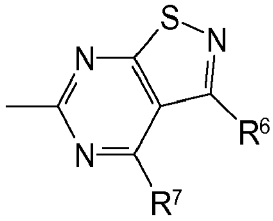 ,

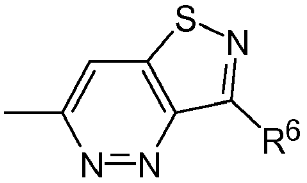 ,
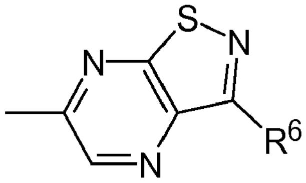 ,
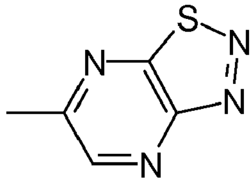 ,

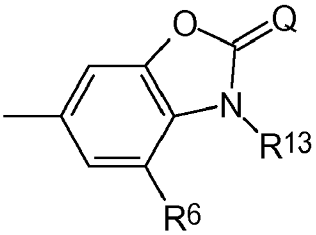 ,
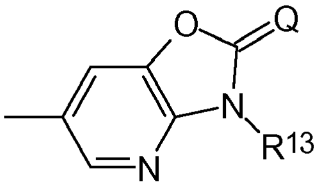 ,
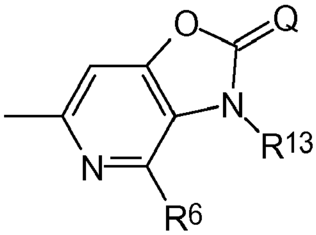 ,

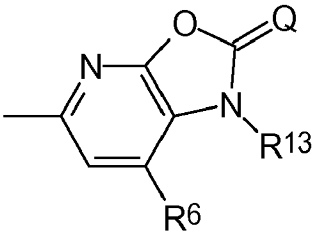 ,
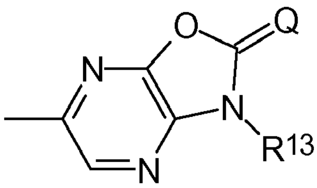 ,
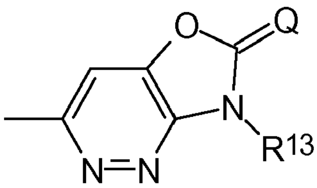 ,

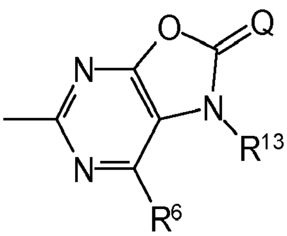 ,
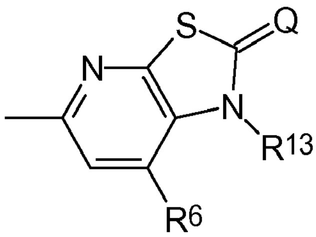 ,
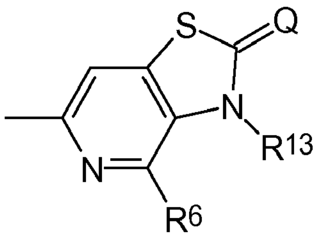 ,

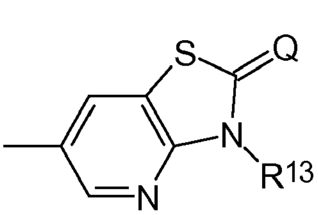 ,
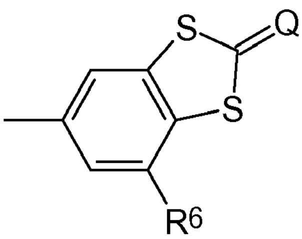 ,
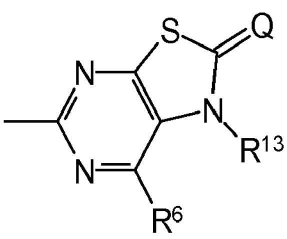 ,

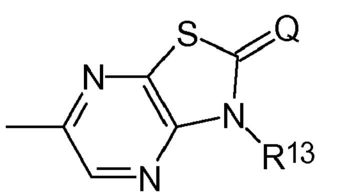
,
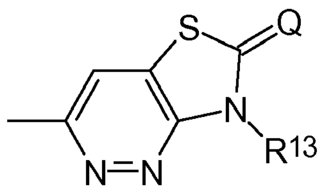
,
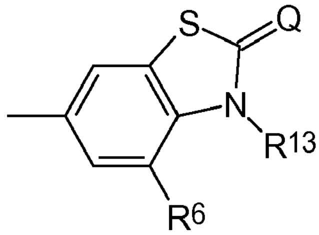
,

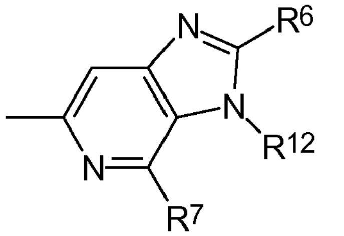
,
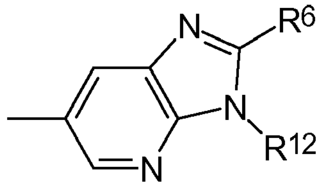
,
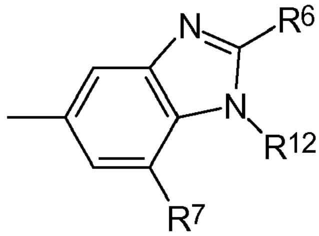
,

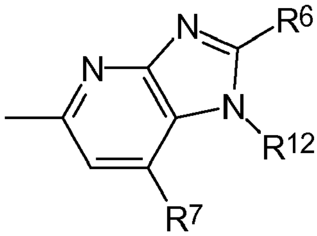
,
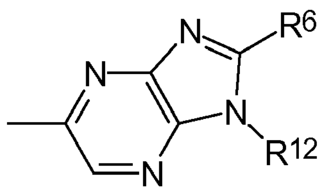
,
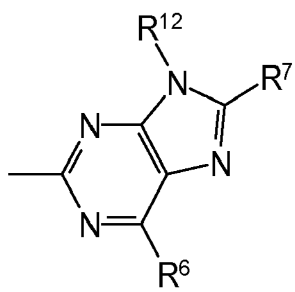
,

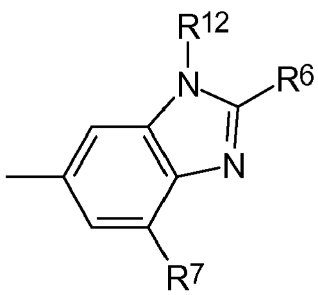
,
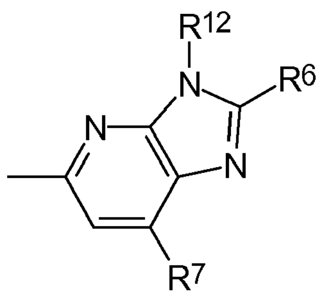
,
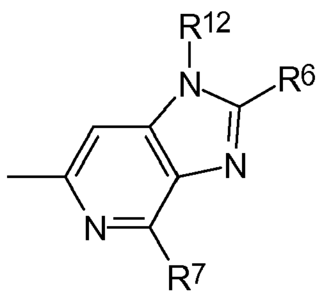
,

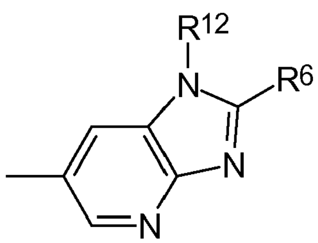
,
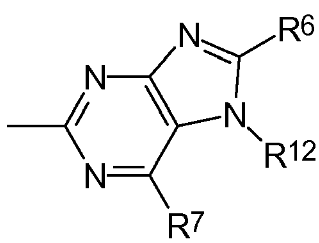
,
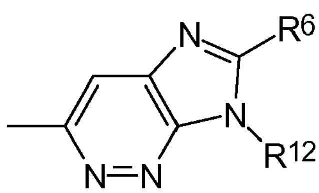
,

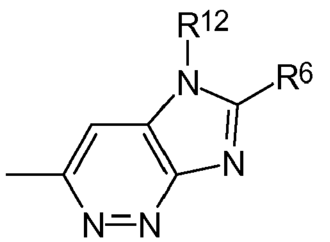
,
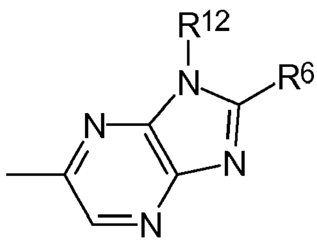
,
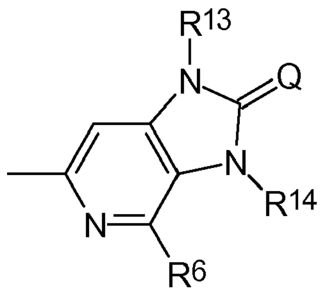
,
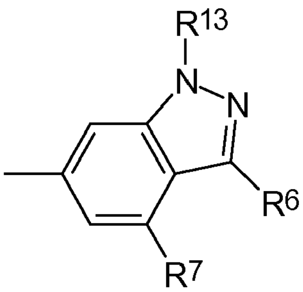
,
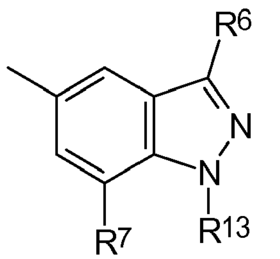
,
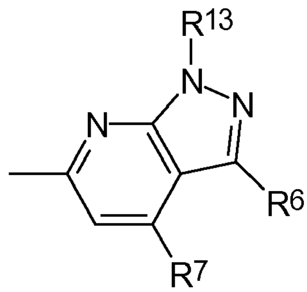
,
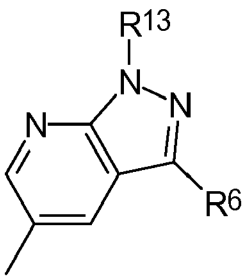
,
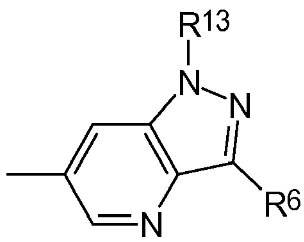
,
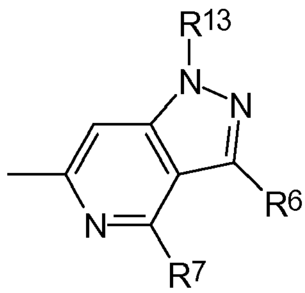
,
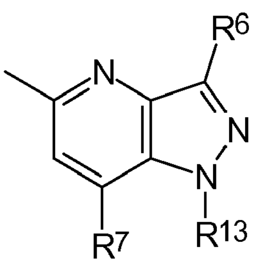
,
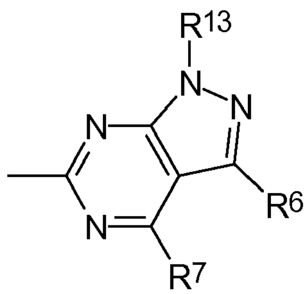
,
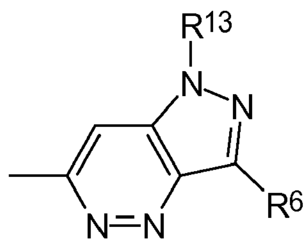
,
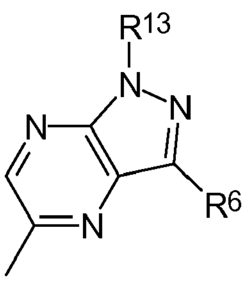
,
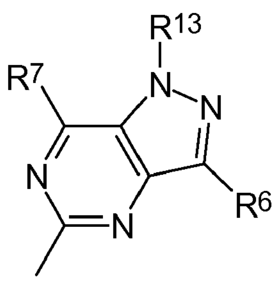
,
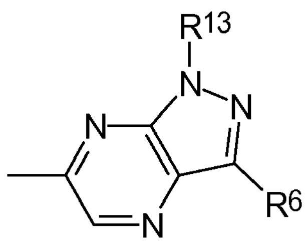
,

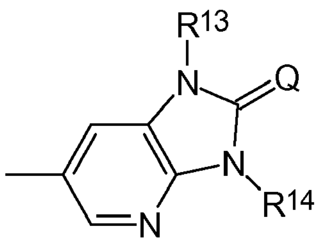 ,

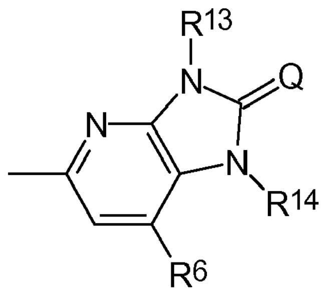 ,

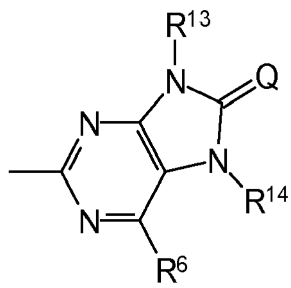 ,

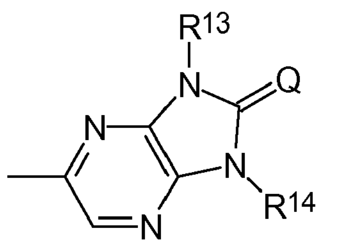 ,

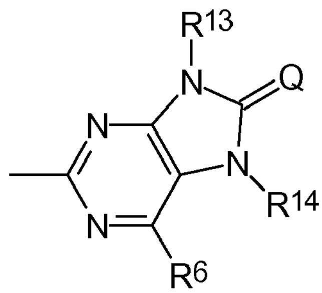 ,

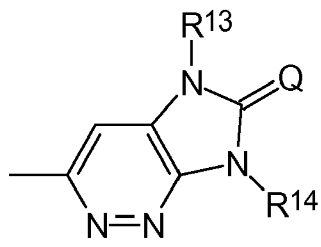 ,

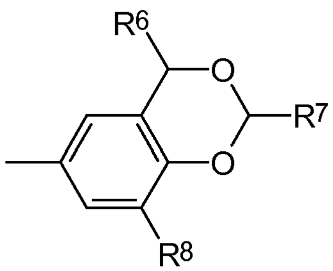 ,

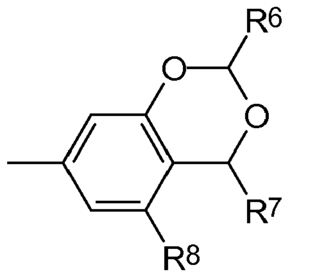 ,

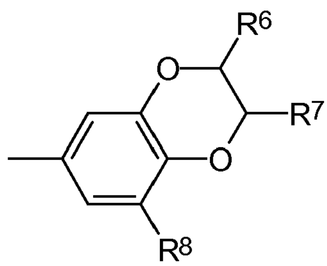 ,

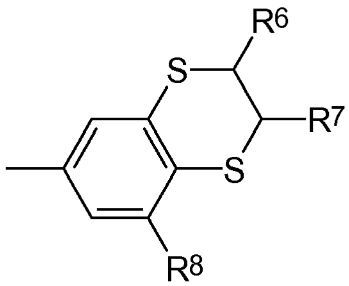 ,

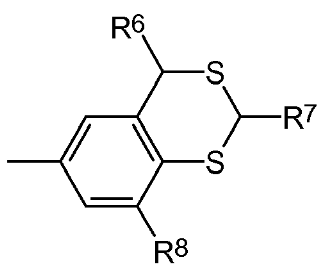 ,

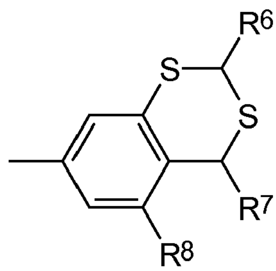 ,

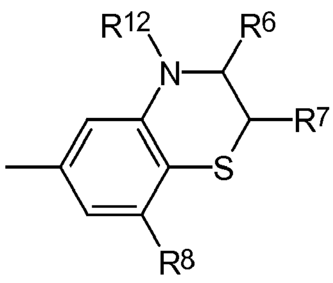 ,

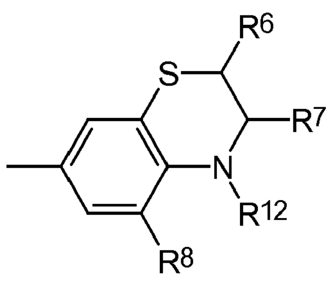 ,

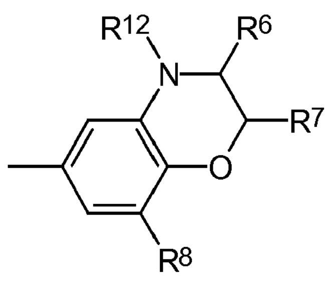 ,

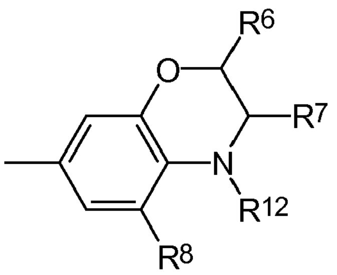 , 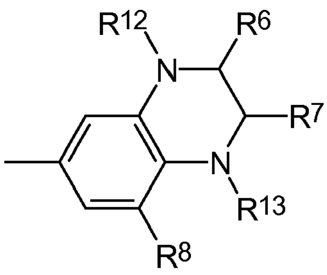 , 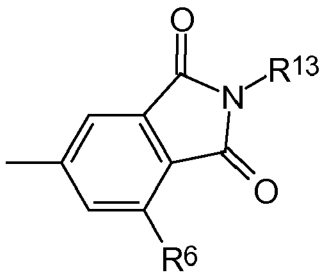 ,

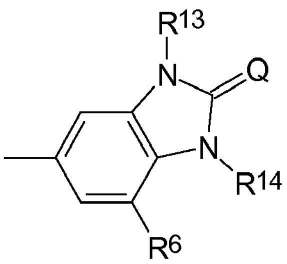 , 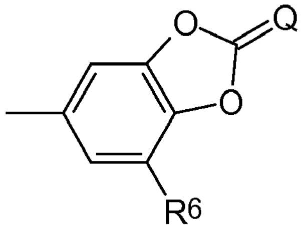 , 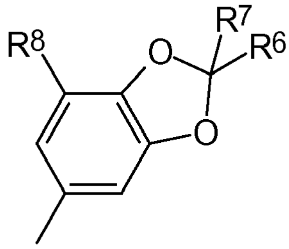 ,

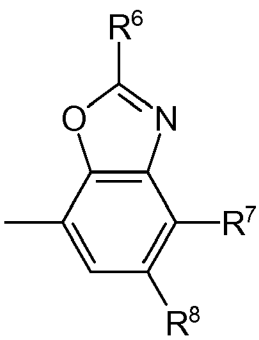 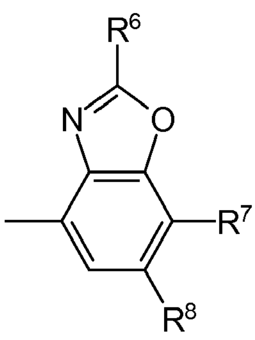 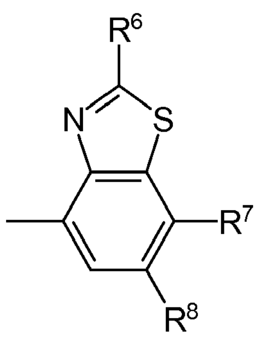 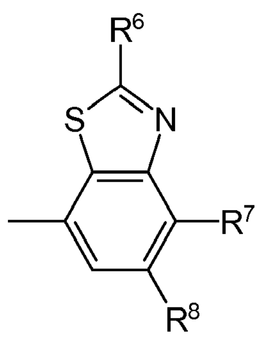

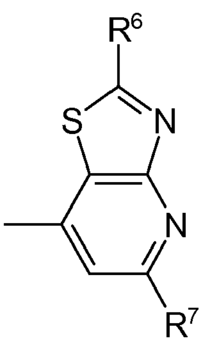 , 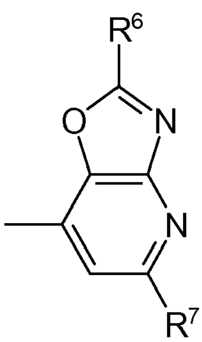 , 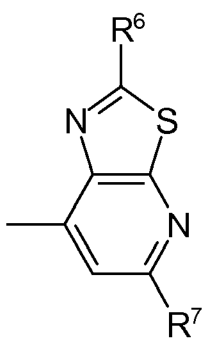 or 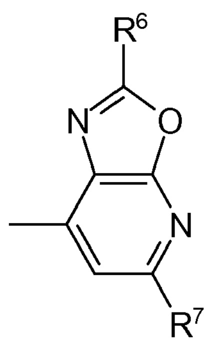 ;

Q represents =O, =S, or =N-$R^{12}$;

**$R^C$** represents -OH, $-CH_2-OH$, -CHO, $-C_2H_4-OH$, $-C_3H_6-OH$, $-O-CH_3$, $-O-C_2H_5$, $-O-CH_2-OH$, $-O-CH(CH_3)_2$, $-O-CH_2-O-CH_3$, $-O-C_2H_4-O-CH_3$, $-CH_2-O-CH_3$, $-CH_2-O-CH_2-OH$, $-CH_2O-C_2H_5$, $-CH_2-O-CH(CH_3)_2$, $-CH_2-O-C_3H_7$, $-CO-CH_3$, $-CH_2-CO-CH_3$, $-CO-CH_2-OH$, $-CH(OH)-CH_3$, $-C(OH)(CH_3)_2$, $-CH(CH_3)CH_2OH$, $-CH(OH)-CH_2-OH$, $-CH_2-CH(OH)-CH_3$, $-CH_2-CH(OH)-CH_2-OH$, $-CH(OCH_3)-CH_2OH$, $-CH(OC_2H_5)-CH_2OH$, $-CH(OCH_3)-CH_2OCH_3$, $-CH(OC_2H_5)-CH_2OCH_3$, $-CH(OC_2H_5)-CH_2OC_2H_5$, $-CH(OAc)-CH_2OH$, $-CH(OAc)-CH_2OAc$, $-CH(OH)-CH_2OAc$, $-CH(OH)-CH_2-NH_2$, $-CH_2-CH(OH)-CH_2-NH_2$, $-CH(OCH_3)-CH_2-NH_2$, $-CH(OC_2H_5)-CH_2-NH_2$, $-CH_2-CH(OCH_3)-CH_2-NH_2$, $-CH_2-CH(OC_2H_5)-CH_2-NH_2$, $-CH(OH)-CH_2-NHCH_3$, $-CH(OH)-CH_2-NHC_2H_5$, $-CH_2-CH(OH)-CH_2-NHCH_3$, $-CH_2-CH(OH)-CH_2-NHC_2H_5$, $-CH(OCH_3)-CH_2NHCH_3$, $-CH(OC_2H_5)-CH_2NHCH_3$, $-CH_2-CH(OCH_3)-CH_2-NHCH_3$, $-CH_2-CH(OC_2H_5)-CH_2-NHCH_3$,

$-CH(OCH_3)-CH_2NHC_2H_5$, $-CH(OC_2H_5)-CH_2NHC_2H_5$, $-CH(OCH_3)-CH_2N(CH_3)_2$, $-CH(OC_2H_5)-CH_2N(CH_3)_2$, $-NH_2$, $-NHCH_3$, $-N(CH_3)_2$, $-CH_2-NH_2$, $-CH_2-NHCH_3$, $-CH_2-N(CH_3)_2$, $-C_2H_4-NH_2$, $-C_2H_4-NHCH_3$, $-C_2H_4-N(CH_3)_2$, $-CH(NHCH_3)CH_3$, $-CH(NHC_2H_5)CH_3$, $-CH(N(CH_3)_2)CH_3$, $-CH(N(C_2H_5)_2)CH_3$, $-CH(NH_2)CH_2OH$, $-CH(NH-CH_3)CH_2OH$, $-CH(NHC_2H_5)CH_2OH$, $-CH(N(CH_3)_2)CH_2OH$, $-CH(N(C_2H_5)_2)CH_2OH$, $-CH(NH_2)CH_2OCH_3$, $-CH(NHCH_3)CH_2OCH_3$, $-CH(NHC_2H_5)CH_2OCH_3$, $-CH(N(CH_3)_2)CH_2OCH_3$, $-CH(N(C_2H_5)_2)CH_2OCH_3$, $-CH(NH_2)CH_2OC_2H_5$, $-CH(NHCH_3)CH_2OC_2H_5$, $-CH(NHC_2H_5)CH_2OC_2H_5$, $-CH(N(CH_3)_2)CH_2OC_2H_5$, $-CH(N(C_2H_5)_2)CH_2OC_2H_5$, $-CH(NH_2)CH_2OAc$, $-CH(NHCH_3)CH_2OAc$, $-CH(NHC_2H_5)CH_2OAc$, $-CH(N(CH_3)_2)CH_2OAc$, $-CH(N(C_2H_5)_2)CH_2OAc$, $-CH_2-CH(NHAc)CH_2OH$, $-CH_2-CH(NHAc)CH_2OCH_3$, $-CH_2-CH(NHAc)CH_2OC_2H_5$,- $CH_2-CH(NHCH_3)CH_3$, $-CH_2-CH(NHC_2H_5)CH_3$, $-CH_2-CH(N(CH_3)_2)CH_3$, $-CH_2-CH(N(C_2H_5)_2)CH_3$, $-CH_2-CH(NH_2)CH_2OH$, $-CH_2-CH(NHCH_3)CH_2OH$, $-CH_2-CH(NHC_2H_5)CH_2OH$, $-CH_2-CH(N(CH_3)_2)CH_2OH$, $-CH_2-CH(N(C_2H_5)_2)CH_2OH$, $-CH_2-CH(NH_2)CH_2OCH_3$, $-CH_2-CH(NH-CH_3)CH_2OCH_3$, $-CH_2-CH(NHC_2H_5)CH_2OCH_3$, $-CH_2-CH(N(CH_3)_2)CH_2OCH_3$, $-CH_2-CH(N(C_2H_5)_2)CH_2OCH_3$, $-CH_2-CH(NH_2)CH_2OC_2H_5$, $-CH_2-CH(NHCH_3)CH_2OC_2H_5$, $-CH_2-CH(NHC_2H_5)CH_2OC_2H_5$, $-CH_2-CH(N(CH_3)_2)CH_2OC_2H_5$, $-CH_2-CH(N(C_2H_5)_2)CH_2OC_2H_5$, $-CH_2-CH(NH_2)CH_2OAc$, $-CH_2-CH(NH-CH_3)CH_2OAc$, $-CH_2-CH(NHC_2H_5)CH_2OAc$, $-CH_2-CH(N(CH_3)_2)CH_2OAc$, $-CH_2-CH(N(C_2H_5)_2)CH_2OAc$, $-CH_2-CH(NHAc)CH_2OH$, $-CH_2-CH(NHAc)CH_2OCH_3$, $-CH_2-CH(NHAc)CH_2OC_2H_5$, $-NHCOCH_3$, $-CH_2-NH-COCH_3$, $-C_2H_4-NHCOCH_3$, $-NHCHO$, $-CH_2-NHCHO$, $-C_2H_4-NHCHO$, $-NHSO_2CH_3$, $-NHSO_2CF_3$, $-NH-SO_2CH_2CF_3$, $-CH_2-NHSO_2CH_3$, $-CH_2-NHSO_2CF_3$, $-CH_2-NHSO_2CH_2CF_3$, $-C_2H_4-NHSO_2CH_3$, $-C_2H_4-NHSO_2CF_3$, $-C_2H_4-NHSO_2CH_2CF_3$, $-NO_2$, $-CH_2-NO_2$, $-C_2H_4-NO_2$, $-CH(OH)-NO_2$, $-CH(NO_2)-OH$, $-CO_2H$, $-CH_2-CO_2H$, $-C_2H_4-CO_2H$, $-CH=CH-CO_2H$, $-CO_2CH_3$, $-CO_2C_2H_5$, $-CO_2CH(CH_3)_2$, $-CH_2-CO_2CH_3$, $-CH_2-CO_2C_2H_5$, $-CH_2-CO_2CH(CH_3)_2$, $-C_2H_4-CO_2CH_3$, $-C_2H_4-CO_2C_2H_5$, $-C_2H_4-CO_2CH(CH_3)_2$, $-CO_2NH_2$, $-CO_2NHCH_3$, $-CO_2N(CH_3)_2$, $-CH_2-CO_2NH_2$, $-CH_2-CO_2NHCH_3$, $-CH_2-CO_2N(CH_3)_2$, $-C_2H_4-CO_2NH_2$, $-C_2H_4-CO_2NHCH_3$, $-C_2H_4-CO_2N(CH_3)_2$, $-O-Si(CH_3)_3$, $-O-Si(C_2H_5)_3$, -CO-CHO, $-CO-CO-CH_3$, $-C(OH)-CO-CH_3$, $-CO-C(OH)-CH_3$, $-CO-CH_2-CO-CH_3$, $-C(OH)-CH_2-CO-CH_3$, $-CO-CH_2-C(OH)-CH_3$, $-C(OH)-CH_2-C(OH)-CH_3$, -F, -Cl, -Br, $-CH_2-F$, $-CHF_2$, $-CF_3$, $-C_2H_4-F$, $-CH_2-CF_3$, $-CF_2-CF_3$, $-O-CHF_2$, $-O-CF_3$, $-O-CH_2-CF_3$, $-O-C_2F_5$, $-CH_3$, $-CH_2CH_3$, $-C_3CH_7$, $-CH(CH_3)_2$, $-CH=CH_2$, $-C\equiv CH$, $-CH_2-CH=CH_2$, or $-CH_2-C\equiv CH$;

**$R^1$ - $R^{10}$** represent independently of each other -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, $-OCH_2-COOH$, -OPh, $-OCH_2-Ph$, $-OCPh_3$, $-CH_2-OCH_3$, $-CH_2-OH$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2$-O-cyclo-$C_3H_5$, $-C_2H_4$-o-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, $-Cl_3$ -Br, -I, $-P(O)(OH)_2$, $-P(O)(OCH_3)_2$, $-P(O)(OC_2H_5)_2$,- $P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, -OOC-cyclo-$C_3H_5$, $-OOC-CH(CH_3)_2$, $-OOC-C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3$-cyclo-$C_3H_5$, $-SO_3CH(CH_3)_2$, $-SO_3C(CH_3)_3$, $-SO_2NH_2$, $-SO_2NHCH_3$, $-SO_2NHC_2H_5$, $-SO_2NHC_3H_7$, $-SO_2NH$-cyclo-$C_3H_5$, $-SO_2NHCH(CH_3)_2$, $-SO_2NHC(CH_3)_3$, $-SO_2N(CH_3)_2$, $-SO_2N(C_2H_5)_2$, $-SO_2N(C_3H_7)_2$, $-SO_2N$(cyclo-$C_3H_5)_2$, $-SO_2N[CH(CH_3)_2]_2$, $-SO_2N[C(CH_3)_3]_2$, $-O-S(=O)CH_3$, $-O-S(=O)C_2H_5$, $-O-S(=O)C_3H_7$, -O-S(=O)-cyclo-$C_3H_5$, $-O-S(=O)CH(CH_3)_2$, $-O-S(=O)C(CH_3)_3$, $-S(=O)(=NH)CH_3$, $-S(=O)(=NH)C_2H_5$, $-S(=O)(=NH)C_3H_7$, -S(=O)(=NH)-cyclo-$C_3H_5$, $-S(=O)(=NH)CH(CH_3)_2$, $-S(=O)(=NH)C(CH_3)_3$, $-NH-SO_2-CH_3$, $-NH-SO_2-C_2H_5$, $-NH-SO_2-C_3H_7$, $-NH-SO_2$-cyclo-$C_3H_5$, $-NH-SO_2-CH(CH_3)_2$, $-NH-SO_2-C(CH_3)_3$, $-O-SO_2-CH_3$, $-O-SO_2-C_2H_5$, $-O-SO_2-C_3H_7$, $-O-SO_2$-cyclo-$C_3H_5$, $-O-SO_2-CH(CH_3)_2$, $-O-SO_2-C(CH_3)_3$, $-OCF_3$, $-CH_2-OCF_3$, $-C_2H_4-OCF_3$, $-C_3H_6-OCF_3$, $-OC_2F_5$, $-CH_2-OC_2F_5$, $-C_2H_4-OC_2F_5$, $-C_3H_6-OC_2F_5$, $-O-COOCH_3$, $-O-COOC_2H_5$, $-O-COOC_3H_7$, -O-COO-cyclo-$C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CS-N(C_3H_7)_2$, $-NH-CO-NHC_3H_7$, $-NH-CO-N(C_3H_7)_2$, $-NH-CO-NH[CH(CH_3)_2]$, $-NH-CO-NH[C(CH_3)_3]$, $-NH-CO-N(CH_3)_2$, $-NH-CO-N(C_2H_5)_2$, -NH-CO-NH-cyclo-$C_3H_5$, -NH-CO-N(cyclo-$C_3H_5)_2$, $-NH-CO-N[CH(CH_3)_2]_2$, $-NH-CS-N(C_2H_5)_2$, $-NH-CO-N[C(CH_3)_3]_2$, $-NH-CS-NH_2$, $-NH-CS-NHCH_3$, $-NH-CS-N(CH_3)_2$, $-NH-CS-NHC_2H_5$, $-NH-CS-NHC_3H_7$, -NH-CS-NH-cyclo-$C_3H_5$, $-NH-CS-NH[CH(CH_3)_2]$, $-NH-CS-NH[C(CH_3)_3]$, -NH-CS-N(cyclo-$C_3H_5)_2$, -NH-CS-

$N[CH(CH_3)_2]_2$, $-NH-CS-N[C(CH_3)_3]_2$,- $NH-C(=NH)-NH_2$, $-NH-C(=NH)-NHCH_3$, $-NH-C(=NH)-NHC_2H_5$, -NH-$C(=NH)-NHC_3H_7$, -O-CO-NH-cyclo-$C_3H_5$, -NH-C(=NH)-NH-cyclo-$C_3H_5$, $-NH-C(=NH)-NH[CH(CH_3)_2]$, -O-CO-$NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$, -NH-$C(=NH)-N(C_3H_7)_2$, -NH-C(=NH)-N(cyclo-$C_3H_5)_2$, $-O-CO-NHC_3H_7$, $-NH-C(=NH)-N[CH(CH_3)_2]_2$, -NH-$C(=NH)-N[C(CH_3)_3]_2$, $-O-CO-NH_2$, $-O-CO-NHCH_3$, $-O-CO-NHC_2H_5$, $-O-CO-NH[C(CH_3)_3]$, $-O-CO-N(CH_3)_2$, -O-$CO-N(C_2H_5)_2$, $-O-CO-N(C_3H_7)_2$, -O-CO-N(cyclo-$C_3H_5)_2$, $-O-CO-N[CH(CH_3)_2]_2$, $-O-CO-N[C(CH_3)_3]_2$, -O-CO-$OCH_3$, $-O-CO-OC_2H_5$, $-O-CO-OC_3H_7$, -O-CO-O-cyclo-$C_3H_5$, $-O-CO-OCH(CH_3)_2$, $-O-CO-OC(CH_3)_3$, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, -cyclo-$C_3H_5$, -cyclo-$C_4H_7$, -cyclo-$C_5H_9$, -cyclo-$C_6H_{11}$, -cyclo-$C_7H_{13}$, -cyclo-$C_8H_{15}$, -Ph, $-CH_2$-Ph, $-CH_2-CH_2$-Ph, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, -CH=CH-$CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, -CH=CH-$C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH$-$C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH_2-C(CH_3)_2-CH=CH_2$, $-C(CH_3)_2-CHrCH=CH_2$,- $CH_2-C(CH_3)=C(CH_3)_2$, $-CH(CH_3)-CH=C(CH_3)_2$, $-C(CH_3)_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_3H_6C\equiv C-CH_3$, $-CH(CH_3)-CH_2-C\equiv CH$, $-CH(CH_3)-C\equiv C-CH_3$, $-C_2H_4-CH(CH_3)-C\equiv CH$, $-CH_2-CH(CH_3)-CH_2-C\equiv CH$, $-CH_2-CH(CH_3)-C\equiv CH$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C$-$C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C$-$CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, or $-CH_2-CH(C\equiv CH)_2$;

**$R^{15}$** represents **$-R^{20}$,** -CN, $-CH_2-CN$, $-CH_2-OR^{17}$, $-CH_2-CH_2-OR^{17}$, $-CH_2-NR^{17}R^{18}$, $-CH_2-NR^{17}COR^{19}$, $-CH_2-CH_2-NR^{17}R^{18}$, $-CH_2-CH_2-NR^{17}COR^{19}$, $-CO_2R^{17}$, $-CO-NR^{17}R^{18}$, $-CH_2-CO_2R^{17}$, or $-CH_2-CO-NR^{17}R^{18}$;

**$R^{16}$** represents **$-R^{21}$,** -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH_2OH$, $-CH_2-SH$, $-CH(OH)CH_3$, $-C_2H_4OH$, $-C_3H_6OH$, $-C_4H_8OH$, $-CH(CH_3)-C_2H_4OH$, $-C_5H_{10}OH$, $-CH_2-S-CH_3$, $-CH_2-CH_2-S-CH_3$, $-C_3H_6-S-CH_3$, $-CH_2OCH_3$, $-C_2H_4OCH_3$, $-C_3H_6OCH_3$, $-C_4H_8OCH_3$, $-CH(CH_3)-C_2H_4OCH_3$, $-C_5H_{10}OCH_3$, $-CH_2NH_2$, $-C_2H_4NH_2$, $-C_3H_6NH_2$, $-C_4H_8NH_2$, $-CH(CH_3)-C_2H_4NH_2$, $-C_5H_{10}NH_2$, $-CH_2-CH_2-CH_2-NH-C(NH)NH_2$, $-CH_2-CO_2H$, $-CH_2-CONH_2$, $-CH_2-CH_2-CO_2H$, $-CH_2-CH_2-CONH_2$, $-CH_2-CO_2CH_3$, $-CH_2-CONHCH_3$, $-CH_2-CON(CH_3)_2$, $-CH_2-CH_2-CO_2CH_3$, $-CH_2-CH_2-CONHCH_3$, $-CH_2-CH_2-CONH(CH_3)_2$, $-CH=CH-CO_2H$, $-CH=CH-CO_2CH_3$, $-CH=CH-CONHCH_3$, $-CH=CH-CONHC_2H_5$,- $CH=CH-CON(CH_3)_2$, $-CH=CH-CON(C_2H_5)_2$, $-CH_2-CH=CH-CO_2H$, $-CH_2-CH=CH-CO_2CH_3$, $-CH_2-CH=CH$-$CONHCH_3$, $-CH_2-CH=CH-CON(CH_3)_2$, $-CH_2-CH=CH-CONHC_2H_5$, $-CH_2-CH=CH-CON(C_2H_5)_2$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$,

$-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C=CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$,- $C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-CH_2-Ph$,

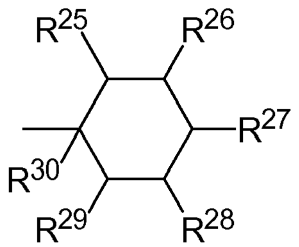
,
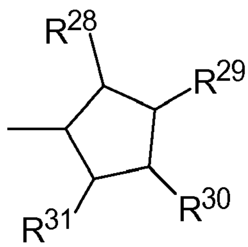
,
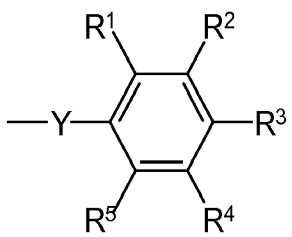
,
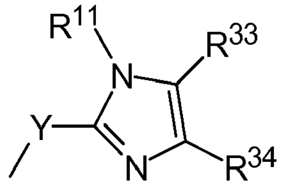
,
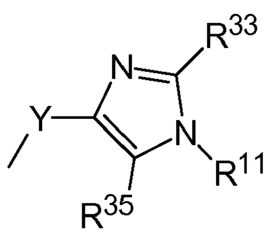
,
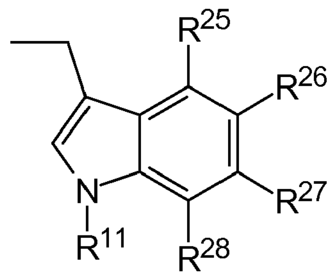
,

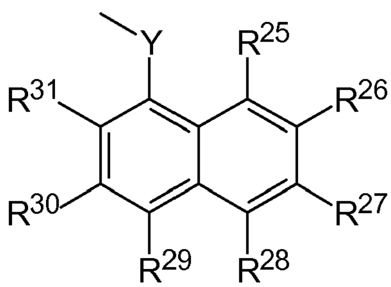
,
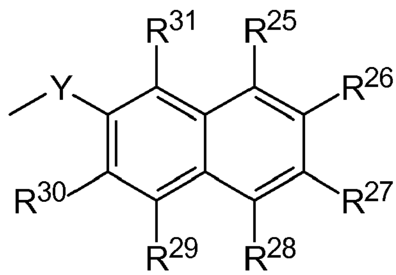
,

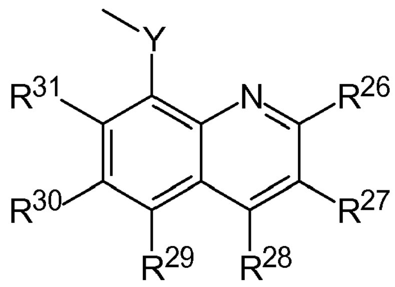
or
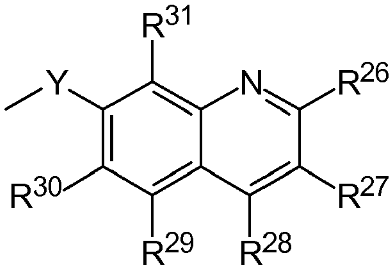
;

**$R^{11}$ - $R^{14}$** and **$R^{17}$** - **$R^{21}$** represent independently of each other $-H$, $-CH_2F$, $-CHF_2$, $-CH_2-OCH_3$, $-CH_2-OH$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2-O\text{-cyclo-}C_3H_5$, $-C_2H_4-O\text{-cyclo-}C_3H_5$, $-C_3H_6-O\text{-cyclo-}C_3H_5$,- $CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2-OPh$, $-C_2H_4-OPh$, $-C_3H_6-OPh$, $-CH_2-OCH_2-Ph$, $-C_2H_4-OCH_2-Ph$, $-C_3H_6-OCH_2-Ph$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, $\text{-cyclo-}C_3H_5$, $\text{-cyclo-}C_4H_7$, $\text{-cyclo-}C_5H_9$, $\text{-cyclo-}C_6H_{11}$, $\text{-cyclo-}C_7H_{13}$, $\text{-cyclo-}C_8H_{15}$, $-Ph$, $-CH_2-Ph$, $-CH_2-CH_2-Ph$, $-CH=CH-Ph$, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$,- $C[CH(CH_3)(C_2H_5)]=CH_2$, $-C[CH_2-CH(CH_3)_2]=CH_2$, $-C_3H_6-C\equiv C-CH_{33}$, $-CH(CH_3)-CH_2-C\equiv CH$, $-CH(CH_3)-C\equiv C-CH_3$, $-C_2H_4-CH(CH_3)-C\equiv CH$, $-CH_2-CH(CH_3)-CH_2-C\equiv CH$, $-CH_2-CH(CH_3)-C\equiv CH$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-$

C$_2$H$_5$, -CH$_2$-C≡C-C$_3$H$_7$, -C≡C-C$_4$H$_9$, -C≡C-C(CH$_3$)$_3$, -CH(CH$_3$)-C$_2$H$_4$-C≡CH, -CH$_2$-CH(CH$_3$)-C≡C-CH$_3$, -CH(CH$_3$)-CH$_2$-C≡C-CH$_3$, -CH(CH$_3$)-C≡C-C$_2$H$_5$, -CH$_2$-C≡C-CH(CH$_3$)$_2$, -C≡C-CH(CH$_3$)-C$_2$H$_5$, -C≡C-CH$_2$-CH(CH$_3$)$_2$, -CH(C$_2$H$_5$)-C≡C-CH$_3$, -C(CH$_3$)$_2$-C≡C-CH$_3$, -CH(C$_2$H$_5$)-CH$_2$-C≡CH, -CH$_2$-CH(C$_2$H$_5$)-C≡CH, -C(CH$_3$)$_2$-CH$_2$-C≡CH, -CH$_2$-C(CH$_3$)$_2$-C≡CH, -CH(CH$_3$)-CH(CH$_3$)-C≡CH, -CH(C$_3$H$_7$)-C≡CH, -C(CH$_3$)(C$_2$H$_5$)-C≡CH, or -CH$_2$-CH(C≡CH)$_2$;

**R$^{22}$ - R$^{37}$** represent independently of each other -H, -OH, -OCH$_3$, -OC$_2$H$_5$, -OC$_3$H$_7$, -O-cyclo-C$_3$H$_5$, -OCH(CH$_3$)$_2$, -OC(CH$_3$)$_3$, -OC$_4$H$_9$, -OCH$_2$-COOH, -OPh, -OCH$_2$-Ph, -OCPh$_3$, -CH$_2$-OH, -C$_2$H$_4$-OH, -C$_3$H$_6$-OH$_3$ -CH(OH)-CH$_2$-OH, -CH$_2$-OCH$_3$, -C$_2$H$_4$-OCH$_3$, -C$_3$H$_6$-OCH$_3$, -CH$_2$-OC$_2$H$_5$, -C$_2$H$_4$-OC$_2$H$_5$, -C$_3$H$_6$-OC$_2$H$_5$, -CH$_2$-OC$_3$H$_7$, -C$_2$H$_4$-OC$_3$H$_7$, -C$_3$H$_6$-OC$_3$H$_7$, -CH$_2$-O-cyclo-C$_3$H$_5$, -C$_2$H$_4$-O-cyclo-C$_3$H$_5$, -C$_3$H$_6$-O-cyclo-C$_3$H$_5$, -CH$_2$-OCH(CH$_3$)$_2$, -C$_2$H$_4$-OCH(CH$_3$)$_2$, -C$_3$H$_6$-OCH(CH$_3$)$_2$, -CH$_2$-OC(CH$_3$)$_3$, -C$_2$H$_4$-OC(CH$_3$)$_3$, -C$_3$H$_6$-OC(CH$_3$)$_3$, -CH$_2$-OC$_4$H$_9$, -C$_2$H$_4$-OC$_4$H$_9$, -C$_3$H$_6$-OC$_4$H$_9$, -CH$_2$-OPh, -C$_2$H$_4$-OPh, -C$_3$H$_6$-OPh, -CH$_2$-OCH$_2$-Ph, -C$_2$H$_4$-OCH$_2$-Ph, -C$_3$H$_6$-OCH$_2$-Ph, -SH, -SCH$_3$, -SC$_2$H$_5$, -SC$_3$H$_7$, -S-cyclo-C$_3$H$_5$, -SCH(CH$_3$)$_2$, -SC(CH$_3$)$_3$, -NO$_2$, -F, -Cl$_3$ -Br, -I, -P(O)(OH)$_2$, -P(O)(OCH$_3$)$_2$, -P(O)(OC$_2$H$_5$)$_2$, -P(O)(OCH(CH$_3$)$_2$)$_2$, -C(OH)[P(O)(OH)$_2$]$_2$, -Si(CH$_3$)$_2$(C(CH$_3$)$_3$), -Si(C$_2$H$_5$)$_3$, -Si(CH$_3$)$_3$, -N$_3$, -CN, -OCN, -NCO, -SCN, -NCS, -CHO, -COCH$_3$, -COC$_2$H$_5$, -COC$_3$H$_7$, -CO-cyclo-C$_3$H$_5$, -COCH(CH$_3$)$_2$, -COC(CH$_3$)$_3$, -COOH, -COCN, -COOCH$_3$, -COOC$_2$H$_5$, -COOC$_3$H$_7$, -COO-cyclo-C$_3$H$_5$, -COOCH(CH$_3$)$_2$, -COOC(CH$_3$)$_3$, -OOC-CH$_3$, -OOC-C$_2$H$_5$, -OOC-C$_3$H$_7$, -OOC-cyclo-C$_3$H$_5$, -OOC-CH(CH$_3$)$_2$, -OOC-C(CH$_3$)$_3$, -CONH$_2$, -CH$_2$-CONH$_2$, -CONHCH$_3$, -CONHC$_2$H$_5$, -CONHC$_3$H$_7$, -CONH-cyclo-C$_3$H$_5$, -CONH[CH(CH$_3$)$_2$], -CONH[C(CH$_3$)$_3$], -CON(CH$_3$)$_2$, -CON(C$_2$H$_5$)$_2$, -CON(C$_3$H$_7$)$_2$, -CON(cyclo-C$_3$H$_5$)$_2$, -CON[CH(CH$_3$)$_2$]$_2$, -CON[C(CH$_3$)$_3$]$_2$, -NHCOCH$_3$, -NHCOC$_2$H$_5$, -NHCOC$_3$H$_7$, -NHCO-cyclo-C$_3$H$_5$, -NHCO-CH(CH$_3$)$_2$, -NHCO-C(CH$_3$)$_3$, -NHCO-OCH$_3$, -NHCO-OC$_2$H$_5$, -NH-CO-OC$_3$H$_7$, -NHCO-O-cyclo-C$_3$H$_5$,- NHCO-OCH(CH$_3$)$_2$, -NHCO-OC(CH$_3$)$_3$, -NH$_2$, -NHCH$_3$, -NHC$_2$H$_5$, -NHC$_3$H$_7$, -NH-cyclo-C$_3$H$_5$, -NHCH(CH$_3$)$_2$, -NHC(CH$_3$)$_3$, -N(CH$_3$)$_2$, -N(C$_2$H$_5$)$_2$, -N(C$_3$H$_7$)$_2$, -N(cyclo-C$_3$H$_5$)$_2$, -N[CH(CH$_3$)$_2$]$_2$, -N[C(CH$_3$)$_3$]$_2$, -SOCH$_3$, -SOC$_2$H$_5$, -SOC$_3$H$_7$, -SO-cyclo-C$_3$H$_5$, -SOCH(CH$_3$)$_2$, -SOC(CH$_3$)$_3$, -SO$_2$CH$_3$, -SO$_2$C$_2$H$_5$, -SO$_2$C$_3$H$_7$, -SO$_2$-cyclo-C$_3$H$_5$, -SO$_2$CH(CH$_3$)$_2$, -SO$_2$C(CH$_3$)$_3$, -SO$_3$H, -SO$_3$CH$_3$, -SO$_3$C$_2$H$_5$, -SO$_3$C$_3$H$_7$, -SO$_3$-cyclo-C$_3$H$_5$, -SO$_3$CH(CH$_3$)$_2$, -SO$_3$C(CH$_3$)$_3$, -SO$_2$NH$_2$, -SO$_2$NHCH$_3$, -SO$_2$NHC$_2$H$_5$, -SO$_2$NHC$_3$H$_7$, -SO$_2$NH-cyclo-C$_3$H$_5$, -SO$_2$NHCH(CH$_3$)$_2$, -SO$_2$NHC(CH$_3$)$_3$, -SO$_2$N(CH$_3$)$_2$, -SO$_2$N(C$_2$H$_5$)$_2$, -SO$_2$N(C$_3$H$_7$)$_2$, -SO$_2$N(cyclo-C$_3$H$_5$)$_2$, -SO$_2$N[CH(CH$_3$)$_2$]$_2$, -SO$_2$N[C(CH$_3$)$_3$]$_2$, -O-S(=O)CH$_3$, -O-S(=O)C$_2$H$_5$, -O-S(=O)C$_3$H$_7$, -O-S(=O)-cyclo-C$_3$H$_5$, -O-S(=O)CH(CH$_3$)$_2$, -O-S(=O)C(CH$_3$)$_3$, -S(=O)(=NH)CH$_3$, -S(=O)(=NH)C$_2$H$_5$, -S(=O)(=NH)C$_3$H$_7$, -S(=O)(=NH)-cyclo-C$_3$H$_5$, -S(=O)(=NH)CH(CH$_3$)$_2$, -S(=O)(=NH)C(CH$_3$)$_3$, -NH-SO$_2$-CH$_3$, -NH-SO$_2$-C$_2$H$_5$, -NH-SO$_2$-C$_3$H$_7$, -NH-SO$_2$-cyclo-C$_3$H$_5$, -NH-SO$_2$-CH(CH$_3$)$_2$, -NH-SO$_2$-C(CH$_3$)$_3$, -O-SO$_2$-CH$_3$, -O-SO$_2$-C$_2$H$_5$, -O-SO$_2$-C$_3$H$_7$, -O-SO$_2$-cyclo-C$_3$H$_5$, -O-SO$_2$-CH(CH$_3$)$_2$, -O-SO$_2$-C(CH$_3$)$_3$, -OCF$_3$, -CH$_2$-OCF$_3$, -C$_2$H$_4$-OCF$_3$, -C$_3$H$_6$-OCF$_3$, -OC$_2$F$_5$, -CH$_2$-OC$_2$F$_5$, -C$_2$H$_4$-OC$_2$F$_5$, -C$_3$H$_6$-OC$_2$F$_5$, -O-COOCH$_3$, -O-COOC$_2$H$_5$, -O-COOC$_3$H$_7$, -O-COO-cyclo-C$_3$H$_5$, -O-COOCH(CH$_3$)$_2$, -O-COOC(CH$_3$)$_3$, -NH-CO-NH$_2$, -NH-CO-NHCH$_3$, -NH-CO-NHC$_2$H$_5$, -NH-CS-N(C$_3$H$_7$)$_2$, -NH-CO-NHC$_3$H$_7$, -NH-CO-N(C$_3$H$_7$)$_2$, -NH-CO-NH[CH(CH$_3$)$_2$], -NH-CO-NH[C(CH$_3$)$_3$], -NH-CO-N(CH$_3$)$_2$, -NH-CO-N(C$_2$H$_5$)$_2$, -NH-CO-NH-cyclo-C$_3$H$_5$, -NH-CO-N(cyclo-C$_3$H$_5$)$_2$, -NH-CO-N[CH(CH$_3$)$_2$]$_2$, -NH-CS-N(C$_2$H$_5$)$_2$, -NH-CO-N[C(CH$_3$)$_3$]$_2$, -NH-CS-NH$_2$, -NH-CS-NHCH$_3$, -NH-CS-N(CH$_3$)$_2$, -NH-CS-NHC$_2$H$_5$, -NH-CS-NHC$_3$H$_7$, -NH-CS-NH-cyclo-C$_3$H$_5$, -NH-CS-NH[CH(CH$_3$)$_2$], -NH-CS-NH[C(CH$_3$)$_3$], -NH-CS-N(cyclo-C$_3$H$_5$)$_2$, -NH-CS-N[CH(CH$_3$)$_2$]$_2$, -NH-CS-N[C(CH$_3$)$_3$]$_2$, -NH-C(=NH)-NH$_2$, -NH-C(=NH)-NHCH$_3$, -NH-C(=NH)-NHC$_2$H$_5$, -NH-C(=NH)-NHC$_3$H$_7$, -O-CO-NH-cyclo-C$_3$H$_5$, -NH-C(=NH)-NH-cyclo-C$_3$H$_5$, -NH-C(=NH)-NH[CH(CH$_3$)$_2$] -O-CO-NH[CH(CH$_3$)$_2$], -NH-C(=NH)-NH[C(CH$_3$)$_3$], -NH-C(=NH)-N(CH$_3$)$_2$, -NH-C(=NH)-N(C$_2$H$_5$)$_2$, -NH-C(=NH)-N(C$_3$H$_7$)$_2$, -NH-C(=NH)-N(cyclo-C$_3$H$_5$)$_2$, -O-CO-NHC$_3$H$_7$, -NH-C(=NH)-N[CH(CH$_3$)$_2$]$_2$, -NH-C(=NH)-N[C(CH$_3$)$_3$]$_2$, -O-CO-NH$_2$, -O-CO-NHCH$_3$, -O-CO-NHC$_2$H$_5$, -O-CO-NH[C(CH$_3$)$_3$], -O-CO-N(CH$_3$)$_2$, -O-CO-N(C$_2$H$_5$)$_2$, -O-CO-N(C$_3$H$_7$)$_2$, -O-CO-N(cyclo-C$_3$H$_5$)$_2$, -O-CO-N[CH(CH$_3$)$_2$]$_2$,- O-CO-N[C(CH$_3$)$_3$]$_2$, -O-CO-OCH$_3$, -O-CO-OC$_2$H$_5$, -O-CO-OC$_3$H$_7$, -O-CO-O-cyclo-C$_3$H$_5$, -O-CO-OCH(CH$_3$)$_2$, -O-CO-OC(CH$_3$)$_3$, -CH$_2$F, -CHF$_2$, -CF$_3$, -CH$_2$Cl, -CH$_2$Br, -CH$_2$I, -CH$_2$-CH$_2$F, -CH$_2$-CHF$_2$, -CH$_2$-CF$_3$, -CH$_2$-CH$_2$Cl, -CH$_2$-CH$_2$Br, -CH$_2$-CH$_2$I, -cyclo-C$_5$H$_9$, -cyclo-C$_6$H$_{11}$, -CH$_2$-cyclo-C$_6$H$_{11}$, -CH$_2$-CH$_2$-cyclo-C$_6$H$_{11}$, -cyclo-C$_7$H$_{13}$, -cyclo-C$_8$H$_{15}$, -Ph, -CH$_2$-Ph, -CH$_2$-CH$_2$-Ph, -CH=CH-Ph, -CPh$_3$, -CH$_3$, -C$_2$H$_5$, -C$_3$H$_7$, -CH(CH$_3$)$_2$, -C$_4$H$_9$, -CH$_2$-CH(CH$_3$)$_2$, -CH(CH$_3$)-C$_2$H$_5$, -C(CH$_3$)$_3$, -C$_5$H$_{11}$, -CH(CH$_3$)-C$_3$H$_7$, -CH$_2$-CH(CH$_3$)-C$_2$H$_5$, -CH(CH$_3$)-CH(CH$_3$)$_2$, -C(CH$_3$)$_2$-C$_2$H$_5$, -CH$_2$-C(CH$_3$)$_3$, -CH(C$_2$H$_5$)$_2$, -C$_2$H$_4$-CH(CH$_3$)$_2$, -C$_6$H$_{13}$, -C$_7$H$_{15}$, -C$_8$H$_{17}$, -C$_3$H$_6$-CH(CH$_3$)$_2$, -C$_2$H$_4$-CH(CH$_3$)-C$_2$H$_5$, -CH(CH$_3$)-C$_4$H$_9$, -CH$_2$-CH(CH$_3$)-C$_3$H$_7$, -CH(CH$_3$)-CH$_2$-CH(CH$_3$)$_2$, -CH(CH$_3$)-CH(CH$_3$)-C$_2$H$_5$, -CH$_2$-CH(CH$_3$)-CH(CH$_3$)$_2$, -CH$_2$-C(CH$_3$)$_2$-C$_2$H$_5$, -C(CH$_3$)$_2$-C$_3$H$_7$, -C(CH$_3$)$_2$-CH(CH$_3$)$_2$, -C$_2$H$_4$-C(CH$_3$)$_3$, -CH(CH$_3$)-C(CH$_3$)$_3$, -CH=CH$_2$, -CH$_2$-CH=CH$_2$, -C(CH$_3$)=CH$_2$, -CH=CH-CH$_3$, -C$_2$H$_4$-CH=CH$_2$, -CH$_2$-CH=CH-CH$_3$, -CH=CH-C$_2$H$_5$, -CH$_2$-C(CH$_3$)=CH$_2$, -CH(CH$_3$)-CH=CH, -CH=C(CH$_3$)$_2$, -C(CH$_3$)=CH-CH$_3$, -CH=CH-CH=CH$_2$, -C$_3$H$_6$-CH=CH$_2$, -C$_2$H$_4$-CH=CH-CH$_3$, -CH$_2$-CH=CH-C$_2$H$_5$, -CH=CH-C$_3$H$_7$, -CH$_2$-CH=CH-CH=CH$_2$, -CH=CH-CH=CH-CH$_3$, -CH=CH-CH$_2$-CH=CH$_2$, -C(CH$_3$)=CH-CH=CH$_2$, -CH=C(CH$_3$)-CH=CH$_2$, -CH=CH-C(CH$_3$)=CH$_2$, -C$_2$H$_4$-C(CH$_3$)=CH$_2$, -CH$_2$-CH(CH$_3$)-CH=CH$_2$, -CH(CH$_3$)-CH$_2$-CH=CH$_2$, -CH$_2$-CH=C(CH$_3$)$_2$, -CH$_2$-C(CH$_3$)=CH-CH$_3$, -CH(CH$_3$)-CH=CH-CH$_3$, -CH=CH-

$CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-C_2H_4-CH=C(CH_3)_2$, $-CH(CH_3)-C_2H_4-CH=CH_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-C[C(CH_3)_3]=CH_2$, $-CH(CH_3)-CH_2-C(CH_3)=CH_2$, $-CH(CH_3)-CH(CH_3)-CH=CH_2$, $-CH=CH-C_2H_4-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$,- $C(CH_3)_2-CH=CH-CH_3$, $-CH=CH-CH_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-C_3H_6-C\equiv C-CH_3$, $-CH_2-CH=CH-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH_2-C\equiv CH$, $-CH(CH_3)-CH=CH-CH=CH_2$, $-CH=CH-CH_2-C(CH_3)=CH_2$, $-CH(CH_3)-C\equiv C-CH_3$, $-CH=CH-CH(CH_3)-CH=CH_2$, $-CH=C(CH_3)-CH_2-CH=CH_2$, $-C_2H_4-CH(CH_3)-C\equiv CH$, $-C(CH_3)=CH-CH_2-CH=CH_2$, $-CH=CH-CH=C(CH_3)_2$, $-CH_2-CH(CH_3)-CH_2-C\equiv CH$, $-CH=CH-C(CH_3)=CH-CH_3$, $-CH=C(CH_3)-CH=CH-CH_3$, $-CH_2-CH(CH_3)-C\equiv CH$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-C_4H_8-C\equiv CH$, $-C_2H_4-C\equiv C-C_2H_5$, $-CH_2-C\equiv C-C_3H_7$, $-C\equiv C-C_4H_9$, $-C\equiv C-C(CH_3)_3$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-CH_2-CH(C\equiv CH)_2$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C(C\equiv CH)_2-CH_3$, $-C\equiv C-CH(CH_3)-C\equiv CH$, $-CH(CH_3)-C\equiv C-C\equiv CH$, $-CH(C\equiv CH)-CH_2-C\equiv CH$, $-CH(C\equiv CH)-C\equiv C-CH_3$,

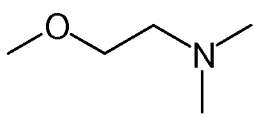,
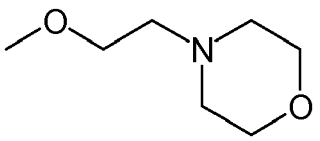,
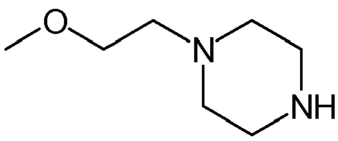,

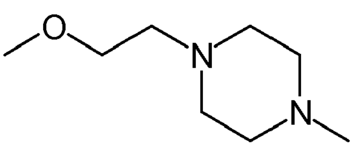, or
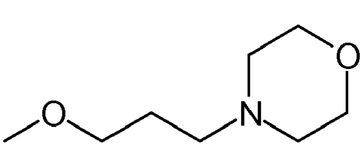;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts;
with a privisio that the compound is not (1S,5S,6R)-10-(3,5-dichlorophenylsulfonyl)-3-(2-(3,4-dimethoxyphenoxy)ethyl)-5-vinyl-3,10-diazabicyclo[4.3.1]decan-2-one.

**2.** Compound according to claim 1 of the general formula (II):

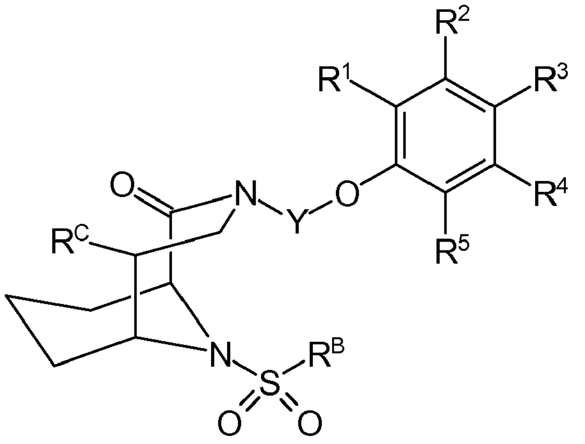

( II )

wherein Y, $R^B$, $R^C$ and $R^1$ - $R^5$ have the meanings as defined in claim 1.

**3.** Compound according to claim 1 of the general formula (VII):

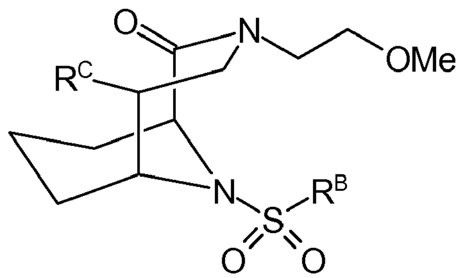

( VII )

wherein $R^B$ and $R^C$ have the meanings as defined in claim 1.

**4.** Compound according to claim 1 selected from the group consisting of:

(1*S*,5*S*,6*R*)-5-acetyl-10-((3,5-dichlorophenyl)sulfonyl)-3-(2-methoxyethyl)-3,10-diazabicyclo[4.3.1]decan-2-one,
2-(((1*S*,5*S*,6*R*)-10-((3,5-dichlorophenyl)sulfonyl)-3-(2-methoxyethyl)-2-oxo-3,10-diazabicyclo[4.3.1]decan-5-yl)acetaldehyde,
(1*S*,5*S*,6*R*)-10-((3,5-dichlorophenyl)sulfonyl)-5-(2-hydroxypropan-2-yl)-3-(2-methoxyethyl)-3,10-diazabicyclo[4.3.1]decan-2-one,
(1*S*,5*S*,6*R*)-10-((3,5-dichlorophenyl)sulfonyl)-5-((R)-1-hydroxyethyl)-3-(2-methoxyethyl)-3,10-diazabicyclo[4.3.1]decan-2-one,
(1*S*,5*S*,6*R*)-10-((3,5-dichlorophenyl)sulfonyl)-5-((S)-1-hydroxyethyl)-3-(2-methoxyethyl)-3,10-diazabicyclo[4.3.1]decan-2-one,
(1*S*,5*S*,6*R*)-10-((3,5-dichlorophenyl)sulfonyl)-5-(2-hydroxyethyl)-3-(2-methoxyethyl)-3,10-diazabicyclo[4.3.1]decan-2-one,
(1*S*,5*S*,6*R*)-10-((3,5-dichlorophenyl)sulfonyl)-3-(2-methoxyethyl)-2-oxo-3,10-diazabicyclo[4.3.1]decane-5-carbaldehyde,
(1S,5S,6R)-10-((3,5-dichlorophenyl)sulfonyl)-3-(2-methoxyethyl)-2-oxo-3,10-diazabicyclo[4.3.1]decane-5-carboxylic acid,
(1*S*,5*S*,6*R*)-10-((3,5-dichlorophenyl)sulfonyl)-3-(2-methoxyethyl)-2-oxo-3,10-diazabicyclo[4.3.1]decane-5-carboxamide,
(1*S*,5*R*,6*R*)-10-((3,5-dichlorophenyl)sulfonyl)-3-(2-methoxyethyl)-5-((methylamino)methyl)-3,10-diazabicyclo[4.3.1]decan-2-one,
(1*S*,5*R*,6*R*)-10-((3,5-dichlorophenyl)sulfonyl)-5-(hydroxymethyl)-3-(2-methoxyethyl)-3,10-diazabicyclo[4.3.1]decan-2-one,
(1*S*,5*R*,6*R*)-10-((3,5-dichlorophenyl)sulfonyl)-3-(2-methoxyethyl)-5-(methoxymethyl)-3,10-diazabicyclo[4.3.1]decan-2-one,
(1S,5S,6R)-10-((3,5-dichlorophenyl)sulfonyl)-5-ethyl-3-(2-methoxyethyl)-3,10-diazabicyclo[4.3.1]decan-2-one,
(1*S*,5*R*,6*R*)-10-((3,5-dichlorophenyl)sulfonyl)-3-(2-hydroxyethyl)-5-(hydroxymethyl)-3,10-diazabicyclo[4.3.1]decan-2-one,
(1*S*,5*S*,6*R*)-10-((3,5-dichlorophenyl)sulfonyl)-5-(1,2-dihydroxyethyl)-3-(2-(3,4-dimethoxyphenoxy)ethyl)-3,10-diazabicyclo[4.3.1]decan-2-one,
(1*S*,5*S*,6*R*)-10-((3,5-dichlorophenyl)sulfonyl)-5-(1,2-dihydroxyethyl)-3-(2-ethoxyethyl)-3,10-diazabicyclo[4.3.1]decan-2-one,
(1S,5S,6R)-5-acetyl-10-((3,5-dichlorophenyl)sulfonyl)-3-(2-(3,4-dimethoxyphenoxy)ethyl)-3,10-diazabicyclo[4.3.1]decan-2-one,
2-(((1S,5S,6R)-10-((3,5-dichlorophenyl)sulfonyl)-3-(2-(3,4-dimethoxyphenoxy)ethyl)-2-oxo-3,10-diazabicyclo[4.3.1]decan-5-yl)acetaldehyde,
(1*S*,5*S*,6*R*)-10-((3,5-dichlorophenyl)sulfonyl)-3-(2-(3,4-dimethoxyphenoxy) ethyl)-5-(2-hydroxypropan-2-yl)-3,10-diazabicyclo[4.3.1]decan-2-one,
(1*S*,5*S*,6*R*)-10-((3,5-dichlorophenyl)sulfonyl)-3-(2-(3,4-dimethoxyphenoxy) ethyl)-5-(2-hydroxypropyl)-3,10-diazabicyclo[4.3.1]decan-2-one,
(1*S*,5*S*,6*R*)-10-((3,5-dichlorophenyl)sulfonyl)-3-(2-(3,4-dimethoxyphenoxy) ethyl)-5-(2-hydroxypropyl)-3,10-diazabicyclo[4.3.1]decan-2-one,
(1*S*,5*S*,6*R*)-10-((3,5-dichlorophenyl)sulfonyl)-3-(2-(3,4-dimethoxyphenoxy) ethyl)-5-((R)-1-hydroxyethyl)-3,10-diazabicyclo[4.3.1]decan-2-one,
(1*S*,5*S*,6*R*)-10-((3,5-dichlorophenyl)sulfonyl)-3-(2-(3,4-dimethoxyphenoxy) ethyl)-5-((R)-1-hydroxyethyl)-3,10-diazabicyclo[4.3.1]decan-2-one,
(1*S*,5*S*,6*R*)-10-((3,5-dichlorophenyl)sulfonyl)-3-(2-(3,4-dimethoxyphenoxy) ethyl)-5-(2-hydroxyethyl)-3,10-diazabicyclo[4.3.1]decan-2-one,
(1S,5S,6R)-10-((3,5-dichlorophenyl)sulfonyl)-5-hydroxy-3,10diazabicyclo-[4.3.1]decan-2-one,
(1S,5R,6R)-5-amino-10-((3,5-dichlorophenyl)sulfonyl)-3,10-diazabicyclo-[4.3.1]decan-2-one,
(1S,5S,6R)-10-((3,5-dichlorophenyl)sulfonyl)-5-methoxy-3-methyl-3,10-diazabicyclo[4.3.1]decan-2-one,
(1S,5R,6R)-10-((3,5-dichlorophenyl)sulfonyl)-5-hydroxy-3,10-diazabicyclo-[4.3.1]decan-2-one,
(1S,5S,6R)-10-((3,5-dichlorophenyl)sulfonyl)-5-(2-methoxyethoxy)-3-(2-methoxy-ethyl)-3,10-diazabicyclo[4.3.1]decan-2-one,
(1S,5S,6R)-10-((3,5-dichlorophenyl)sulfonyl)-5-(methoxymethoxy)-3,10-diaza-bicyclo[4.3.1]decan-2-one,
(1S,5S,6R)-10-((3,5-dichlorophenyl)sulfonyl)-3-(2-(3,4-dimethoxyphenoxy) ethyl)-5-hydroxy-3,10-diazabicyclo[4.3.1]decan-2-one,
(1S,5R,6R)-10-((3,5-dichlorophenyl)sulfonyl)-3-(3-methoxypropyl)-5-vinyl-3,10-diazabicyclo[4.3.1]decan-2-one,
(1S,5R,6R)-10-((3,5-dichlorophenyl)sulfonyl)-3-(4-methoxybutyl)-5-vinyl-3,10-diazabicyclo[4.3.1]decan-2-one,
(1 S,5R,6R)-10-((3,5-dichlorophenyl)sulfonyl)-3-(pyridin-2-ylmethyl)-5-vinyl-3,10-diazabicyclo[4.3.1]decan-2-one,
(1 S,5R,6R)-10-((3,5-dichlorophenyl)sulfonyl)-5-(hydroxymethyl)-3-(3-hydroxypropyl)-3,10-diazabicyclo[4.3.1]decan-2-one,
(1 S,5R,6R)-10-((3,5-dichlorophenyl)sulfonyl)-5-(hydroxymethyl)-3-(3-methoxypropyl)-3,10-diazabicyclo[4.3.1]decan-2-one,
(1 S,5R,6R)-10-((3,5-dichlorophenyl)sulfonyl)-5-(hydroxymethyl)-3-(4-methoxybutyl)-3,10-diazabicyclo[4.3.1]decan-2-one, and
(1 S,5R,6R)-10-((3,5-dichlorophenyl)sulfonyl)-5-(hydroxymethyl)-3-(pyridin-2-ylmethyl)-3,10-diazabicyclo[4.3.1]decan-2-one.

**5.** Compound according to any one of claims 1 - 4 for use as inhibitor of FK506-binding proteins.

**6.** Compound according to claim 5 for use as inhibitor of FK506-binding protein 51 and/or FK506-binding protein 52.

**7.** Compound according to any one of claims 1 - 6 for use as pharmaceutically active agent in medicine.

**8.** Compound according to any one of claims 1 - 7 for use in treatment or prophylaxis of psychiatric disorders, neurological disorders, metabolic diseases, cancers, glucocorticoid hyposensitivity syndromes, peripheral glucocorticoid resistance, infectious diseases, alopecia, abnormally elevated intraocular pressure, macular degeneration, oxidative

damage to eye tissues, vision disorder, sleeping disorders, asthma, diabetes, traumatic brain injury, nerve injury, Alzheimer's disease, Huntington's disease, Parkinson's disease, ischemia, memory impairment and for neuroprotection, neuroregeneration, promoting hair growth, stimulating neurite growth, wound healing, antiglaucomatous medications, improving vision, enhancing memory performance, for the use in treatment or prevention of multi-drug resistance, for the use in induced abortion and the inhibition of embryo implantation, for the use in limiting or preventing hemorrhage or neovascularization and for the use in treatment of diseases relating to neurodegeneration.

**9.** Compound according to claim 8, wherein the psychiatric diseases is an affective disorder or an anxiety disorder and/or the metabolic diseases is a localized adiposity, metabolic syndrome or obesity and/or the cancers is prostate cancer, acute lymphoblastic leukaemia or melanoma and/or wherein the affective disorder is selected from the group consisting of: depression, bipolar disorder, mania, substance induced mood disorder and seasonal affective disorder (SAD) and/or wherein the depression is selected from major depression or major depressive disorder and/or wherein the anxiety disorder is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders and/or wherein the infectious disease is selected from the group consisting of malaria and Legionnaires' disease.

**10.** Pharmaceutical composition comprising at least one compound according to any one of claims 1 - 4 together with at least one pharmaceutically acceptable carrier, solvent or excipient or together with at least one pharmaceutically acceptable carrier, solvent or excipient and at least one active agent selected from the group consisting of an anti-depressant and other psychotropic drugs.

**11.** Pharmaceutical composition according to claim 10, wherein the anti-depressant is selected from amitriptyline, amioxide clomipramine, doxepine, duloxetine, imipramine trimipramine, mirtazapine, reboxetine, citaloprame, fluoxetine, moclobemide and sertraline.

**12.** Method for preparation of compound of the general formula **(I)** comprising the following steps:

(a) providing an intermediate compound of formula **(I-A1)**

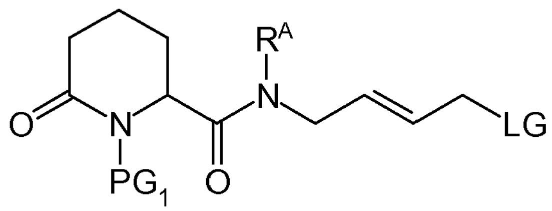

**(I-A1)**

wherein $R^A$ has the meanings as defined in claim 1, LG refers to a leaving group, and $PG_1$ refers to a protecting group for amine;

(b) performing a cyclization reaction and deprotecting $PG_1$ to yield compound of formula **(I-B1)**

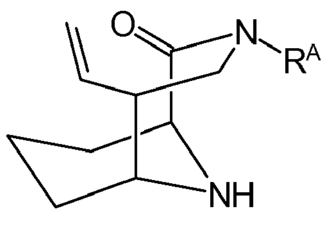

**(I-B1)**

(c) introducing a moiety $-SO_2-R^B$ to yield compound of formula **(I-C)**

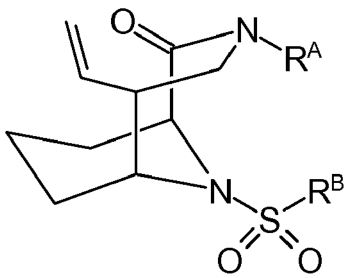

(I-C)

(d) transforming the vinyl group of compound **(I-C)** into the substituent $R^C$ to yield compound of the formula (**I**)

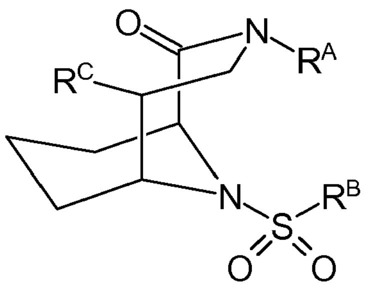

**(I)**

wherein $R^A$, $R^B$ and $R^C$ have the meanings as defined in claim 1.

**13.** Method for preparation of compound of the general formula (I) comprising the following steps:

(a) providing an intermediate compound of the formula **(I-A2)**

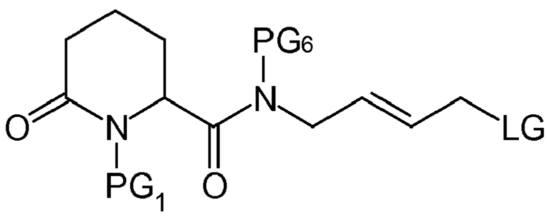

(**I-A2**)

wherein $R^A$ has the meanings as defined herein, LG refers to a leaving group, and $PG_1$ and $PG_6$ refer to protecting groups for amines;

(b) performing a cyclization reaction and deprotecting $PG_1$ to yield compound of the formula (**I-B1**)

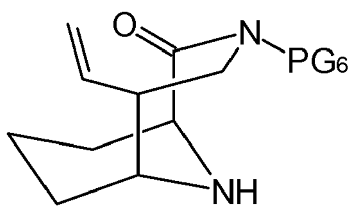

(**I-B2**)

(c) introducing the moiety $-SO_2-R^B$ to yield compound of the formula **(I-B3)**

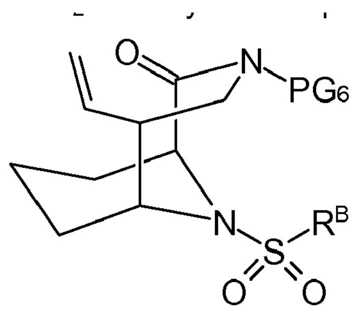

(I-B3)

wherein $R^B$ has the meanings as defined herein;

(d) deprotecing $PG_6$ and introducing the moiety $R^A$ to yield compound of the formula **(I-C)**

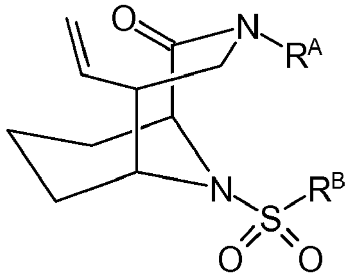

(I-C)

(e) transforming the vinyl group of compound **(I-C)** into the substituent $R^C$ to yield compound of the formula (**I**)

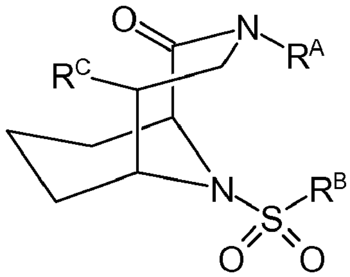

(I)

wherein $R^A$ , $R^B$ and $R^C$ have the meanings as defined herein.

**14.** Method for preparation of compound of the general formula **(I)** comprising the following steps:

(a) providing an intermediate compound of formula **(I-E)**

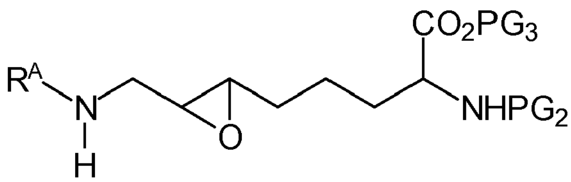

**(I-E)**

wherein $R^A$ has the meanings as defined in claim 1, $PG_2$ refers to a protecting group for amine, and $PG_3$ refers to a protecting group for carboxylic acid;

(b) deprotecting $PG_2$, performing a piperindine ring formation reaction and introducing $PG_4$ to yield compound of formula **(I-F)**

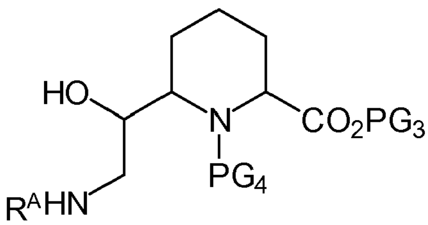

**(I-F)**

wherein $PG_4$ refers to a protecting group for amine;
(c) introducing $PG_5$, deprotecting $PG_3$ and performing an amide coupling reaction to yield compound of formula **(I-G)**

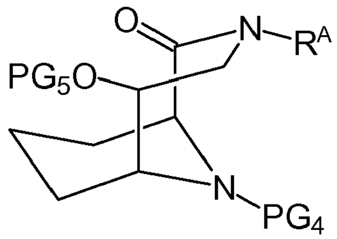

**(I-G)**

wherein $PG_5$ refers to a protecting group for hydroxyl group;
(d) deprotecting $PG_4$ and introducing a moiety $-SO_2-R^B$ to yield compound of formula **(I-H)**

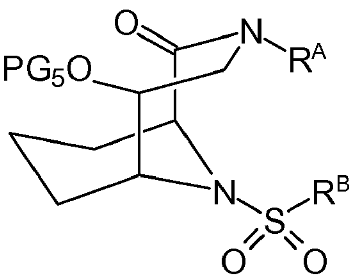

**(I-H)**

wherein $R^B$ has the meanings as defined in claim 1;
(e) transforming a hydroxyl group obtained by deprotecting $PG_5$ to yield compound of the formula (**I**)

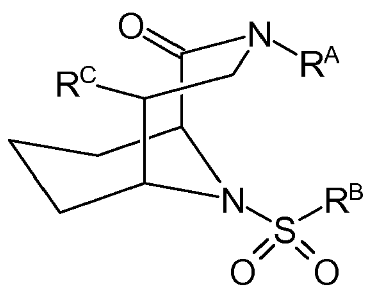

**(I)**

wherein $R^A$, $R^B$ and $R^C$ have the meanings as defined in claim 1.

**15.** Intermediate compound of the formula **(I-E)** or **(I-F):**

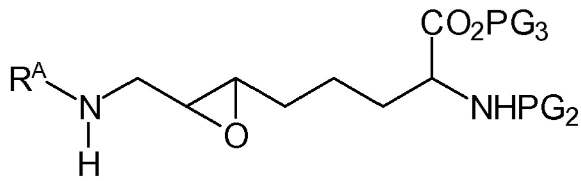

**(I-E)**

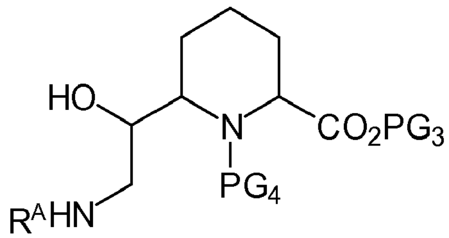

**(I-F)**

wherein $R^A$ has the meanings as defined in claim 1, $PG_2$ and $PG_4$ refer to a protecting group for amine, and $PG_3$ refers to a protecting group for carboxylic acid.

**Figure 1**

**(a)**

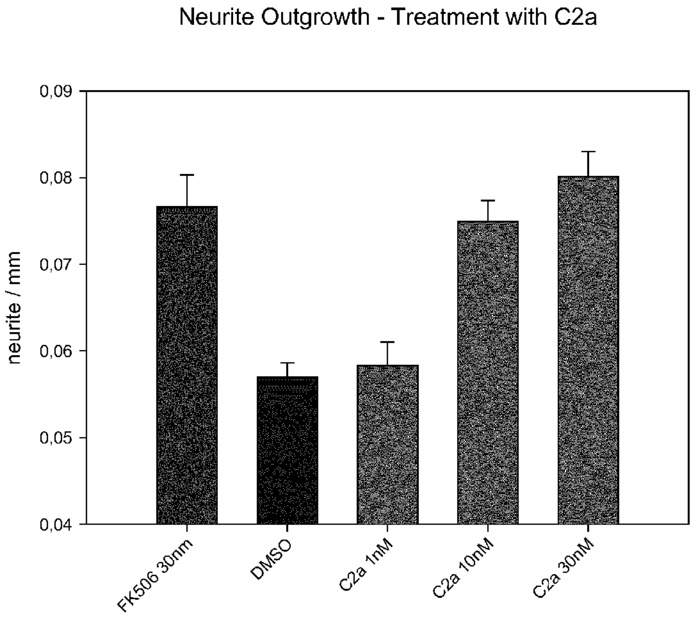


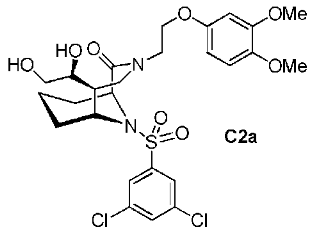

**C2a**

**Figure 1 continued**

**(b)**

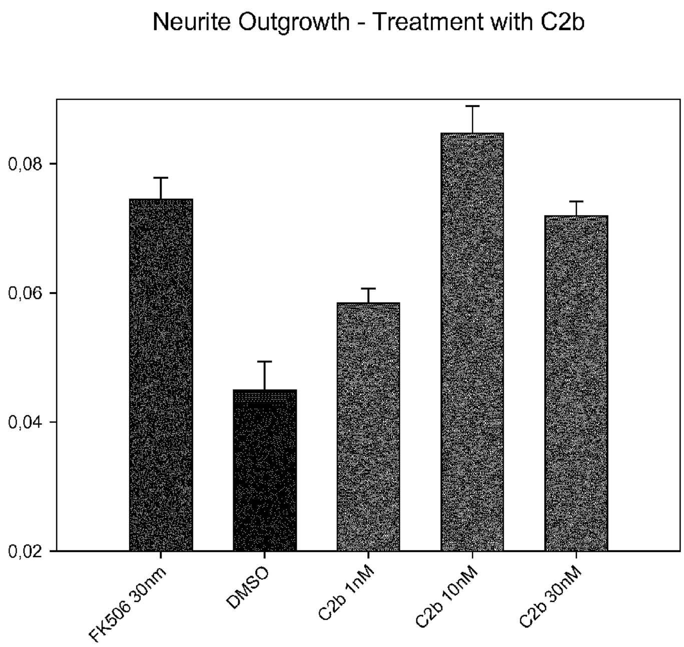


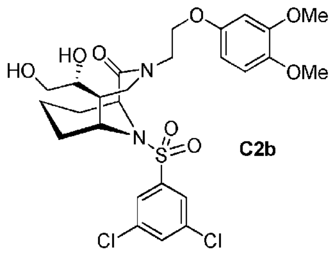

**C2b**

**Figure 1 continued**

**(c)**

Neurite Outgrotwh - Treatment with FK506

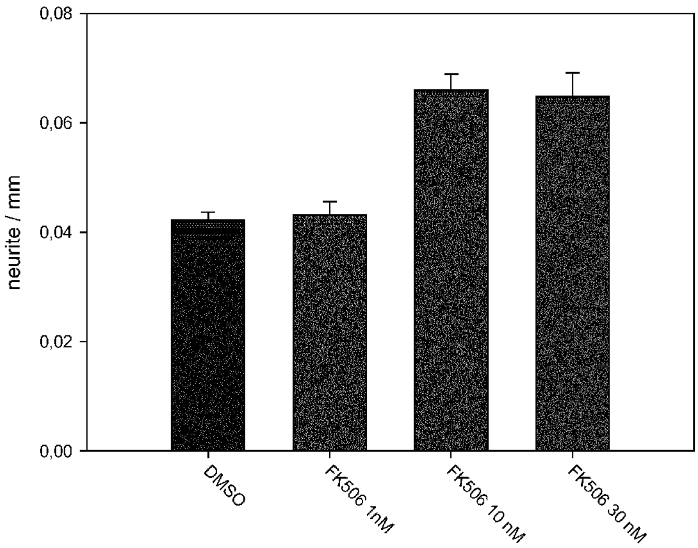


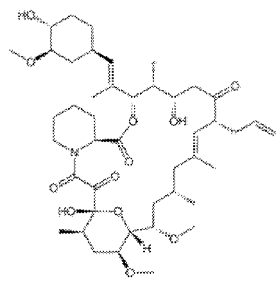

**FK506**

Europäisches Patentamt
European Patent Office
Office européen des brevets

# EUROPEAN SEARCH REPORT

Application Number
EP 14 15 2567

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 2 690 102 A1 (MAX PLANCK GESELLSCHAFT [DE]) 29 January 2014 (2014-01-29) * claim 1 * ----- | 1-15 | INV. C07D471/08 A61K31/551 A61P3/00 A61P25/00 A61P27/00 A61P35/00 |
| A | YANSONG WANG ET AL: "Increasing the Efficiency of Ligands for FK506-Binding Protein 51 by Conformational Control", JOURNAL OF MEDICINAL CHEMISTRY, vol. 56, no. 10, 23 May 2013 (2013-05-23), pages 3922-3935, XP055102631, ISSN: 0022-2623, DOI: 10.1021/jm400087k * the whole document * ----- | 1-15 | |
| A | WO 01/42245 A1 (SQUIBB BRISTOL MYERS CO [US]) 14 June 2001 (2001-06-14) * claim 1 * ----- | 1-15 | |
| A | WO 02/089806 A1 (AGOURON PHARMA [US]; GUO CHUANGXING [US]; AUGELLI-SZAFRAN CORINNE E [U) 14 November 2002 (2002-11-14) * claim 1 * ----- | 1-15 | TECHNICAL FIELDS SEARCHED (IPC) |
| A | WO 00/04020 A1 (AGOURON PHARMA [US]; KATOH SUSUMU [JP]; KAWAKAMI HIROSHI [JP]; TADA HI) 27 January 2000 (2000-01-27) * claim 1 * ----- | 1-15 | C07D |

2

The present search report has been drawn up for all claims

| Place of search | Date of completion of the search | Examiner |
|---|---|---|
| Munich | 17 February 2014 | Bérillon, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 15 2567

This annex lists the patent family members relating to the patent documents cited in the above-mentioned European search report. The members are as contained in the European Patent Office EDP file on
The European Patent Office is in no way liable for these particulars which are merely given for the purpose of information.

17-02-2014

| Patent document cited in search report | Publication date | Patent family member(s) | Publication date |
|---|---|---|---|
| EP 2690102 A1 | 29-01-2014 | EP 2690102 A1 | 29-01-2014 |
| | | WO 2014015993 A1 | 30-01-2014 |
| WO 0142245 A1 | 14-06-2001 | AU 1804801 A | 18-06-2001 |
| | | US 6818643 B1 | 16-11-2004 |
| | | WO 0142245 A1 | 14-06-2001 |
| WO 02089806 A1 | 14-11-2002 | BR 0210060 A | 17-08-2004 |
| | | CA 2446795 A1 | 14-11-2002 |
| | | EP 1423119 A1 | 02-06-2004 |
| | | JP 2004532854 A | 28-10-2004 |
| | | MX PA03010255 A | 07-03-2005 |
| | | WO 02089806 A1 | 14-11-2002 |
| WO 0004020 A1 | 27-01-2000 | AT 268772 T | 15-06-2004 |
| | | AU 756912 B2 | 23-01-2003 |
| | | AU 4996399 A | 07-02-2000 |
| | | BR 9912423 A | 05-06-2001 |
| | | CA 2337377 A1 | 27-01-2000 |
| | | CN 1318067 A | 17-10-2001 |
| | | DE 69917907 D1 | 15-07-2004 |
| | | DE 69917907 T2 | 30-06-2005 |
| | | EE 200100032 A | 17-06-2002 |
| | | EP 1098897 A1 | 16-05-2001 |
| | | ES 2226409 T3 | 16-03-2005 |
| | | GE P20043368 B | 13-04-2004 |
| | | HR P20010118 A2 | 28-02-2002 |
| | | HU 0200637 A2 | 28-08-2002 |
| | | ID 27428 A | 05-04-2001 |
| | | IS 5805 A | 12-01-2001 |
| | | JP 2002520413 A | 09-07-2002 |
| | | LT 2001012 A | 25-07-2001 |
| | | LV 12665 A | 20-05-2001 |
| | | NO 20010191 A | 16-03-2001 |
| | | NZ 509211 A | 25-10-2002 |
| | | OA 11581 A | 23-07-2004 |
| | | PL 345598 A1 | 17-12-2001 |
| | | PT 1098897 E | 29-10-2004 |
| | | SI 20638 A | 28-02-2002 |
| | | SK 462001 A3 | 05-02-2002 |
| | | TR 200100122 T2 | 21-08-2001 |
| | | US 6630472 B1 | 07-10-2003 |
| | | WO 0004020 A1 | 27-01-2000 |
| | | YU P2901 A | 12-05-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SCHMIDT et al.** *ChemMedChem,* 2012, vol. 7, 1351-1359
- **KOZANY et al.** *ChemBioChem,* 2009, vol. 10, 1402-1410
- **GAALI et al.** *Curr Med Chem,* 2011, vol. 18, 5355-5379
- **GALIGNIANA et al.** *J. Neurochem,* 2012, vol. 122, 4-18
- **ERLEJMAN et al.** *Futue Med Chem,* 2013, vol. 5, 591-607
- **SANCHEZ.** *biochim Biophys Acta Mol Cell Res,* 2012, vol. 1823, 722-729
- **ZANNAS, A. S. ; BINDER, E. B.** Gene-environment interactions at the FKBP5 locus: sensitive periods, mechanisms and pleiotropism. *Genes Brain Behav,* 2013
- **BLAIR et al.** *J Clin Invest,* 2013
- **TAJIRI et al.** *PLOS One,* 2013, vol. 8, e65284
- **CALDWELL et al.** *J Allerg Clin Immunol,* 2010, vol. 125, 879-888
- **WARRIER.** Role of FKBP51 and FKBP52 in Glucocorticoid Receptor Regulated Metabolism. *PhD Thesis 2008, University of Toledo, ProQuest LLC,* 2008
- **ROMANO et al.** *Cell Death Dis,* 2013, vol. 4, e578
- **LI.** *Br J Cancer,* 2013
- **JIANG et al.** *Neoplasia,* 2013, vol. 10, 235-243
- **KOMURA et al.** *Cancer Res,* 2005, vol. 65, 3281-3289
- **HOU ; WANG.** *PLOS One,* 2012, 7
- **KOZANY, C. ; MARZ, A. ; KRESS, C. ; HAUSCH, F.** Fluorescent probes to characterise FK506-binding proteins. *Chembiochem,* 2009, vol. 10 (8), 1402-10
- **Z.X. WANG.** *FEBS Lett,* 1995, vol. 360, 111-114